(19) Europäisches Patentamt / European Patent Office / Office européen des brevets

(11) **EP 3 177 713 B1**

(12) **EUROPEAN PATENT SPECIFICATION**

(45) Date of publication and mention of the grant of the patent:
**05.08.2020  Bulletin 2020/32**

(21) Application number: **15829211.0**

(22) Date of filing: **06.08.2015**

(51) Int Cl.:
*A61K 9/00* *(2006.01)*          *A61K 47/34* *(2017.01)*
*A61K 47/60* *(2017.01)*        *A61K 49/00* *(2006.01)*
*C12N 5/09* *(2010.01)*         *C12N 5/02* *(2006.01)*
*A61K 9/10* *(2006.01)*         *A61K 9/127* *(2006.01)*
*A61K 41/00* *(2020.01)*        *A61K 47/30* *(2006.01)*

(86) International application number:
**PCT/US2015/044017**

(87) International publication number:
**WO 2016/022805 (11.02.2016 Gazette 2016/06)**

(54) **COMPOSITIONS AND THEIR USE FOR INDUCING NANOPARTICLE-MEDIATED MICROVASCULAR EMBOLIZATION OF TUMORS**

ZUSAMMENSETZUNGEN UND DEREN VERWENDUNG ZUR INDUZIERUNG VON NANOTEILCHENVERMITTELTER MIKROVASKULÄRER EMBOLISATION VON TUMOREN

COMPOSITIONS ET LEUR UTILISATION POUR L'INDUCTION D'UNE EMBOLISATION MICROVASCULAIRE DES TUMEURS À MÉDIATION PAR DES NANOPARTICULES

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority:  **08.08.2014  US 201414455082**

(43) Date of publication of application:
**14.06.2017  Bulletin 2017/24**

(73) Proprietor: **Poseida Therapeutics, Inc.**
**San Diego, California 92121 (US)**

(72) Inventor: **GHOROGHCHIAN, P. Peter**
**Boston, Massachusetts 02116 (US)**

(74) Representative: **Cooley (UK) LLP**
**Dashwood**
**69 Old Broad Street**
**London EC2M 1QS (GB)**

(56) References cited:
WO-A2-2011/133635     WO-A2-2012/094679
US-A- 5 612 310          US-A- 5 612 310
US-A- 6 133 316          US-A1- 2005 287 145
US-A1- 2005 287 189     US-A1- 2011 223 128

• LINGE WANG ET AL: "Encapsulation of Biomacromolecules within Polymersomes by Electroporation", ANGEWANDTE CHEMIE INTERNATIONAL EDITION, vol. 51, no. 44, 28 September 2012 (2012-09-28), pages 11122-11125, XP055434887, ISSN: 1433-7851, DOI: 10.1002/anie.201204169
• Christelle Kamga ET AL: "Myoglobin and mitochondria: A relationship bound by oxygen and nitric oxide", NITRIC OXIDE: BIOLOGY AND CHEMISTRY., vol. 26, no. 4, 1 May 2012 (2012-05-01), pages 251-258, XP55524700, AMSTERDAM, NL ISSN: 1089-8603, DOI: 10.1016/j.niox.2012.03.005

**Description**

FIELD OF INVENTION

The present application is related to compositions and methods for synthesis and delivery of high-affinity oxygen binding agents to tumors to increase intratumoral partial pressures of oxygen, mitigate the natural selection of tumor cells that demonstrate aggressive molecular behavior and metastatic potential, and potentiate the effects of radiation and chemotherapies. The present application is also related to compositions and methods for generation and delivery of vasoactive compounds that induce tumor-specific hemostasis. The present invention relates to a composition comprising a nitric oxide (NO)-inhibiting agent and a carrier vehicle, wherein the (NO)-inhibiting agent is for use in treating highly vascularized tumors by causing microvascular embolization in the tumor,wherein the carrier vehicle is a nanoparticle, and wherein the nanoparticle is a polymersome, wherein the (NO)-inhibiting agent comprises an (NO)-binding molecule; wherein NO binding is enabled only at oxygen tensions of less than 10 mm Hg; wherein the (NO)-binding molecule is selected from one or more of unmodified human myoglobin, unmodified myoglobin from another biological species, and chemically or genetically modified myoglobin from humans or from another biological species;wherein the (NO)-binding molecule is preloaded with oxygen; and wherein the NO-inhibiting agent is encapsulated within the carrier vehicle.

BACKGROUND OF THE INVENTION

Each year, approximately 1.2 million Americans are diagnosed with solid tumor malignancies, resulting in aggregate health care costs of greater than $55 billion for treatment.[1,2] More than 50% of these patients undergo radiotherapy (XRT) as part of their treatment plan.[3-5] Local tumor recurrence in the radiated field is often implicated as a primary cause of treatment failure in patients undergoing definitive therapy.[4-7] The ability of XRT to eradicate malignant cells depends critically upon the intratumoral content of $O_2$, a potent radiosensitizer involved in mediating DNA damage.[8-10] The intratumoral $O_2$ level is one of the most important determinants of response among tumors of the same type treated with a single fraction of ionizing radiation therapy.[4, 5, 11] Experimental studies suggest that hypoxic cells are 2-3 times more resistant to a single fraction of ionizing radiation than those with normal levels of $O_2$.[5, 10, 12,13] While XRT generates high levels of localized reactive oxygen species (ROS) that are cytotoxic, tumor hypoxia promotes baseline endogenous ROS[14] that result in the stabilization of hypoxia-inducible factor 1 (HIF-1) and lead to a more aggressive tumorigenic phenotype.[3, 5, 8, 15-17] Investigations on the prognostic significance of the pretreatment $O_2$ levels of tumors in patients with head, neck, and cervical cancers have further demonstrated that worsening hypoxia, typically designated in these studies as oxygen tension ($pO_2$) levels below 2.5-10 mmHg, is associated with both radiation and chemotherapy resistance, decreased local tumor control after surgery, as well as lower rates of suirvival.[4, 5, 18-28]

Although hypoxia has been recognized as a cause of treatment failure in solid tumors for more than 50 years, efforts to overcome it have generally been unsuccessfu1.[4, 5, 8, 29-35] A number of strategies have been designed to enhance the radiosensitivity and radiocurability of solid tumors. The most well-studied, hypoxia-altering methods have involved the use of electron-affinity radiosensitizers that mimic the actions of $O_2$ but are more slowly metabolized. During the past three decades, the nitroimidazole compounds have been extensively evaluated as adjuncts to XRT in carcinomas of the head, neck, cervix, and lung.[36-43] Most of these studies have reported disappointing local control and survival outcomes,[36-38, 40, 41, 43] but efforts to maximize their efficacy and safety, as well as to develop newer classes of agents, are ongoing.[44-47] The majority of alternative strategies have relied on the use of bulk alkylating compounds that confer direct cytotoxic effects that are independent of XRT administration. Clinical trials evaluating mitomycin C, tirapazamine, porfiromycin and others have shown statistically and clinically significant improvements in loco-regional control and cause-specific survival of various cancers, but often at the cost of significant toxicities with repeated dosing.[30-32, 48-73]

The most direct and least toxic path to overcoming tumor hypoxia is to increase the intratumoral $pO_2$. The administration of hyperbaric oxygen was initially attempted but is not used clinically as it exhibits inconsistent response, prohibitive cost, inconvenience, and administration-related safety issues.[4, 5, 8, 10, 74] More recent strategies have included administration of carbogen,[45, 75-82] transfusions of blood, synthetic hemoglobin-based oxygen carriers, or perfluorocarbon emulsions,[5, 83, 84] and injections of recombinant human erythropoietin,[5, 85-89] allosteric effectors (RSR13), or angiogenesis inhibitors.[90-92] All of these strategies have met with minimal clinical success due to their reliance on hyperbaric oxygen loading, formulation instabilities, release of hemoglobin-bound oxygen that occurs at $pO_2$ values (20-40 mmHg) that are much higher than those found in hypoxic tumor regions (< 3 mmHg), and/or intravascular regulatory mechanisms that alter blood flow to maintain relatively constant tissue oxygenation levels.[5, 8, 83, 91]

In addition, since angiogenesis is required for tumor growth and metastasis require, such process is also an important point in the control of cancer progression. Over the past 35 years, some therapies that have been developed to target the molecular underpinnings of tumor-specific angiogenesis include anti- vascular endothelial growth factor (VEGF) therapies and mammalian target of rapamycin (mTOR) inhibitors. However, these therapies only partially inhibit angiogenesis, and therefore provide only a slight effect in most cancers. WO 2012/094679 describes compositions and methods for delivering high-affinity oxygen binding agents to tumors. WO 2011/133635 describes biodegradable nanoparticles as hemoglobin-based oxygen carriers. US 5612310 describes the use of an nitric oxide (NO) scavenger or an NO synthase inhibitor to enhance the effectiveness of tumor therapy with hypoxic or acidic chemotherapeutic agents or hyperthermia.

A list of publications referenced in this disclosure follows.

1. American Cancer Society, Cancer Facts and Figures 2010, http://www.cancer.org/research/cancerfactsfigures/cancerfactsfigures/cancer-facts-and-figures-2010 (last visited Oct. 10, 2011).

2. Care Core National, Radiation Therapy Management 2010, http://www.carecorenational.com/radiation-therapy-management.asp (last visited 2010).

3. Feldmann H.J., Molls M., Vaupel P., Blood Flow And Oxygenation Status Of Human Tumors - Clinical Investigations, STRAHLENTHERAPIE UND ONKOLOGIE 1999;175:1-9.

4. Harrison L., Blackwell K., Hypoxia and Anemia: Factors In Decreased Sensitivity To Radiation Therapy And Chemotherapy, ONCOLOGIST 2004; 9:31-40.

5. Harrison L.B., Chadha M., Hill R.J., Hu K., Shasha D., Impact Of Tumor Hypoxia And Anemia On Radiation Therapy Outcomes, ONCOLOGIST 2002; 7:492-508.

6. Mundt A.J., Connell P.P., Campbell T., Hwang J.H., Rotmensch J., Waggoner S., Race And Clinical Outcome In Patients With Carcinoma Of The Uterine Cervix Treated With Radiation Therapy, GYNECOLOGIC ONCOLOGY 1998; 71:151-8.

7. Takeshi K., Katsuyuki K., Yoshiaki T., et al., Definitive Radiotherapy Combined With High-Dose-Rate Brachytherapy For Stage III Carcinoma Of The Uterine Cervix: Retrospective Analysis Of Prognostic Factors Concerning Patient Characteristics And Treatment Parameters, INTERNATIONAL JOURNAL OF RADIATION ONCOLOGY BIOLOGY PHYSICS 1998; 41:319-27.

8. Rockwell S., Dobrucki I.T., Kim E.Y., Marrison S.T., Vu V.T., Hypoxia and Radiation Therapy: Past History, Ongoing Research, and Future Promise, CURRENT MOLECULAR MEDICINE 2009; 9:442-58.

9. Carlson D.J., Keall P.J., Chen Z.J., Stewart R.D., Nath R., Brown J.M., Towards Temporal Optimization of Radiation Fractionation: The Kinetic Effects of Tumor Hypoxia, DNA Damage Repair, and Tumor Cell Repopulation, INTERNATIONAL JOURNAL OF RADIATION ONCOLOGY BIOLOGY PHYSICS 2009; 75:S615-S6.

10. Brown J.M., The Hypoxic Cell: A Target For Selective Cancer Therapy - Eighteenth Bruce F. Cain Memorial Award Lecture, CANCER RESEARCH 1999; 59:5863-70.

11. Dietz A., Rudat V., Vanselow B., et al. Rise Of Oxygenation In Cervical Lymph Node Metastasis During The Initial Course Of Radiochemotherapy, OTOLARYNGOLOGY-HEAD AND NECK SURGERY 1999; 121:789-96.

12. Evans S.M., Jenkins W.T., Shapiro M., Koch C.J., Evaluation Of The Concept Of "Hypoxic Fraction"As A Descriptor Of Tumor Oxygenation Status, ADV. EXP. MED. BIOL 1997; 411:215-25, OXYGEN TRANSPORT TO TISSUE XVIII (Nemoto E.M., LaManna J.C. eds.) (1997).

13. Teicher B.A., Physiological-Mechanisms of Therapeutic Resistance - Blood-Flow and Hypoxia, HEMATOLOGY-ONCOLOGY CLINICS OF NORTH AMERICA 1995; 9:475-506.

14. Kaelin W.G., ROS: Really Involved In Oxygen Sensing. Cell Metabolism 2005; 1:357-8.

15. Alarcon R., Koumenis C., Geyer R.K., Maki C.G., Giaccia A.J., Hypoxia Induces Accumulation Through MDM2 Down-Regulation And Inhibition Of E6-Mediated Degradation, Cancer Research 1999; 59:6046-51.

16. Graeber T.G., Osmanian C., Jacks T., et al. Hypoxia-Mediated Selection Of Cells With Diminished Apoptotic Potential In Solid Tumors, Nature 1996; 379:88-91.

17. Rockwell S., Oxygen Delivery: Implications For The Biology And Therapy Of Solid Tumors, Oncology Research 1997; 9:383-90.

18. Brizel D.M., Dodge R.K., Clough R.W., Dewhirst M.W., Oxygenation Of Head And Neck Cancer: Changes During Radiotherapy And Impact On Treatment Outcome, Radiotherapy and Oncology 1999; 53:113-7.

19. Brizel D.M., Sibley G.S., Prosnitz L.R., Scher R.L., Dewhirst M.W., Tumor Hypoxia Adversely Affects The Prognosis Of Carcinoma Of The Head And Neck, International Journal of Radiation Oncology Biology Physics 1997; 38:285-9.

20. Fyles A.W., Milosevic M., Wong R., et al., Oxygenation Predicts Radiation Response And Survival In Patients With Cervix Cancer. Radiotherapy And Oncology 1998; 48:149-56.

21. Hockel M., Knoop C., Schlenger K., et al., Intratumoral Po2 Predicts Survival In Advanced Cancer Of The Uterine Cervix, Radiotherapy and Oncology 1993; 26:45-50.

22. Hockel M., Schlenger K., Aral B., Mitze M., Schaffer U., Vaupel P., Association Between Tumor Hypoxia And Malignant Progression In Advanced Cancer Of The Uterine Cervix, Cancer Research 1996; 56:4509-15.

23. Hockel M., Vorndran B., Schlenger K., Baussmann E., Knapstein P.G., Tumor Oxygenation - A New Predictive Parameter In Locally Advanced Cancer Of The Uterine Cervix, Gynecologic Oncology 1993; 51:141-9.

24. Knocke T.H., Weitmann H.D., Feldmann H.J., Selzer E., Potter R., Intratumoral Po(2)-Measurements As Predictive Assay In The Treatment Of Carcinoma Of The Uterine Cervix, Radiotherapy and Oncology 1999; 53:99-104.

25. Rofstad E.K., Sundfor K., Lyng H., Trope C.G., Hypoxia-Induced Treatment Failure In Advanced Squamous Cell Carcinoma Of The Uterine Cervix Is Primarily Due To Hypoxia Induced Radiation Resistance Rather Than Hypoxia-Induced Metastasis, British Journal of Cancer 2000; 83:354-9.

26. Rudat V., Vanselow B., Wollensack P., et al. Repeatability And Prognostic Impact Of The Pretreatment Po(2) Histography In Patients With Advanced Head And Neck Cancer, RADIOTHERAPY AND ONCOLOGY 2000; 57:31-7.

27. Stadler P., Becker A., Feldmann H.J., et al., Influence Of The Hypoxic Subvolume On The Survival Of Patients With Head And Neck Cancer, INTERNATIONAL JOURNAL OF RADIATION ONCOLOGY BIOLOGY PHYSICS 1999; 44:749-54.

28. Stadler P., Feldmann H.J., Creighton C., Kau R., Molls M., Changes In Tumor Oxygenation During Combined Treatment With Split-Course Radiotherapy And Chemotherapy In Patients With Head And Neck Cancer, RADIOTHERAPY AND ONCOLOGY 1998; 48:157-64.

29. Karar J., Maity A., Modulating the Tumor Microenvironment to Increase Radiation Responsiveness, CANCER BIOLOGY & THERAPY 2009; 8:1994-2001.

30. Bache M., Kappler M., Said H.M., Staab A., Vordermark D., Detection And Specific Targeting Of Hypoxic Regions Within Solid Tumors: Current Preclinical And Clinical Strategies, CURRENT MEDICINAL CHEMISTRY 2008; 15:322-38.

31. Ahn G.O., Brown M., Targeting Tumors With Hypoxia-Activated Cytotoxins, FRONTIERS IN BIOSCIENCE 2007; 12:3483-501.

32. Nagasawa H., Uto Y., Kirk K.L., Hori H., Design Of Hypoxia-Targeting Drugs As New Cancer Chemotherapeutics. BIOLOGICAL & PHARMACEUTICAL BULLETIN 2006; 29:2335-42.

33. Janssen H.L., Haustermans K.M., Balm A.J., Begg A.C., Hypoxia In Head And Neck Cancer: How Much, How Important? HEAD AND NECK-JOURNAL FOR THE SCIENCES AND SPECIALTIES OF THE HEAD AND NECK 2005; 27:622-38.

34. Wouters B.G., Weppler S.A., Koritzinsky M., et al., Hypoxia As A Target For Combined Modality Treatments, EUROPEAN JOURNAL OF CANCER 2002; 38:240-57.

35. Kondo A., Safaei R., Mishima M., Niedner H., Lin X.J., Howell S.B., Hypoxia-Induced Enrichment And Mutagenesis Of Cells That Have Lost DNA Mismatch Repair, CANCER RESEARCH 2001; 61:7603-7.

36. Grigsby P.W., Winter K., Wasserman T.H., et al., Irradiation With Or Without Misonidazole For Patients With Stages IIIB And IVA Carcinoma Of The Cervix: Final Results of RTOG 80-05. INTERNATIONAL JOURNAL OF RADIATION ONCOLOGY BIOLOGY PHYSICS 1999; 44:513-7.

37. Lee D.J., Cosmatos D., Marcial V.A., et al., Results Of An RTOG Phase-III Trial (RTOG-85-27) Comparing Radiotherapy Plus Etanidazole With Radiotherapy Alone For Locally Advanced Head And Neck Carcinomas, INTERNATIONAL JOURNAL OF RADIATION ONCOLOGY BIOLOGY PHYSICS 1995; 32:567-76.

38. Lee D.J., Pajak T.F., Stetz J., Order S.E., Weissberg J.B., Fischer J.J., A Phase-I Phase-II Study Of The Hypoxic Cell Sensitizer Misonidazole As An Adjunct To High Fractional Dose Radiotherapy In Patients With Unresectable Squamous-Cell Carcinoma Of The Head And Neck - A RTOG Randomized Study (79-04), INTERNATIONAL JOURNAL OF RADIATION ONCOLOGY BIOLOGY PHYSICS 1989; 16:465-70.

39. Overgaard J., Sensitization Of Hypoxic Tumor-Cells - Clinical-Experience, INTERNATIONAL JOURNAL OF RADIATION BIOLOGY 1989; 56:801-11.

40. Overgaard J., Bentzen S.M., Kolstad P., et al., Misonidazole Combined With Radiotherapy In The Treatment Of Carcinoma Of The Uterine Cervix, INTERNATIONAL JOURNAL OF RADIATION ONCOLOGY BIOLOGY PHYSICS 1989; 16:1069-72.

41. Overgaard J., Hansen H.S., Andersen A.P., et al. Misonidazole Combined With Split-Course Radiotherapy In The Treatment Of Invasive-Carcinoma Of Larynx And Pharynx - Report From The Dahanca-2 Study, INTERNATIONAL JOURNAL OF RADIATION ONCOLOGY BIOLOGY PHYSICS 1989; 16:1065-8.

42. Overgaard J., Hansen H.S., Overgaard M., et al., A Randomized Double-Blind Phase III Study Of Nimorazole As A Hypoxic Radiosensitizer Of Primary Radiotherapy In Supraglottic Larynx And Pharynx Carcinoma, Results Of The Danish Head And Neck Cancer Study (DAHANCA) Protocol 5-85, RADIOTHERAPY AND ONCOLOGY 1998; 46:135-46.

43. Wasserman T.H., Lee D.J., Cosmatos D., et al., Clinical-Trials With Etanidazole (Sr-2508) By The Radiation-Therapy Oncology Group (RTOG), RADIOTHERAPY AND ONCOLOGY 1991; 20:129-35.

44. Lim SH, Lee JY, Park SH, et al. Effect of Combination of Anticancer Agents and Nitroimidazoles on the Survival of Human Hepatocellular Carcinoma Cells under Hypoxic Conditions, JOURNAL OF THE KOREAN SURGICAL SOCIETY 2009; 76:337-47.

45. Fenton B.M., Lord E.M., Paoni S.E., Enhancement Of Tumor Perfusion And Oxygenation By Carbogen And Nicotinamide During Single- And Multifraction Irradiation, RADIATION RESEARCH 2000; 153:75-83.

46. Rosenthal D.I., Nurenberg P., Becerra C.R., et al. A Phase I Single-Dose Trial Of Gadolinium Texaphyrin (Gd-Tex), A Tumor Selective Radiation Sensitizer Detectable By Magnetic Resonance Imaging, CLINICAL CANCER RESEARCH 1999; 5:739-45.

47. Eisbruch A., Robertson J.M., Johnston C.M., et al., Bromodeoxyuridine Alternating With Radiation For Advanced Uterine Cervix Cancer: A Phase I And Drug Incorporation Study, JOURNAL OF CLINICAL ONCOLOGY 1999; 17:31-40.

48. Rischin D., Peters L.J., O'Sullivan B., et al., Tirapazamine, Cisplatin, and Radiation Versus Cisplatin and Radiation for Advanced Squamous Cell Carcinoma of the Head and Neck (TROG 02.02, HeadSTART): A Phase III Trial of the Trans-Tasman Radiation Oncology Group, JOURNAL OF CLINICAL ONCOLOGY 2010; 28:2989-95.

49. Fogh S., Machtay M., Werner-Wasik M., et al., Phase I Trial Using Patupilone (Epothilone B) And Concurrent Radiotherapy For Central Nervous System Malignancies, INTERNATIONAL JOURNAL OF RADIATION ONCOLOGY BIOLOGY PHYSICS 2010; 77:1009-16.

50. de la Fouchardiere C., Negrier S., Labrosse H., et al., Phase I Study Of Daily Irinotecan As A Radiation Sensitizer For Locally Advanced Pancreatic Cancer, INTERNATIONAL JOURNAL OF RADIATION ONCOLOGY BIOLOGY PHYSICS 2010; 77:409-13.

51. Williams K.J., Albertella M.R., Fitzpatrick B., et al., In Vivo Activation Of The Hypoxia-Targeted Cytotoxin AQ4N In Human Tumor Xenografts, MOLECULAR CANCER THERAPEUTICS 2009; 8:3266-75.

52. Hay M.P., Pruijn F.B., Gamage S.A., et al., DNA-Targeted 1,2,4-Benzotriazine 1,4-Dioxides: Potent Analogues Of The Hypoxia-Selective Cytotoxin Tirapazamine, JOURNAL OF MEDICINAL CHEMISTRY 2004; 47:475-88.

53. Shibamoto Y., Zhou L., Hatta H., Mori M., Nishimoto S., In Vivo Evaluation Of A Novel Antitumor Prodrug, 1-(2'-Oxopropyl)-5-Fluorouracil (OFU001), Which Releases 5-Fluorouracil Upon Hypoxic Irradiation, INTERNATIONAL JOURNAL OF RADIATION ONCOLOGY BIOLOGY PHYSICS 2001; 49:407-13.

54. Koch C.J., Hahn S.M., Rockwell K., Covey J.M., McKenna W.G., Evans S.M., Pharmacokinetics Of EF5 2-(2-Nitro-1-H-Imidazol-1-Yl)-N-(2,2,3,3,3-Pentafluoropropyl) Acetamide In Human Patients: Implications For Hypoxia Measurements In Vivo By 2-Nitrolmidazoles, CANCER CHEMOTHER PHARMACOL 2001; 48:177-87.

55. von Pawel J., von Roemeling R., Gatzemeier U., et al., Tirapazamine Plus Cisplatin Versus Cisplatin In Advanced Non-Small-Cell Lung Cancer: A Report Of The International CATAPULT I Study Group, JOURNAL OF CLINICAL ONCOLOGY 2000; 18:1351-9.

56. Roberts K.B., Urdaneta N., Vera R., et al., Interim Results Of A Randomized Trial Of Mitomycin C As An Adjunct To Radical Radiotherapy In The Treatment Of Locally Advanced Squamous-Cell Carcinoma Of The Cervix, INTERNATIONAL JOURNAL OF CANCER 2000; 90:206-23.

57. Papadopoulou M.V., Ji M., Rao M.K., Bloomer W.D., 4- 3-(2-Nitro-1-Imidazolyl)Propylamino-7-Chloroquinoline Hydrochloride (NLCQ-1), A Novel Bioreductive Compound As A Hypoxia-Selective Cytotoxin, ONCOLOGY RESEARCH 2000; 12:185-92.

58. Papadopoulou M.V., Ji M., Rao M.K., Bloomer W.D., 4-3-(2-Nitro-1-Imidazolyl)Propylamino -7-Chloroquinoline Hydrochloride (NLCQ-1), A Novel Bioreductive Agent As Radiosensitizer In Vitro And In Vivo: Comparison With Tirapazamine, ONCOLOGY RESEARCH 2000; 12:325-33.

59. Craighead P.S., Pearcey R., Stuart G., A Phase I/II Evaluation Of Tirapazamine Administered Intravenously Concurrent With Cisplatin And Radiotherapy In Women With Locally Advanced Cervical Cancer, INTERNATIONAL JOURNAL OF RADIATION ONCOLOGY BIOLOGY PHYSICS 2000; 48:791-5.

60. Weitman S., Mangold G., Marty J., et al., Evidence Of Enhanced In Vivo Activity Using Tirapazamine With Paclitaxel And Paraplatin Regimens Against The MV-522 Human Lung Cancer Xenograft, CANCER CHEMOTHER PHARMACOL 1999; 43:402-8.

61. Treat J., Johnson E., Langer C., et al., Tirapazamine With Cisplatin In Patients With Advanced Non-Small-Cell Lung Cancer: A Phase II Study, JOURNAL OF CLINICAL ONCOLOGY 1998; 16:3524-7.

62. Siemann D.W., Hinchman C.A., Potentiation Of Cisplatin Activity By The Bioreductive Agent Tirapazamine, RADIOTHERAPY AND ONCOLOGY 1998; 47:215-20.

63. Lee D.J., Trotti A., Spencer S., et al., Concurrent Tirapazamine And Radiotherapy For Advanced Head And Neck Carcinomas: A Phase II Study, INTERNATIONAL JOURNAL OF RADIATION ONCOLOGY BIOLOGY PHYSICS 1998; 42:811-5.

64. Harrison L.B., Raben A., Pfister D.G., et al. A Prospective Phase II Trial Of Concomitant Chemotherapy And Radiotherapy With Delayed Accelerated Fractionation In Unresectable Tumors Of The Head And Neck, HEAD AND NECK-JOURNAL FOR THE SCIENCES AND SPECIALTIES OF THE HEAD AND NECK 1998; 20:497-503.

65. Gatzemeier U., Rodriguez G., Treat J., et al., Tirapazamine-Cisplatin: The Synergy, BRITISH JOURNAL OF CANCER 1998; 77:15-7.

66. Brown J.M., Wang L.H., Tirapazamine: Laboratory Data Relevant To Clinical Activity, ANTI-CANCER DRUG DESIGN 1998; 13:529-39.

67. Haffty B.G., Son Y.H., Papac R., et al., Chemotherapy As An Adjunct To Radiation In The Treatment Of Squamous Cell Carcinoma Of The Head And Neck: Results Of The Yale Mitomycin Randomized Trials, JOURNAL OF CLINICAL ONCOLOGY 1997; 15:268-76.

68. Adelstein D.J., Saxton J.P., Lavertu P., et al., A Phase III Randomized Trial Comparing Concurrent Chemotherapy And Radiotherapy With Radiotherapy Alone In Resectable Stage III And IV Squamous Cell Head And Neck Cancer: Preliminary Results, HEAD AND NECK-JOURNAL FOR THE SCIENCES AND SPECIALTIES OF THE HEAD AND NECK 1997; 19:567-75.

69. Sartorelli A.C., Hodnick W.F., Belcourt M.F., et al., Mitomycin-C - A Prototype Bioreductive Agent, ONCOLOGY RESEARCH 1994; 6:501-8.

70. Dobrowsky W., Mitomycin-C, 5-Fluorouracil And Radiation In Advanced, Locally Recurrent Rectal-Cancer, BRITISH JOURNAL OF RADIOLOGY 1992; 65:143-7.

71. Sivanesaratnam V., Jayalakshmi P., Mitomycin-C Adjuvant Chemotherapy After Wertheim Hysterectomy For Stage-Ib Cervical-Cancer, CANCER 1989; 64:798-800.

72. Keyes S.R., Rockwell S., Sartorelli A.C., Enhancement Of Mitomycin-C Cyto-Toxicity To Hypoxic Tumor-Cells By Dicoumarol Invivo And Invitro. CANCER RESEARCH 1985; 45:213-6.

73. Keyes S.R, Rockwell S., Sartorelli A.C., Porfiromycin As A Bioreductive Alkylating Agent With Selective Toxicity To Hypoxic Emt6 Tumor-Cells Invivo And Invitro, CANCER RESEARCH 1985; 45:3642-5.

74. Overgaard J., Horsman M.R., Modification Of Hypoxia-Induced Radioresistance In Tumors By The Use Of Oxygen And Sensitizers, SEMINARS IN RADIATION ONCOLOGY 1996; 6:10-21.

75. Aquino-Parsons C., Lim P., Green A., Minchinton A.I., Carbogen Inhalation In Cervical Cancer: Assessment Of Oxygenation Change, GYNECOLOGIC ONCOLOGY 1999; 74:259-64.

76. Bernier J., Denekamp J., Rojas A., et al., ARCON: Accelerated Radiotherapy With Carbogen And Nicotinamide In Head And Neck Squamous Cell Carcinomas: The Experience Of The Cooperative Group Of Radiotherapy Of The European Organization For Research And Treatment Of Cancer (EORTC), RADIOTHERAPY AND ONCOLOGY 2000; 55:111-9.

77. Bussink J., Kaanders J., Van der Kogel A.J., Clinical Outcome And Tumour Microenvironmental Effects Of Accelerated Radiotherapy With Carbogen And Nicotinamide, ACTA ONCOLOGICA 1999; 38:875-82.

78. Hoskin P.J., Saunders M.I., Dische S., Hypoxic Radiosensitizers In Radical Radiotherapy For Patients With Bladder Carcinoma - Hyperbaric Oxygen, Misonidazole, And Accelerated Radiotherapy, Carbogen, And Nicotinamide, CANCER 1999; 86:1322-8.

79. Miralbell R., Mornex F., Greiner R., et al., Accelerated Radiotherapy, Carbogen, And Nicotinamide In Glioblastoma Multiforme: Report Of European Organization For Research And Treatment Of Cancer Trial 22933, JOURNAL OF CLINICAL ONCOLOGY 1999; 17:3143-9.

80. Saunders M., Dische S., Clinical Results Of Hypoxic Cell Radiosensitisation From Hyperbaric Oxygen To Accelerated Radiotherapy, Carbogen And Nicotinamide, BRITISH JOURNAL OF CANCER 1996; 74:S271-S8.

81. Stuben G., Stuschke M., Knuhmann K., Horsman M.R., Sack H., The Effect Of Combined Nicotinamide And Carbogen Treatments In Human Tumour Xenografts: Oxygenation And Tumour Control Studies, RADIOTHERAPY AND ONCOLOGY 1998; 48:143-8.

82. Zhan H.W., Liu H.B., Bao C.K., Ye X.J., Zhang H., He G.Q., Effect Of Carbogen On Tumour Oxygenation And 32P-Colloid Interstitial Irradiation Response, MEDICAL SCIENCE MONITOR 2010; 16:BR11-BR6.

83. Yu M.H., Dai M., Liu Q., Xiu R.J., Oxygen Carriers And Cancer Chemo- And Radiotherapy Sensitization: Bench To Bedside And Back, CANCER TREATMENT REVIEWS 2007; 33:757-61.

84. Hoogsteen I.J., Marrest A.M., van der Kogel A.J., Kaanders J., The Hypoxic Tumour Microenvironment, Patient Selection And Hypoxia-Modifying Treatments, CLINICAL ONCOLOGY 2007; 19:385-96.

85. Shasha D., The Negative Impact Of Anemia On Radiotherapy And Chemoradiation Outcomes, SEMINARS IN HEMATOLOGY 2001; 38:8-15.

86. Shasha D., George M.J., Harrison L.B., Once-Weekly Dosing Of Epoetin Alfa Increases Hemoglobin And Improves Quality Of Life In Anemic Cancer Patients Receiving Radiation Therapy Either Concomitantly Or Sequentially With Chemotherapy, BLOOD 2000; 96:1866.

87. Henke M., Guttenberger R., Barke A., Pajonk F., Potter R., Frommhold H., Erythropoietin For Patients Undergoing Radiotherapy: A Pilot Study, RADIOTHERAPY AND ONCOLOGY 1999; 50:185-90.

88. Dusenbery K.E., McGuire W.A., Holt P.J., et al., Erythropoietin Increases Hemoglobin During Radiation-Therapy For Cervical-Cancer, INTERNATIONAL JOURNAL OF RADIATION ONCOLOGY BIOLOGY PHYSICS 1994; 29:1079-84.

89. Lavey R.S., Dempsey W.H., Erythropoietin Increases Hemoglobin In Cancer-Patients During Radiation-Therapy, INTERNATIONAL JOURNAL OF RADIATION ONCOLOGY BIOLOGY PHYSICS 1993; 27:1147-52.

90. McGee M.C., Hamner J.B., Williams R.F., et al., Improved Intratumoral Oxygenation Through Vascular Normalization Increases Glioma Sensitivity To Ionizing Radiation, INTERNATIONAL JOURNAL OF RADIATION ONCOLOGY BIOLOGY PHYSICS 2010; 76:1537-45.

91. Katz D., Ito E., Liu F.F., On The Path To Seeking Novel Radiosensitizers, INTERNATIONAL JOURNAL OF RADIATION ONCOLOGY BIOLOGY PHYSICS 2009; 73:988-96.

92. Gali-Muhtasib H., Sidani M., Geara F., et al., Quinoxaline 1,4-Dioxides Are Novel Angiogenesis Inhibitors That Potentiate Antitumor Effects Of Ionizing Radiation, INTERNATIONAL JOURNAL OF ONCOLOGY 2004; 24:1121-31.

93. Ordway G.A., Garry D.J., Myoglobin: An Essential Hemoprotein In Striated Muscle, JOURNAL OF EXPERIMENTAL BIOLOGY 2004; 207:3441-6.

94. Wittenberg J.B., Wittenberg B.A., Myoglobin Function Reassessed, JOURNAL OF EXPERIMENTAL BIOLOGY 2003; 206:2011-20.

95. Kooyman G.L., Ponganis P.J., The Physiological Basis Of Diving To Depth: Birds And Mammals, ANNUAL REVIEW OF PHYSIOLOGY 1998; 60:19-32.

96. Hochachka P.W., The Metabolic Implications Of Intracellular Circulation, PROCEEDINGS OF THE NATIONAL ACADEMY OF SCIENCES OF THE UNITED STATES OF AMERICA 1999; 96:12233-9.

97. Conley K.E., Jones C., Myoglobin Content And Oxygen Diffusion: Model Analysis Of Horse And Steer Muscle, AMERICAN JOURNAL OF PHYSIOLOGY-CELL PHYSIOLOGY 1996; 271:C2027-C36.

98. Galluzzo M., Pennacchietti S., Rosano S., Comoglio P.M., Michieli P., Prevention Of Hypoxia By Myoglobin Expression In Human Tumor Cells Promotes Differentiation And Inhibits Metastasis, JOURNAL OF CLINICAL INVESTIGATION 2009; 119:865-75.

99. Floegel U., Dang C.V., Myoglobin Tames Tumor Growth And Spread, JOURNAL OF CLINICAL INVESTIGATION 2009; 119:766-8.

100. Bunn H.F., The Role Of Hemoglobin Based Substitutes In Transfusion Medicine, TRANSFUS CLIN. BIOL. 1995; 2:433-9.

101. Gottschalk A., Raabe A., Hommel M., Rempf C., Freitag M., Standl T., Influence Of The Hemoglobin Solution HBOC-201 On Tissue Oxygenation In The Rat RIH-Tumor, ARTIF. CELLS BLOOD SUBSTIT. BIOTECHNOL. 2005; 33:379-89.

102. Gundersen S.I., Palmer A.F., Hemoglobin-Based Oxygen Carrier Enhanced Tumor Oxygenation: A Novel Strategy for Cancer Therapy, BIOTECHNOL. PROG. 2008; 24:1353-64.

103. Palaparthy R., Wang H.S., Gulati A., Current Aspects In Pharmacology Of Modified Hemoglobins, ADV DRUG DELIV. REV 2000; 40:185-98.

104. Robinson M.F., Dupuis N.P., Kusumoto T., Liu F., Menon K., Teicher B.A., Increased Tumor Oxygenation And Radiation Sensitivity In 2 Rat-Tumors By A Hemoglobin-Based, Oxygen-Carrying Preparation, ARTIF. CELLS BLOOD SUBSTIT. IMMOBIL. BIOTECHNOL. 1995; 23:431-8.

105. Rowinsky E.K., Novel Radiation Sensitizers Targeting Tissue Hypoxia, ONCOLOGY-NY 1999; 13:61-70.

106. Teicher B.A., Herman T.S., Hopkins R.E., Menon K., Effect Of A Bovine Hemoglobin Preparation On The Response Of The Fsaiic Fibrosarcoma To Chemotherapeutic Alkylating-Agents, J. CANCER RES. CLIN. ONCOL. 1992; 118:123-8.

107. Teicher B.A., Holden S.A., Dupuis N.P., et al., Oxygenation Of The Rat-91 Gliosarcoma And The Rat-13672 Mammary-Carcinoma With Various Doses Of A Hemoglobin Solution, ARTIF. CELLS BLOOD SUBSTIT. IMMOBIL. BIOTECHNOL. 1994; 22:827-33.

108. Teicher B.A., Holden S.A., Menon K., Hopkins R.E., Gawryl M.S., Effect Of Hemoglobin Solution On The Response Of Intracranial And Subcutaneous Tumors To Antitumor Alkylating-Agents, CANCER CHEMOTHER. PHARMACOL. 1993; 33:57-62.

109. Teicher B.A., Schwartz G.N., Sotomayor E.A., Robinson M.F., Dupuis N.P., Menon K., Oxygenation Of Tumors By A Hemoglobin Solution, J. CANCER RES. CLIN. ONCOL. 1993; 120:85-90.

110. Christian D.A., Cai S., Bowen D.M., Kim Y., Pajerowski J.D., Discher D.E., Polymersome Carriers: From Self-Assembly To siRNA And Protein Therapeutics, EUR. J. PHARM. BIOPHARM. 2009; 71:463-74.

111. Levine D.H., Ghoroghchian P.P., Freudenberg J., et al., Polymersomes: A New MultiFunctional Tool For Cancer Diagnosis And Therapy, METHODS 2008; 46:25-32.

112. Letchford K., Burt H., A Review Of The Formation And Classification Of Amphiphilic Block Copolymer Nanoparticulate Structures: Micelles, Nanospheres, Nanocapsules And Polymersomes, EUR. J. PHARM. BIOPHARM. 2007; 65:259-69.

113. Discher B.M., Hammer D.A., Bates F.S., Discher D.E., Polymer Vesicles In Various Media, CURR. OPIN. COLLOID INTERFACE SCI. 2000; 5:125-31.

114. Discher D.E., Ortiz V., Srinivas G., et al., Emerging Applications Of Polymersomes In Delivery: From Molecular Dynamics To Shrinkage Of Tumors, PROG. POLYM. SCI. 2007; 32:838-57.

115. Discher B.M., Won Y.Y., Ege D.S., et al., Polymersomes: Tough Vesicles Made From Diblock Copolymers, SCIENCE 1999; 284:1143-6.

116. O'Neil C.P., Suzuki T., Demurtas D., Finka A., Hubbell J.A., A Novel Method For The Encapsulation Of Biomolecules Into Polymersomes Via Direct Hydration, LANGMUIR 2009; 25:9025-9.

117. Lee J.C.M., Bermudez H., Discher B.M., et al., Preparation, Stability, And In Vitro Performance Of Vesicles Made With Diblock Copolymers, BIOTECHNOL. BIOENG. 2001; 73:135-45.

118. Ghoroghchian P.P., Li G.Z., Levine D.H., et al., Bioresorbable Vesicles Formed Through Spontaneous Self-Assembly Of Amphiphilic Poly(Ethylene Oxide)-Block-Polycaprolactone, MACROMOLECULES 2006; 39:1673-5.

119. Kim Y.H., Tewari M., Pajerowski J.D., et al., Polymersome Delivery Of siRNA And Antisense Oligonucleotides, J. CONTROL RELEASE 2009; 134:132-40.

120. Hearnden V., Lomas H., MacNeil S., et al., Diffusion Studies of Nanometer Polymersomes Across Tissue Engineered Human Oral Mucosa, PHARMACEUTICAL RESEARCH 2009; 26:1718-28.

121. Meng F.H., Engbers G.H.M., Feijen J., Biodegradable Polymersomes As A Basis For Artificial Cells: Encapsulation, Release And Targeting, J. CONTROL RELEASE 2005; 101:187-98.

122. Photos P.J., Bacakova L., Discher B., Bates F.S., Discher D.E., Polymer Vesicles In Vivo: Correlations With PEG Molecular Weight, J. CONTROL RELEASE 2003; 90:323-34.

123. Ghoroghchian P.P., Lin J.J., Brannan A.K., et al., Quantitative Membrane Loading Of Polymer Vesicles, SOFT MATTER 2006; 2:973-80.

124. Ghoroghchian P.P., Frail P.R., Susumu K., et al., Near-Infrared-Emissive Polymersomes: Self-Assembled Soft Matter For In Vivo Optical Imaging, PROC NATL. ACAD. SCI. U.S.A. 2005; 102:2922-7.

125. Bermudez H., Hammer D.A., Discher D.E., Effect Of Bilayer Thickness On Membrane Bending Rigidity, LANGMUIR 2004; 20:540-3.

126. Bermudez H., Brannan A.K., Hammer D.A., Bates F.S., Discher D.E., Molecular Weight Dependence Of Polymersome Membrane Structure, Elasticity, And Stability, MACROMOLECULES 2002; 35:8203-8.

127. Massignani M., LoPresti C., Blanazs A., et al., Controlling Cellular Uptake by Surface Chemistry, Size, and Surface Topology at the Nanoscale, SMALL 2009; 5:2424-32.

128. Blanazs A., Massignani M., Battaglia G., Armes S.P., Ryan A.J., Tailoring Macromolecular Expression at Polymersome Surfaces, ADVANCED FUNCTIONAL MATERIALS 2009; 19:2906-14.

129. van Dongen S.F.M., Nallani M., Schoffelen S., Cornelissen J., Nolte R.J.M., van Hest J.C.M., A Block Copolymer For Functionalisation Of Polymersome Surfaces, MACROMOLECULAR RAPID COMMUNICATIONS 2008; 29:321-5.

130. Christian N.A., Milone M.C., Ranka S.S., et al., Tat-Functionalized Near-Infrared Emissive Polymersomes For Dendritic Cell Labeling, BIOCONJUGATE. CHEM. 2007; 18:31-40.

131. Binder W.H., Sachsenhofer R, Farnik D, Blaas D. Guiding The Location Of Nanoparticles Into Vesicular Structures: A Morphological Study, PHYSICAL CHEMISTRY CHEMICAL PHYSICS 2007; 9:6435-41.

132. Lin J.J., Ghoroghchian P., Zhang Y., Hammer D.A., Adhesion Of Antibody-Functionalized Polymersomes, LANGMUIR 2006; 22:3975-9.

133. Lin J.J., Silas J.A., Bermudez H., Milam V.T., Bates F.S., Hammer D.A., The Effect Of Polymer Chain Length And Surface Density On The Adhesiveness Of Functionalized Polymersomes, LANGMUIR 2004; 20:5493-500.

134. Pangu G.D., Davis K.P., Bates F.S., Hammer D.A., Ultrasonically Induced Release from Nanosized Polymer Vesicles, MACROMOL. BIOSCI. 2010; 10:546-54.

135. Kim M.S., Lee D.S., Biodegradable And Ph-Sensitive Polymersome With Tuning Permeable Membrane For Drug Delivery Carrier, CHEMICAL COMMUNICATIONS 2010; 46:4481-3.

136. Chen W., Meng F.H., Cheng R., Zhong Z.Y., pH-Sensitive Degradable Polymersomes For Triggered Release Of Anticancer Drugs: A Comparative Study With Micelles, J CONTROL RELEASE 2010; 142:40-6.

137. Robbins G.P., Jimbo M., Swift J., Therien M.J., Hammer D.A., Dmochowski I.J., Photoinitiated Destruction of Composite Porphyrin-Protein Polymersomes, J AM CHEM SOC 2009; 131:3872-+.

138. Kim K.T., Cornelissen J., Nolte R.J.M., van Hest J.C.M., A Polymersome Nanoreactor with Controllable Permeability Induced by Stimuli-Responsive Block Copolymers, ADVANCED MATERIALS 2009; 21:2787.

139. Sanson C., Le Meins J.F., Schatz C., Soum A., Lecommandoux S., Temperature Responsive Poly(Trimethylene Carbonate)-Block-Poly(L-Glutamic Acid) Copolymer: Polymersomes Fusion And Fission, SOFT MATTER 2010; 6:1722-30.

140. Castillo R.V., Muller A.J., Raquez J.M., Dubois P., Crystallization Kinetics and Morphology of Biodegradable Double Crystalline PLLA-b-PCL Diblock Copolymers, MACROMOLECULES 2010; 43:4149-60.

141. Wang F., Wang Y.C., Yan L.F., Wang J., Biodegradable Vesicular Nanocarriers Based On Poly(Epsilon-Caprolactone)-Block-Poly(Ethyl Ethylene Phosphate) For Drug Delivery, POLYMER 2009; 50:5048-54.

142. Schatz C., Louguet S., Le Meins J.F., Lecommandoux S., Polysaccharide-Block-Polypeptide Copolymer Vesicles: Towards Synthetic Viral Capsids, ANGEW. CHEM. INT. EDIT 2009; 48:2572-5.

143. Rabotyagova O.S., Cebe P., Kaplan D.L., Self-Assembly of Genetically Engineered Spider Silk Block Copolymers, BIOMACROMOLECULES 2009; 10:229-36.

144. Katz J.S., Levine D.H., Davis K.P., Bates F.S., Hammer D.A., Burdick J.A., Membrane Stabilization of Biodegradable Polymersomes, LANGMUIR 2009; 25:4429-34.

145. Bromley E.H.C, Channon K., Moutevelis E., Woolfson D.N., Peptide And Protein Building Blocks For Synthetic Biology: From Programming Biomolecules To Self-Organized Biomolecular Systems, Acs CHEMICAL BIOLOGY 2008; 3:38-50.

146. Najafi F., Sarbolouki M.N., Biodegradable Micelles/Polymersomes From Fumaric\Sebacic Acids And Poly(Ethylene Glycol), BIOMATERIALS 2003;24:1175-82.

147. Rameez S., Bamba I., Palmer A.F., Large Scale Production of Vesicles by Hollow Fiber Extrusion: A Novel Method for Generating Polymersome Encapsulated Hemoglobin Dispersions, LANGMUIR 2010; 26:5279-85.

148. Shum H.C., Kim J.W., Weitz D.A., Microfluidic Fabrication Of Monodisperse Biocompatible And Biodegradable Polymersomes With Controlled Permeability, J AM CHEM SOC 2008; 130:9543-9.

149. Yildiz M.E., Prud'homme R.K., Robb I., Adamson D.H., Formation And Characterization Of Polymersomes Made By A Solvent Injection Method, POLYMERS FOR ADVANCED TECHNOLOGIES 2007; 18:427-32.

150. Dewhirst M.W., Relationships Between Cycling Hypoxia, HIF-1, Angiogenesis And Oxidative Stress, RADIATION RESEARCH 2009; 172:653-65.

151. Palmer G.M., Boruta R.J., Viglianti B.L., Lan L., Spasojevic I., Dewhirst M.W., Non-Invasive Monitoring Of Intra-Tumor Drug Concentration And Therapeutic Response Using Optical Spectroscopy, J CONTROL RELEASE 2010; 142:457-64.

152. Dewhirst M.W., Thrall D.E., Palmer G., et al., Utility Of Functional Imaging In Prediction Or Assessment Of Treatment Response And Prognosis Following Thermotherapy, INTERNATIONAL JOURNAL OF HYPERTHERMIA 2010; 26:283-93.

153. Zhang G.Q., Palmer G.M., Dewhirst M., Fraser C.L., A Dual-Emissive-Materials Design Concept Enables Tumour Hypoxia Imaging, NATURE MATERIALS 2009; 8:747-51.

154. Palmer G.M., Viola R.J., Schroeder T., Yarmolenko P.S., Dewhirst M.W., Ramanujam N., Quantitative Diffuse Reflectance And Fluorescence Spectroscopy: Tool To Monitor Tumor Physiology In Vivo, JOURNAL OF BIOMEDICAL OPTICS 2009; 14.

155. Hardee M.E., Dewhirst M.W., Agarwal N., Sorg B.S., Novel Imaging Provides New Insights into Mechanisms of Oxygen Transport in Tumors, CURRENT MOLECULAR MEDICINE 2009; 9:435-41.

156. Sorg B.S., Hardee M.E., Agarwal N., Moeller B.J., Dewhirst M.W., Spectral Imaging Facilitates Visualization And Measurements Of Unstable And Abnormal Microvascular Oxygen Transport In Tumors, JOURNAL OF BIOMEDICAL OPTICS 2008; 13.

157. Dewhirst M.W., Cao Y., Moeller B., Cycling Hypoxia And Free Radicals Regulate Angiogenesis And Radiotherapy Response, NATURE REVIEWS CANCER 2008; 8:425-37.

158. Sorg B.S., Moeller B.J., Donovan O., Cao Y.T., Dewhirst M.W., Hyperspectral Imaging Of Hemoglobin Saturation In Tumor Microvasculature And Tumor Hypoxia Development, JOURNAL OF BIOMEDICAL OPTICS 2005; 10.

159. Dewhirst M.W., Cao Y.T., Li C.Y., Moeller B., Exploring The Role Of HIF-1 In Early Angiogenesis And Response To Radiotherapy, RADIOTHERAPY AND ONCOLOGY 2007; 83:249-55.

160. Moeller B., Dewhirst M.W., HIF-I and tumour radiosensitivity, BRITISH JOURNAL OF CANCER 2006; 95:1-5.

161. Moeller B.J., Dreher M.R., Rabbani Z.N., et al., Pleiotropic Effects Of HIF-1 Blockade On Tumor Radiosensitivity, CANCER CELL 2005; 8:99-110.

162. Arifin D.R., Palmer A.F., Polymersome Encapsulated Hemoglobin: A Novel Type Of Oxygen Carrier, BIOMACROMOLECULES 2005; 6:2172-81.

163. Rameez S., Alosta H., Palmer A.F., Biocompatible And Biodegradable Polymersome Encapsulated Hemoglobin: A Potential Oxygen Carrier, BIOCONJUGATE CHEM 2008; 19:1025-32.

164. Ghoroghchian P.P., Therien M.J., Hammer D.A., In Vivo Fluorescence Imaging: A Personal Perspective, WILEY INTERDISCIP. REV-NANOMED NANOBIOTECHNOL. 2009; 1:156-67.

165. Duncan T.V., Ghoroghchian P.P., Rubtsov I.V., Hammer D.A., Therien M.J., Ultrafast Excited-State Dynamics Of Nanoscale Near-Infrared Emissive Polymersomes, J AM. CHEM. SOC 2008; 130:9773-84.

166. Ghoroghchian P.P., Frail P.R., Li G.Z., et al., Controlling Bulk Optical Properties Of Emissive Polymersomes Through Intramembranous Polymer-Fluorophore Interactions, CHEM. MAT 2007; 19:1309-18.

167. Ghoroghchian P.P., Frail P.R., Susumu K., et al., Broad Spectral Domain Fluorescence Wavelength Modulation Of Visible And Near-Infrared Emissive Polymersomes, J. AM. CHEM. SOC. 2005; 127:15388-90.

168. Christian N.A., Benencia F., Milone M.C., et al., In Vivo Dendritic Cell Tracking Using Fluorescence Lifetime Imaging and Near-Infrared-Emissive Polymersomes, MOL. IMAGING BIOL. 2009; 11:167-77.

169. Hearnden V., Battaglia G., MacNeil S., Murdoch C., Thornhill M., Penetration Of Polymersome Drug And Gene Delivery Nanoparticles Into In Vitro Models Of Head And Neck Cancer And Tissue Engineered Oral Mucosa, ORAL ONCOLOGY 2009:66.

170. Li S.L., Byrne B., Welsh J., Palmer A.F., Self-Assembled Poly(Butadiene)-B-Poly(Ethylene Oxide) Polymersomes As Paclitaxel Carriers, BIOTECHNOL. PROG. 2007; 23:278-85.

171. Ahmed F., Pakunlu R.I., Brannan A., Bates F., Minko T., Discher D.E., Biodegradable Polymersomes Loaded With Both Paclitaxel And Doxorubicin Permeate And Shrink Tumors, Inducing Apoptosis In Proportion To Accumulated Drug, J. CONTROL RELEASE 2006; 116:150-8.

172. Zhang X.L., Liu C.S., Yuan Y., et al., Key Parameters Affecting The Initial Leaky Effect Of Hemoglobin-Loaded Nanoparticles As Blood Substitutes, JOURNAL OF MATERIALS SCIENCE-MATERIALS IN MEDICINE 2008; 19:2463-70.

173. Dewhirst M.W., Kimura H., Rehmus S.W.E., et al., Microvascular Studies On The Origins Of Perfusion-Limited Hypoxia, BRITISH JOURNAL OF CANCER 1996; 74:S247-S51.

174. Zupancich J.A., Bates F.S., Hillmyer M.A., Aqueous Dispersions Of Poly(Ethylene Oxide)-B-Poly(Gamma-Methyl-Epsilon-Caprolactone) Block Copolymers, MACROMOLECULES 2006; 39:4286-8.

175. Hahn J.S., Braun R.D., Dewhirst M.W., et al., Stroma-Free Human Hemoglobin A Decreases R3230Ac Rat Mammary Adenocarcinoma Blood Flow And Oxygen Partial Pressure, RADIATION RESEARCH 1997; 147:185-94.

176. Arifin D.R., Palmer A.F., Determination Of Size Distribution And Encapsulation Efficiency Of Liposome-Encapsulated Hemoglobin Blood Substitutes Using Asymmetric Flow Field-Flow Fractionation Coupled With Multi-Angle Static Light Scattering, BIOTECHNOL PROG 2003; 19:1798-811.

177. Sakai H., Sato A., Sobolewski P., et al., NO And CO Binding Profiles Of Hemoglobin Vesicles As Artificial Oxygen Carriers, BIOCHIMICA ET BIOPHYSICA ACTA-PROTEINS AND PROTEOMICS 2008; 1784:1441-7.

178. Sakai H., Hamada K., Takeoka S., Nishide H., Tsuchida E., Functional Evaluation Of Hemoglobin- And Lipidheme-Vesicles As Red Cell Substitutes, POLYMERS FOR ADVANCED TECHNOLOGIES 1996; 7:639-44.

179. Frauenfelder H., McMahon B.H., Fenimore P.W., Myoglobin: The Hydrogen Atom Of Biology And A Paradigm Of Complexity, PROCEEDINGS OF THE NATIONAL ACADEMY OF SCIENCES OF THE UNITED STATES OF AMERICA 2003; 100:8615-7.

180. Herold S., Exner M., Nauser T., Kinetic And Mechanistic Studies Of The NO Center Dot-Mediated Oxidation Of Oxymyoglobin And Oxyhemoglobin, BIOCHEMISTRY 2001; 40:3385-95.

181. Nienhaus G.U., Mourant J.R., Chu K., Frauenfelder H., Ligand-Binding To Heme-Proteins - The Effect Of Light On Ligand-Binding In Myoglobin, BIOCHEMISTRY 1994; 33:13413-30.

182. Ansari A., Jones C.M., Henry E.R., Hofrichter J., Eaton W.A., Conformational Relaxation And Ligand-Binding In Myoglobin, BIOCHEMISTRY 1994; 33:5128-45.

183. Mourant J.R., Braunstein D.P., Chu K., et al., Ligand-Binding To Heme-Proteins .2. Transitions In The Heme Pocket Of Myoglobin, BIOPHYSICAL JOURNAL 1993; 65:1496-507.

184. Kanner J., Harel S., Granit R., Nitric-Oxide As An Antioxidant, ARCHIVES OF BIOCHEMISTRY AND BIOPHYSICS 1991; 289:130-6.

185. Chance B., Optical Method. ANNUAL REVIEW OF BIOPHYSICS AND BIOPHYSICAL CHEMISTRY 1991; 20:1-28.

186. Chance B., Nioka S., Kent J., et al., Time-Resolved Spectroscopy Of Hemoglobin And Myoglobin In Resting And Ischemic Muscle, ANALYTICAL BIOCHEMISTRY 1988; 174:698-707.

187. Privalov P.L., Griko Y.V., Venyaminov S.Y., Kutyshenko V.P., Cold Denaturation Of Myoglobin, JOURNAL OF MOLECULAR BIOLOGY 1986; 190:487-98.

188. Helcke G.A., Ingram D.J.E., Slade E.F., Electron Resonance Studies Of Haemoglobin Derivatives .3. Line-Width And G-Value Measurements Of Acid-Met Myoglobin And Of Met Myoglobin Azide Derivatives, PROCEEDINGS OF THE ROYAL SOCIETY OF LONDON SERIES B-BIOLOGICAL SCIENCES 1968; 169:275.

189. Moeller B.J., Richardson R.A., Dewhirst M.W., Hypoxia And Radiotherapy: Opportunities For Improved Outcomes In Cancer Treatment, CANCER AND METASTASIS REVIEWS 2007; 26:241-8.

190. Kong G., Braun R.D., Dewhirst M.W., Characterization Of The Effect Of Hyperthermia On Nanoparticle Extravasation From Tumor Vasculature, CANCER RESEARCH 2001; 61:3027-32.

191. Kong G., Braun R.D., Dewhirst M.W., Hyperthermia Enables Tumor-Specific Nanoparticle Delivery: Effect Of Particle Size, CANCER RESEARCH 2000; 60:4440-5.

192. Moeller B.J., Dewhirst M.W., Raising The Bar - How HIF-1 Helps Determine Tumor Radiosensitivity, CELL CYCLE 2004; 3:1107-10.

193. Mozzarelli A., Faggiano S., Bettati S, Bruno S., Haemoglobin-based oxygen carriers: research and reality towards an alternative to blood transfusions, BLOOD TRANSFUS 2010; 8 (Suppl 3):s59-s68.

194. Aranda-Espinoza H., Bermudez H., Bates F.S., Discher D.E., Electromechanical Limits of Polymersomes, PHYSICAL REVIEW LETTERS 2001; 87:208301.

195. Chin A.I., Lam J.S., Figlin R.A., Belldegrun A.S., Surveillance Strategies for Renal Cell Carcinoma Patients Following Nephrectomy, REVIEWS IN UROLOGY 2006; 8:1-7.

196. Ather M.H., Masood N, T.S. Current management of advanced and metastatic renal cell carcinoma, UROLOGY JOURNAL 2010; 7:1-9.

197. Benjamini Y., Hochberg Y., Controlling the false discovery rate - a practical and powerful approach to multiple testing, JOURNAL OF THE ROYAL STATISTICAL SOCIETY SERIES B-METHODOLOGICAL 1995; 57:289-300.

198. Brahmer J.R. et al., Safety and Activity of Anti-PD-LI Antibody in Patients with Advanced Cancer, NEW ENGLAND JOURNAL OF MEDICINE 2012; 366:2455-65.

199. Cabrales P. FJ, HBOC vasoactivity: interplay between nitric oxide scavenging and capacity to generate bioactive nitric oxide species, ANTIOXID REDOX SIGNAL 2013; 18:2284-97.

200. Caroline R., Jean-Charles S., Alexander M. ME, Drug of the year: Programmed Death-1 receptor/Programmed Death-1 Ligand-1 receptor monoclonal antibodies, EUROPEAN JOURNAL OF CANCER 2013; 49:2968-71.

201. Chance B. et al., Time-Resolved Spectroscopy of Hemoglobin and Myoglobin in Resting and Isechmic Muscle, ANALYTICAL BIOCHEMISTRY 1988;174:698-707.

202. Chance B., Optical Method, ANNUAL REVIEW OF BIOPHYSICS AND BIOPHYSICAL CHEMISTRY 1991; 20:1-28.

203. Chang A., Finelli A., Berns J.S., Rosner M., Chronic Kidney Disease in Patients With Renal Cell Carcinoma, ADVANCES IN CHRONIC KIDNEY DISEASE 2014; 21:91-5.

204. Chang T. MS., Blood replacement with nanobiotechnologically engineered hemoglobin and hemoglobin nanocapsules, WILEY INTERDISCIPLINARY REVIEWS: NANOMEDICINE AND NANOBIOTECHNOLOGY 2010; 2:418-30.

205. Chang Y. et al., Sorafenib (BAY 43-9006) inhibits tumor growth and vascularization and induces tumor apoptosis and hypoxia in RCC xenograft models, CANCER CHEMOTHER PHARMACOL 2007; 59:561-74.

206. Cho D., Signoretti S., Regan M., Mier J.W., Atkins M.B., The role of mammalian target of rapamycin inhibitors in the treatment of advanced renal cancer, CLIN CANCER RES 2007; 13:758s-63s.

207. Cho D. et al., The Efficacy of the Novel Dual PI3-Kinase/mTOR Inhibitor NVP-BEZ235 Compared with Rapamycin in Renal Cell Carcinoma, CLINICAL CANCER RESEARCH 2010; 16:3628-38.

208. Choueiri T.K. et al, Clinical factors associated with outcome in patients with metastatic clear-cell renal cell carcinoma treated with vascular endothelial growth factor-targeted therapy, CANCER 2007; 110:543-50.

209. Choueiri T.K. et al., Prognostic factors associated with long-term survival in previously untreated metastatic renal cell carcinoma, ANN ONCOL 2007; 18:249-55.

210. Choueiri T.K., Efficacy of cabozantinib (XL184) in patients (pts) with metastatic, refractory renal cell carcinoma (RCC), 2012 ASCO Annual Meeting; 2012.

211. Choueiri T.K., Factors associated with outcome in patients with advanced renal cell carcinoma in the era of antiangiogenic agents, CLIN GENITOURIN CANCER 2008; 6:15-20.

212. Christian D.A., Garbuzenko O.B., Minko T., Discher D.E., Polymer Vesicles with a Red Cell-like Surface Charge: Microvascular Imaging and in vivo Tracking with Near-Infrared Fluorescence, MACROMOLECULAR RAPID COMMUNICATIONS 2010; 31:135-41.

213. Clifford S.C., Maher E.R., Von Hippel-Lindau disease: clinical and molecular perspectives, ADV CANCER RES 2001; 82:85-105.

214. Cora N.S. et al., A randomised, double-blind phase III study of pazopanib in patients with advanced and/or metastatic renal cell carcinoma: Final overall survival results and safety update, EUROPEAN JOURNAL OF CANCER 2013; 49:1287-96.

215. Courtney K.D., Choueiri T.K., Optimizing recent advances in metastatic renal cell carcinoma, CURR ONCOL REP 2009; 11:218-26.

216. Courtney K.D., Choueiri T.K., Review: Updates on novel therapies for metastatic renal cell carcinoma, THERAPEUTIC ADVANCES IN MEDICAL ONCOLOGY 2010; 2:209.

217. David S.T. et al., Sickle Erythrocytes Target Cytotoxics to Hypoxic Tumor Microvessels and Potentiate a Tumoricidal Response, PLOS ONE 2013; 8:e52543.

218. Doehn C. et al., Mode-of-Action, Efficacy, and Safety of a Homologous Multi-Epitope Vaccine in a Murine Model for Adjuvant Treatment of Renal Cell Carcinoma, EUROPEAN UROLOGY 2009; 56:123-31.

219. Escudier B, et al., Randomized, Controlled, Double-Blind, Cross-Over Trial Assessing Treatment Preference for Pazopanib Versus Sunitinib in Patients With Metastatic Renal Cell Carcinoma: PISCES Study, JOURNAL OF CLINICAL ONCOLOGY 2014; 32:1412-8.

220. Feldman D.R. et al., Phase I Trial of Bevacizumab Plus Escalated Doses of Sunitinib in Patients With Metastatic Renal Cell Carcinoma, JOURNAL OF CLINICAL ONCOLOGY 2009; 27:1432-9.

221. Figlin R.A. et al., Overall survival with sunitinib versus interferon (IFN)-alfa as first-line treatment of metastatic renal cell carcinoma (mRCC), JOURNAL OF CLINICAL ONCOLOGY 2008 (Meeting Abstracts); 26:abstr 5024.

222. Fontanella A.N. et al., Quantitative Mapping of Hemodynamics in the Lung, Brain, and Dorsal Window Chamber-Grown Tumors Using a Novel, Automated Algorithm, MICROCIRCULATION 2013; 20:724-35.

223. Golshayan A.R. et al., Metastatic Sarcomatoid Renal Cell Carcinoma Treated With Vascular Endothelial Growth Factor-Targeted Therapy, JOURNAL OF CLINICAL ONCOLOGY 2009; 27:235-41.

224. Gregory M.P. et al., In vivo optical molecular imaging and analysis in mice using dorsal window chamber models applied to hypoxia, vasculature and fluorescent reporters, NATURE PROTOCOLS 2011; 6:1355-66.

225. Grepin R. et al., Acceleration of clear cell renal cell carcinoma growth in mice following bevacizumab/Avastin treatment: the role of CXCL cytokines, ONCOGENE 2012; 31:1683-94.

226. Grisanzio C. et al., Orthotopic xenografts of RCC retain histological, immunophenotypic and genetic features of tumours in patients, JOURNAL OF PATHOLOGY, 2011; 225:212-21.

227. Sonpavde G., Choueiri T., Precision medicine for metastatic renal cell carcinoma, UROLOGIC ONCOLOGY 2014; 32:5-15 (DOI: 10.1016/j.urolonc.2013.07.010).

228. Hai C., Systems Biology of HBOC-Induced Vasoconstriction, CURRENT DRUG DISCOVERY TECHNOLOGIES 2012; 9:204-11.

229. Hillman G.G. et al., Progression of renal cell carcinoma is inhibited by genistein and radiation in an orthotopic model, BMC CANCER, 2007; 7:4.

230. Hodi F.S. et al., MPDL3280A (anti-PDLI): Clinical activity, safety and biomarkers of an engineered PD-L1 antibody in patients with locally advanced or metastatic tumors, EUROPEAN JOURNAL OF CANCER 2013; 49:S 184.

231. Hudes G. et al., Temsirolimus, Interferon Alfa, or Both for Advanced Renal-Cell Carcinoma, NEW ENGLAND JOURNAL OF MEDICINE 2007; 356:2271-81.

232. Hutson TE, Axitinib versus sorafenib as first-line therapy in patients with metastatic renal cell carcinoma (mRCC), JOURNAL OF CLINICAL ONCOLOGY 2013; 31(suppl 6): abstr LBA348.

233. Hylander B.L. et al., Origin of the vasculature supporting growth of primary patient tumor xenografts, JOURNAL OF TRANSLATIONAL MEDICINE 2013; 11:1-14.

234. Jason S.H. et al., Stroma-Free Human Hemoglobin A Decreases R3230Ac Rat Mammary Adenocarcinoma Blood Flow and Oxygen Partial Pressure, RADIATION RESEARCH 1997; 147:185-94.

235. Chen J.Y., Scerbo M., Kramer G., A Review of Blood Substitutes: Examining The History, Clinical Trial Results, and Ethics of Hemoglobin-Based Oxygen Carriers, CLINICS (SAO PAULO) 2009; 64:803-13.

236. Kaelin W.G. Jr., The von Hippel-Lindau tumor suppressor protein and clear cell renal carcinoma, CLIN CANCER RES 2007;13:680s-684s.

237. Kaelin W.G. Jr., The Von Hippel-Lindau Tumor Suppressor Gene and Kidney Cancer, CLIN CANCER RES 2004; 10:6290s-6295s.

238. Kersey F.R., Zhang G., Palmer G.M., Dewhirst M.W., Fraser C.L., Stereocomplexed Poly(lactic acid)-Polyethylene glycol) Nanoparticles with Dual-Emissive Boron Dyes for Tumor Accumulation, ACS NANO 2010; 4:4989-96.

239. Kobayashi M., Morita T., Chun N., Matsui A., Takahashi M., Murakami T., Effect of host immunity on metastatic potential in renal cell carcinoma: the assessment of optimal in vivo models to study metastatic behavior of renal cancer cells, TUMOR BIOLOGY 2012; 33:551-9.

240. Kondo K., Klco J., Nakamura E., Lechpammer M., Kaelin W.G. Jr., Inhibition of HIF is necessary for tumor suppression by the von Hippel-Lindau protein, CANCER CELL 2002; 1:237-46.

241. Kondo K. et al., Comprehensive mutational analysis of the VHL gene in sporadic renal cell carcinoma: relationship to clinicopathological parameters, GENES CHROMOSOMES CANCER 2002; 34:58-68.

242. Larkin J. et al., Efficacy of Sequential Treatment with Sunitinib-Everolimus in an Orthotopic Mouse Model of Renal Cell Carcinoma, ANTICANDER RESEARCH 2012; 32:2399-2406.

243. Lee J., Siemann D.W., Koch C.J., Lord E.M., Direct relationship between radiobiological hypoxia in tumors and monoclonal antibody detection of EF5 cellular adducts, INTERNATIONAL JOURNAL OF CANCER 1996; 67:372-8.

244. Lee Y.S. et al., Coexpression of erythropoietin and erythropoietin receptor in von Hippel-Lindau disease-associated renal cysts and renal cell carcinoma, CLIN CANCER RES 2005; 11:1059-1064.

245. Leiping F., Gang W., Shevchuk M.M., Nanus D.M., Gudas L.J., Generation of a Mouse Model of Von Hippel-Lindau Kidney Disease Leading to Renal Cancers by Expression of a Constitutively Active Mutant of HIFloc, CANCER RESEARCH 2011; 71:6848-6856.

246. Lin W. et al., Laboratory Studies: A novel murine model of human renal cell carcinoma spinal metastasis, JOURNAL OF CLINICAL NEUROSCIENCE 2012; 19:881-3.

247. Liu G., Chen C., Ji J., Biocompatible and biodegradable polymersomes as delivery vehicles in biomedical applications, SOFT MATTER 2012; 8:8811-21.

248. Lomas H. et al., Polymersome-Loaded Capsules for Controlled Release of DNA, SMALL 2011; 7:2109-19.

249. Ma W.W., Adjei A.A., Novel agents on the horizon for cancer therapy, CA CANCER J CLIN 2009; 59:111-37.

250. Malik A., Kundu J., Mukherjee S.K., Chowdhury P.K., Myoglobin Unfolding in Crowding and Confinement, JOURNAL OF PHYSICAL CHEMISTRY B 2012;116:12895-8904.

251. Marzilli L., Golden T., Cotter R., Woods A., Peptide sequence information derived by pronase digestion and ammonium sulfate in-source decay matrix-assisted laser desorption/ionization time-of-flight mass spectrometry, J AM SOC SPECTROM 2000;11:1000-1008.

252. Massignani M. et al., Controlling Cellular Uptake by Surface Chemistry, Size, and Surface Topology at the Nanoscale, SMALL 2009; 5:2424-32.

253. Massimo C., Chusilp C., Gabriel N.H., Angiogenesis modulation in cancer research: novel clinical approaches, NATURE REVIEWS DRUG DISCOVERY 2002; 1:415-426.

254. McGee M.C. et al., Improved Intratumoral Oxygenation Through Vascular Normalization Increases Glioma Sensitivity To Ionizing Radiation, INTERNATIONAL JOURNAL OF RADIATION ONCOLOGY BIOLOGY PHYSICS 2010; 76:1537-1545.

255. Melichar B. et al., First-line bevacizumab combined with reduced dose interferon-alpha2a is active in patients with metastatic renal cell carcinoma, ANN ONCOL 2008; 19:1470-76.

256. Meng F. H., Engbers G.H.M., Feijen J., Biodegradable Polymersomes As A Basis For Artificial Cells: Encapsulation, Release And Targeting, J. CONTROL RELEASE 2005; 101:187-198.

257. Meric-Bernstam F., Gonzalez-Angulo A.M., Targeting the mTOR signaling network for cancer therapy, J CLIN ONCOL 2009; 27:2278-287.

258. Minoru K. et al., Investigative Urology: Establishment and Characterization of Transplantable, Luminescence Labeled Rat Renal Cell Carcinoma Cell Lines, J UROL 2009; 183:2029-35.

259. Miralbell R. et al., Accelerated Radiotherapy, Carbogen, And Nicotinamide In Glioblastoma Multiforme: Report Of European Organization For Research And Treatment Of Cancer Trial 22933, J CLIN ONCOL 1999; 17:3143-49.

260. Moeller B., Dewhirst M.W., HIF-1 and tumour radiosensitivity, BRITISH JOURNAL OF CANCER 2006; 95:1-5.

261. Moeller B.J., Dewhirst M.W., Raising The Bar-How HIF-1 Helps Determine Tumor Radiosensitivity, CELL CYCLE 2004; 3:1107-10.

262. Moeller B.J. et al., Pleiotropic Effects Of HIF-1 Blockade On Tumor Radiosensity, CANCER CELL 2005; 8:99-110.

263. Moeller B.J., Richardson R.A., Dewhirst M.W., Hypoxia And Radiotherapy: Opportunities For Improved Outcomes In Cancer Treatment, CANCER AND METASTASIS REVIEWS 2007; 26:241-48.

264. Molina A.M. et al., Phase 1 trial of everolimus plus sunitinib in patients with metastatic renal cell carcinoma, CANCER 2012; 118:1868-76.

265. Molino D., Sepe J., Anastasio P., De Santo N.G., The history of von Hippel-Lindau disease, J NEPHROL 2006;19 Supp:S119-23.

266. Moore E.E. et al., Human Polymerized Hemoglobin for the Treatment of Hemorrhagic Shock when Blood Is Unavailable: The USA Multicenter Trial, JOURNAL OF THE AMERICAN COLLEGE OF SURGEONS 2009;208:1-13.

267. Motzer R.J. et al., A phase 3 comparative study of nivolumab (anti-PD-1; BMS-936558; ONO-4538) versus everolimus in patients with advanced or metastatic renal cell carcinoma (mRCC) previously treated with anti-angiogenic therapy, BJU INTERNATIONAL 2013; 112 Suppl:10.

268. Motzer R.J., Escudier B., Oudard S., et al., Efficacy of everolimus in advanced renal cell carcinoma: a double-blind, randomised, placebo-controlled phase III trial, LANCET 2008; 372:449-56.

269. Motzer R.J. et al., Phase I Trial of Sunitinib Malate plus Interferon-oc for Patients with Metastatic Renal Cell Carcinoma, CLINICAL GENITOURINARY CANCER 2009; 7:28-33.

270. Motzer R.J. et al., Overall survival and updated results for sunitinib compared with interferon alfa in patients with metastatic renal cell carcinoma, J CLIN ONCOL 2009; 27:3584-90.

271. Motzer R.J. et al., Overall survival and updated results for sunitinib compared with interferon alfa in patients with metastatic renal cell carcinoma, J CLIN ONCOL 2009; 27:3584-90.

272. Motzer R.J. et al., Dovitinib versus sorafenib for third-line targeted treatment of patients with metastatic renal cell carcinoma: an open-label, randomised phase 3 trial, LANCET ONCOLOGY 2014; 15:286-96.

273. Motzer R.J. et al., Tivozanib versus sorafenib as initial targeted therapy for patients with advanced renal cell carcinoma: Results from a phase III randomized, open-label, J CLIN ONCOL 2012; 30(suppl 15):277s, abstr 4501.

274. Mourant J.R. et al., Ligand-Binding To Heme-Proteins: II. Transitions In The Heme Pocket Of Myoglobin, BIOPHYSICAL JOURNAL 1993; 65:1496-507.

275. Mundt A..J., Connell P.P., Campbell T., Hwang J.H., Rotmensch J., Waggoner S., Race And Clinical Outcome In Patients With Carcinoma Of The Uterine Cervix Treated With Radiation Therapy, GYNECOLOGIC ONCOLOGY 1998; 71:151-8.

276. Musumeci F., Radi M., Brullo C., Schenone S., Vascular Endothelial Growth Factor (VEGF) Receptors: Drugs and New Inhibitors, J. MED. CHEM. 2012; 55:10797-822.

277. Nagasawa H., Uto Y., Kirk K.L., Hori H., Design Of Hypoxia-Targeting Drugs As New Cancer Chemotherapeutics, BIO PHARM BULL 2006; 29:2335-42.

278. Najafi F., Sarbolouki M.N., Biodegradable Micelles/Polymersomes From Fumaric/Sebacic Acids And Poly(Ethylene Glycol), BIOMATERIALS 2003; 24:1175-82.

279. Natanson C., Kern S.J., Lurie P., Banks S.M., Wolfe S.M., Cell-free hemoglobin-based blood substitutes and risk of myocardial infarction and death: A meta-analysis, JAMA 2008; 299:2304-12.

280. Nienhaus G.U., Mourant J.R., Chu K., Frauenfelder H., Ligand-Binding To Heme-Proteins - The Effect Of Light On Ligand-Binding In Myoglobin, BIOCHEMISTRY 1994; 33:13413-30.

281. O'Neil C.P., Suzuki T., Demurtas D., Finka A., Hubbell J.A., A Novel Method For The Encapsulation Of Biomolecules Into Polymersomes Via Direct Hydration, LANGMUIR 2009; 25:9025-9.

282. Ordway G.A., Garry D.J., Myoglobin: An Essential Hemoprotein In Striated Muscle, JOURNAL OF EXPERIMENTAL BIOLOGY 2004; 207:3441-6.

283. Overgaard J. et al., Misonidazole Combined With Radiotherapy In The Treatment Of Carcinoma Of The Uterine Cervix, IJROBP 1989; 16:1069-72.

284. Overgaard J. et al., Misonidazole Combined With Split-Course Radiotherapy In The Treatment Of Invasive-Carcinoma Of Larynx And Pharynx - Report From The Dahanca-2 Study, IJROBP 1989; 16:1065-8.

285. Overgaard J. et al., A Randomized Double-Blind Phase III Study OfNimorazole As A Hypoxic Radiosensitizer Of Primary Radiotherapy In Supraglottic Larynx And Pharynx Carcinoma, Results Of The Danish Head And Neck Cancer Study (DAHANCA) Protocol 5-85, RADIOTHERAPY AND ONCOLOGY 1998; 46:135-46.

286. Overgaard J., Horsman M.R., Modification Of Hypoxia-Induced Radioresistance In Tumors By The Use Of Oxygen And Sensitizers, SEMINARS IN RADIATION ONCOLOGY 1996; 6:10-21.

287. Overgaard J., Sensitization Of Hypoxic Tumor-Cells - Clinical-Experience, INTERNATIONAL JOURNAL OF RADIATION BIOLOGY 1989; 56:801-11.

288. Cabrales P., Examining and mitigating acellular hemoglobin vasoactivity, ANTIOXID REDOX SIGNAL 2013; 18:2329-41.

289. Palaparthy R., Wang H.S., Gulati A., Current Aspects In Pharmacology Of Modified Hemoglobins, ADV DRUG DELIV. REV 2000; 40:185-98.

290. Palmer A.F., Sun G., Harris D.R., Tangential flow filtration of hemoglobin, BIOTECHNOLOGY PROGRESS 2009; 25:189-99.

291. Palmer G.M., Boruta R.J., Viglianti B.L., Lan L., Spasojevic I., Dewhirst M.W., Non-Invasive Monitoring Of Intra-Tumor Drug Concentration And Therapeutic Response Using Optical Spectroscopy, J CONTROL RELEASE 2010; 142:457-64.

292. Palmer G.M., Viola R.J., Schroeder T., Yarmolenko P.S., Dewhirst M.W., Ramanujam N., Quantitative Diffuse Reflectance And Fluorescence Spectroscopy: Tool To Monitor Tumor Physiology In Vivo, JOURNAL OF BIOMEDICAL OPTICS 2009; 14:024010.

293. Palmer G.M. et al., In vivo optical molecular imaging and analysis in mice using dorsal window chamber models applied to hypoxia, vasculature and fluorescent reporters, NAT PROTOC 2011; 6:1355-66.

294. Palmer G.M., Fontanella A.N., Zhang G., Hanna G., Fraser C.L., Dewhirst M.W., Optical imaging of tumor hypoxia dynamics, J BIOMED OPT. 2010; 15:066021.

295. Pangu G.D., Davis K.P., Bates F.S., Hammer D.A., Ultrasonically Induced Release from Nanosized Polymer Vesicles, MACROMOL. BIOSCI. 2010; 10:546-54.

296. Papadopoulou M.V., Ji M., Rao M.K., Bloomer W.D., 4- 3-(2-Nitro-1-Imidazolyl)Propylamino -7-Chloroquinoline Hydrochloride (NLCQ-1), A Novel Bioreductive Compound As A Hypoxia-Selective Cytotoxin, ONCOLOGY RESEARCH 2000; 12:185-92.

297. Papadopoulou M.V., Ji M., Rao M.K., Bloomer W.D., 4-3-(2-Nitro-1-Imidazolyl)Propylamino -7-Chloroquinoline Hydrochloride (NLCQ-1), A Novel Bioreductive Agent As Radiosensitizer In Vitro And In Vivo: Comparison With Tirapazamine, ONCOLOGY RESEARCH 2000; 12:325-33.

298. Patel P.H., Senico P.L., Curiel R.E., Motzer R.J., Phase I Study Combining Treatment with Temsirolimus and Sunitinib Malate in Patients with Advanced Renal Cell Carcinoma, CLINICAL GENITOURINARY CANCER 2009;7:24-7.

299. Patton J.N., Palmer A.F., Physical Properties of Hemoglobin-Poly(acrylamide) Hydrogel-Based Oxygen Carriers: Effect of Reaction pH, LANGMUIR 2006; 22:2212-21.

300. Photos P.J., Bacakova L., Discher B., Bates F.S., Discher D.E., Polymer Vesicles In Vivo: Correlations With PEG Molecular Weight, J. CONTROL RELEASE 2003; 90:323-34.

301. P.L., Griko Y.V., Venyaminov S.Y., Kutyshenko V.P., Cold Denaturation Of Myoglobin, JOURNAL OF MOLECULAR BIOLOGY 1986; 190:487-98.

302. Qi W., Ghoroghchian P.P., Li G., Hammer D.A., Therien M.J., Aqueous self-assembly of polyethylene oxide)-block-poly([varepsilon]-caprolactone) (PEO-b-PCL) copolymers: disparate diblock copolymer compositions give rise to nano- and meso-scale bilayered vesicles, NANOSCALE 2013; 5:10908-15.

303. Rabotyagova O.S., Cebe P., Kaplan D.L., Self-Assembly of Genetically Engineered Spider Silk Block Copolymers, BIOMACROMOLECULES 2009; 10:229-36.

304. Rameez S., Banerjee U., Fontes J., Roth A., Palmer A.F., Reactivity of Polymersome Encapsulated Hemoglobin with Physiologically Important Gaseous Ligands: Oxygen, Carbon Monoxide, and Nitric Oxide, MACROMOLECULES 2012; 45:2385-9.

305. Rameez S., Alosta H., Palmer A.F., Biocompatible And Biodegradable Polymersome Encapsulated Hemoglobin: A Potential Oxygen Carrier, BIOCONJUGATE CHEM 2008; 19:1025-32.

306. Rameez S., Bamba I., Palmer A.F., Large Scale Production of Vesicles by Hollow Fiber Extrusion: A Novel Method for Generating Polymersome Encapsulated Hemoglobin Dispersions, LANGMUIR 2010; 26:5279-85.

307. Raval R.R. et al., Contrasting properties of hypoxia-inducible factor 1(H1F-1) and HIF-2 in von Hippel-Lindau-associated renal cell carcinoma, MOL CELL BIOL 2005; 25:5675-86.

308. Rini B. et al., AMG 386 in combination with sorafenib in patients with metastatic clear cell carcinoma of the kidney, CANCER 2012; 118:6152-61.

309. Rini B. et al., Bevacizumab plus interferon alfa compared with interferon alfa monotherapy in patients with metastatic renal cell carcinoma: CALGB 90206, J CLIN ONCOL 2008; 26:5422-8.

310. Rischin D. et al., Tirapazamine, Cisplatin, and Radiation Versus Cisplatin and Radiation for Advanced Squamous Cell Carcinoma of the Head and Neck (TROG 02.02, HeadSTART): A Phase III Trial of the Trans-Tasman Radiation Oncology Group, J CLIN ONCOL 2010; 28:2989-95.

311. Robbins G.P., Jimbo M., Swift J., Therien M.J., Hammer D.A., Dmochowski I.J., Photoinitiated Destruction of Composite Porphyrin-Protein Polymersomes, J AM CHEM SOC 2009; 131:3872-4.

312. Roberts K.B. et al., Interim Results Of A Randomized Trial Of Mitomycin C As An Adjunct To Radical Radiotherapy In The Treatment Of Locally Advanced Squamous-Cell Carcinoma Of The Cervix, INT. J. CANCER 2000; 90:206-23.

313. Robinson M.F., Dupuis N.P., Kusumoto T., Liu F., Menon K., Teicher B.A., Increased Tumor Oxygenation And Radiation Sensitivity In 2 Rat-Tumors By A Hemoglobin-Based, Oxygen-Carrying Preparation, ARTIF. CELLS BLOOD SUBSTIT. IMMOBIL. BIOTECHNOL. 1995; 23:431-8.

314. Rockwell S., Dobrucki I.T., Kim E.Y., Marrison S.T., Vu V.T., Hypoxia and Radiation Therapy: Past History, Ongoing Research, and Future Promise, CURRENT MOLECULAR MEDICINE 2009; 9:442-58.

315. Rockwell S., Oxygen Delivery: Implications For The Biology And Therapy Of Solid Tumors, ONCOLOGY RESEARCH 1997; 9:383-90.

316. Rofstad E.K., Sundfor K., Lyng H., Trope C.G., Hypoxia-Induced Treatment Failure In Advanced Squamous Cell Carcinoma Of The Uterine Cervix Is Primarily Due To Hypoxia Induced Radiation Resistance Rather Than Hypoxia-Induced Metastasis, BRITISH JOURNAL OF CANCER 2000; 83:354-9.

317. Rosenthal D.I., Nurenberg P., Becerra C.R., et al. A Phase I Single-Dose Trial Of Gadolinium Texaphyrin (Gd-Tex), A Tumor Selective Radiation Sensitizer Detectable By Magnetic Resonance Imaging, CLINICAL CANCER RESEARCH 1999; 5:739-45.

318. Rowinsky E.K., Novel Radiation Sensitizers Targeting Tissue Hypoxia, ONCOLOGY (WILLISTON PARK) 1999; 13:61-70.

319. Rosenthal D.I., Nurenberg P., Becerra C.R., et al. A Phase I Single-Dose Trial Of Gadolinium Texaphyrin (Gd-Tex), A Tumor Selective Radiation Sensitizer Detectable By Magnetic Resonance Imaging, CLINICAL CANCER RESEARCH 1999; 5:739-45.

320. Rudat V., Vanselow B., Wollensack P., et al. Repeatability And Prognostic Impact Of The Pretreatment Po(2) Histography In Patients With Advanced Head And Neck Cancer, RADIOTHERAPY AND ONCOLOGY 2000; 57:31-7.

321. Sabatini D.M., MTOR and cancer: insights into a complex relationship, NAT REV CANCER 2006; 6:729-34.

322. Sakai H., Hamada K., Takeoka S., Nishide H., Tsuchida E., Functional Evaluation Of Hemoglobin- And Lipidheme-Vesicles As Red Cell Substitutes, POLYMERS FOR ADVANCED TECHNOLOGIES 1996; 7:639-44.

323. Sakai H. et al., NO And CO Binding Profiles Of Hemoglobin Vesicles As Artificial Oxygen Carriers, BIOCHIMICA ET BIOPHYSICA ACTA-PROTEINS AND PROTEOMICS 2008; 1784:1441-7.

324. Salumbides B.C., Lehet K.M., Ndikuyeze G., Pili R., Pre-clinical models of renal carcinoma and their utility in drug development, CURR PROTOC PHARMACOL. Dec. 2009; Chapter 14:Unit 14.13.

325. Sanson C., Le Meins J.F., Schatz C., Soum A., Lecommandoux S., Temperature Responsive Poly(Trimethylene Carbonate)-Block-Poly(L-Glutamic Acid) Copolymer: Polymersomes Fusion And Fission, SOFT MATTER 2010; 6:1722-30.

326. Sartorelli A.C. et al., Mitomycin-C - A Prototype Bioreductive Agent, ONCOLOGY RESEARCH 1994; 6:501-8.

327. Saunders M., Dische S., Clinical Results Of Hypoxic Cell Radiosensitisation From Hyperbaric Oxygen To Accelerated Radiotherapy, Carbogen And Nicotinamide, BRITISH JOURNAL OF CANCER 1996; 74:S271-8.

328. Schatz C., Louguet S., Le Meins J.F., Lecommandoux S., Polysaccharide-Block-Polypeptide Copolymer Vesicles: Towards Synthetic Viral Capsids, ANGEW. CHEM. INT. EDIT 2009; 48:2572-5.

329. Schraml P. et al., VHL mutations and their correlation with tumour cell proliferation, microvessel density, and patient prognosis in clear cell renal cell carcinoma, J PATHOL 2002; 196:186-93.

330. Shasha D., George M.J., Harrison L.B., Once-Weekly Dosing Of Epoetin-Alfa Increases Hemoglobin And Improves Quality Of Life In Anemic Cancer Patients Receiving Radiation Therapy Either Concomitantly Or Sequentially With Chemotherapy, CANCER 2003; 98:1072-9.

331. Shasha D., The Negative Impact Of Anemia On Radiotherapy And Chemoradiation Outcomes, SEMINARS IN HEMATOLOGY 2001; 38:8-15.

332. Shibamoto Y., Zhou L., Hatta H., Mori M., Nishimoto S., In Vivo Evaluation Of A Novel Antitumor Prodrug, 1-(2'-Oxopropyl)-5-Fluorouracil (OFU001), Which Releases 5-Fluorouracil Upon Hypoxic Irradiation, IJROBP 2001; 49:407-13.

333. Shibuya M., Vascular endothelial growth factor-dependent and -independent regulation of angiogenesis, BMB REP 2008; 41:278-86.

334. Shum H.C., Kim J.W., Weitz D.A., Microfluidic Fabrication Of Monodisperse Biocompatible And Biodegradable Polymersomes With Controlled Permeability, J AM CHEM SOC 2008; 130:9543-9.

335. Siemann D.W., Hinchman C.A., Potentiation Of Cisplatin Activity By The Bioreductive Agent Tirapazamine, RADIOTHERAPY AND ONCOLOGY 1998; 47:215-20.

336. Sivanand S. et al., A Validated Tumorgraft Model Reveals Activity of Dovitinib Against Renal Cell Carcinoma, SCIENCE TRANSLATIONAL MEDICINE 2012; 4:137ra75.

337. Sivanesaratnam V., Jayalakshmi P., Mitomycin-C Adjuvant Chemotherapy After Wertheim Hysterectomy For Stage-Ib Cervical-Cancer, CANCER 1989; 64:798-800.

338. Smaldone M.C., Fung C., Uzzo R.G., Haas N.B., Adjuvant and Neoadjuvant Therapies in High-Risk Renal Cell Carcinoma, HEMATOLOGY/ONCOLOGY CLINICS OF NORTH AMERICA 2011; 25:765-91.

339. Sonpavde G., Willey C.D., Sudarshan S., Fibroblast growth factor receptors as therapeutic targets in clear-cell renal cell carcinoma, EXPERT OPINION ON INVESTIGATIONAL DRUGS 2014; 23:305-15.

340. Sonveaux P. et al., Oxygen Regulation of Tumor Perfusion by S-Nitrosohemoglobin Reveals a Pressor Activity of Nitric Oxide. Circulation Research 2005; 96:1119-26.

341. Sorg B.S., Hardee M.E., Agarwal N., Moeller B.J., Dewhirst M.W., Spectral Imaging Facilitates Visualization And Measurements Of Unstable And Abnormal Microvascular Oxygen Transport In Tumors, J. BIOMED. OPT. 2008; 13:014026.

342. Sorg B.S., Moeller B.J., Donovan O., Cao Y.T., Dewhirst M.W., Hyperspectral Imaging Of Hemoglobin Saturation In Tumor Microvasculature And Tumor Hypoxia Development, JOURNAL OF BIOMEDICAL OPTICS 2005; 10:044004.

343. Stadler P. et al., Influence Of The Hypoxic Subvolume On The Survival Of Patients With Head And Neck Cancer, IJROBP 1999; 44:749-54.

344. Stadler P., Feldmann H.J., Creighton C., Kau R., Molls M., Changes In Tumor Oxygenation During Combined Treatment With Split-Course Radiotherapy And Chemotherapy In Patients With Head And Neck Cancer, RADIOTHERAPY AND ONCOLOGY 1998; 48:157-64.

345. Strube A., Stepina E., Mumberg D., Scholz A., Hauff P., Kakonen S.M., Characterization of a new renal cell carcinoma bone metastasis mouse model, CLIN EXP METASTASIS 2010;27:319-30.

346. Stuben G., Stuschke M., Knuhmann K., Horsman M.R., Sack H., The Effect Of Combined Nicotinamide And Carbogen Treatments In Human Tumour Xenografts: Oxygenation And Tumour Control Studies, RADIOTHERAPY AND ONCOLOGY 1998; 48:143-8.

347. Takeshi K. et al., Definitive Radiotherapy Combined With High-Dose-Rate Brachytherapy For Stage III Carcinoma Of The Uterine Cervix: Retrospective Analysis Of Prognostic Factors Concerning Patient Characteristics And Treatment Parameters, IJROBP 1998; 41:319-27.

348. Tang P.A., Heng D.Y.C., Programmed death 1 pathway inhibition in metastatic renal cell cancer and prostate cancer, CURRENT ONCOLOGY REPORTS 2013; 15:98-104.

349. Teicher B.A., Herman T.S., Hopkins R.E., Menon K., Effect Of A Bovine Hemoglobin Preparation On The Response Of The Fsaiic Fibrosarcoma To Chemotherapeutic Alkylating-Agents, J. CANCER RES CLIN ONCOL 1992; 118:123-8.

350. Teicher B.A. et al., Oxygenation Of The Rat-9l Gliosarcoma And The Rat-13672 Mammary-Carcinoma With Various Doses Of A Hemoglobin Solution, ARTIF. CELLS BLOOD SUBSTIT. IMMOBIL. BIOTECHNOL. 1994; 22:827-33.

351. Teicher B.A., Holden S.A., Menon K., Hopkins R.E., Gawryl M.S., Effect Of Hemoglobin Solution On The Response Of Intracranial And Subcutaneous Tumors To Antitumor Alkylating-Agents, CANCER CHEMOTHER. PHARMACOL. 1993; 33:57-62.

352. Teicher B.A., Physiological-Mechanisms of Therapeutic Resistance - Blood-Flow and Hypoxia, HEMATOLOGY-ONCOLOGY CLINICS OF NORTH AMERICA 1995; 9:475-506.

353. Teicher B.A., Schwartz G.N., Sotomayor E.A., Robinson M.F., Dupuis N.P., Menon K., Oxygenation Of Tumors By A Hemoglobin Solution, J. CANCER RES CLIN ONCOL 1993; 120:85-90.

354. Treat J. et al., Tirapazamine With Cisplatin In Patients With Advanced Non-Small-Cell Lung Cancer: A Phase II Study, J CLIN ONCOL 1998; 16:3524-7.

355. Van Dongen S.F.M., Nallani M., Schoffelen S., Cornelissen J., Nolte R.J.M., Van Hest J.C.M., A Block Copolymer For Functionalisation Of Polymersome Surfaces, MACROMOLECULAR RAPID COMMUNICATIONS 2008; 29:321-5.

356. Von Pawel J. et al., Tirapazamine Plus Cisplatin Versus Cisplatin In Advanced Non-Small-Cell Lung Cancer: A Report Of The International CATAPULT I Study Group, Cisplatin and Tirapazamine in Subjects with Advanced Previously Untreated Non-Small-Cell Lung Tumors, J CLIN ONCOL 2000; 18:1351-9.

357. Walsh L. et al., Efficacy of ablative high-dose-per-fraction radiation for implanted human renal cell cancer in a nude mouse model, EUROPEAN UROLOGY 2006; 50:795-800.

358. Walter S. et al., Multipeptide immune response to cancer vaccine IMA901 after single-dose cyclophosphamide associates with longer patient survival, NATURE MEDICINE 2012; 18:1254-61.

359. Wang F., Wang Y.C., Yan L.F., Wang J., Biodegradable Vesicular Nanocarriers Based On Poly(Epsilon-Caprolactone)-Block-Poly(Ethyl Ethylene Phosphate) For Drug Delivery, POLYMER 2009; 50:5048-54.

360. Wang L. et al., Encapsulation of Biomacromolecules within Polymersomes by Electroporation, ANGEW CHEM INT ED ENGL.2012; 51:11122-5.

361. Wasserman T.H. et al., Clinical-Trials With Etanidazole (Sr-2508) By The Radiation-Therapy Oncology Group (RTOG), RADIOTHERAPY AND ONCOLOGY 1991; 20:129-35.

362. Weitman S. et al., Evidence Of Enhanced In Vivo Activity Using Tirapazamine With Paclitaxel And Paraplatin Regimens Against The MV-522 Human Lung Cancer Xenograft, CANCER CHEMOTHER PHARMACOL 1999; 43:402-8.

363. Williams K.J. et al., In Vivo Activation Of The Hypoxia-Targeted Cytotoxin AQ4N In Human Tumor Xenografts, MOLECULAR CANCER THERAPEUTICS 2009; 8:3266-75.

364. Wilson D., Evans S., Jenkins W., Vinogradov S., Ong E., Dewhirst M., Oxygen Distributions within R3230AC Tumors Growing in Dorsal Flap Window Chambers in Rats, ADV EXP MED BIOL. 1998; 454:603-9.

365. Wittenberg J.B., Wittenberg B.A., Myoglobin Function Reassessed, JOURNAL OF EXPERIMENTAL BIOLOGY 2003; 206:2011-20.

366. Wood C.G., Figlin R.A., ADAPT: An Ongoing international phase 3 randomized trial of autologous dendritic cell Immunotherapy (AGS 003) plus standard treatment in advanced renal cell carcinoma (RCC), BJU INTERNATIONAL 2013; 112:11-2.

367. Wouters B.G. et al., Hypoxia As A Target For Combined Modality Treatments, EUROPEAN JOURNAL OF CANCER 2002; 38:240-57.

368. Yildiz M.E., Prud'homme R.K., Robb I., Adamson D.H., Formation And Characterization Of Polymersomes Made By A Solvent Injection Method, POLYMERS FOR ADVANCED TECHNOLOGIES 2007; 18:427-32.

369. Yu M.H., Dai M., Liu Q., Xiu R.J., Oxygen Carriers And Cancer Chemo- And Radiotherapy Sensitization: Bench To Bedside And Back, CANCER TREATMENT REVIEWS 2007; 33:757-61.

370. Yuen J.S.P. et al., Inhibition of angiogenic and non-angiogenic targets by sorafenib in renal cell carcinoma (RCC) in a RCC xenograft model, BRITISH JOURNAL OF CANCER 2011; 104:941-7.

371. Zhan H.W., Liu H.B., Bao C.K., Ye X.J., Zhang H., He G.Q., Effect Of Carbogen On Tumour Oxygenation And 32P-Colloid Interstitial Irradiation Response, MED SCI MONIT 2010; 16:BR11-6.

372. Zhang G.Q., Palmer G.M., Dewhirst M., Fraser C.L., A Dual-Emissive-Materials Design Concept Enables Tumour Hypoxia Imaging, NATURE MATERIALS 2009; 8:747-51.

373. Zhang X.L. et al., Key Parameters Affecting The Initial Leaky Effect Of Hemoglobin-Loaded Nanoparticles As Blood Substitutes, J MATER SCI METER MED. 2008; 19:2463-70.

374. Zupancich J.A., Bates F.S., Hillmyer M.A., Aqueous Dispersions Of Poly(Ethylene Oxide)-B-Poly(Gamma-Methyl-Epsilon-Caprolactone) Block Copolymers, MACROMOLECULES 2006; 39:4286-8.

375. IARC CancerBase No. 10. Lyon, France: International Agency for Research on Cancer; 2010.

376. Lam, John S.; Leppert, John T.; Belldegrun, Arie S., Figlin, Robert A., Novel approaches in the therapy of metastatic renal cell carcinoma, WORLD JOURNAL OF UROLOGY 20015; 23:202-12.

377. Patil S., Manola J., Elson P., Negrier S., Escudier B., Eisen T., Atkins M., Bukowski R., Motzer R.J., Improvement in Overall Survival of Patients with Advanced Renal Cell Carcinoma: Prognostic Factor Trend Analysis from an International Data Set of Clinical Trials, JUROL. 2012; 188: 2095-100.

378. Shih Y.C., Chien C.R., Xu Y., Pan I.W., Smith G.L., Buchholz T.A., Economic burden of renal cell carcinoma in the US: Part II--an updated analysis, PHARMACOECONOMICS 2011; 29:331-41.

379. Helfand C., Top twenty orphan drugs by 2018, FREEPHARMA (July 23, 2013), http://www.fiercepharma.com/special-reports/top-20-orphan-drugs-2018.

380. Global Industry Analysts, Inc., Kidney Cancer Drugs: A Global Strategic Business Report (2011), available at http://www.strategyr.com/Kidney_Cancer_Drugs_Market_ Report.asp.

381. Vítecek J., Lojek A., Valacchi G., Kubala L., Arginine-Based Inhibitors of Nitric Oxide Synthase: Therapeutic Potential and Challenges, MEDIATORS OF INFLAMMATION 2012; Article ID 318087.

## SUMMARY OF THE INVENTION

The invention is defined according to the appended claims. Various aspects of the disclosure include methods of causing nanoparticle-mediated microvascular embolization (NME) in a tumor that includes delivering a nitric oxide (NO)-affecting agent to a tumor. In some aspects, delivering the NO-affecting agent to the tumor includes introducing the NO-affecting agent into systemic circulation, in which the NO-affecting agent does not affect normal activity of NO in systemic circulation and accumulation of the NO-affecting agent within the tumor is based at least in part on enhanced retention and permeability of the tumor microvasculature.

In some aspects, the NO-affecting agent is NO-binding molecules encapsulated within carrier particles, and selectively preventing normal activity of NO includes selectively scavenging NO in the tumor microvasculature. In some aspects, the carrier particles are selected from the group consisting of nanoparticles and microparticles, and wherein the carrier particles include at least one of phospholipids, synthetic polymers, polypeptides, and polynucleic acids. In some aspects, the nanoparticles are polymersomes.

In some aspects, the NO-binding molecules competitively bind oxygen ($O_2$) and NO, in which introducing the NO-affecting agent into systemic circulation includes introducing oxygenated NO-binding molecules into systemic circulation, in which the NO-binding molecules become deoxygenated upon accumulation of the carrier particles in the tumor, thereby enabling the selective scavenging of NO in the tumor microvasculature.

In some aspects, the accumulation of the NO-affecting agent in the tumor allows diffusion of NO into the carrier particles, in which the selective scavenging of NO is performed at least in part by deoxygenation of the encapsulated NO -binding molecules. In some aspects, the NO-affecting agent further includes surface-associated NO-binding molecules, where the selective scavenging of NO is performed at least in part by deoxygenation of the surface-associated NO-binding molecule. In some aspects, the NO-binding molecules only release oxygen at tensions less than 10 mmHg.

In some aspects, NO-binding molecules are selected from one or more of unmodified human myoglobin, unmodified myoglobin from another biological species, and chemically or genetically modified myoglobin from humans or from another biological species. In some aspects, the selective prevention of normal NO activity in the tumor vasculature causes vasoconstriction and platelet aggregation in the tumor vasculature. In some aspects the persistent hydrodynamic pressure in the tumor vasculature causes rupture of the platelet aggregation and bleeding into the tumor. In some aspects, the bleeding into the tumor causes thrombosis of tumor vasculature and necrosis of tumor tissue.

In some aspects, surface-associated NO-binding molecules include surface-bound myoglobin. In some aspects, delivering the NO-affecting agent to the tumor includes administering the NO-affecting agent in conjunction with at least one of a vascular endothelial growth factor receptor (VEGFR) tyrosine kinase inhibitor (TKI), a mammalian target of rapamycin (mTOR) inhibitor, and radiotherapy. In some aspects, the NO-affecting agent further includes at least one of a chemotherapy agent and an angiogensis inhibiting agent co-encapsulated with the NO-binding molecules within the carrier particles. In some aspects, the NO-affecting agent includes at least one of a NO synthase (NOS) inhibitor and an antioxidant.

Compositions in further aspects include a nitric oxide (NO)-inhibiting agent, and a carrier vehicle, in which the NO-inhibiting agent is chemically or non-covalently incorporated with the carrier vehicle such that the NO activity is not affected when the carrier vehicle is in systemic circulation, and NO activity is inhibited following extravasation of the carrier vehicle from circulation into a tumor. In some aspects, the inhibition of NO activity involves binding of NO, in which the NO binding is enabled only at oxygen tensions of less than 5 mmHg.

In some aspects, the carrier vehicle is a synthetic polymer vesicle, in which the NO-inhibiting agent is within an aqueous core of the polymer vesicle. In other aspects, the carrier vehicle comprises a synthetic polymer vesicle, and the NO-inhibiting agent is within a membranous portion of the polymer vesicle. In some aspects, the carrier vehicle is a synthetic polymer vesicle, and the NO-inhibiting agent is attached to the outside surface of the polymer vesicle. In other aspects, the carrier vehicle is a uni- or multi-lamellar polymersome.

In some embodiment compositions, the carrier vehicle includes a plurality of biodegradable polymers. In other aspects, the plurality of biodegradable polymers form a nanoparticle. In some aspects, the nanoparticle is less than 200 nanometers in diameter, and in other aspects the nanoparticle is less than 100 nanometers in diameter.

In some aspects, the carrier vehicle co-encapsulates the NO-inhibiting agent with at least one other radiation-sensitizing or chemotherapeutic agent. In other aspects, the carrier vehicle is selected from at least one of a micelle, a solid nanoparticle, a polymersome, and a lipsome . In further aspects, the carrier vehicle is a nanoparticle, and the composition further includes a plurality of the nanoparticles configured to accumulate at sites of interest via passive diffusion or via a targeting modality comprised of a conjugation of a targeting molecule separate from the nanoparticles. In some aspects, the at least some of the plurality of nanoparticles are biodegradable polymer vesicles and at least some of the plurality of polymer vesicles are biocompatible polymer vesicles. In some aspects, the biocompatible polymer vesicles include poly(ethylene oxide) or poly(ethylene glycol). In other aspects, the biodegradable polymer vesicles include poly($\varepsilon$-caprolactone). In other embodiment compositions, the biodegradable polymer vesicles include poly($\gamma$-methyl $\varepsilon$-caprolactone). In other aspects, the biodegradable polymer vesicles include poly(trimethylcarbonate).

In some aspects, the biodegradable polymer vesicles include at least one block copolymer of poly(ethylene oxide) and poly($\varepsilon$-caprolactone). In other aspects, the biodegradable polymer vesicles include at least one block copolymer of poly(ethylene oxide) and poly($\gamma$-methyl $\varepsilon$-caprolactone). In other aspects, the biodegradable polymer vesicles include at least one block copolymer of poly(ethylene oxide) and poly(trimethylcarbonate). In other aspects, the biodegradable polymer vesicles are either pure or blends of multiblock copolymer, in which the copolymer includes at least one of poly(ethylene oxide) (PEO), poly(lactide) (PLA), poly(glycolide) (PLGA), poly(lactic-co-glycolic acid) (PLGA), poly($\varepsilon$-caprolactone) (PCL), and poly (trimethylene carbonate) (PTMC), poly(lactic acid), poly(methyl $\varepsilon$-caprolactone).

BRIEF DESCRIPTION OF THE DRAWINGS

The accompanying drawings illustrate exemplary aspects of the invention, and together with the general description given above and the detailed description given below, serve to explain the features of the invention.

FIG. 1 is a graph illustrating the oxygen dissociation curve of hemoglobin.

FIG. 2 is a graph illustrating the oxygen dissociation curves of hemoglobin and example agents that may be used to manipulate oxygen levels in tissues in accordance with various embodiments.

FIG. 3 is a graph illustrating the oxygen dissociation curves of hemoglobin and myoglobin.

FIG. 4A is an illustration of biodegradable polymers that may be a component of a biodegradable cellular oxygen carrier in various embodiments.

FIG. 4B is an illustration of water-soluble near-infrared fluorophores "◊" and water-soluble oxygen-binding proteins " ○" that may be used as components of a biodegradable cellular oxygen carrier in various embodiments.

FIG.4C is an illustration of the synthesis of nanoscale polymersome-encapsulated myoglobin (PEM) and processing procedures (heat, sonication, and extrusion) used to yield nanoscale PEM in accordance with various embodiments.

FIG. 4D is an illustration of an encapsulation schematic of an embodiment polymersome.

FIG. 4E is a cryogenic transmission electron micrograph and a confocal micrograph of PEM.

FIG. 5 is a set of photographs illustrating the (A) bright field, (B) oxygen tension in % oxygen, and (C) functional blood vasculature for a window chamber tumor.

FIG. 6A is a cryogenic transmission electron micrograph of PEO(2K)-*b*-PCL(12K)-based polymersomes in de-ionized water (5 mg/ml) that illustrates the membrane core thickness of the vesicles as being 22.5 $\pm$ 2.3 nanometer.

FIG. 6B a graph illustrating the cumulative *in situ* release of doxorubicin, loaded within 200 nm diameter PEO(2K)-b-PCL(12K) based polymersomes, under various physiological conditions (pH 5.5 and 7.4; T= 37 °C) as measured fluorometrically over 14 days.

FIG. 7A is an *in vivo* optical image of encapsulated oligo(porphyrin)-based near-infrared (NIR) fluorophores (NIRFs) that illustrates the accumulation of an embodiment carrier in tumors.

FIG. 7B is a graph of *in vivo* tumor growth as inhibited by phosphate buffered saline (PBS), doxorubicin (DOX), liposome-encapsualted DOX, and polymersome-encapsualted DOX.

FIG. 8A is a bar chart illustrating the hemoglobin (Hb) encapsulation efficiencies of four polymersome-encapsulated bovine and human Hb formulations after extrusion through 200 nm diameter polycarbonate membranes.

FIG. 8B is a bar chart illustrating the $P_{50}$ (mmHg) of red blood cells, hemoglobin and four polymersome-encapsulated hemoglobin formulations after extrusion through 200 nm polycarbonate membranes.

FIG. 9 is a process flow diagram illustrating an embodiment method for the preparation and delivery of a myoglobin-based oxygen carrier (MBOC).

FIG. 10 is a process flow diagram illustrating an embodiment method for preparing a polymersome including at least one biocompatible polymer and at least one biodegradable polymer.

FIG. 11 is a schematic illustration of optimized steps for the generation of PEM.

FIG. 12 is a set of bar graphs quantifying myoglobin concentrations and weight percentages of myoglobin-to-polymer in PEM suspensions formed by the method of FIG. 11.

FIG. 13A is graph illustrating oxygen saturation of PEM having myoglobin associated both on the surface and in the aqueous cavaties of the polymersomes (i.e. PEM-SE), PEM having myoglobin associated only in the aqueous cavaties of the polymersomes (i.e. PEM-E), and free myoglobin as a function of partial pressure of oxygen.

FIG. 13B is a graph illustrating the kinetic time course for the dissociation of oxygen from PEM, where all unencapsulated and surface-associated myoglobin had been removed by proteolysis and in which the remaining myoglobin had been reduced to oxymyoglobin in the presence of 1.5 mg/mL of $Na_2S_2O_4$.

FIG. 13C is a graph illustrating the kinetic time course for NO-mediated deoxygenation of PEM, where all unencapsulated and surface-associated myoglobin had been removed by proteolysis and in which the remaining myoglobin had been reduced to oxymyoglobin.

FIG. 14 is a table showing kinetic rate constants for oxygen dissociation ($K_{off, O_2}$) and NO-mediated deoxygenation ($K_{ox, NO}$) for various PEM formulations in comparison to free (unencapsulated) myoglobin (Mb).

FIG. 15A is a set of *in vivo* optical images of the biodistribution of NIR-myoglobin, empty polymersomes, and PEM at four time points following intravenous injection.

FIG. 15B is a graph illustrating tumor accumulations of NIR-myoglobin, empty polymersomes, and PEM as functions of time.

FIG. 15C is a graph illustrating plasma myoglobin concentrations of NIR-myoglobin, and PEM as functions of time.

FIG. 16 is a set of images showing PEM treatment of syngeneic orthotopic 4T1 mammary tumors.

FIG. 17 is a graph illustrating total intratumoral hemoglobin concentration as a function of time for the PEM-treated tumors shown in FIG. 16, as well as for tumors treated with NIR-myoglobin and empty polymersomes.

FIG. 18A is a set of brightfield images of tumor and surrounding normal tissues over time following treatment with PEM.

FIG. 18B is a set of images of the tumor FIG. 18A at 24 h after administration of PEM.

FIG. 19A is a set of images showing hemoglobin saturation (%) in tumors before and at around 4 hours post treatment with PEM and empty polymersomes.

FIG. 19B is a set of images showing flow velocity ($\mu$m/s) in tumors before and at around 4 hours post treatment with PEM and empty polymersomes.

FIG. 20 is a set of representative fluorescent images and hematoxylin and eosin images of excised tumors for NIR-myoglobin control and PEM-treated animal.

FIG. 21 is a table illustrating the results of a serum chemistry panel for PEM-treated animals.

FIGs. 22A and 22B are sets of hematoxylin and eosin images of excised livers from tumor-mice treated with PEM and empty polymersomes.

FIG. 22C is a set of images showing allograft RENCA tumors in mice treated with PEM.

FIG. 22D is a set of images showing allograft RENCA tumors in mice treated with empty polymersomes.

FIGs. 22E and 22F are sets of representative fluorescent images and hematoxylin and eosin images of excised tumors from mice that were treated with PEM and with empty polymersomes, respectively.

FIG. 23A is a table illustrating study parameters for determining safe dosing levels and schedules for PEM.

FIG. 23B is a table illustrating study parameters for determining minimum effective doses of PEM to induce tumor-specific nanoparticle-mediated microvascular embolization (NME) as visualized in window chambers.

FIG. 24A is a table illustrating study parameters for determining an optimal PEM dosing schedule and therapeutic combination with a VEGF receptor TKI for inhibiting tumor growth as a first-line therapy for RCC.

FIG. 24B is a table illustrating study parameters for determining an optimal PEM dosing schedule and therapeutic combination with an mTOR inhibitor for inhibiting tumor growth as a second-line therapy for RCC.

FIG. 25 is a table illustrating study parameters for determining an optimal PEM dosing schedule with radiation therapy for inhibiting tumor growth as palliative therapy for RCC.

## DETAILED DESCRIPTION OF THE INVENTION

The present invention may be understood more readily by reference to the following detailed description taken in connection with the accompanying figures and examples, which form a part of this disclosure.

References to values stated in ranges includes each and every value within that range.

The word "about" is used herein when referring to a measurable value such as an amount, a temporal duration, and the like, is meant to encompass variations of $\pm20\%$, $\pm10\%$, $\pm5\%$, $\pm1\%$, or $\pm0.1\%$ from the specified value, as such variations are appropriate to perform the disclosed methods.

The word "exemplary" is used herein to mean "serving as an example, instance, or illustration." Any implementation described herein as "exemplary" is not necessarily to be construed as preferred or advantageous over other implementations.

The word "plurality" is used herein to mean more than one. When a range of values is expressed, another embodiment includes from the one particular value and/or to the other particular value. Similarly, when values are expressed as approximations, by use of the antecedent "about," it will be understood that the particular value forms another embodiment. All ranges are inclusive.

The terms "subject" and "patient" are used interchangeably herein to refer to human patients, whereas the term "subject" may also refer to any animal. It should be understood that in various embodiments, the subject may be a mammal, a non-human animal, a canine and/or a vertebrate.

The term "monomeric units" is used herein to mean a unit of polymer molecule containing the same or similar number of atoms as one of the monomers. Monomeric units, as used in this specification, may be of a single type (homogeneous) or a variety of types (heterogeneous).

The term "polymers" is used according to its ordinary meaning of macromolecules comprising connected monomeric molecules.

The term "amphiphilic substance" is used herein to mean a substance containing both polar (water-soluble) and hydrophobic (water-insoluble) groups.

The term *"in vivo* delivery" is used herein to refer to delivery of a biologic by routes of administration such as topical, transdermal, suppository (rectal, vaginal), pessary (vaginal), intravenous, oral, subcutaneous, intraperitoneal, intrathecal, intramuscular, intracranial, inhalational, oral, and the like.

The term "an effective amount" is used herein to refer to an amount of a compound, material, or composition effective to achieve a particular biological result such as, but not limited to, biological results disclosed, described, or exemplified herein. Such results may include, but are not limited to, the effective reduction of symptoms associated with any of the disease states mentioned herein, as determined by any means suitable in the art.

The term "membrane" is used herein to mean a spatially distinct collection of molecules that defines a two-dimensional surface in three-dimensional space, and thus separates one space from another in at least a local sense.

The term "pharmaceutically active agent" is used herein to refer to any a protein, peptide, sugar, saccharide, nucleoside, inorganic compound, lipid, nucleic acid, small synthetic chemical compound, or organic compound that appreciably alters or affects the biological system to which it is introduced.

The term "drug delivery" is used herein to refer to a method or process of administering a pharmaceutical compound to achieve a therapeutic effect in humans or animals.

The term, "vehicle" is used herein to refer to agents with no inherent therapeutic benefit but when combined with an pharmaceutically active agent for the purposes of drug delivery result in modification of the pharmaceutical active agent's properties, including but not limited to its mechanism or mode of in vivo delivery, its concentration, bioavailability, absorption, distribution and elimination for the benefit of improving product efficacy and safety, as well as patient convenience and compliance.

The term "carrier" is used herein to describe a delivery vehicle that is used to incorporate a pharmaceutically active agent for the purposes of drug delivery.

The term "oxygen-binding agent" or "oxygen-binding compound" is used herein to refer to any molecule or macromolecule that binds, stores, and releases oxygen.

The term "allosteric effector" is used herein to refer to a molecule that modulates the rate or amount of oxygen binding to or releasing from of an oxygen carrier.

The term "high-oxygen affinity" agent or "high oxygen affinity compound" is used herein to refer to any molecule or macromolecule that binds and stores oxygen but only releases it at partial pressures of oxygen that are lower than the levels at which natural human hemoglobin normally releases oxygen. High-oxygen affinity agents include oxygen-binding compounds. High-oxygen affinity agents may include oxygen-binding compounds with a P50 for oxygen then is less than that of human adult or fetal hemoglobins with or without their interactions with natural allosteric modulators, carbon monoxide or strong reducing or oxidizing agents.

The term "oxygen-binding carrier" or "oxygen carrier" is used herein to refer to a carrier comprised of a synthetic or partially synthetic vehicle that incorporates a single or plurality of oxygen-binding agents.

The term "homopolymer" is used herein to refer to a polymer derived from one monomeric species of polymer.

The term "copolymer" is used herein to refer to a polymer derived from two (or more) monomeric species of polymer, as opposed to a homopolymer where only one monomer is used. Since a copolymer consists of at least two types of constituent units (also structural units), copolymers may be classified based on how these units are arranged along the chain.

The term "block copolymers" is used herein to refer to a copolymer that includes two or more homopolymer subunits linked by covalent bonds in which the union of the homopolymer subunits may require an intermediate non-repeating subunit, known as a junction block. Block copolymers with two or three distinct blocks are referred to herein as "diblock copolymers" and "triblock copolymers," respectively.

The term "areal strain" is used herein to refer to the change in the surface area of a particle under an external force or tension divided by the original surface area of the particle prior to application of said external force or tension (denoted by "A" and expressed as %).

The term "critical lysis tension" or "Tc" is used herein to refer to the tension at which a particle ruptures when subject to an external force as measured by micropipette aspiration and expressed as milliNewtons/meter (mN/m).

The term "critical areal strain" or "Ac" is used herein to refer to the areal strain realized by the oxygen carrier or polymersome at the critical lysis tension.

The term "loading ratio" is used herein to refer to a measurement of a oxygen biding carrier and may be defined as the weight of oxygen binding agent within the oxygen carrier divided by the dry weight of the inert vehicle.

The term "myoglobin loading capacity" is used herein to refer to a measurement of a myoglobin-based oxygen carrier and may be defined as the weight of myoglobin within the oxygen carrier divided by the total weight of carrier.. The term "myoglobin loading efficiency" is used herein to refer to a measurement of a myoglobin-based oxygen carrier and may be defined as the weight of myoglobin that is encapsulated and/or incorporated within a carrier suspension divided by the weight of the original myoglobin in solution prior to encapsulation (expressed as a %).

The term a "unit dose" is used herein to refer to a discrete amount of the pharmaceutical composition comprising a predetermined amount of the active ingredient.

It should be understood that P50 is the partial pressure of oxygen (pO2) at which the oxygen-binding compound becomes 50% saturated with oxygen. As the P50 decreases, oxygen affinity increases, and visa verse. Normal adult Hemoglobin A has a P50 of 26.5 mm Hg while Fetal Hemoglobin F has a P50 of 20 mm Hg and sickle cell anemia Hemoglobin S has a P50 of 34 mm Hg.

The various embodiments provide a nanoparticle-based therapeutic carrier to deliver high-oxygen affinity agents (e.g., molecules and proteins such as myoglobin) to tumors in order to increase intratumoral pO2, to stunt their aggressive molecular phenotypes, and to increase the efficacy of radiation and chemo-therapies directed against the tumor.

Generally, radiation treatment may be augmented by increasing the oxygen levels of tumors, in order to generate more oxygen-based free radicals with concomitant radiation therapy, or by delivering another non-$O_2$ dependent radiation sensitizer to tumor-specific sites. Conventional methods for manipulating the oxygen levels of tumors are reliant upon increasing the systemic level of oxygen in order to eventually deliver this increased oxygen capacity to the tumor. One such method that delivers artificial blood substitutes using natural hemoglobin (Hb) is disclosed in U.S. Patent Application No. 13/090,076 entitled "Biodegradable Nanoparticles as Novel Hemoglobin-Based Oxygen Carriers and Methods of Using the Same" filed on April 19,2011.

Hemoglobin is an oxygen-transporting protein in human red blood cells. Hemoglobin's structure makes it efficient at binding to oxygen, and efficient at unloading the bound oxygen in human tissues/blood stream. Hemoglobin consists of two pairs of globin dimers held together by non-covalent bonds to form a larger four subunit (tetrameric) hemoglobin molecule. The oxygen binding capacity of tetrameric hemoglobin depends on the presence of a non-protein unit called the heme group (i.e., one molecule of hemoglobin can bind with four oxygen molecules).

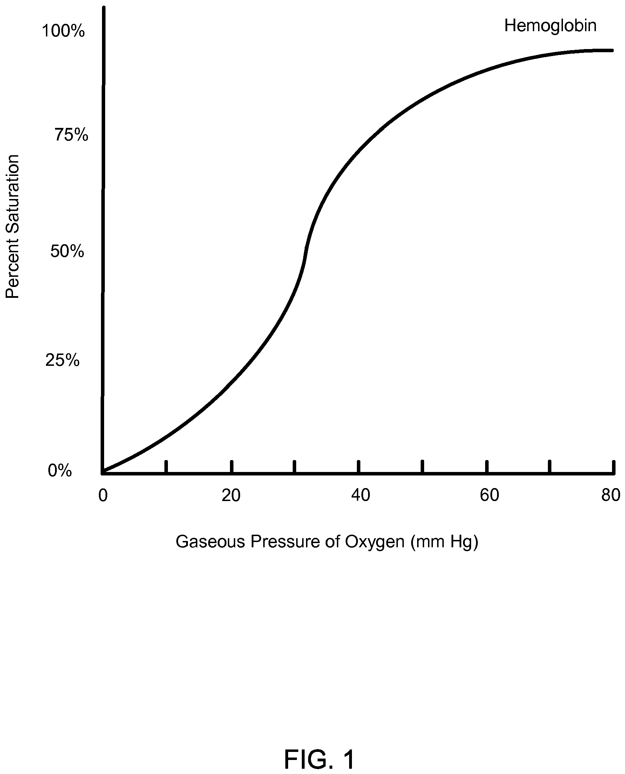

FIG. 1 illustrates the oxygen dissociation curve of hemoglobin. Cooperative binding of oxygen to hemoglobin gives native hemoglobin a sigmoidal-shaped oxygen dissociation curve and allows oxygen to be bound and released within a narrow physiological range of pO$_2$s (from 40-100 mmHg). Conventional methods for manipulating the oxygen levels of tumors have attempted to use hemoglobin or agents (proteins, molecules, etc.) having a similar oxygen dissociation curve as native hemoglobin. Others have attempted to use agents having oxygen dissociation curves that are shifted to the right of the hemoglobin oxygen dissociation curve (i.e., agents having a lower affinity for oxygen than hemoglobin).

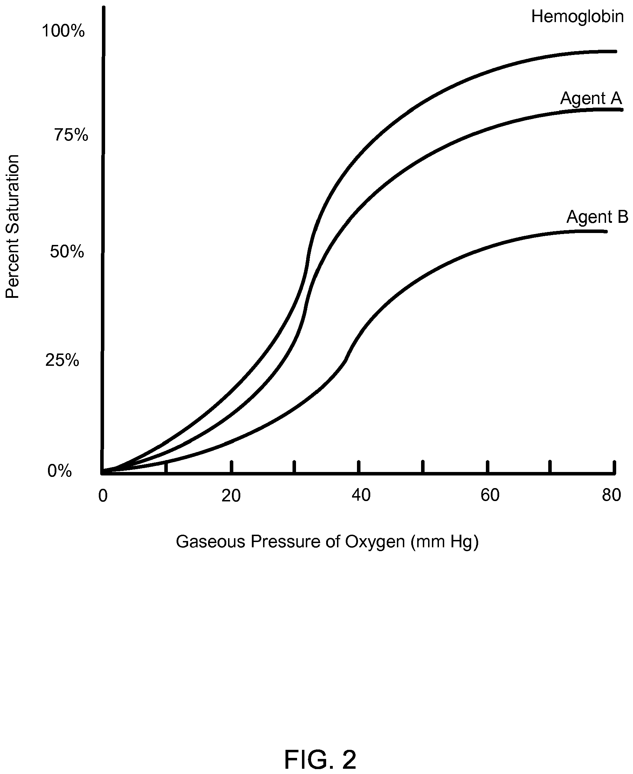

FIG. 2 illustrates the oxygen dissociation curves of hemoglobin and two other example agents (e.g., Agent A, Agent B) which may be used to manipulate oxygen levels in tumors. Specifically, FIG. 2 illustrates that the example agents (Agent A, Agent B) have oxygen dissociation curves that are shifted to the right of the hemoglobin oxygen dissociation curve, which increases the amount of oxygen delivered by the example agents.

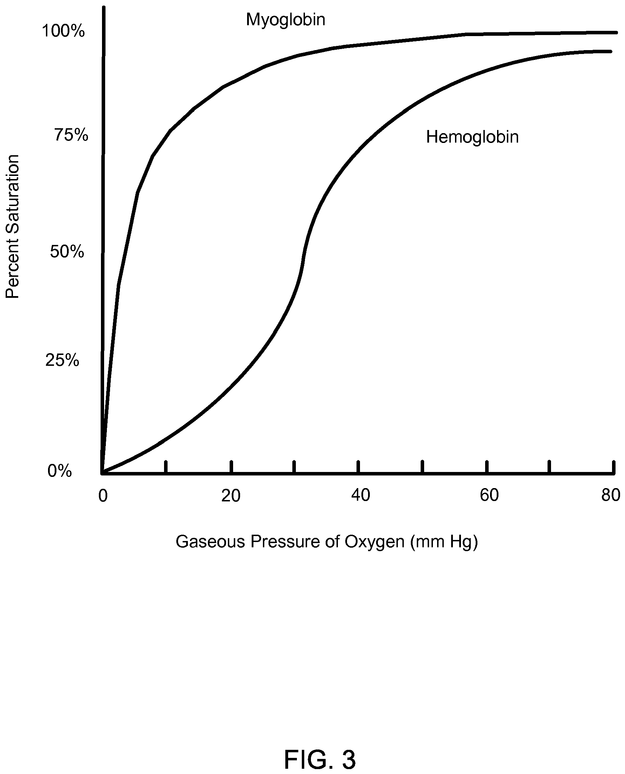

FIG. 3 illustrates the oxygen dissociation curves of hemoglobin and myoglobin, a ubiquitous protein involved in regulating oxygen levels in muscle tissues. FIG. 3 shows that the oxygen dissociation curve of myoglobin is a rectangular hyperbola with a very low P50 that lies to the left of the sigmoid-shaped hemoglobin oxygen dissociation curve (i.e., myoglobin has a much higher affinity for oxygen than hemoglobin). That is, in contrast to the example agents (e.g., Agent A, Agent B) discussed above with reference to FIG. 2, myoglobin has an oxygen dissociation curve that is shifted to the left of the hemoglobin oxygen dissociation curve. This is due in part to the fact that, unlike hemoglobin (which has affinity for oxygen of about 20 to 50 mmHg), myoglobin binds oxygen very tightly and only releases it at a very low oxygen tension (2 to 3 mmHg). The various embodiments benefit from the fact that a similar level of oxygen tension (2 to 5 mmHg) exists in the center of solid tumors, and this same level (2 to 5 mmHg) has been shown to be the level at which the efficacy of radiation therapy falls below fifty percent of its maximal value.

The various embodiments provide compositions and methods for delivering high-oxygen affinity agents (e.g., myoglobin, modified hemoglobin, synthetic proteins) having oxygen affinity similar to native myoglobin to solid tumors to improve the efficacy of radiation therapy. Since the oxygen tension at the center of a solid tumor is similar to the oxygen tension (2 to 3 mmHg) at which oxygen releases from myoglobin, the use of high-oxygen affinity agents ensures that the oxygen is not released from the agents until they are positioned around or within the tumor.

As mentioned above, the oxygen tensions at the center of solid tumors are similar to the oxygen tensions (2 to 3 mmHg) at which oxygen releases from myoglobin. As also mentioned above, the efficacy of radiation treatment may be improved by increasing the oxygen levels of tumors, and conventional methods for manipulating the oxygen levels are reliant upon increasing the systemic level of oxygen. For example, existing techniques for delivering oxygen to tumors may involve increasing the amount of blood flow to the tumor, increasing the amount of dissolved oxygen in blood, or increasing the overall blood concentration of hemoglobin. Conventional methods achieve this by using agents having a similar affinity for oxygen as natural red blood cells (e.g., derivatives of human or xenotic hemoglobin and/or other agents having the same or less affinity for oxygen as natural human hemoglobin) in order to increase the oxygen carrying capacity of the blood in hopes that this may translate to increased tumor oxygen delivery. In contrast to these conventional treatment methods, the various embodiments describe compositions of matter and methodology to deliver oxygen to tumor tissues by utilizing agents that have much higher affinity for oxygen than that of natural human hemoglobin and that possess an oxygen dissociation curve similar to that of natural myoglobin. Since the partial pressure of oxygen at the center of a solid tumor is similar to the oxygen tension at which oxygen releases from myoglobin (2 to 3 mmHg), oxygen is not released until the high-oxygen affinity agents are around or within the tumor.

While delivering high-oxygen affinity agents (e.g., myoglobin, other synthetic proteins having similar oxygen affinity as myoglobin, etc.) to solid tumors may improve the efficacy of radiation therapy, in order to achieve proper localization to the tumor, a large amount of the high-oxygen affinity agents must be injected into the blood stream. Injecting a large amount of such proteins into the bloodstream is dangerous, as the injected agent (e.g., myoglobin) may be nephrotoxic and/or cause hypertensive urgency or emergency (via sequestration of the vasodilator nitric oxide that normally controls blood vessel tone). For example, in the case of myoglobin, such phenomena is commonly observed in people who have heart attacks, run marathons, engage in other strenuous exercises, and/or use various drugs such as cocaine. In such people, muscles may begin to break down very quickly, thereby releasing a large amount of myoglobin into the blood stream. This extra myoglobin may result in the protein getting trapped in the body's filter apparatus (i.e., kidney glomeruli) and/or cause a life threatening condition known as rhabdomyolysis. A large amount of myoglobin in the blood stream may also increase blood pressure and lead to organ damage. This is because, in the bloodstream, myoglobin and other free oxygen-binding proteins sequester nitric oxide (NO) that is a mediator of vascular tone and blood flow. Thus, when myoglobin floods in the bloodstream, it acts to extract nitric oxide from the blood vessel walls, causing the blood vessels to constrict. This may cause an increase in the overall blood pressure and possibly lead to a hypertensive crisis, damaging major organs such as the kidneys, the heart, and the brain. For these and other reasons, injecting an oxygen binding protein, such as myoglobin, directly into the blood stream in its free-form is dangerous.

To address these and other issues, various embodiments may encapsulate the high-affinity oxygen binding agents in a carrier vehicle (e.g., a nanoparticle shell) that will protect the encapsulated agents from being released into the blood stream or interacting with biological components (e.g. proteins, cells, and the blood vessel walls) while in the blood circulation. In some embodiments, the inert carrier may be a PEGylated or polymerized version of the high-affinity oxygen-binding agent itself.

In various embodiments, high-affinity oxygen binding agents may be encapsulated in carrier vehicles having characteristics that allow for their accumulation around tumor regions and/or are capable of targeting tumors experiencing low oxygen tension. The carrier vehicle may also have characteristics such that oxygen will diffuse from within the vehicle to regions of low oxygen tension (as exist in the center of tumors) while the high-affinity oxygen binding agents remain encapsulated. The high-oxygen affinity agents may be delivered to tumors in a manner that allows the delivered agent to release oxygen at the oxygen tension required to increase the efficacy of radiation due to the oxygen partial pressure gradient characteristics of the agent.

In various embodiments, the high-oxygen affinity agents may be encapsulated in a vehicle comprised of biodegradable polymers (e.g., polymersomes, nanoparticles, etc.). According to the invention, the nitric oxide-inhibiting agent is encapsulated within the carrier vehicle, which is a nanoparticle, which is a polymersome. Encapsulation of the high-oxygen affinity agents (e.g., oxygen binding proteins and/or molecules) in biodegradable polymeric vehicles protects the agents from contact with blood and tissues, thereby reducing toxicity while maintaining high internal oxygen concentrations until the vehicles are positioned within hypoxic tumor tissues. In an embodiment, the agents may be encapsulated such that they are highly concentrated within the aqueous interior of the carrier vehicle. The agents may be encapsulated such that the carrier vehicle (e.g., nanoparticle shell) shields the encapsulated proteins from interacting with the blood vessel walls, preventing nitric oxide from being taken up into the nanoparticle and/or binding to encapsulated oxygen binding proteins and/or molecules. The agents may include proteins and/or molecules that have very high affinity for oxygen (e.g., proteins having a low P50 for oxygen) and/or have oxygen binding proprieties such that oxygen becomes unbound from the proteins and/or molecules only at the lowest oxygen tensions, such as those found in the most hypoxic tumors (i.e., heterogeneous tumors where pockets of tissue have oxygen tensions that are below the P50 of the high-affinity oxygen carrying agent). The agents may include proteins and/or molecules for which oxygen is released at an oxygen tension of less than 10 millimeters mercury (mmHg).

In various embodiments, the high-affinity oxygen-binding agents may be unmodified human myoglobin, unmodified myoglobin from another biological species, chemically or genetically modified myoglobin from humans or from another biological species, unmodified hemoglobin from another biological species, or a small molecule, metal-chelator complex, or a biological agent, including a peptide, protein, nucleic acid, or polysaccharide that binds oxygen tightly at physiological oxygen binding tensions as found in the lungs and that releases it only at lowest oxygen tensions as found in hypoxic tumors (i.e., molecules that posses P50 for oxygen of < or = 10 mm Hg). According to the invention, the (NO)-binding molecule is selected from one or more of unmodified human myoglobin, unmodified myoglobin from another biological species, and chemically or genetically modified myoglobin from humans or from another biological species.

In various embodiments, the inert carrier vehicle may be any one or more of a liposome, polymersome, micelle, modified lipoprotein, solid nanoparticle, solid micron-sized particle, lipid or perfluorocarbon emulsion, dendrimer, virus, or virus-like particle. In other embodiments, the inert carrier vehicle may be a PEGylated or polymerized version of the high-affinity oxygen-binding agent or agents. In a preferred embodiment, human myoglobin may be encapsulated within nanoparticles, polymer vesicles and/or polymersomes. In various embodiments, the nanoparticles, polymer vesicles and/or polymersomes may be constructed from one of a number of different biodegradable materials.

As mentioned above, in an embodiment, the high-affinity oxygen-binding agents may include myoglobin. Myoglobin (Mb) is a cytoplasmic heme protein that plays a well-characterized role in $O_2$ transport and free radical scavenging in skeletal and cardiac muscle (two tissues, notably, with low incidences of malignancy).[93, 94] Myoglobin's oxygen-related functions are multiple and include at least 3 different activities. First, myoglobin acts as an oxygen reservoir, possessing a much higher $O_2$ affinity than that of hemoglobin (Mb) (P50-Mb = 2.75 vs. P50-Hb = 25-50 mmHg). Myoglobin thus binds $O_2$ in aerobic conditions and releases it under hypoxic conditions,[95] as found in tumors. Second, myoglobin is capable of buffering intracellular $O_2$ by unloading its oxygen as cytoplasmic $pO_2$ falls to low levels, promoting continuous oxidative phosophorylation.[96] Third, myoglobin supplements simple $O_2$ delivery by working as a carrier in a process known as facilitated $O_2$ diffusion.[97]

Myoglobin has recently been shown to be a modulator of tumor hypoxia.[98, 99] Myoglobin gene transfer in a mouse xenotransplanted human lung tumor provided a valid model for studying the role of $O_2$ and ROS in tumor progression. By enabling oxidative phosphorylation under low $pO_2$, myoglobin further prevents baseline ROS formation under hypoxic conditions and mitigates the tumorigenic response.[98, 99] In these mouse models of cancer, myoglobin expression resulted in delayed tumor implantation, reduced xenograft growth, and generated minimal HIF-1 levels.[98] Angiogenesis and invasion were also strongly inhibited.[98] These effects were not observed using point-mutated forms of myoglobin unable to bind $O_2$ but capable of scavenging free radicals.[98] Together, these data suggest that hypoxia is not just an epiphenomenon associated with dysregulated growth, but also a key factor driving tumor progression. They also suggest that the pleiotropic functions of myoglobin affect cancer biology in multiple ways.

While myoglobin has shown to modulate tumor hypoxia, its clinical utility as an $O_2$ therapeutic requires overcoming two major obstacles related to its free intravascular infusion: 1) vasoconstriction, hypertension, reduced blood flow, and vascular damage in animals due to entrapment of endothelium-derived nitric oxide (NO); and 2) nephrotoxicity as seen with rhabdomyolysis. In the various embodiments, the limitations of using myoglobin as an oxygen carrier may be overcome by encapsulating myoglobin within an appropriate polymeric vehicle (e.g., polymersome) to improve its tumor-specific delivery and to mitigate its systemic exposure.

Polymersomes[38, 39] are synthetic polymer vesicles that are formed in nanometric dimensions (50 to 300 nm in diameter) and exhibit several favorable properties as cellular oxygen carriers. For example, polymersomes belong to the class of bi- and multi-layered vesicles that can be generated through self-assembly and can encapsulate hydrophilic compounds such as hemoglobin (Hb) and myoglobin (Mb) in their aqueous core.[40, 41] Moreover, polymersomes offer several options to be designed from fully biodegradable FDA-approved components and exhibit no *in vitro* or acute *in vivo* toxicities.

Polymersomes exhibit several superior properties over liposomes and other nanoparticle-based delivery vehicles that make them effective myoglobin-based oxygen carriers MBOCs. For example, depending on the structure of their component copolymer blocks, polymersome membranes may be significantly thicker (~ 9-22 nm) than those of liposomes (3-4 nm), making them 5-50 times mechanically tougher and at least 10 times less permeable to water than liposomes.[46, 47] The circulatory half-life of polymersomes, with poly(ethylene oxide) (PEO) brushes ranging from 1.2-3.7 kDa, is analogous to that of poly (ethylene glycol)-based liposomes (PEG-lyposomes) of similar sizes (-24-48 hours) and can be further specifically tailored by using a variety of copolymers as composite building blocks.[48] Polymersomes have been shown to be stable for several months *in situ,* and for several days in blood plasma under well-mixed quasi-physiological conditions, without experiencing any changes in vesicle size and morphology.[40, 48] They do not show in-surface thermal transitions up to 60 °C. [37, 48] In addition, early animal studies on PEO-b-PCL and poly(ethylene-oxide)-block-poly(butadiene)- (PEO-b-PBD-)based polymersomes formulations encapsulating doxorubicin have shown no acute or subacute toxicities. Finally, the production and storage of polymersomes is economical. Polymersomes may be readily produced and stored on a large-scale without requiring costly post-manufacturing purification processes.

Most promising biodegradable polymersome-encapsulated myoglobin (PEM) formulations have been hypothesized to be comprised of block copolymers that consist of the hydrophilic biocompatible poly(ethylene oxide) (PEO), which is chemically synonymous with PEG, coupled to various hydrophobic aliphatic poly(anhydrides), poly(nucleic acids), poly(esters), poly(ortho esters), poly(peptides), poly(phosphazenes) and poly(saccharides), including but not limited by poly(lactide) (PLA), poly(glycolide) (PLGA), poly(lactic-co-glycolic acid) (PLGA), poly($\varepsilon$-caprolactone) (PCL), and poly (trimethylene carbonate) (PTMC). Polymersomes comprised of 100% PEGylated surfaces possess improved *in vitro* chemical stability, augmented *in vivo* bioavailablity, and prolonged blood circulatory half-lives.[42, 43] For example, aliphatic polyesters, constituting the polymersomes' membrane portions, are degraded by hydrolysis of their ester linkages in physiological conditions such as in the human body. Because of their biodegradable nature, aliphatic polyesters have received a great deal of attention for use as implantable biomaterials in drug delivery devices, bioresorbable sutures, adhesion barriers, and as scaffolds for injury repair via tissue engineering.[44, 45]

Compared to the other biodegradable aliphatic polyesters, poly($\varepsilon$-caprolactone) (PCL) and its derivatives have several advantageous properties including: 1) high permeability to small drug molecules; 2) maintenance of a neutral pH environment upon degradation; 3) facility in forming blends with other polymers; and 4) suitability for long-term delivery afforded by slow erosion kinetics as compared to PLA, PGA, and PLGA.[45] Utilization of $\varepsilon$-caprolactone (or derivatives such as $\gamma$-methyl $\varepsilon$-caprolactone) as the membrane-forming shells in polymersome-encapsulated myoglobin (PEM) formulations promises that the resultant cellular myoglobin-based oxygen carriers (MBOCs) will have safe and complete in vivo degradation.

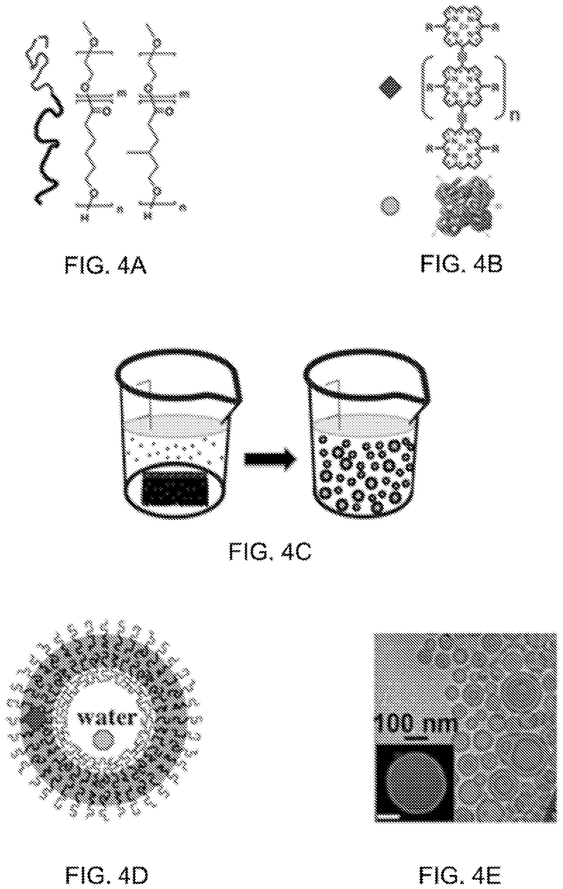

Components of biodegradable polymersomes may include biodegradable polymers, as shown in FIG. 4A, and water-soluble near-infrared fluorophores and water-soluble oxygen binding proteins, as shown in FIG. 4B. FIG. 4C illustrates processing procedures involved in the synthesis of nanoscale PEM in various embodiments, which include heat, sonication, and extrusion. An embodiment PEM is shown schematically in FIG. 4D, and in cryogenic transmission electron micrograph and confocal micrographs in FIG. 4E. Fully biodegradable and bioresorbable polymersomes have previously been demonstrated to be generated via self-assembly upon aqueous hydration of amphiphilic diblock copolymers of PEO-b-PCL[39]. Over 20 PEO-b-PCL copolymers, varying in molecular weights of the component building blocks, have previously been tested for the generation of stable bilayered polymersomes. However, only diblock copolymers of PEO-b-PCL in which the PEO block was 1-5 kDa and 10-20% of the polymer mass by weight have demonstrated a consistent and significant yield of stable mono-dispersed polymersomes, with mean particle diameters of < 200 nm and membrane thicknesses of 9-22 nm after extrusion through 200-nm diameter pore cut-off membranes. PEO-b-PCL polymersomes have subsequently been shown to be capable of loading the anti-neoplastic drug doxorubicin (DOX) using an ammonium sulfate gradient. The in vitro stability, mechanism of degradation, and rate of drug release from DOX-loaded PEO(2kDa)-b-PCL(12kDa) polymersomes were evaluated as a function of pH over 14 days. While the kinetics of release varied under neutral and acidic pH conditions (5.5 and 7.4, at 37 °C), an initial burst release phase (approx. 20% of the initial payload within the first 8 h) was observed at both pH conditions followed by a more controlled, pH-dependent release over the several days. At a pH of 7.4, kinetic release studies suggest that the encapsulated molecules initially escape the polymersome through passive diffusion of the drug across intact poly($\varepsilon$-caprolactone) (PCL) membrane (days 1-4), and subsequently through hydrolytic matrix degradation of PCL (days 5-14). At a pH of 5.5, however, it appears that the dominant mechanism of release, at both short and long times, is acid-catalyzed hydrolysis of the PCL membrane. Notably, these fully-biodegradable polymersomes have a half-life ($\tau_{1/2}$) of circulation (24-48 h) that is much shorter than their half-life ($\tau_{1/2}$) of release (2 weeks at pH 7.4).

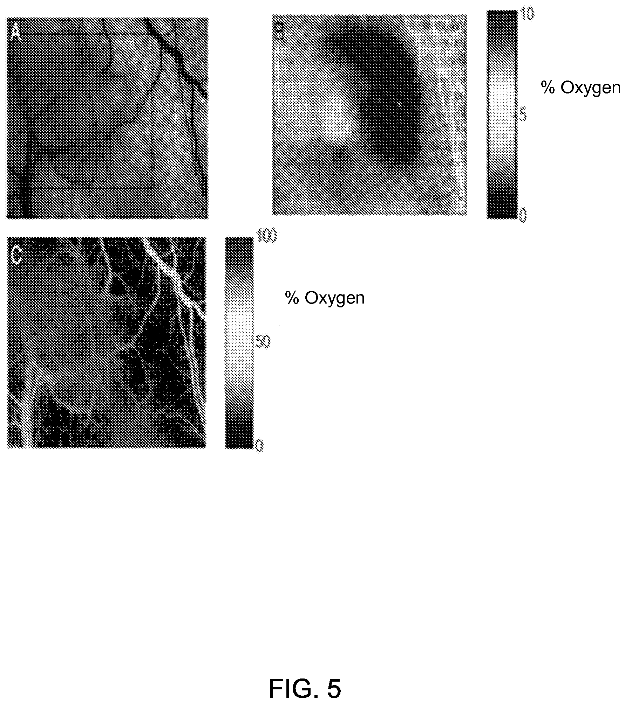

FIG. 5 illustrates the (A) bright field micrograph of tumor vasculature, (B) distribution of oxygen tensions in the tumor parenchyma (in % oxygen), and (C) overlay of (A) and (B), demonstrating functional oxygen delivery, utilizing an in vivo window chamber tumor model system. In this illustration the tumor is the relatively dark region in panel A in the center-left. The oxygen saturation (C) is shown on a color scale whose brightness is modulated by the total $O_2$ content (thus well vascularized regions appear bright.) The tumor region displays highly heterogeneous oxygen concentration (B), with a central peak in oxygen tension, as well as a peripheral (upper and right) region that is highly hypoxic. The composite map shows significant contrast with the surrounding normal tissue due to angiogenesis throughout the tumor, making it appear hazy bright. As is evident in the illustrated example of FIG. 5, the O2 content (as measured by the partial pressure of oxygen at various points) is heterogeneous throughout the tumor parenchyma but the lowest oxygen-tensions (darkest areas as demarcated by pO2 of < 10mmHg) can be found within the center of the tumor. It is within these low pO2 laden areas where tumors tend to up-regulate the HIF-1 signaling cascade, leading to a more aggressive tumorigenic phenotype that is resistant to radiation and chemotherapies and that has a higher tendency to metastasize to other locations. As such, increasing the minimum oxygen tensions found within the heterogeneous tumor may be as important as increasing the overall tumor pO2 when it comes to a therapeutic goal.

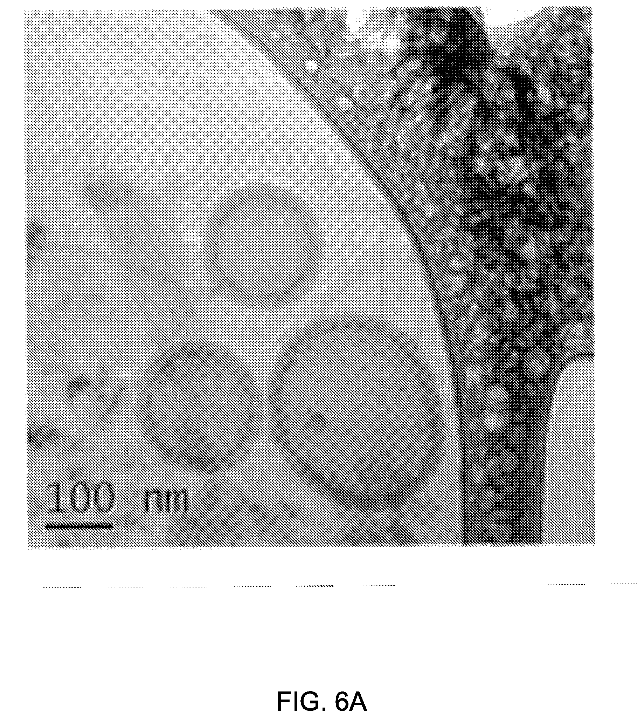

FIG. 6A illustrates that only diblock copolymers of poly(ethylene oxide)-block-poly(e-caprolactone) (PEO-b-PCL) in which the PEO block was 1-5 kDa and 10-20% of the polymer mass by weight have demonstrated a consistent and significant yield of stable mono-dispersed polymersomes, with mean particle diameters of < 200 nm and membrane thicknesses of 9-22 nm after extrusion through 200-nm diameter pore-cutoff membranes. PEO-b-PCL polymersomes have subsequently been shown to be capable of loading the anti-neoplastic drug doxorubicin (DOX) using an ammonium sulfate gradient.

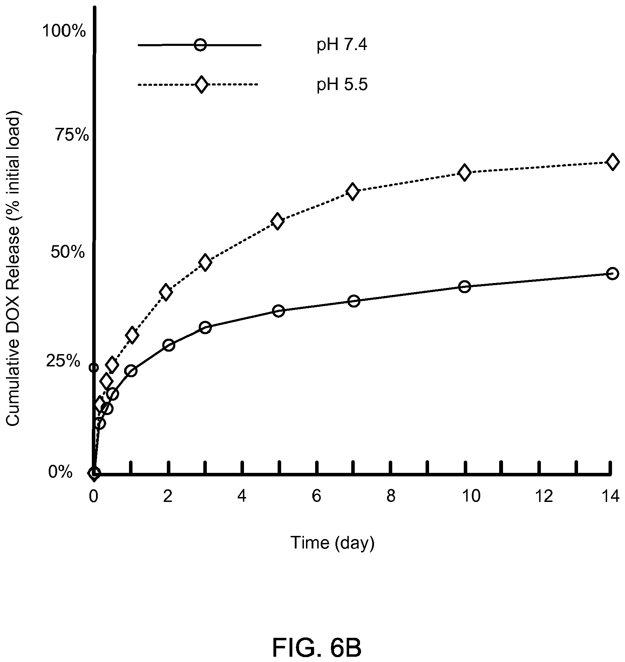

FIG. 6B illustrates the in vitro stability, mechanism of degradation and rate of drug release from DOX-loaded PEO(2kDa)-b-PCL(12kDa) polymersomes evaluated as a function of pH over 14 days. FIG. 6B shows that, while the kinetics of release varied under neutral and acidic pH conditions (5.5 and 7.4, at 37 °C), an initial burst release phase (approx. 20% of the initial payload within the first 8 h) was observed at both pH conditions followed by a more controlled, pH-dependent release over the several days. At a pH of 7.4, kinetic release studies show that the encapsulated molecules initially escape the polymersome through passive diffusion of the drug across the intact PCL membrane (days 1-4), and subsequently through hydrolytic matrix degradation of PCL (days 5-14). At a pH of 5.5, however, the dominant mechanism of release, at both short and long times, is acid-catalyzed hydrolysis of the PCL membrane. Notably, these fully-biodegradable polymersomes have a t1/2 half-life of circulation (24-48 h) that is much shorter than their t1/2 half-life of release (2 weeks at pH 7.4). As such, polymersomes can be expected to circulate in the blood stream relatively intact and will release their encapsulated contents in an accelerated fashion only when exposed to lower pH environments, as found in hypoxic tumors.

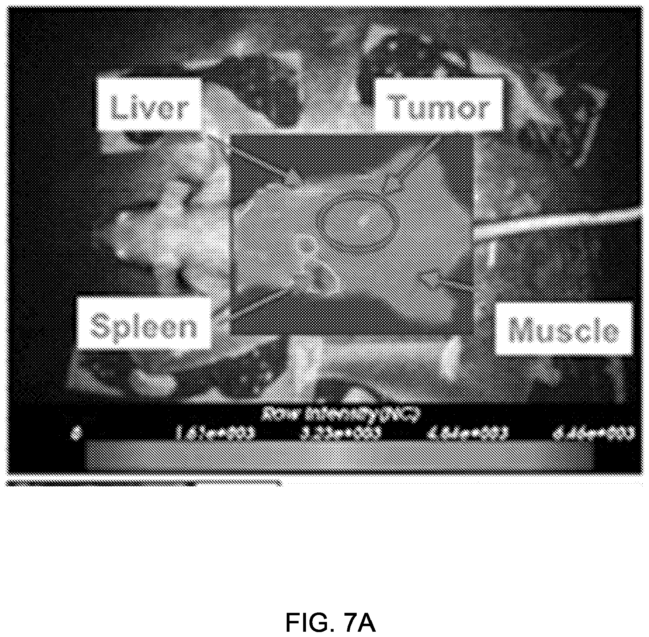

FIG. 7A illustrates the accumulation of an embodiment carrier (Polymersomes) in tumors as demonstrated through *in vivo* optical imaging of oligo(porphyrin)-based near-infrared (NIR) fluorophores (NIRFs) that are incorporated within the membrane shells of the polymersomes. This figure illustrates that polymersomes may accumulate around the tumor through a passive targeting modality due to the enhanced permeation and retention effect (EPR) associated with leaky tumor microvasculature. Further increases in polymersome accumulation may be aided through the inclusion of targeting molecules that will enhance the concentration of polymersomes at the tumor site.

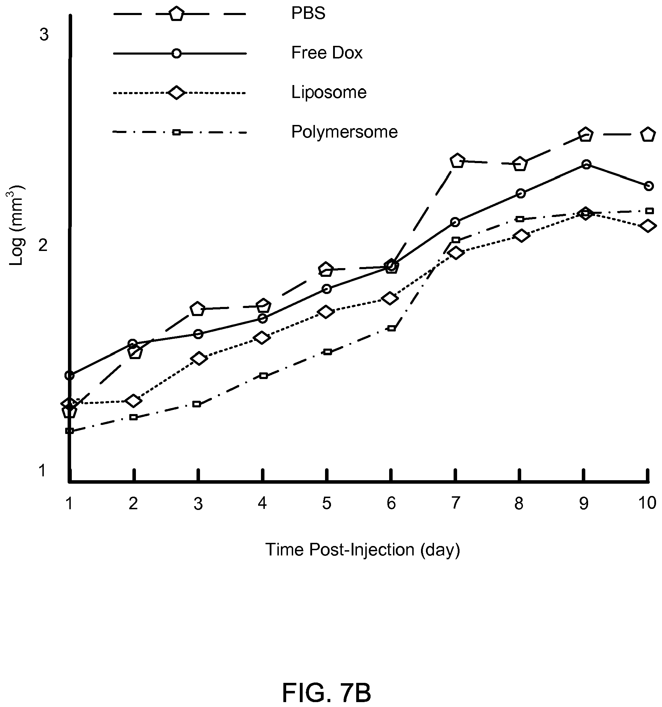

FIG. 7B is a line chart of *in vivo* tumor growth (depicted as tumor size in log ($mm^3$)) as inhibited by phosphate buffered saline (PBS), doxorubicin (DOX), liposome-encapsulated DOX, and polymersome-encapsulated DOX. This figure illustrates that not only are polymersomes able to accumulate around tumors (as seen in FIG. 7A) but that they do so in sufficient quantities and with preserved intravascular stabilities so as to enable effective release of their encapsulant payload at the tumor site so as to alter tumor biology. When comparing different biodegradable delivery vehicles (e.g. polymersomes vs. liposomes vs. free drug), the superior ability of polymersomes to achieve these outcomes is evident.

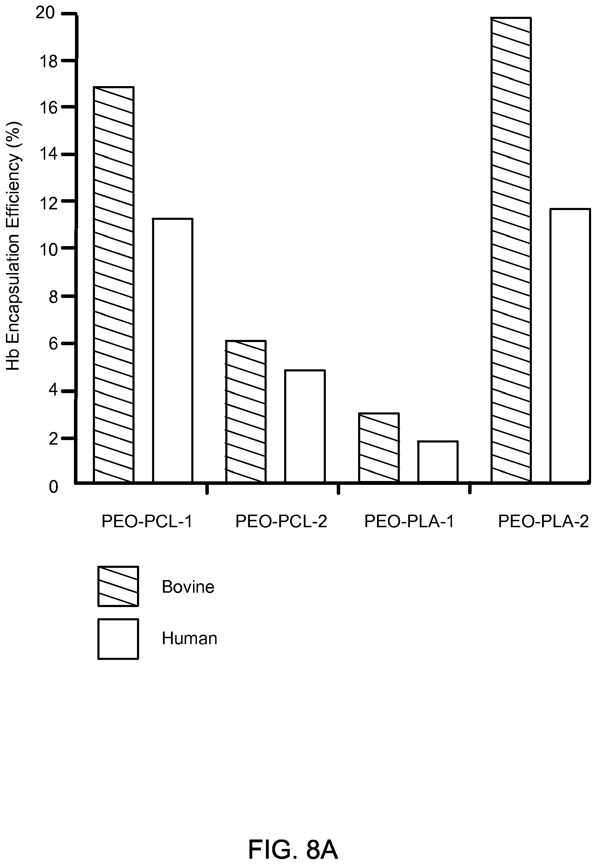

FIG. 8A illustrates hemoglobin (Hb) encapsulation efficiencies of four polymersome-encapsulated bovine and human Hb formulations after extrusion through 200 nm diameter polycarbonate membranes. Specifically, FIG. 8A illustrates the hemoglobin encapsulation efficiency of PEO-b-PCL-1(1.65 KDa), PEO-b-PCL-2(15 KDa), PEO-b-PLA-1(10 kDa) and PEO-b-PLA-2 (2.45 KDa). As discussed above, the various embodiments provide methodology for generating constructs that have an average radius between 100-125 nm with polydispersity index < 1.1 and a hemoglobin encapsulation efficiency > 50%.

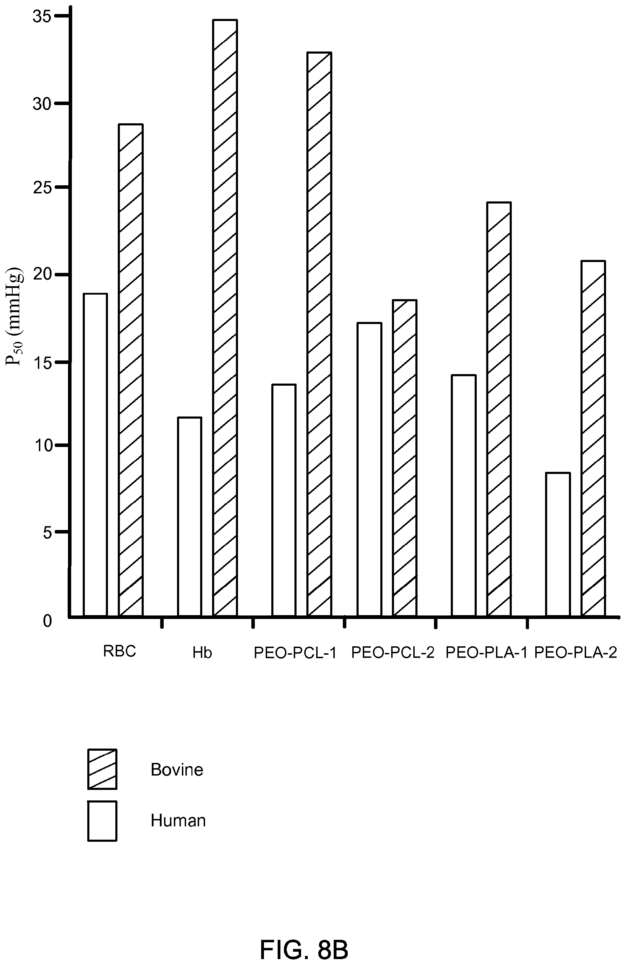

FIG. 8B illustrates the $P_{50}$ (mmHg) of red blood cells, hemoglobin and four polymersome-encapsulated hemoglobin formulations (PEO-b-PCL-1(1.65 KDa), PEO-b-PCL-2(15 KDa), PEO-b-PLA-1(10 kDa) and PEO-b-PLA-2 (2.45 KDa)) extruded through 200 nm diameter polycarbonate membranes. As mentioned above, the various embodiments provide methodology for generating PEM constructs that have a $P_{50}$ < 10 mm mercury and at least an order of magnitude smaller NO binding rate constant than that measured for liposome-encapsulated hemoglobin dispersions (LEHs) at similar hemoglobin loading concentrations.

As discussed above, polymers are macromolecules comprising chemically conjugated monomeric molecules, wherein the monomeric units being either of a single type (homogeneous) or of a variety of types (heterogeneous). The physical behavior of polymers may be dictated by several factors, including: the total molecular weight, the composition of the polymer (e.g., the relative concentrations of different monomers), the chemical identity of each monomeric unit and its interaction with a solvent, and the architecture of the polymer (whether it is single chain or consists of branched chains). For example, in polyethylene gylcol (PEG), which is a polymer of ethylene gylcol (EG), the chain lengths of which, when covalently attached to a phospholipid, optimize the circulation life of a liposome, is known to be in the approximate range of 34 -114 covalently linked monomers (EG34 to EG114). The preferred embodiments comprise hydrophilic copolymers of polyethylene oxide (PEO), a polymer that is related to PEG), and one of several hydrophobic blocks that drive self-assembly of the polymersomes, up to microns in diameter, in water and other aqueous media.

As discussed above, an amphiphilic substance is one containing both polar (water-soluble) and hydrophobic (water-insoluble) groups. To form a stable membrane in water, a potential minimum requisite molecular weight for an amphiphile must exceed that of methanol $HOCH_3$, which is the smallest canonical amphiphile, with one end polar (HO-) and the other end hydrophobic (-CH3). Formation of a stable lamellar phase requires an amphiphile with a hydrophilic group whose projected area is approximately equal to the volume divided by the maximum dimension of the hydrophobic portion of the amphiphile.

In some embodiments, the oxygen carrier, nanoparticle and/or polymersome does not include polyethylene glycol (PEG) or polyethylene oxide (PEO) as one of its plurality of polymers. In some embodiments, the oxygen carrier, nanoparticle and/or polymersome include least one hydrophilic polymer that is polyethylene glycol (PEG) or polyethyelene oxide (PEO). In some embodiments, the PEG or PEO polymer may vary in molecular weight from about 5 kDaltons (kDa) to about 50 kDa in molecular weight.

The most common lamellae-forming amphiphiles may have a hydrophilic volume fraction between 20 and 50%. In some embodiments, the hydrophilic volume fraction of the oxygen carriers, nanoparticles and/or polymersomes is up to about 20%. In some embodiments, the hydrophilic volume fraction of the oxygen carriers, nanoparticles and/or polymersomes is up to about 19%. In some embodiments, the hydrophilic volume fraction of the oxygen carriers, nanoparticles and/or polymersomes is up to about 18%. In some embodiments, the hydrophilic volume fraction of the oxygen carriers, nanoparticles and/or polymersomes is up to about 17%. In some embodiments, the hydrophilic volume fraction of the oxygen carriers, nanoparticles and/or polymersomes is up to about 16%. In some embodiments, the hydrophilic volume fraction of the oxygen carriers, nanoparticles and/or polymersomes is up to about 15%. In some embodiments, the hydrophilic volume fraction of the oxygen carriers, nanoparticles and/or polymersomes is less than 20%. In some embodiments, the hydrophilic volume fraction of the oxygen carriers, nanoparticles and/or polymersomes is from about 1% to about 20%. It should be noted that the ability of amphiphilic and super-amphiphilic molecules to self-assemble can be largely assessed, without undue experimentation, by suspending the synthetic super-amphiphile in aqueous solution and looking for lamellar and vesicular structures as judged by simple observation under any basic optical microscope, cryogenic transmission electron microscope, or through the scattering of light.

The effective amount of the composition may be dependent on any number of variables, including without limitation, the species, breed, size, height, weight, age, overall health of the subject, the type of formulation, the mode or manner of administration, the type and/or severity of the particular condition being treated, or the need to modulate the activity of the molecular pathway induced by association of the analog to its receptor. The appropriate effective amount can be routinely determined by those of skill in the art using routine optimization techniques and the skilled and informed judgment of the practitioner and other factors evident to those skilled in the art.

A therapeutically effective dose of the oxygen carriers of the various embodiments may provide partial or complete biological activity as compared to the biological activity of a patient's or subject's physiologically mean, median or minimum tissue oxygenation. A therapeutically effective dose of the oxygen carriers of the various embodiments may provide a complete or partial amelioration of symptoms associated with a disease, disorder or ailment for which the subject is being treated.

The oxygen carriers of the various embodiments may delay the onset or lower the chances that a subject develops one or more symptoms associated with the disease, disorder, or ailment for which the subject is being treated. In some embodiments, an effective amount is the amount of a compound required to treat or prevent a consequence resulting from low or poor tissue oxygenation. According to the various embodiments, the effective amount of active compound(s) used for therapeutic treatment of conditions caused by or contributing to low or poor tissue oxygenation varies depending upon the manner of administration, the age, body weight, and general health of the patient.

Soluble amphiphiles, proteins, ligands, allosteric effectors, oxygen binding compounds can bind to and/or intercalate within a membrane. Such a membrane must also be semipermeable to solutes, sub-microscopic in its thickness (d), and result from a process of self-assembly or directed assembly. The membrane can have fluid or solid properties, depending on temperature and on the chemistry of the amphiphiles from which it is formed. At some temperatures, the membrane can be fluid (having a measurable viscosity), or it can be solid-like, with an elasticity and bending rigidity. The membrane can store energy through its mechanical deformation, or it can store electrical energy by maintaining a transmembrane potential. Under some conditions, membranes can adhere to each other and coalesce (fuse).

In various embodiments, myoglobin may be used as the oxygen-binding compound. In some embodiments, the oxygen-binding compound is protein with oxygen binding properties that are similar to myoglobin. In some embodiments, the oxygen-binding compound is genetically- or chemically-modified myoglobin or an oxygen binding protein isolated from another species that possesses gaseous binding characteristics that are similar to human myoglobin. In some embodiments, the oxygen-binding compound is chosen from a protein, small molecule, polypeptide, nucleic acid molecule, a metal-chelator complex or any combination thereof. In some embodiments, the oxygen-binding compound is a protein. In some embodiments, the oxygen-binding compound is a polypeptide. In some embodiments, the oxygen-binding compound is a polypeptide with a genetically or chemically modified heme group. In some embodiments, the oxygen-binding compound is a small molecule comprising a heme group.

In some embodiments, the oxygen carrier transports an effective amount of oxygen in order to treat a subject or to prevent a subject from suffering from a disease or disorder in which their blood does not carry or release sufficient levels of oxygen to tissues. In some embodiments the oxygen carrier comprises an effective amount of oxygen in order to treat or prevent the spread of cancer in a subject in need thereof. In some embodiments, the oxygen carrier comprises an effective amount of oxygen in order to promote wound healing in a subject in need thereof.

In some embodiments, the allosteric effector is 2,3-Bisphosphoglycerate or an isomer derived there from. Allosteric effectors such as 2,3-Bisphosphoglycerate may increase the offload of oxygen from the oxygen carrier or polymersome of the various embodiments to a tissue or cell that is deoxygenated within a subject.

As mentioned above, critical lysis tension (Tc) is the tension at which a particle ruptures when subject to an external force, as measured by micropipette aspiration and expressed as milliNewtons/meter (mN/m). The change in critical lysis tension of an oxygen carrier or polymersome may be measured before and after loading of the oxygen carrier, nanoparticle and/or polymersome with myoglobin, another oxygen-binding compound, or a mixture of one or more oxygen-binding compounds.

In various embodiments, the oxygen carriers, nanoparticles and/or polymersomes have a change of critical lysis tension of no more than 20%. In various embodiments, the oxygen carriers, nanoparticles and/or polymersomes have a change of critical lysis tension of no more than 19%. In various embodiments, the oxygen carriers, nanoparticles and/or polymersomes have a change of critical lysis tension of no more than 18%. In various embodiments, the oxygen carriers, nanoparticles and/or polymersomes have a change of critical lysis tension of no more than 17%. In various embodiments, the oxygen carriers, nanoparticles and/or polymersomes have a change of critical lysis tension of no more than 16%. In various embodiments, the oxygen carriers, nanoparticles and/or polymersomes have a change of critical lysis tension of no more than 15%. In various embodiments, the oxygen carriers, nanoparticles and/or polymersomes have a change of critical lysis tension of no more than 14%. In various embodiments, the oxygen carriers, nanoparticles and/or polymersomes have a change of critical lysis tension of no more than 13%. In various embodiments, the oxygen carriers, nanoparticles and/or polymersomes have a change of critical lysis tension of no more than 12%. In various embodiments, the oxygen carriers, nanoparticles and/or polymersomes have a change of critical lysis tension of no more than 11%. In various embodiments, the oxygen carriers, nanoparticles and/or polymersomes have a change of critical lysis tension of no more than 10%. In various embodiments, the oxygen carriers, nanoparticles and/or polymersomes have a change of critical lysis tension from about 5% to about 10%. In various embodiments, the oxygen carriers, nanoparticles and/or polymersomes may have a change of critical lysis tension from about 10% to about 15%. In various embodiments, the oxygen carriers, nanoparticles and/or polymersomes have a change of critical lysis tension from about 15% to about 20%. In various embodiments, the oxygen carriers, nanoparticles and/or polymersomes have a change of critical lysis tension from about 1% to about 5%.

As mentioned above, critical areal strain (Ac) is the areal strain realized by the oxygen carriers, nanoparticles and/or polymersomes at the critical lysis tension. In various embodiments, the oxygen carriers, nanoparticles and/or polymersomes have a critical areal strain from about 20% to about 50%. In various embodiments, the oxygen carriers, nanoparticles and/or polymersomes have a critical areal strain from about 20% to about 25%. In various embodiments, the oxygen carriers, nanoparticles and/or polymersomes have a critical areal strain from about 25% to about 30%. In various embodiments, the oxygen carriers, nanoparticles and/or polymersomes have a critical areal strain from about 30% to about 35%. In various embodiments, the oxygen carriers, nanoparticles and/or polymersomes have a critical areal strain from about 35% to about 40%. In various embodiments, the oxygen carriers, nanoparticles and/or polymersomes have a critical areal strain from about 40% to about 45%. In various embodiments, the oxygen carriers, nanoparticles and/or polymersomes have a critical areal strain from about 45% to about 50%.

As mentioned above, a "myoglobin loading capacity" is a measurement of a myglobin-based oxygen carrier and is defined as the weight of myoglobin within the oxygen carrier divided by the total weight of carrier. In various embodiments, the oxygen carriers, nanoparticles and/or polymersomes have a myoglobin loading capacity of greater than about 5. In various embodiments, the oxygen carriers, nanoparticles and/or polymersomes have a myoglobin loading capacity of greater than 10. In various embodiments, the oxygen carriers, nanoparticles and/or polymersomes have a myoglobin loading capacity of greater than 15. In various embodiments, the oxygen carriers, nanoparticles and/or polymersomes have a myoglobin loading capacity of greater than 20. In various embodiments, the oxygen carriers, nanoparticles and/or polymersomes have a myoglobin loading capacity of greater than 25. In various embodiments, the oxygen carriers, nanoparticles and/or polymersomes have a myoglobin loading capacity of greater than 26. In various embodiments, the oxygen carriers, nanoparticles and/or polymersomes have a myoglobin loading capacity of greater than 27. In various embodiments, the oxygen carriers, nanoparticles and/or polymersomes have a myoglobin loading capacity of greater than 28. In various embodiments, the oxygen carriers, nanoparticles and/or polymersomes have a myoglobin loading capacity of greater than 29. In various embodiments, the oxygen carriers, nanoparticles and/or polymersomes have a myoglobin loading capacity of greater than 30.

As mentioned above, a "myoglobin loading efficiency" is a fundamental measurement of a myoglobin-based oxygen carrier and is defined as the weight of myoglobin that is encapsulated and/or incorporated within a carrier suspension divided by the weight of the original myoglobin in solution prior to encapsulation (expressed as a %). In various embodiments, the oxygen carriers, nanoparticles and/or polymersomes have a myoglobin loading efficiency of greater than about 10%. In various embodiments, the oxygen carriers, nanoparticles and/or polymersomes have a myoglobin loading efficiency of greater than about 11%. In various embodiments, the oxygen carriers, nanoparticles and/or polymersomes have a myoglobin loading efficiency of greater than about 12%. In various embodiments, the oxygen carriers, nanoparticles and/or polymersomes have a myoglobin loading efficiency of greater than about 13%. In various embodiments, the oxygen carriers, nanoparticles and/or polymersomes have a myoglobin loading efficiency of greater than about 14%. In various embodiments, the oxygen carriers, nanoparticles and/or polymersomes have a myoglobin loading efficiency of greater than about 15%. In various embodiments, the oxygen carriers, nanoparticles and/or polymersomes have a myoglobin loading efficiency of greater than about 16%. In various embodiments, the oxygen carriers, nanoparticles and/or polymersomes have a myoglobin loading efficiency of greater than about 17%. In various embodiments, the oxygen carriers, nanoparticles and/or polymersomes have a myoglobin loading efficiency of greater than about 18%. In various embodiments, the oxygen carriers, nanoparticles and/or polymersomes have a myoglobin loading efficiency of greater than about 19%. In various embodiments, the oxygen carriers, nanoparticles and/or polymersomes have a myoglobin loading efficiency of greater than about 20%. In various embodiments, the oxygen carriers, nanoparticles and/or polymersomes have a myoglobin loading efficiency of greater than about 21%. In various embodiments, the oxygen carriers, nanoparticles and/or polymersomes have a myoglobin loading efficiency of greater than about 22%. In various embodiments, the oxygen carriers, nanoparticles and/or polymersomes have a myoglobin loading efficiency of greater than about 23%. In various embodiments, the oxygen carriers, nanoparticles and/or polymersomes have a myoglobin loading efficiency of greater than about 24%. In various embodiments, the oxygen carriers, nanoparticles and/or polymersomes have a myoglobin loading efficiency of greater than about 25%. In various embodiments, the oxygen carriers, nanoparticles and/or polymersomes have a myoglobin loading efficiency of greater than about 26%. In various embodiments, the oxygen carriers, nanoparticles and/or polymersomes have a myoglobin loading efficiency of greater than about 27%. In various embodiments, the oxygen carriers, nanoparticles and/or polymersomes have a myoglobin loading efficiency of greater than about 28%. In various embodiments, the oxygen carriers, nanoparticles and/or polymersomes have a myoglobin loading efficiency of greater than about 29%. In various embodiments, the oxygen carriers, nanoparticles and/or polymersomes have a myoglobin loading efficiency of greater than about 30%.

In various embodiments, the oxygen carriers, nanoparticles and/or polymersomes have a myoglobin loading efficiency from about 10% to about 35%. In various embodiments, the oxygen carriers, nanoparticles and/or polymersomes have a myoglobin loading efficiency from about 15% to about 35%. In various embodiments, the oxygen carriers, nanoparticles and/or polymersomes have a myoglobin loading efficiency from about 18% to about 35%. In various embodiments, the oxygen carriers, nanoparticles and/or polymersomes have a myoglobin loading efficiency from about 20% to about 35%. In various embodiments, the oxygen carriers, nanoparticles and/or polymersomes have a myoglobin loading efficiency from about 22% to about 35%. In various embodiments, the oxygen carriers, nanoparticles and/or polymersomes have a myoglobin loading efficiency from about 24% to about 35%. In various embodiments, the oxygen carriers, nanoparticles and/or polymersomes have a myoglobin loading efficiency from about 26% to about 35%. In various embodiments, the oxygen carriers, nanoparticles and/or polymersomes have a myoglobin loading efficiency from about 28% to about 35%. In various embodiments, the oxygen carriers, nanoparticles and/or polymersomes have a myoglobin loading efficiency from about 30% to about 35%.

In various embodiments, the subject may be a mammal. In various embodiments, the subject may be a non-human animal. In various embodiments, the subject may be a canine. In various embodiments, the subject may be a vertebrate.

The various embodiments include compositions and methods for making, storing and administering oxygen carriers comprising of an oxygen-binding compound encapsulated in a nanoparticle such as a polymersome. In various embodiments, the oxygen-binding compound may be comprised of myoglobin. In various embodiments, the oxygen-binding compound may be comprised of human or animal hemoglobin. In various embodiments, the oxygen-binding compound may be comprised of a genetically- or chemically-altered form of human or animal hemoglobin. In various embodiments, the oxygen-binding compound may be derived from a peptide, protein, or nucleic acid that possess oxygen affinities (P50, cooperativity coefficient n) similar to that of human myoglobin. In various embodiments, the oxygen-binding compound may be derived from a small molecule or metal-chelator complex that possess oxygen affinities (P50, cooperativity coefficient n) similar to that of human myoglobin. In various embodiments, the oxygen-binding compound may be derived from a nucleic acid or polysaccharide that possess oxygen affinities (P50, cooperativity coefficient n) similar to that of human myoglobin. In various embodiments, the oxygen carriers, nanoparticle and/or polymersomes may be comprised of a mixture of oxygen-binding compounds.

The various embodiments include compositions and methods for making, storing and administering oxygen carriers comprising of an oxygen-binding compound encapsulated in a vehicle such as a polymersome. In various embodiments, the oxygen carriers may comprise myoglobin. In various embodiments, the oxygen carriers may comprise a genetically- or chemically-altered form of human or animal hemoglobin. In various embodiments, the oxygen carriers, nanoparticle and/or polymersomes may comprise a mixture of oxygen-binding compounds.

Some embodiments may further include compositions and methods for developing polymersome-encapsulated myoglobin (PEM) as oxygen carriers. In various embodiments, the PEM may include polymersomes comprising of poly(ethylene oxide)-block-poly(ε-caprolactone) (PEO-b-PCL) and related diblock copolymers of poly(ethylene oxide)-block-poly(γ-methyl ε-caprolactone) (PEO-b-PMCL). PEO may provide the polymersomes improved *in vitro* chemical stability, augmented *in vivo* bioavailability and prolonged blood circulation half-lives. Both PEO-b-PCL and PEO-b-PMCL may afford complete and safe *in vivo* biodegradation of polymersome membranes via hydrolysis of their ester linkages. In various embodiments, the biodegradable polymersome-encapsulated myoglobin (PEM) dispersions may be comprised of diblock copolymers of PEO-b-PCL with a PEO block size of ~1.5-2kDa and with a block fraction of ~10-20% by weight. In various embodiments, the biodegradable polymersome-encapsulated myoglobin (PEM) dispersions may be comprised of diblock copolymers of PEO-b-PCL with a PCL block size of ~8kDa-23kDa and with a block weight fraction of about ~50 to 85 percent. In other embodiments, the PEM dispersions may be comprised of diblock copolymers of PEO-b-PMCL. PEO-b-PCL and PEO-b-PMCL polymersomes may be preferred cellular myoglobin-based oxygen carriers (MBOCs) and possess all the requisite properties for effective oxygen delivery, including tunable oxygen-binding capacities, uniform and appropriately small size distributions, human bloodlike viscosities and oncotic properties, as well as ease of mass production and affordable storage.

In an embodiment, a supramolecular self-assembly approach may be used to prepare mono-disperse unilamellar polymersomes (50-300 nm diameter) that incorporate high quantum yield oligo(porphyrin)-based near-infrared (NIR) fluorophores (NIRFs) within their bilayer membranes[124, 130, 164-167]. These bright, NIR-emissive polymersomes may possess the requisite photophysical properties and biocompatibility for ultra-sensitive *in vivo* optical imaging. [111, 124, 164, 168] Imaging studies of tumor-bearing mice have shown that non-targeted polymersomes are able to accumulate in tumors after intravascular injection due to the Enhanced Permeability and Retention (EPR) effect associated with leaky tumor microvasculature; quantitative fluorescence analysis has shown that a greater than two times tumor accumulation is readily achieved (FIGURE 7A).[164] Tumor-specific accumulation may further be enhanced by modifying polymersome surfaces through chemical conjugation to targeting ligands, such as small molecules, peptides, proteins (e.g. antibodies), and nucleic acids. [111, 127, 164]

Some embodiments include an operating methodology to synthesize PEM dispersions that consistently meet the following standard characteristics: (i) average radius between 100-200 nm with polydispersity index < 1.1 (ii) Mb encapsulation efficiency > 50 mol%; (iii) weight ratio of encapsulated Mb:polymer > 2; (iv) solution metMb level < 5%; (v) suspension viscosity between 3-4 cP; (vi) P50 between 2-3 mm Hg; and, (vii) at least an order of magnitude smaller NO binding rate constant as that measured for free Mb at similar weight per volume of distribution; (viii) final suspension concentration of between 80 to 180 mg Mb/mL solution; and (ix) excellent stability under different storage and flow conditions as determined by intact morphology, change in average particle diameter < 5 nm and unaltered Mb concentration (change < 0.5 g/dL) and unchanged metMb level (change < 2%).

The various embodiment PEMs may differ in their combination of particle size, deformability and concentration. Each of these parameters may independently affect the amount of Mb per particle, particle stability, and the numbers of particles that will accumulate at the tumor site. PEMs may be formed that are either 100 nm or 200 nm in mean particle diameter. Polymersomes, like other nanoparticles that are smaller than 250 nm in diameter may accrue in solid tumors due to the EPR effect[111, 114, 164] Although polymersomes with 200 nm mean particular diameter may deliver more Mb per particle, those that are ~100 nm in diameter may exhibit longer blood circulation half-lives (before eventual clearance by the RES))[102, 111, 114, 136, 172] and may demonstrate enhanced tumor accumulation by traversing plasma channels[173] (small microvessels that exclude RBCs).

An embodiment PEM may be constructed from either PEO-b-PCL, poly(ethylene oxide)-block-poly(y-methyl ε-caprolactone) (PEO-b-PMCL), and/or poly(ethylene oxide)-block-poly(trimethylcarbonate) (PEO-b-PTMC) diblock copolymers in order to determine the ultimate balance of particle stability versus deformability that may maximize *in vivo* tumor delivery. PMCL, as a derivative of PCL, similarly forms fully bioresorbable polymersomes that degrade via non-enzymatic hydrolysis of ester linkages.[174] PEO-b-PCL, however, may yield ultra-stable, solid vesicle membranes while PEO-b-PMCL and PEO-b-TMC may generate more deformable polymersomes, a characteristic that may aid in PEM passage through tortuous tumor blood vessels.

The various embodiments may include a polymersomes nanoparticle, or other oxygen carriers with varying sizes. In various embodiments, the polymersome or oxygen carrier includes a roughly spherical shape and has a diameter of about 50 nm to about 1 μm. In various embodiments, the polymersome or oxygen carrier has a diameter of about 50 nm to about 250 nm. In various embodiments, the polymersome or oxygen carrier has a diameter of about 100 nm to about 200 nm. In various embodiments, the polymersome or oxygen carrier has a diameter of about 200 nm to about 300 nm. In various embodiments, the polymersome or oxygen carrier has a diameter of about 300 nm to about 400 nm. In various embodiments, the polymersome or oxygen carrier has a diameter of about 400 nm to about 500 nm. In various embodiments, the polymersome or oxygen carrier has a diameter of about 500 nm to about 600 nm. In various embodiments, the polymersome or oxygen carrier has a diameter of about 600 nm to about 700 nm. In various embodiments, the polymersome or oxygen carrier has a diameter of about 700 nm to about 800 nm. In various embodiments, the polymersome or oxygen carrier has a diameter of about 800 nm to about 900 nm. In various embodiments, the polymersome or oxygen carrier has a diameter of about 900 nm to about 1 μm.

In various embodiments, the oxygen carrier consists of a nanoparticle that has a diameter of about 5 nm to about 100 nm. In various embodiments, the oxygen carrier has a diameter of about 5 nm to about 10 nm. In various embodiments, the oxygen carrier has a diameter of about 10 nm to about 50 nm. In various embodiments, the oxygen carrier has a diameter of about 50 nm to about 100 nm. In various embodiments, the oxygen carrier has a diameter of about 100 nm to about 300 nm. In various embodiments, the oxygen carrier has a diameter of about 300 nm to about 500 nm. In various embodiments, the oxygen carrier has a diameter of about 500 nm to about 1 μm.

In a further embodiment, the oxygen carriers, nanoparticle and/or polymersomes may include varying membrane thicknesses. The thickness of the membrane may depend upon the molecular weight of the polymers and the types of polymers used in the preparation of the oxygen carriers or polymersomes. In various embodiments, the membrane may be a single, double, triple, quadruple, or more layers of polymers. In various embodiments, the oxygen carriers, nanoparticle and/or polymersomes have a polymer membrane thickness from about 5 nm to about 35 nm. In various embodiments, the oxygen carriers, nanoparticle and/or polymersomes have a membrane thickness from about 5 nm to about 10 nm. In various embodiments, the oxygen carriers, nanoparticle and/or polymersomes have a membrane thickness from about 10 nm to about 15 nm. In various embodiments, the oxygen carriers, nanoparticle and/or polymersomes have a membrane thickness from about 15 nm to about 20 nm. In various embodiments, the oxygen carriers, nanoparticle and/or polymersomes have a membrane thickness from about 20 nm to about 25 nm. In various embodiments, the oxygen carriers, nanoparticle and/or polymersomes have a membrane thickness from about 25 nm to about 30 nm. In various embodiments, the oxygen carriers, nanoparticle and/or polymersomes have a membrane thickness from about 30 nm to about 35 nm. In various embodiments, the oxygen carriers, nanoparticle and/or polymersomes have a polymer membrane that is no more than about 5 nm in thickness. In various embodiments, the oxygen carriers, nanoparticle and/or polymersomes have a polymer membrane that is no more than about 10 nm in thickness. In various embodiments, the oxygen carriers, nanoparticle and/or polymersomes have a polymer membrane that is no more than about 15 nm in thickness. In various embodiments, the oxygen carriers, nanoparticle and/or polymersomes have has a polymer membrane that is no more than about 20 nm in thickness. In various embodiments, the oxygen carriers, nanoparticle and/or polymersomes have a polymer membrane that is no more than about 25 nm in thickness. In various embodiments, the oxygen carriers, nanoparticle and/or polymersomes have a polymer membrane that is no more than about 30 nm in thickness. In various embodiments, the oxygen carriers, nanoparticle and/or polymersomes have a polymer membrane that is no more than about 35 nm in thickness.

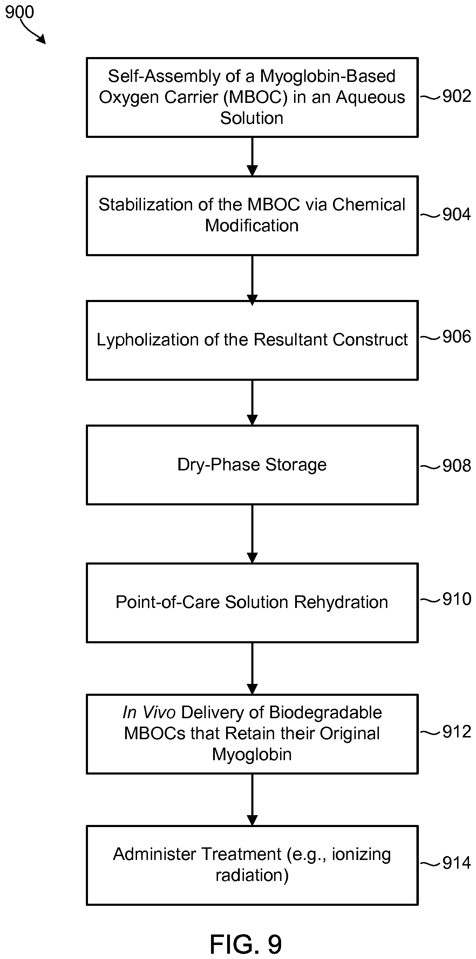

FIG. 9 illustrates a method 900 for MBOC preparation and delivery. In step 902, the myoglobin-based oxygen carrier (MBOC) is self-assembled in aqueous solution. In step 904, the myoglobin-based oxygen carrier is stabilized via chemical modification. In step 906, the resultant construct is lypholized. In step 908, the resultant construct is stored via dry-phase storage. In step 910, point-of-care solution rehydration. In step 912, biodegradable MBOCs that retain their original myoglobin are delivered *in vivo.* As a non-limiting example, polymersome-encapsulated Mb may be prepared and generated via such an MBOC preparation method. In step 914, the treatment may be administered by, for example, administering the MBOC and/or high-oxygen affinity agent to the patient and administering ionizing radiation to the tumor.

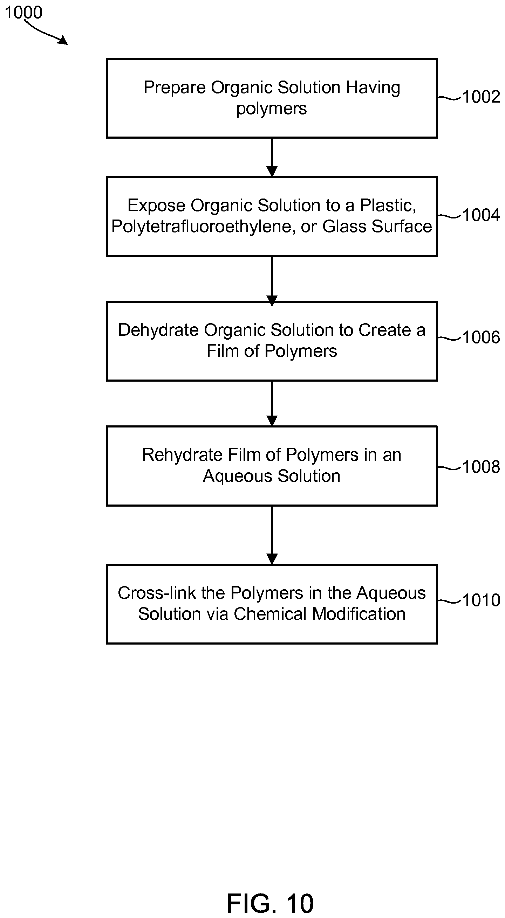

FIG. 10 illustrates a method 1000 for preparing a polymersome comprising at least one biocompatible polymer and at least one biodegradable polymer. It should be noted that FIG. 10 provides a high-level overview of the method steps and that details for each step are provided further below. In step 1002, an organic solution having a plurality of polymers may be prepared. In step 1004, the organic solution comprising the plurality of polymers may be exposed to a plastic, polytetrafluoroethylene (i.e., Teflon™) (herein "PTFE"), or glass surface. In step 1006, the organic solution may be dehydrated on the plastic, PTFE, or glass surface to create a film of polymers. In step 1008, the film of polymers may be rehydrated in an aqueous solution. In step 1010, the polymers may be cross-linked in the aqueous solution via chemical modification.

Polymersomes of the various embodiment PEM may comprise copolymers that are synthesized to include polymerizable groups within either their hydrophilic or hydrophobic blocks. The polymerizable biodegradable polymers may be utilized to form polymersomes that co-incorporate Mb and a water-soluble initiator in their aqueous interiors, or alternatively, by compartmentalizing Mb in their aqueous cavities and a water-insoluble initiator in their hydrophobic membranes.

The various embodiments may further include a method for preparing a polymersome comprising at least one biocompatible polymer and at least one biodegradable polymer comprising: (a) preparing an organic solution comprising a plurality of polymers and exposing the organic solution comprising the plurality of polymers to a plastic, polytetrafluoroethylene (PTFE) (a.k.a. Teflon®), or glass surface; (b) dehydrating the organic solution on the plastic, Teflon®, or glass surface to create a film of polymers; and (c) rehydrating the film of polymers in an aqueous solution; (d) cross-linking the polymers in the aqueous solution via chemical modification.

The compositions of the various embodiments may be made by direct hydration methods as described in O'Neil, et al.,, Langmuir 2009, 25(16), 9025-9029. Briefly, polymersomes of the various embodiments may be made and encapsulated using the following method: To prepare formulations, 20 total mgs of polymer may be weighed into a 1.5mL centrifuge tube, heated at 95 °C for 20 min, and mixed. After the samples are cooled to room temperature (15 min minimum), 10 µL of protein solution may be added and diluted with 20, 70, and 900 µL of 10 mmol phosphate buffered saline (PBS), pH 7.4, with mixing after each addition. As a control, the polymersomes may be formed via dilution with PBS (10, 20, 70, 890 µL of PBS with mixing after each addition) and finally add 10 µL of the protein solution after the formation of the polymersomes. In this way, the encapsulation efficiency and loading may be calculated by subtraction. All samples may be prepared in triplicate. Encapsulation efficiencies may be quantified from standard curves generated from the fluorescently labeled crosslinked to the polymers of choice under investigation.

In a further embodiment method, polymersome preparation may involve large-scale fractionation of vesicular particles. Briefly, a total of 1.25 g of diblock copolymer may be hydrated with 25mL of 10mM phosphate buffer (PB) at pH 7.3. Because of the lows solubility of diblock copolymers in PB, the aqueous polymer mixture may be sonicated (Branson Sonifier 450, VWR Scientific,West Chester, PA) for 8-10 h at room temperature to yield the stock copolymer solution. The stock copolymer solution may be then mixed with 25mL of purified Mb (250-300 g/L) to yield a copolymer concentration of 12.5 mg/mL in the Mb copolymer mixture. Empty polymersomes may be prepared by diluting the stock copolymer solution in PB, instead of purified Mb solution, to yield a copolymer concentration of 12.5 mg/mL. For the 1 mL volume manual extrusion method, the Mb-copolymer/copolymer mixture may be extruded 20 times through either 100 nm or 200 nm diameter polycarbonate membranes (Avanti PolarLipids, Alabaster, AL). However, for the large scale Hollow Fiber (HF) extrusion method (Figures 4 and 5), the Mb-copolymer/copolymer mixture may be extruded through a 0.2 $\mu$m HF membrane (Spectrum Laboratories Inc., Rancho Dominguez, CA). For both extrusion methods, extruded PEM dispersions may be dialyzed overnight using 300 kDa molecular weight cutoff (MWCO) dialysis bags (Spectrum Laboratories Inc., Rancho Dominguez, CA) in PB at 4 °C at a 1:1000 Volume/Volume ration (v/v)(extruded PEM/PB) ratio to remove unencapsulated Mb from the vesicular dispersion. An Eclipse asymmetric flow field-flow fractionator (Wyatt Technology Corp., Santa Barbara,CA) coupled in series to an 18 angle Dawn Heleos multi-angle static light scattering photometer (Wyatt Technology Corp., Santa Barbara, CA) may be used to measure the size distribution of empty polymersomes and PEM particles. The light scattering photometer is equipped with a 30 mW GaAs laser operating at a laser wavelength of 658 nm. Light scattering spectra may be analyzed using the ASTRA software package (Wyatt Technology Corp., Santa Barbara, CA) to calculate the particle size distribution. The elution buffer consisted of 10 mM PB at pH 7.3.

It should be noted that while diameter rages are given above, the final diameter of polycarbonate membrane through which polymersomes are extruded will define the ultimate size distribution (diameter) of the polymersomes in that suspension.

Mb Encapsulation in PEM: To measure the amount of Mb that was encapsulated inside PEM particles, dialyzed PEM dispersions were first lysed using 0.5% v/v Triton X100 (Sigma-Aldrich, St. Louis, MO) in PB. Lysed PEM samples may be centrifuged at 14,000 rpm for 15 min, and the supernatant collected for analysis. The concentration of encapsulated Mb obtained after lysing the PEM particles (mg/mL) may be measured using the Bradford method via the Coomassie Plus protein assay kit (Pierce Biotechnology, Rockford, IL).

As a consequence of the reaction of two or more of the polymerizable groups facilitated by the initiator, stabilized PEM dispersions may be generated via formation of covalent bonds between chains of the copolymers forming the polymersome membranes. These stabilized PEM constructs may be further dried via well-established lyophilization protocols without disrupting the formed polymersome structure or losing the encapsulated Mb. In various embodiments, the polymersomes are administered in the aqueous solution. If lyophilized, in various embodiments, the polymersomes are reconstituted in an appropriate aqueous solution and administered to a subject. Lyophilized biodegradable PEM may be stored in a dessicator (free of $O_2$) at 4 °C for varying periods of time without polymer or Mb degradation as the dried suspensions are free of aqueous free radicals, protons, etc. The polymersomes may be rehydrated at point-of-care prior to delivery.

To generate stabilized polymersomes, polymerizable units may be chemically linked to either the hydrophilic or hydrophobic ends of the copolymer after synthesis. One or more cross-links between multiblock copolymer chains may be formed between the polymerizable units and the hydrophilic or hydrophobic polymers of the various embodiments. These cross-links may be suitably formed by introducing a composition having multiple polymerizable groups to the chains of multiblock copolymer, although in various cases, the multiblock copolymer itself includes multiple polymerizable groups. In various embodiments, the multiple polymerizable groups are chosen from acrylates, methacrylates, acrylamides, methacrylamides, vinyls, vinyl sulfone units or a combination thereof. In various embodiments, the oxygen carriers, nanoparticle and/or polymersomes comprise the polymerizable groups from about 0 weight (wt) % to about 5 wt % of the total weight of the composition. In various embodiments, the oxygen carriers, nanoparticle and/or polymersomes comprise the polymerizable groups from about 5 wt % to about 10 wt % of the total weight of the composition. In various embodiments, the oxygen carriers, nanoparticle and/or polymersomes comprise the polymerizable groups from about 10 wt % to about 20 wt % of the total weight of the composition. In various embodiments, the oxygen carriers, nanoparticle and/or polymersomes comprise the polymerizable groups from about 20 wt % to about 30 wt % of the total weight of the composition. In various embodiments, the oxygen carriers, nanoparticle and/or polymersomes comprise the polymerizable groups from about 30 wt % to about 40 wt % of the total weight of the composition. In various embodiments, the oxygen carriers, nanoparticle and/or polymersomes comprise the polymerizable groups from about 40 wt % to about 50 wt % of the total weight of the composition. In various embodiments, the oxygen carriers, nanoparticle and/or polymersomes comprise the polymerizable groups from about 50 wt % to about 60 wt% of the total weight of the composition. In various embodiments, the oxygen carriers, nanoparticle and/or polymersomes comprise the polymerizable groups from about 60 wt % to about 70 wt % of the total weight of the composition. In various embodiments, the oxygen carriers or the polymersomes comprise the polymerizable groups from about 70 wt % up to about 80 wt % of the total weight of the composition. In various embodiments, the oxygen carriers, nanoparticle and/or polymersomes comprise the polymerizable groups from about 80 wt % up to about 90 wt % of the total weight of the composition. In various embodiments, the oxygen carriers, nanoparticle and/or polymersomes comprise the polymerizable groups from about 90 wt % up to about 95 wt % of the total weight of the composition. In various embodiments, the oxygen carriers, nanoparticle and/or polymersomes comprise the polymerizable groups from about 95 wt % up to about 100 wt % of the total weight of the composition.Cross-linking between chains of a membrane is achieved via activation of the polymerization reaction by an initiator and results in enhancing the rigidity of the polymersome composition. In certain embodiments, the polymerizable group may be conjugated to copolymer's hydrophilic block consisting of either poly(ethylene oxide), poly(ethylene glycol), poly(acrylic acid), and the like. In other embodiments, the polymerizable group may be conjugated to the copolymer's hydrophobic block consisting of either poly(ε-caprolactone), poly(γ-methyl ε-caprolactone), poly(trimethylcarbonate), poly(menthide), poly(lactide), poly(glycolide), poly(methylglycolide), poly(dimethylsiloxane), poly(isobutylene), poly(styrene), poly(ethylene), poly(propylene oxide), etc. The initiator may be a molecule that generates/reacts to heat, light, pH, solution ionic strength, osmolarity, pressures, etc. In various embodiments, the initiator may be photoreactive and cross-links the polymers of the oxygen carrier or polymersome via exposure to ultraviolet light.

The compositions of the various embodiments may be prepared without the use of organic solvents. The compositions of the various embodiments may include polymersomes comprising poly(ethylene oxide)-block-poly(ε-caprolactone), poly(ethylene oxide)-block-poly(γ-methyl ε-caprolactone) and/or), and/or poly(ethylene oxide)-block-poly(trimethylcarbonate) copolymers that have been modified with an acrylate moiety at the hydrophobic block terminus. In various embodiments, the oxygen carriers, nanoparticle and/or polymersomes may comprise cross-linked polymers formed between the hydrophobic block terminus and a diacrylate using a UV initiator, such as 2,2-dimethoxy-2-phenylacetophenone (DMPA). In various embodiments, DMPA is compartmentalized in the polymersome membrane during polymersome assembly while Mb occupies the internal aqueous compartment of the carrier.

The composition of the various embodiments may also comprise polymersomes, nanoparticles or oxygen carriers that have increased degradative half-lives. Circulation times of oxygen carrier and polymersomes may be generally limited to hours (or up to one day) because of either rapid clearance by the mononuclear phagocytic system (MPS) of the liver and spleen, or by excretion. Clinical studies have shown that circulation times of spherical carriers may be generally extended threefold in humans over rats. As proposed for clinically used drug formulations of PEG-liposomes, oxygen carriers and polymersomes with long circulating lifetime may increase the drug exposure to cancer cells, low oxygenated tissues, or healing wounds, and thereby increase the time-integrated dose, commonly referred to in drug delivery as "the area under the curve." Additionally, the enhanced permeation and retention effect that allows small solutes and micelles to permeate the leaky blood vessels of a rapidly expanding tumor might also allow oxygen carriers and polymersomes to transport into the tumor stroma. Persistent circulation of the oxygen carriers and polymersomes has many practical applications because these vehicles can increase exposure of drugs to cancer cells, low or poor oxygenated tissues, or healing wounds.

The compositions of the various embodiments may comprise polymersomes, nanoparticles or oxygen carriers that have increased circulatory half-lives. In various embodiments, the compositions have a certain percent mass composition of polymer designed to have a circulatory half-life from about 12 hours to about 36 hours, and a degradative half-life from about 38 to about 60 hours.

In various embodiments, the compositions have a certain percent mass composition of polymer designed to have circulatory half-life about 12 hours less than the degradative half-life of the oxygen-carrier or polymersome. This delay in degradation may vary depending upon the route of administration and/or the targeted micro-compartment, the size of the oxygen-carrier or polymesome or subcellular microenvironment where the polymersome or oxygen carrier deploys its contents for treatment or prevention of the disease states or disorders disclosed herein. In various embodiments, the compositions comprising polymersomes, nanoparticles or oxygen carriers have circulatory half-life about 11 hours less than the degradative half-life of the oxygen-carrier or polymersome. In various embodiments, the compositions comprising polymersomes, nanoparticles or oxygen carriers have circulatory half-life about 10 hours less than the degradative half-life of the oxygen-carrier or polymersome. In various embodiments, the compositions comprising polymersomes, nanoparticles or oxygen carriers have circulatory half-life about 9 hours less than the degradative half-life of the oxygen-carrier or polymersome. In various embodiments, the compositions comprising polymersomes, nanoparticles or oxygen carriers have circulatory half-life about 8 hours less than the degradative half-life of the oxygen-carrier or polymersome. In various embodiments, the compositions comprising polymersomes, nanoparticles or oxygen carriers have circulatory half-life about 7 hours less than the degradative half-life of the oxygen-carrier or polymersome. In various embodiments, the compositions comprising polymersomes, nanoparticles or oxygen carriers have circulatory half-life about 6 hours less than the degradative half-life of the oxygen-carrier or polymersome. In various embodiments, the compositions comprising polymersomes, nanoparticles or oxygen carriers have circulatory half-life about 5 hours less than the degradative half-life of the oxygen-carrier or polymersome. In various embodiments, the compositions comprising polymersomes, nanoparticles or oxygen carriers have circulatory half-life about 4 hours less than the degradative half-life of the oxygen-carrier or polymersome. In various embodiments, the compositions comprising polymersomes, nanoparticles or oxygen carriers have circulatory half-life about 3 hours less than the degradative half-life of the oxygen-carrier or polymersome. In various embodiments, the compositions comprising polymersomes, nanoparticles or oxygen carriers have circulatory half-life about 2 hours less than the degradative half-life of the oxygen-carrier or polymersome. In various embodiments, the compositions comprising polymersomes, nanoparticles or oxygen carriers have circulatory half-life about 1 hours less than the degradative half-life of the oxygen-carrier or polymersome. In various embodiments, the compositions comprising polymersomes, nanoparticles or oxygen carriers have circulatory half-life about 14 hours less than the degradative half-life of the oxygen-carrier or polymersome. In various embodiments, the compositions comprising polymersomes, nanoparticles or oxygen carriers have circulatory half-life from about 1 hour to about 20 hours less than the degradative half-life of the oxygen-carrier or polymersome. In various embodiments, the compositions comprising polymersomes, nanoparticles or oxygen carriers have a certain percent mass composition of polymer designed to have a circulatory half-life of about 36 hours, and a degradative half-life greater than about 48 hours. In various embodiments, the compositions comprising polymersomes, nanoparticles or oxygen carriers have a certain percent mass composition of polymer designed to have a circulatory half-life from about 24 hours to about 36 hours, and a degradative half-life from about 38 to about 60 hours. In various embodiments, the compositions comprising polymersomes, nanoparticles or oxygen carriers have a certain percent mass composition of polymer designed to have a circulatory half-life from about 28 hours to about 36 hours, and a degradative half-life from about 38 to about 60 hours. In various embodiments, the compositions comprising polymersomes, nanoparticles or oxygen carriers have a certain percent mass composition of polymer designed to have a circulatory half-life from about 30 hours to about 36 hours, and a degradative half-life from about 38 to about 60 hours. In various embodiments, the compositions comprising polymersomes, nanoparticles or oxygen carriers have a certain percent mass composition of polymer designed to have a circulatory half-life of no more than 36 hours, and a degradative half-life from about 38 to about 60 hours.

In various embodiments, the degradation half-life is 6 hours greater than the circulatory half-life. In various embodiments, the degradation half-life is between 6 hours and 24 hours greater than the circulatory half-life. In various embodiments, the degradation half-life is more than 24 hours greater than the circulatory half-life. In various embodiments, the degradation half-life is about 6 hours greater than the circulatory half-life. In various embodiments, the degradation half-life is about 7 hours greater than the circulatory half-life. In various embodiments, the degradation half-life is about 8 hours greater than the circulatory half-life. In various embodiments, the degradation half-life is about 9 hours greater than the circulatory half-life. In various embodiments, the degradation half-life is about 10 hours greater than the circulatory half-life. In various embodiments, the degradation half-life is about 11 hours greater than the circulatory half-life. In various embodiments, the degradation half-life is about 12 hours greater than the circulatory half-life. In various embodiments, the degradation half-life is about 13 hours greater than the circulatory half-life. In various embodiments, the degradation half-life is about 14 hours greater than the circulatory half-life. In various embodiments, the degradation half-life is about 15 hours greater than the circulatory half-life. In various embodiments, the degradation half-life is about 16 hours greater than the circulatory half-life. In various embodiments, the degradation half-life is about 17 hours greater than the circulatory half-life. In various embodiments, the degradation half-life is about 18 hours greater than the circulatory half-life. In various embodiments, the degradation half-life is about 19 hours greater than the circulatory half-life. In various embodiments, the degradation half-life is about 20 hours greater than the circulatory half-life. In various embodiments, the degradation half-life is about 21 hours greater than the circulatory half-life. In various embodiments, the degradation half-life is about 22 hours greater than the circulatory half-life. In various embodiments, the degradation half-life is about 23 hours greater than the circulatory half-life. In various embodiments, the degradation half-life is about 24 hours greater than the circulatory half-life. In various embodiments, the degradation half-life is about 36 hours greater than the circulatory half-life. In various embodiments, the degradation half-life is about 48 hours greater than the circulatory half-life. In various embodiments, the degradation half-life is about 60 hours greater than the circulatory half-life. In various embodiments, the degradation half-life is about 72 hours greater than the circulatory half-life. In various embodiments, the degradation half-life is more than 96 hours greater than the circulatory half-life.

In various embodiments, *in vivo* delivery is achieved by intravenous, inhalational, transmucosal (e.g. buccal) or transcutaneous routes of administration. Dosages for a given host may be determined using conventional considerations, e.g., by customary comparison of the differential activities of the subject preparations and a known appropriate, conventional pharmacological protocol.

In an embodiment, different final concentrations of PEMs may be used in order to test the effects of Mb dose on improving oxygenation and mitigating tumor hypoxia. 100 uL injections of PEMs that contain either 90 or 180 mg Mb/mL may result in 450 or 900 mg/kg injection doses of Mb, respectively, assuming a 20 g mouse. These doses correspond to the total hemoglobin injection dose found in 0.5 and 1 unit of whole blood, assuming 15 g/dL blood concentrations, 450 mL blood/unit, and a 70 kg human. While larger PEM doses may likely enhance Mb tumor delivery, increased amounts of free Mb (released during PEM degradation) may also result in local NO uptake, decreased microperfusion, and ineffective oxygenation.[175] In a preferred embodiment, the associated polymer concentrations and subject injection doses may range between 2.5-18 mg/mL and 12.5-90 mg/kg, assuming a final weight ratio of encapsulated Mb:polymer ranging between 10-35, both of which fall well within the range of previous animal studies that demonstrated no subacute or acute in vivo toxicities from various polymersome compositions.[111, 122, 164, 168, 171]

The pharmaceutical composition of the various embodiments may be an oxygen carrier that possess different "loading ratios" of oxygen binding agents to inert vehicle. In various embodiments, the pharmaceutical composition comprises < 5 mg oxygen binding agent/mg inert vehicle. In various embodiments, the pharmaceutical composition comprises from about 5 to about 40 mg oxygen binding agent/mg inert vehicle. In various embodiments, the pharmaceutical composition comprises from about 10 to about 40 mg oxygen binding agent/mg polymer inert vehicle. In various embodiments, the pharmaceutical composition comprises from about 20 to about 40 mg oxygen binding agent/mg inert vehicle. In various embodiments, the pharmaceutical composition comprises from about 30 to about 40 mg oxygen binding agent /mg inert vehicle. In various embodiments, the pharmaceutical composition comprises from about 35 to about 40 mg oxygen binding agent/mg inert vehicle. In various embodiments, the pharmaceutical composition comprises from about 25 to about 40 mg oxygen binding agent/mg inert vehicle. In various embodiments, the pharmaceutical composition comprises from about 25 to about 35 mg oxygen binding agent/mg inert vehicle. In various embodiments, the pharmaceutical composition comprises from about 25 to about 30 mg oxygen binding agent/mg inert vehicle. In various embodiments, the pharmaceutical composition comprises from about 20 to about 25 mg oxygen binding agent/mg inert vehicle. In various embodiments, the pharmaceutical composition comprises from about 10 to about 15 mg oxygen binding agent/mg inert vehicle.

In various embodiments, the pharmaceutical composition comprises from about 5 to about 35 mg Mb/mg polymer. In various embodiments, the pharmaceutical composition comprises from about 10 to about 35 mg Mb/mg polymer. In various embodiments, the pharmaceutical composition comprises from about 20 to about 35 mg Mb/mg polymer. In various embodiments, the pharmaceutical composition comprises from about 30 to about 35 mg Mb/mg polymer. In various embodiments, the pharmaceutical composition comprises from about 25 to about 35 mg Mb/mg polymer. In various embodiments, the pharmaceutical composition comprises from about 25 to about 30 mg Mb/mg polymer. In various embodiments, the pharmaceutical composition comprises from about 20 to about 25 mg Mb/mg polymer. In various embodiments, the pharmaceutical composition comprise from about 10 to about 15 mg Mb/mg polymer. In various embodiments, the pharmaceutical composition comprise from about 5 to about 10 mg Mb/mg polymer. In various embodiments the Mb dosages may be replaced by the same weight of Mb.

In various embodiments, the pharmaceutical composition is a liquid formulation, wherein the dosage may be from about 1 unit of compositions to about 50 units of oxygen-carrier suspension, wherein a unit of suspension comprises from about 40 g of Mb to about 85 g of Mb.

In an embodiment, the high-oxygen affinity agent/compound has a P50 for oxygen that is less than 25 mmHg. In an embodiment, the high-oxygen affinity agent/compound has a P50 for oxygen that is less than 20 mmHg. In an embodiment, the high-oxygen affinity agent/compound has a P50 for oxygen that is less than 15 mmHg. In an embodiment, the high-oxygen affinity agent/compound has a P50 for oxygen that is less than 10 mmHg. In an embodiment, the high-oxygen affinity agent/compound has a P50 for oxygen that is less than 5 mmHg.

In various embodiments, a unit of a liquid formulation comprising the pharmaceutical composition comprises about 41 grams of Mb. In various embodiments, a unit of a liquid formulation comprising the pharmaceutical composition comprises about 45 grams of Mb. In various embodiments, a unit of a liquid formulation comprising the pharmaceutical composition comprises about 50 grams of Mb. In various embodiments, a unit of a liquid formulation comprising the pharmaceutical composition comprises about 55 grams of Mb: In various embodiments, a unit of a liquid formulation comprising the pharmaceutical composition comprises about 60 grams of Mb. In various embodiments, a unit of a liquid formulation comprising the pharmaceutical composition comprises about 65 grams of Mb. In various embodiments, a unit of a liquid formulation comprising the pharmaceutical composition comprises about 70 grams of Mg. In various embodiments, a unit of a liquid formulation comprising the pharmaceutical composition comprises about 75 grams of Mb. In various embodiments, a unit of a liquid formulation comprising the pharmaceutical composition comprises about 80 grams of Mb. In various embodiments, a unit of a liquid formulation comprising the pharmaceutical composition comprises about 85 grams of Mb.

Generally, a pharmaceutical composition according to the various embodiments may comprise a dose of an oxygen-binding protein that is suspended within a solution and administered in units, where a unit is equal to 81 grams of oxygen-binding protein. If a subject undergoes surgery or experiences blood loss, the pharmaceutical composition may be administered to the subject according to the following dosing regimen, where blood is replaced with units of liquid formulation: In various embodiments, the pharmaceutical composition comprises from about 40 g of oxygen binding protein/unit of solution administered to about 81 g of oxygen binding protein/unit of solution administered.

| Examples Of Blood Use | Average # Unites Required per Patient |
|---|---|
| Automobile Accident | 50 units of blood |
| Heart Surgery | 6 units of blood<br>6 units of platelets |
| Organ Transplant | 40 units of blood<br>30 units of platelets<br>20 bags of cryoprocipitate<br>25 units of fresh frozen plasma |
| Bone Marrow Transplant | 120 units of platelets<br>20 units of blood |

In various embodiments, the pharmaceutical composition comprises a dose from about 40 g of Mb/unit of solution administered to about 80 g of Mb/unit of solution administered. In various embodiments, the pharmaceutical composition comprises a dose from about 50 g of Mb/unit of solution administered to about 80 g of Mb/unit of solution administered. In various embodiments, the pharmaceutical composition comprises a dose from about 60 g of Mb/unit of solution administered to about 80 g of Mb/unit of solution administered. In various embodiments, the pharmaceutical composition comprises a dose from about 70 g of Mb/unit of solution administered to about 80 g of Mb/unit of solution administered. In various embodiments, the pharmaceutical composition comprises a dose from about 60 g of Mb/unit of solution administered to about 70 g of Mb/unit of solution administered.

The dose of the pharmaceutical composition of the various embodiments may also be measured in grams of polymersome administered per kg of a subject. In various embodiments, the total dose administered comprises from about 12.5 mg of polymer to about 90 mg of polymer per kg of a subject. In various embodiments, the total dose administered comprises from about 15 mg of polymer to about 90 mg of polymer per kg of a subject. In various embodiments, the total dose administered comprises from about 25 mg of polymer to about 90 mg of polymer per kg of a subject. In various embodiments, the total dose administered comprises from about 35 mg of polymer to about 90 mg of polymer per kg of a subject. In various embodiments, the total dose administered comprises from about 45 mg of polymer to about 90 mg of polymer per kg of a subject. In various embodiments, the total dose administered comprises from about 55 mg of polymer to about 90 mg of polymer per kg of a subject. In various embodiments, the total dose administered comprises from about 65 mg of polymer to about 90 mg of polymer per kg of a subject. In various embodiments, the total dose administered comprises from about 75 mg of polymer to about 90 mg of polymer per kg of a subject. In various embodiments, the total dose administered comprises from about 80 mg of polymer to about 90 mg of polymer per kg of a subject. In various embodiments, the total dose administered comprises from about 85 mg of polymer to about 90 mg of polymer per kg of a subject.

In various embodiments, the pharmaceutical composition is a liquid formation that comprises an allosteric effector such as 2,3-Bisphosphoglycerate, wherein the formulation comprises from about 1 to about 100 mmol/L of formulation. In various embodiments, the formulation comprises from about 1 to about 100 mmol of a isomer of 2,3-Bisphosphoglycerate per L of formulation. In various embodiments, the formulation comprises from about 1 to about 10 mmol of a isomer of 2,3Bisphosphoglycerate per L of formulation. In various embodiments, the formulation comprises about 5 mmol of 2,3-Bisphosphoglycerate or isomer derived thereof per L of formulation. In various embodiments, the formulation comprises about 2.25 mmol of 2,3-Bisphosphoglycerate or isomer derived thereof per Unit (450 mL) of formulation.

The pharmaceutical compositions may be prepared, packaged, or sold in the form of a sterile, injectable, aqueous or oily suspension or solution. This suspension or solution may be formulated according to the known art, and may comprise, in addition to the active ingredient, additional ingredients such as the dispersing agents, wetting agents, or suspending agents described herein. Such sterile injectable formulations may be prepared using a non-toxic parenterally acceptable diluent or solvent, such as water or 1,3 butane diol, for example. Other acceptable diluents and solvents include, but are not limited to, Ringer's solution, isotonic sodium chloride solution, and fixed oils such as synthetic mono or di-glycerides. Other parentally-administrable formulations which are useful include those which comprise the active ingredient in microcrystalline form, in a liposomal preparation, or as a component of biodegradable polymer systems. Compositions for sustained release or implantation may comprise pharmaceutically acceptable polymeric or hydrophobic materials such as an emulsion, an ion exchange resin, a sparingly soluble polymer, or a sparingly soluble salt. The formulations described herein, are also useful for pulmonary delivery and the treatment of such cancers of the respiratory system or lung, are also useful for intranasal delivery of a pharmaceutical composition of the various embodiments. Such formulation suitable for intranasal administration is a coarse powder comprising the active ingredient and having an average particle from about 0.2 to 500 micrometers, administered by rapid inhalation through the nasal passage from a container of the powder held close to the nares.

The various embodiment pharmaceutical compositions may be administered to deliver a dose of from about 0.1 g/kg/day to about 100 g/kg/day, where the gram measurement is equal to the total weight of Mb and polymer in the pharmaceutical composition. In various embodiments, the dosage is from about 0.1 to 1 g/kg/day. In another embodiment, the dosage is from about 0.5 g/kg/day to about 1.0 g/kg/day. In another embodiment, the dosage is from about 1.0 g/kg/day to about 1.5 g/kg/day. In another embodiment, the dosage is from about 1.5 g/kg/day to about 2.0 g/kg/day. In another embodiment, the dosage is from about 2.5 g/kg/day to about 3.0 g/kg/day. In another embodiment, the dosage is 1.0, 2.0, 5.0, 10, 15, 20, 25, 30, 35, 40, 45, or 50 g/kg/day, where the gram measurement is equal to the total weight of Mb and polymer in the pharmaceutical composition. In one embodiment, administration of a dose may result in a therapeutically effective concentration of the drug, protein, active agent, etc., between 1 $\mu$M and 10 $\mu$M in a diseased or cancer-affected tissue, or tumor of a mammal when analyzed *in vivo.*

In an embodiment, a pharmaceutical composition, especially one used for prophylactic purposes, can comprise, in addition, a pharmaceutically acceptable adjuvant filler or the like. Suitable pharmaceutically acceptable carriers are well known in the art. Examples of typical carriers include saline, buffered saline and other salts, lipids, and surfactants.

The oxygen carrier or polymersome may also be lyophilized and administered in the forms of a powder. Taking appropriate precautions not to denature any protein component disclosed herein, the preparations can be sterilized and if desired mixed with auxiliary agents, e.g., lubricants, preservatives, stabilizers, wetting agents, emulsifiers, salts for influencing osmotic pressure, buffers, and the like that do not deleteriously react with the oxygen carrier or polymersome discussed herein. They also can be combined where desired with other biologically active agents, e.g., antisense DNA or mRNA.

A pharmaceutical composition of the various embodiments may be prepared, packaged, or sold in bulk, as a single unit dose, or as a plurality of single unit doses. The amount of the active ingredient is generally equal to the dosage of the active ingredient which would be administered to a subject, or a convenient fraction of such a dosage, such as, for example, one-half or one-third of such a dosage, as would be known in the art.

The relative amounts of the active ingredient, the pharmaceutically acceptable carrier, and any additional ingredients in a pharmaceutical composition of the various embodiments may vary, depending upon the identity, size, and condition of the subject treated and further depending upon the route by which the composition is to be administered. By way of example, the composition may comprise from about 0.1% to about 100% (w/w) active ingredient.

The compositions and methods described herein may be useful for preventing or treating cancer or any blood disorder including but not necessarily limited to anemia, wherein a blood disorder causes low or poor oxygenation of tissues in a subject. In various embodiment the composition and methods described herein may be used in treatment of cancer in conjunction with radiation therapy.

The compositions and methods described herein can be useful for preventing the dissemination or improving the chemotherapy and/or radiation therapy of cancers including leukemias, lymphomas, meningiomas, mixed tumors of salivary glands, adenomas, carcinomas, adenocarcinomas, sarcomas, dysgerminomas, retinoblastomas, Wilms' tumors, neuroblastomas, melanomas, and mesotheliomas; as represented by a number of types of cancers, including but not limited to breast cancer, sarcomas and other neoplasms, bladder cancer, colon cancer, lung cancer, pancreatic cancer, gastric cancer, cervical cancer, ovarian cancer, brain cancers, various leukemias and lymphomas. One would expect that any other human tumor cell, regardless of expression of functional p53, would be subject to treatment or prevention by the methods discussed herein, although the particular emphasis is on mammary cells and mammary tumors. The various embodiments may also encompass a method of treatment, according to which a therapeutically effective amount of the drug, protein, active agent, etc., or a vector comprising same according to the various embodiments may be administered to a patient requiring such treatment. The various embodiments should not be construed as being limited solely to these examples, as other cancer-associated diseases which are at present unknown, once known, may also be treatable using the methods of the various embodiments.

Also useful in conjunction with the methods provided in the various embodiments may be chemotherapy, phototherapy, anti-angiogenic or irradiation therapies, separately or combined, which may be used before, contemporaneously, or after the enhanced treatments discussed here, but will be most effectively used after the cells have been sensitized by the present methods. As used herein, the phrase "chemotherapeutic agent" means any chemical agent or drug used in chemotherapy treatment, which selectively affects tumor cells, including but not limited to, such agents as adriamycin, actinomycin D, camptothecin, colchicine, taxol, cisplatinum, vincristine, vinblastine, and methotrexate. Other such agents are well known in the art.

The various embodiments may include methods for stimulating wound healing in a subject in need thereof comprising administering the oxygen carrier or polymersome of the various embodiments to a subject in need thereof. Some embodiments may include methods for treating or preventing diseases, illnesses or conditions in mammals. In various embodiments, the compositions of the various embodiments may be used for canine anemia. In various embodiments, the compositions of the various embodiments may be useful to treat or prevent symptoms associated with iron deficiency. Some embodiments may provide methods for treating a blood disorder or low oxygenation of tissues in patients susceptible to, symptomatic of, or at elevated risk for developing hypertension.

The various embodiments may also include kits comprising any of the aforementioned compositions or pharmaceutical compositions comprising an oxygen carrier or a polymersome, wherein the oxygen carrier or a polymersome comprises at least one biocompatible polymer and at least one biodegradable polymer. According to various embodiments, the formulation may be supplied as part of a kit. The kit may comprise the pharmaceutical composition comprising an oxygen carrier or a polymersome. In another embodiment, the kit may comprise a lyophilized oxygen carrier or polymersome with an aqueous rehydration mixture. In another embodiment, the oxygen carrier or polymersome may be in one container while the rehydration mixture is in a second container. The rehydration mixture may be supplied in dry form, to which water may be added to form a rehydration solution prior to administration by mouth, venous puncture, injection, or any other mode of delivery. In various embodiments, the kit may further comprise a vehicle for administration of the composition such as tubing, a catheter, syringe, needle, and/or combination of any of the foregoing.

While nanoparticles may be used to increase intratumoral $pO_2$ in order to increase the efficacy of radiation and chemotherapies as discussed above, in some embodiments various nanoparticle formulations may be designed for tumor reduction through alternative mechanisms. A particular of such alternative mechanisms is a unique process of "nanoparticle-mediated microvascular embolization" (NME). In NME processes, nanoparticles may be used to selectively deliver vasoactive substances to tumors for cutting off blood supply in a manner that is not reliant upon the expression of molecular angiogenic targets.

Current tumor embolization attempts generally involve techniques that are performed by trained interventional radiologists and involve transcatheter arterial chemoembolization (TACE) of hepatocellular carcinoma (HCC) or of liver-dominant metastases from other primary malignancies. While such techniques typically induce direct tumor necrosis in more than half of patients, the infusion agents that are used may distribute only amongst branching vessels in the tumor and be excluded from the collateral microvascular circulation. Therefore, TACE is highly effective for temporary, palliative and localized treatment but cannot be administered systemically to treat all areas of tumor in patients with metastatic disease. Moreover, TACE may also be associated with a self-limiting post-embolization syndrome (pain, fever, and malaise), chemical hepatitis, and a leukemoid reaction due to necrosis and release of cytokines from hepatocytes and embolized tumor cells. Further, conventional agents for tumor embolization are typically polymeric microparticles embedded with chemotherapies. Such agents are not amenable to systemic administration because their sizes and toxic payloads promote severe damage to the vascular beds of vital organs (e.g. liver, heart, lungs, kidneys, etc).

The vasoactivity of HBOCs is generally attributed at least in part to infiltration of modified iron-containing hemoglobin into the endothelium of blood vessels, resulting in the consumption of nitric oxide (NO). The treatment techniques of the various embodiments may involve encapsulating molecules that are capable of binding nitric oxide ("NO-binding molecules"), such as myoglobin, within the aqueous cavities of nanoparticles, for example, polymersomes. Such encapsulated NO-binding molecules may be the same as or different from those that also have a high affinity for binding oxygen and that are discussed above with respect to tumor oxygenation (e.g., PEM). As discussed above, NO is a paracrine signaling factor that promotes vasodilation, while NO depletion results in vasoconstriction. Other techniques may involve encapsulating molecules that are capable of inhibiting normal NO activity through other mechanisms. Generally, NO-binding molecules and other NO activity inhibitors may be referred to herein as "NO-inhibiting" or "NO-affecting" molecules. Generally, NO-binding molecules may also bind $O_2$, and therefore the term "NO-binding" molecule may be used herein to refer to an NO- and O-binding molecule.

In various embodiments, encapsulated NO-binding molecules may mediate a tumor through NME effects by local vasoactivity that ensues only after accumulation of the nanoparticles therein. Such accumulation may be due to enhanced permeability and retention ("EPR") from active tumor angiogenesis. In general, the EPR effect is a property describing the tendency of molecules to accumulate in tumor tissue more than they would in normal tissue, which may be based on the creation of abnormal vessels in the rapid angiogenesis stimulated by active tumor growth. Such abnormal vasculature may be dysfunctional, and may be referred to herein as "leaky" vasculature. Further, tumor tissues usually lack effective lymphatic drainage, which together with leaky vasculature may lead to abnormal molecular and fluid transport, i.e., the EPR effect.

The result of NME may be eventual microvascular occlusion and tumor necrosis, but without any systemic vasoactivity. As such, the nanoparticles of the various embodiments may have particular utility for the treatment of the most highly vascular tumors, including renal cell carcinoma (RCC), Hepatocellular carcinoma (HCC), glioblastoma multiforme (GBM), and multiple myeloma (MM). Without wishing to be bound to a particular theory, the introduction of nanoparticles containing myoglobin or another NO-binding molecule into the systemic circulation may selectively induce NME in tumors by selectively delaying the binding of NO until the particles have extravasated via the EPR effect or become lodged within the tumor microvaculature. In various example procedures, NO-binding molecules, such as a myoglobin, may be encapsulated in nanoparticles, such as polymersomes. In some embodiments, additional imaging agents and/or therapeutic molecules may also be encapsulated within the nanoparticles, such as to promote a complementary activity against the tumor. The NO-binding molecule may be delivered to tumors via passive accumulation and/or active targeting by nanoparticles. Upon delivery, the NO-binding molecule may induce NME in the tumor tissue.

Without wishing to be bound by a particular theory, the NME may be based on a number of specific characteristics, properties, and adjustments to previously investigated polymersome encapsulation formulations and other HBOCs. As compared to the free NO-binding molecule, NO sequestration by the encapsulated NO-binding molecule may be significantly delayed in time and/or amount, thereby allowing tumor tissue specificity. Such delay may be based on physical and/or chemical properties of the NO-binding molecule (i.e., binding affinity for NO and/or other molecules, etc.) and of the nanoparticles (i.e., size, type, membrane, etc.). Various non-limiting theories of these properties affecting NO sequestration time are discussed below.

First, the NME effect may be due to the interaction of nanoparticles with NO. Specifically, in some embodiments the molecules encapsulated in nanoparticles may be oxygen carriers that competitively bind both oxygen and NO (e.g., myoglobin). These encapsulated molecules may be preloaded with oxygen, and therefore unable to bind NO until oxygen is released. Some embodiment oxygen carriers may also have particularly high affinities for oxygen binding, providing an additional barrier to NO sequestration while in systemic circulation since NO binding only occurs after deoxygenation. In some embodiments, such deoxygenation may only occur once the encapsulated oxygen carriers are able to accumulate in tumor tissue.

In an example embodiment, the encapsulated oxygen carrier may be PEM. As discussed above, myoglobin has a high affinity (low $P_{50}$) for oxygen, which enables it to bind and retain oxygen to a much greater extent than hemoglobin found in natural red blood cells within the circulation. The specific molecular site (i.e. the iron prophyrin) to which NO binds on myoglobin is occupied by oxygen under physiologic conditions. In such physiologic conditions, which are seen while the nanoparticle is in the systemic circulation, encapsulated myoglobin is therefore unable to bind significant amounts of NO.

**[0149]** Second, the tumor specific NME effect may also be due to sequestration of the NO-binding molecule from the environment due to incorporating within or upon nanoparticles. For example, embodiments in which the nanoparticles that encapsulate oxygen carriers are polymersomes, their synthetic polymer membranes may be significantly thicker than those of other natural vesicles, such as liposomes. Such thick membranes may provide an additional barrier against diffusions of polar gas molecules, including NO, into the aqueous cavities of polymersomes until reaching the hypoxic tumor. As discussed above, other agents may be coencapsulated within the nanoparticle in order to provide complementary activity against a tumor. Examples of such additional agents may include, without limitation, additional chemotherapy agents or biologic agents that inhibit angiogenesis (i.e. the process of molecular signals generated by the tumor that prompt the growth of new blood vessels), thereby enhancing the NME effects.

In some embodiments, molecules can be attached to the surface of nanoparticles that incorporate the NO-binding molecules, which may allow for targeting to particular organs or uptake by specific cells. In some embodiments, the nanoparticles are polymersomes. In some embodiments, the surface molecules may be surface-bound myoglobin, which may be used in addition to or instead of the encapsulated myoglobin.

*Proposed NME Mechanisms:*

Following their administration (e.g. via intraperitoneal or intravenous injection), nanoparticles may accumulate in tumors due to the EPR effect, discussed above. While nanoparticles in circulation may be unable to penetrate through tight endothelial junctions of normal blood vessels, they may selectively extravasate in tumor tissues as a result of the leaky vasculature, in which they may become trapped and accumulate, which may be helped by the lack of effective lymphatic drainage.

Since equilibrium oxygen tension in the tumor may be much lower than that of normal tissue, oxygen bound to the encapsulated NO-binding molecules that are accumulated in tumor tissue may then dissociate and diffuse out of the nanoparticles. In various embodiments, oxygen dissociation from the nanoparticles may occur when the partial pressure of oxygen in the tumor falls below the equilibrium binding pressure (i.e., partial pressure of oxygen required for 50% saturation) of the encapsulated NO-binding molecule. The equilibrium binding pressure in various embodiments is based on the properties/composition of the specific NO-binding molecule.

The deoxygenation of the encapsulated NO-binding molecule may then enable it to bind NO that has passively diffused into the nanoparticles, which may the result in vasoconstriction due to depletion of NO from vascular endothelium. Downstream results of such vasoconstriction due to endothelial NO depletion may include damage to the tumor microvasculature, followed by platelet adhesion, activation, and aggregation resulting in clot formation (i.e., thrombosis). Ultimately, clotting in the tumor microvasculature may lead to local hemostasis (i.e., slowing/stopping blood flow) in the surrounding tumor tissue, thereby creating a persistent hydrodynamic pressure in tumor capillaries. As a result, the newly formed clot may rupture and cause bleeding into the body of the tumor (i.e., hemorrhage). Subsequent to the hemorrhage, complete thrombosis of the tumor capillary bed may progress to stop all blood flow to the tumor, including further delivery of red blood cells. In this manner, the NME effect may lead to necrosis of tumor cells.

*Alternatives to NO-binding Molecules:*

In various embodiments, other molecules that affect NO inhibition may be encapsulated in the nanoparticles in addition to, or instead of, o oxygen carriers that bind NO-binding molecules. Such agents may include compounds that inhibit NO production (e.g. NO synthase (NOS) inhibitors), compounds that scavenge NO radicals, and commercially-available NO-binding reagents.

NO is produced enzymatically by three different NOSs: Neuronal NOS (nNOS) and endothelial NOS (eNOS) are constitutive enzymes important for homeostatic processes, such as neurotransmission and vascular tone, respectively, and inducible NOS (iNOS), which is normally not expressed, but rather is synthesized *de novo* in response to inflammation. Thus, various embodiments nanoparticles may be created that encapsulate nNOS inhibitors, eNOS inhibitors, and/or iNOS inhibitors. Further, since NOS enzymes make NO from L-arginine, competitive L-arginine analogues may prevent the NOS enzymes from producing NO, and may also be encapsulated in some embodiment nanoparticles. These encapsulated analogues may include, but are not limited to, NG-monomethyl-L-arginine (L-NMMA), Nω-nitro-L-arginine (L-NNA), and NG-Nitroarginine methyl ester (L-NAME).

NO-scavenging compounds may be encapsulated and used in the various embodiment nanoparticle formulations. Such compounds may include, but are not limited to, nitronyl nitroxides (e.g., carboxy-PTIO), dithiocarbamate derivatives, a chemically modified human-derived hemoglobin conjugate pyridoxalated hemoglobin polyoxyethylene (PHP), etc. Additional NO-scavenging compounds that are specific for NO radicals may be developed in the future for use in the various embodiments.

Further, commercially available NO-binding reagents may be encapsulated in nanoparticles for use in the various embodiment nanoparticle formulations. Such reagents may be those that have shown to induce some degree of NO sequestration for other purposes/treatments. Examples of such NO-binding reagents may include small molecule NO-inhibitors and low molecular weight nitric oxide metabolite compounds developed by Medinox, Inc. for treatment of septic shock, type-2 diabetes, arthritis and other inflammatory diseases, and sickle cell anemia.

Polymersome Alternatives (not part of the claimed invention):

In various embodiments, nanoparticle formulations other than polymersomes may be used instead of or in addition to polymersomes to encapsulate the NO-binding or NO-affecting, molecules. Further, a variety of commercially available acellular HBOCs may be used in addition, or as alternatives, to developing the encapsulated NO-binding molecules. These commercially available HBOCs may be, without limitation, polymerized hemoglobin products (e.g., PolyHeme™ by Northfield Laboratories Inc., USA, Hemopure™ and Oxyglobin™ by Biopure, Inc., USA), conjugated hemoglobin products (e.g., Hemospan™ by Sangart Inc., USA), which may have larger overall particles based on the surface modification with inert polymers (e.g., polyethylene glycol (PEG, with a molecular weight greater than 5 kDa)), and/or cross-linked hemoglobin products (e.g., HemoAssist™ by Baxter, Optro™ by Somatogen, etc.). However, since they lack the ability to autoregulate the oxidative state of iron in their heme groups, iron in HBOCs may freely convert from the ferrous to the ferric state, creating a larger than normal amount of methemoglobin.

Further, in some embodiments, microparticles and/or nanoparticles that encapsulate oxygen carriers/NO-binding or NO-affecting molecules may be modified by surface glycoproteins, nucleic acids, or small molecules that mediate immune recognition. In an example, CD47, which mediates mononuclear phagocytic system (MPS) (primarily the liver and spleen) via engagement of the CD172 a receptor, may be conjugated onto nanoparticle surfaces.

Various other commercially available nanoparticles that may be used to encapsulate an NO-inhibiting/NO-affecting molecule instead of the nanoparticle formulations discussed above may include, without limitation, Abraxane® (i.e., a nanoparticle-albumin bound paclitaxel), Doxil® (i.e., doxorubicin encapsulated by liposomes), and/or various other nanoparticles that have been developed specifically for cancer therapy.

Specific Applications for NME:

In various embodiments, the use of the encapsulated NO-inhibiting/NO-effecting molecules (e.g., PEM) to cause NME may be especially effective for highly vascularized tumors, such as RCC, HCC, GBM, and MM, all of which are cancers with unsatisfactory treatment options. In some embodiments, PEM may be effective with many or all solid tumors, including vascular tumors with poor treatment options, such as ovarian cancer, non-small cell lung cancer, breast cancer and colon cancer. In various embodiments, the use of encapsulated NO-affecting molecules to cause NME may use the PEM constructs that are described above with respect to tumor oxygenation to augment radiation and/or chemotherapy.

Deregulation of angiogenesis is a key pathophysiologic factor in the development of highly vascularized tumors, such as RCC tumors. In particular, conventional or clear cell RCC is the most common histological subtype of kidney cancer and is characterized by somatic loss, secondary to mutation or silencing by methylation, of the von Hippel-Lindau (VHL) tumor suppressor gene. These VHL aberrations lead to the upregulation of hypoxia inducible factor (HIF), a transcription factor that amplifies multiple pro-angiogenic molecules including vascular endothelial growth factor receptor (VEGFR).

Therefore, VEGFR tyrosine kinase inhibitors (TKIs) have emerged as front-line treatments for RCC, such as sunitinib, pazopanib, and sorafenib. Further, mammalian target of rapamycin (mTOR) inhibitors, such as temsirolimus and everolimus, have demonstrated benefits in increasing progression free survival after the development of VEGFR TKI-resistant disease. No combination therapies have resulted in further improvements and have only vastly increased side effects.

First, the efficacy and safety of PEM as a novel therapeutic modality for highly vascular tumors may be demonstrated by showing therapeutic use as a single agent in murine models of RCC, and developing optimal dose levels and dosing schedules. Then, the efficacy and safety of PEM in synergistic combination with VEGFR-directed therapies (first-line therapy), and/or in cases of VEGFR TKI-resistant disease (second-line therapy) may be shown. PEM may also have utility as a novel palliative treatment with and without radiation therapy (XRT), improving outcomes (or in lieu) of palliative nephrectomy. Thus, effective combinations of PEM with established anti-angiogenesis therapies and/or XRT may be shown in various embodiments using murine models of RCC.

In particular, PEM may be used in synergistic combinations with a variety of different cancers, agent classes and agent names below.

As discussed above, some embodiment nanoparticles may also be used to co-incorporate multiple agents within a single nanoparticle. For example, embodiment PEM constructs may be loaded with any two or more of NO binding molecules and molecules that affect NO (e.g., NOS inhibitors, VEGFR TKIs, and mTOR inhibitors), thereby producing maximal anti-angiogenesis activity and inhibition of tumor growth. Moreover, PEM-induced NME is likely synergistic with anti-angiogenesis therapies (e.g. VEGFR TKIs or mTOR inhibitors) and may demonstrate similar efficacy in treatment naive, heavily treated and poor risk patient populations.

The various embodiments may be illustrated, but are not limited to, the following examples.

**Example I**

**Methods and materials to construct biodegradable PEM dispersions with varying physicochemical properties:**

Poly(ethyleneoxide)-block-poly($\varepsilon$-caprolactone) (PEO-b-PCL) possessing a PEO block size of ~1.5- 4 kDa and with a PEO block fraction of ~10-20% by weight are utilized to form biodegradable PEM dispersions. Poly(ethylene oxide)-block-poly($\gamma$-methyl $\varepsilon$-caprolactone) (PEO-b-PMCL) and Poly(ethylene oxide)-block-poly(trimethylcarbonate) (PEO-b-PTMC) copolymers of varying molecular weight, hydrophobic-to-hydrophilic block fraction, and resulting polymersome membrane-core thickness are further incorporated to generate PEM constructs that are not only slowly biodegradable but also uniquely deformable, enabling passage through compromised capillary beds, via infra. PMCL, as a derivative of PCL, is a similarly fully bioresorbable polymer that degrades via non-enzymatic cleavage of its ester linkages. Polymersomes composed from PEO-b-PTMC and/or PEO-b-PMCL are spontaneously formed at lower temperatures, in greater yields, and possess more deformable and viscoelastic membranes as compared to those composed from PEO-b-PCL. They also similarly degrade much more slowly than vesicles formed from PEO-b-PGA, PEO-b-PLA, or PEO-b-PLGA. As such, PEO-b-PCL and PEO-b-PMCL-derived PEM dispersions demonstrate larger Mb-encapsulation efficiencies, smaller average particle diameters, and lower levels of met-myoglobin generation as compared to biodegradable cellular MBOCs claimed in the literature.

*Synthesis of PEM dispersions:*

To synthesize PEM dispersions, Purified human Mb may be purchased from Sigma-Aldrich® to be used as starting materials. PEO-b-PCL, PEO-b-PMCL, and PEO-b-PTMC copolymers with PEO molecular weight ranging from 1kDa-4kDa have previously been shown to give a stable and high yield of polymersomes[53]. For example, the PEO may have a molecular weight of 2kDa and the PMCL may have a molecular weight of 9.4kDa. By varying the initial amounts of polymer (from 5 mg - 20 mg per sample), as well as the initial Mb concentrations used in polymersome formation (from 100 mg/ml to 300 mg/ml), PEM dispersions that differ in the degree of Mb encapsulation are generated.

PEM dispersions will be formed by using three different methodologies: 1) "thin-film rehydration", which involves the deposition of an organic solution of dissolved polymer on a Teflon film, drying of the film under vacuum oven overnight to remove all organic solvent, immersion of the dry thin-film of polymer in an aqueous solution of purified Mb and subsequent high-frequency sonication with heat, and, finally, extruding through a series of different pore-size membranes in order to yield the desired nanometric PEM dispersion; 2) "direct hydration",[116, 117] where dry polymer is mixed with an equal weight of PEG 500 DME at a 1:1 molar ratio, heated to 95 °C for 30 minutes, mixed vigorously, allowed to cool to room temperature for 20 minutes prior to addition of Mb solution, then followed by further vigorous mixing and sonication, extrusion through a series of different pore-size membranes in order to yield the desired nanometric PEM dispersion, and finally, by separation of PEG 500 by running on a size-exclusion column; and 3) thin-film direct hydration, where dry polymer is mixed with an equal weight of PEG 500 DME at a 1:1 molar ratio prior to deposition on Teflon, followed by then drying of this film over night, heating to 95 °C for 30 minutes, vigorously mixing, allowing to cool to room temperature for 20 minutes prior to addition of Mb solution, further vigorous mixing and sonication, extrusion through a series of different pore-size membranes in order to yield the desired nanometric PEM dispersion, and finally, by separation of PEG 500 by running on a size-exclusion column.

Each of these methods produces a high yield of stable polymersomes that can be effectively controlled through membrane extrusion to yield unilamellar, mono-dispersed suspensions of PEMs that vary from 100 nm - 1 $\mu$m in diameter in average size. Although thin-film rehydration may yields very narrow PEM size distribution, and relatively higher Mb encapsulation % due to larger core volumes available for encapsulation,[116] the stability of Mb and the resultant PEM dispersions can be demonstrably lower;[163] these results may be due to the fact that the hydration and optimal sonication temperatures necessary for generating a given polymeric-composition of polymersomes may be close to the denaturation temperature of free Mb (e.g. 60 °C used to generate PEO-b-PCL-based PEM dispersions via thin-film rehydration). PEO-b-PMCL and PEO-b-PTMC polymersomes will be formed by direct or thin-film direct hydration at room temperature (under ambient $pO_2$) and expectedly enable a higher yield of PEMs with greater Mb encapsulation efficiency. For concomitant NIR imaging studies, NIR-emissive PEM constructs may be generated via co-incorporation of oligo(porphyrin)-based NIRFs with dried polymer (at a mol ratio of 1:40),[166] prior to exposure to the aqueous Mb solution. Unencapsulated Mb are separated from all PEM dispersions using dialysis, ultra-filtration, and/or size exclusion chromatography.

**Example II**

**Characterization of physicochemical properties of PEM dispersions:**

To verify PEM generation, each Mb/polymer formulation are characterized for particle size distribution using dynamic light scattering (DLS). PEM structure and morphology are directly visualized using cryogenic transmission electron microscopy (cryo-TEM). The viscosity of the various PEM dispersions are measured using a microviscometer. To measure Mb encapsulation%, two independent methods are used. In the first method, PEM dispersions are initially lysed with a detergent (e.g. triton X-100) and the UV absorbance of the resulting lysate is measured to determine the mass of Mb and subsequent Mb encapsulation% of the original PEM composition.[162] While this calculation is relatively straight forward, it may overestimate the encapsulation% through some assumptions on total Mb dispersion volume. As such, an asymmetric field-flow fractionator coupled with a differential interferometric refractometer is used to measure the concentration of eluting, unencapsulated Mb from the encapsulation% is determined.[162, 176] From these measurements, the final weight ratio of Mb:polymer in the various PEM dispersions is further calculated. The % metMb in each of the PEM dispersions is determined by analogous methodology to the well-established cyanometMb assay.[162, 163]

**Example III**

**Characterization of the oxygen-carrying properties of biodegradable PEM dispersions:**

The oxygen binding properties of PEO-b-PCL and PEO-b-PMCL-based PEM dispersions are measured using established techniques. The equilibrium oxygen binding properties are thoroughly characterized as well as the diffusion kinetics of oxygen across polymersome membranes. With the aid of these measurements, oxygen permeabilities and oxygen-membrane diffusion coefficients for these various PEM dispersions are determined. These very fundamental parameters are critical for the optimal design of a successful cellular MBOC. Nitric oxide (NO) binding profiles of various PEO-b-PCL and PEO-bPMCL-based PEM dispersions are further determined. Acellular MBOCs can be expected to induce vasoconstriction, hypertension, reduced blood flow, and vascular damage in animals due to their entrapment of endothelium-derived NO. Mb-encapsulated in nanoparticles such as polymersomes, liposomes, micelles, etc, however, is not been expected to be similarly "vasoactive"; analogous to those of natural RBCs, liposome and polymersome membranes should effectively retard NO binding through effective Mb sequestration from the surrounding vascular environment. PEM dispersions will likely exhibit more resistance to NO scavenging owing to their thicker membranes and lower permeabilities. Finally, different measurements on PEO-b-PCL and PEO-b-PMCL-based PEM dispersions will be performed in order to test their stability and integrity under physiological conditions for extended durations of time.

**Experimental:**

*Characterization of oxygen binding properties:*

Equilibrium oxygen binding properties such as $P_{50}$ of PEO-b-PCL, PEO-b-PMCL-and PEO-b-PTMC-based PEM dispersions are measured using a Hemox-analyzer[51, 52]. Dependence of these properties on the composition of PEM dispersions are determined using a series of Mb-loading concentrations, as well as by adding an allosteric effector such as inositol hexaphosphate into the aqueous phase of the polymersomes. This is especially important in order to determine the suitability of PEO-b-PCL, PEO-b-PMCL, and PEO-b-PTMC-based PEM constructs to deliver oxygen to tissues experiencing normal oxygenation as well as in low oxygenation conditions. Results of these experiments will be compared with respect to $P_{50}$ and n values of free Mb solution, as well as those values of Oxyglobin® (Biopure Corp., Cambridge, MA), which is the only oxygen therapeutic approved by the FDA for veterinary use.

In addition to these equilibrium measurements, the kinetics of oxygen diffusion across PEM membranes and binding to/release of Mb for different PEO-b-PCL, PEO-b-PMCL, and PEO-b-PTMC-based PEM dispersions are determined using a highly sensitive oxygen microelectrode. Measurements of various PEMs are compared to those from free Mb and empty polymersome dispersions (without Mb) in order to delineate the roles of diffusion and binding in $O_2$ take-up. The results of these experiments are analyzed with the help of a diffusion-reaction transport model to determine oxygen permeability of different polymersome membranes; a correlation between diffusive properties of various diblock copolymer membranes and measured oxygen binding properties of PEM formulations is expected.

*Characterization of NO binding properties:*

NO binding of PEO-b-PCL, PEO-b-PMCL, and PEO-b-PTMC-based PEM dispersions under oxygenated and deoxygenated conditions are systematically studied using stopped flow spectroscopy. [177, 178] The time-course of binding is measured by taking rapid absorbance scans of the various oxygenated or deoxygenated PEM dispersions rapidly mixed with NO-containing solution. A range of Mb loading concentrations, PEM dispersion concentrations, and PEM sizes are expected to alter the results of these experiments. Similarly, the roles of NO diffusion and binding in NO uptake by PEO-b-PCL, PEO-b-PMCL, and PEO-b-PTMC-based PEM constructs are further characterized by conducting experiments comparing PEM, free Mb, and empty polymersomes using a NO microelectrode. Through these comprehensive studies, the NO binding rate constants for PEO-b-PCL, PEO-b-PMCL, and PEO-b-PTMC-based PEM dispersions under different conditions are established and compared with the results for free Mb in solution, liposome encapsulated Mb (LEM), and Oxyglobin®.

*Characterization of the stability and integrity of PEM dispersions:*

To test the stability of various PEO-b-PCL, PEO-b-PMCL, and PEO-b-PTMC-based PEM dispersions, they are stored in saline solution and in blood plasma at 4 °C and at 37 °C for several days; changes in PEM morphology and size distribution are assessed using cryo-TEM and DLS, respectively. Similarly, in situ changes in Mb concentration, metMb level, NO uptake, and Mb release from biodegradable PEMs under various solution conditions (e.g. temperature, pH, $pO_2$, and pNO) and at various time points is tested using techniques described herein. These studies utilize electronic absorption spectroscopy and concentration calculations based on known extinction coefficients for methylated, NO-bound, and oxygenated Mb.[179-188]

*Measurement of critical lysis tension, critical areal strain using micropipette aspiration:*

Micropipet aspiration of Mb-encapsulating polymersomes follows analogous procedures to those described in previous references. Briefly, micropipets made of borosilicate glass tubing (Friedrich and Dimmock, Milville, NJ) are prepared using a needle/pipette puller (model 730, David Kopf Instruments, Tujunga, CA) and microforged using a glass bead to give the tip a smooth and flat edge. The inner diameters of the micropipets range from 1 um to 6 um and are measured using computer imaging software. The pipettes are used to pick up the Mb-loaded and unloaded polymersomes and apply tension to their membranes. Micropipets are filled with PBS solution and connected to an aspiration station mounted on the side of a Zeiss inverted microscope, equipped with a manometer, Validyne pressure transducer (models DP 15-32 and DP 103-14, Validyne Engineering Corp., Northridge, CA), digital pressure read-outs, micromanipulators (model WR-6, Narishige, Tokyo, Japan), and MellesGriot millimanipulators (course x,y,z control). Suction pressure is applied via a syringe connected to the manometer. Experiments are performed in PBS solutions that has osmolalities of 310-320 mOsm in order to make the polymersomes flaccid (internal vesicle solution was typically 290-300 mOsm sucrose). The osmolalities of the solutions are measured using an osmometer. Since sucrose and PBS have different densities and refractive indices, the polymersomes settle in solution and are readily visible under phase contrast or DIC optics.

Example IV

Development of PEO-b-PCL, PEO-b-PMCL, and PEO-b-PTMC-based PEM dispersions that are capable of dry storage, point-of-care rehydration, and *in vivo* delivery:

*Polymer Synthesis:*

Acrylate-modified diblock copolymers (e.g. an acryl modified PEO-b-PCL-based polymer deemed PEO-b-PCL-acryl) are synthesized according to standard procedures using stannous octoate as the catalyst. For example, PEO-b-PCL-acryl is found to have a number average molecular weight of 14 kDa (12 and 2 kDa for the PCL and PEO blocks, respectively). These are determined by calibrating the NMR peaks to the terminal methoxy group on the PEO at approximately 3.4 ppm. The polydispersity of the polymer is < 1.5. Acrylation of the OH terminus of the PCL block does not lead to a significant change in the polymer size or distribution following the second purification. The acrylation efficiency has been found to be 99%.

*Formation of PEM Dispersions:*

To synthesize PEM dispersions comprised of acryl-modified polymers (e.g. PEO-b-PCL-acryl-based PEM dispersions), pure human Mb is used as starting materials. Pure human Mb may be purchased from Sigma-Aldrich®. PEO(2k)-b-PCL(12k)-acryl polymer and 2,2-dimethoxy-2-phenylacetophenone (DMPA) are dried on roughened Teflon® via dissolution in methylene chloride at a molar ratio of 1:1, deposition on Teflon®, and evaporation of the organic solvent. Varying the amount of acryl-modified polymer (e.g. PEO(2k)-b-PCL(12k)-acryl polymer, from 5 mg - 20 mg per sample), as well as the initial aqueous Mb concentrations used in polymersome formation (from 100 mg/ml to 300 mg/ml), PEM dispersions that compartmentalize DMPA in their membranes and that differ in the degree of aqueous Mb encapsulation are generated. PEM dispersions are formed by using three well-established methodologies: 1) thin-film rehydration, 2) direct hydration, and 3) thin-film direct hydration (see Example I). Each of these methods produces a high yield of stable polymersomes that can be effectively controlled through membrane extrusion to yield unilamellar, mono-dispersed suspensions of PEMs that vary from 100 nm - 1 $\mu$m in diameter in average size. Although thin-film rehydration may yields very narrow PEM size distribution, and possibly higher Mb encapsulation % due to larger core volumes available for encapsulation,[116] the stability of Mb and the resultant PEO-b-PCL-based PEM dispersions can be demonstrably low;[163] these results may be due to the fact that the hydration temperature for PEO-b-PCL is close to the denaturation temperature of free Mb. PEO-b-PMCL and PEO-b-PTMC polymersomes are formed by direct hydration or thin-film direct hydration at room temperature (under ambient pO$_2$) and expectedly enable a higher yield of PEMs with greater Mb encapsulation efficiency. For concomitant NIR imaging studies, NIR-emissive PEM constructs are generated via co-incorporation of oligo(porphyrin)-based NIRFs with dried polymer (at a mol ratio of 1:40),[166] prior to exposure to the aqueous Mb solution. Unencapsulated Mb is separated from all PEM dispersions using dialysis, ultra-filtration, or size exclusion chromatography.

*Stabilization of PEM Membranes After Formation:*

Once assembled, acryl-modified polymersomes comprising the membranes of the PEM dispersions (e.g. PEO-b-PCL-acryl) can be crosslinked via UV light exposure that induces a radical polymerization of the acryl groups via activation of the photoinitiator DMPA incorporated in the polymersome membranes. This approach does not hinder hydrolysis of the biodegradable block (e.g. the PCL chain of PEO-b-PCL-acryl) and yields degraded monomers (e.g. oligo-caprolactone units), PEO, and kinetic chains of poly(acrylic acid) as the degradation products. Mb is protected from photo-induced degradation of metMb formation by co-ecapsulation of NAC or methylene blue with Mb within the polymersomess' aqueous core. Polymerization of the vescicles' membranes proceeds by exposure of the DMPA-incorporated acryl-modified polymers (e.g. PEO-b-PCL-acryl) that compose thePEM dispersions using UV light generated from an OmniCure Series 1000 spot-curing lamp with a collimating lens (Exfo, Ontario, Canada; 365 nm, 55 mW/cm2) for 10-30 min.*Lyophilization and Dry-phase Storage:*

Lyophilization proceeds by freeze-drying the acryl-modified PEM dispersions (e.g. PEO-b-PCL acryl PEM) after UV light exposure by placement in liquid nitrogen until bubbling ceases. The frozen PEM dispersions are then placed on a benchtop lyophilizer (FreeZone 4.5 L Benchtop Freeze Dry System, Labconco, Kansas City, MO; Model 77500) for 24 h until samples are dry. The dry, collapsed PEM dispersions are then stored in a dessicator under argon gas and placed at 4 °C.

*Point-of-Care Hydration:*

The dried acry-modified PEM dispersions are taken out of the dessicator and placed in a vial. The same original volume of aqueous solution is added back to the samples to hydrate the vesicles. Polymersome rehydration is further augmented by gentle vortexing for 10 minutes to achieve full vesicle resuspension. Intact polymersomes are verified by DLS, which shows minimal vesicle aggregation and no destruction into micelles. Mb retention is verified by running the PEM dispersion over an aqueous size-exclusion column and taking aliquots of the running bands for UV-vis analysis. Only bands corresponding to polymersomes, as verified further by DLS of the elution aliquots, contain Mb as assessed by UV-vis spectroscopy. The stability of the retained Mb is further verified by the UV-vis spectra that show no bands corresponding to metMb generation or any further Mb breakdown products.

**Development of Molecularly-targeted PEO-b-PCL, PEO-b-PMCL, and PEO-b-PTMC-based PEM Dispersions:**

Through well-established chemical conjugation methods, polymersome surfaces are modified with various biological ligands to impart specific multi-avidity biological adhesion. Similar methodology may are adopted to generate molecularly-and cellular-targeted polymersome-encapsulated PEM dispersions that are able to promote, amongst other things, wound healing and improved efficacy of radiation therapy to hypoxia tissues. Biological ligands are conjugated to these nanoparticles via a carbodiimide- poly-vinyl sulfone-mediated aqueous phase reactions. The degree of polymersome-surface coverage with ligand is systematically varied (from 1% to > 10% of the total surface area of the polymersomes) by using ligands of different concentrations and PEM dispersions that are synthesized from mixtures containing different ratios of functionalized to unfunctionalized polymers. After verifying peptide conjugation to polymersome surfaces, the kinetic binding of the resultant PEM formulations to recombinant molecular targets/receptors are characterized via surface plasmon resonance (Biacore SPR) measurements; dose-dependent curves are analyzed in a manner similar to that described for the free biological ligand. These studies reveal kinetic parameters of the interaction between PEM dispersions and molecular targets (on-rate, *kon* and off-rate, *koff*) and the change in affinity of ligands (dissociation constant, K) as affected by their conjugation to polymersomes.

Experimental:

Established chemical modification procedures are used to functionalize the PEO terminus of biodegradable polymers (e.g. PEO-b-PCL diblock copolymers) with carboxyl groups and to verify the reactions by 1H NMR spectroscopy. PEM dispersions are created and purified from various combinations of functionalized and unfunctionalized copolymers using standard separation methods to yield mono-dispersed suspensions of unilamellar vesicles that are stable for several months. PEM size distributions are determined by dynamic light scattering (DLS). Ligand identity and purity are confirmed by reverse phase high performance liquid chromatography and MALDI mass spectrometry. Ligand conjugation to carboxyl-terminated PEO groups on the polymersome surface is carried in an aqueous reaction mediated by 1-ethyl-3-(3-dimethylaminopropyl) carbodiimide (EDC) and Nhydroxysuccinimide (NHS). The extent of ligand conjugation is determined using a micro-BCA assay. The resultant targeted PEM dispersions are extensively imaged by cryogenic transmission electron microscopy (cryo-TEM) to verify their stability after ligand conjugation. Their size distributions are again measured by DLS. The degree of ligand conjugation is verified using flow cytometry. SPR measurements are carried out on biosensor instruments Biacore X and Biacore 2000 (Biacore AG, Uppsala, Sweden) at 25°C. Recombinant purified recombinant ligand targets (e.g. protein receptors) are purchased commercially and immobilized by attachment to the dextran hydrogel on the sensor surface. Targeted PEM constructs are injected in various concentrations and their binding is monitored in real time. The kinetic rate constants ($k_{on}$ and $k_{off}$) and the equilibrium binding constant (KD) for receptor/PEM binding are estimated from kinetic analysis of the sensorgrams. PEMs without targeting ligands or irrelevant ligand-conjugated PEMs are used as controls.
Alternative ligand conjugation chemistries can also be employed. For example, organic phase reactions where the diblock polymer is chemically functionalized and conjugated with select ligands (small molecules, peptides that have organic-phase solubility) prior to forming PEM dispersions are possible; this organic coupling methods ensures that the PEO terminus is conjugated with ligand before it is exposed to aqueous solution where it might lose many of its modified surface reactive groups via competing hydrolysis. Also, as an alternative method to vary the degree of ligand surface conjugation, PEM dispersions composed of PEO-b-PCL copolymers that vary with respect to PEO and PCL block sizes are created. This approach controls the kinetics of ligand conjugation to polymersome surfaces as well as the degree of ligand surface coverage for a given PEM formulation. It is possible for targeted PEM formulations to bind to the sensor surface in a non-specific manner during SPR measurements, thereby affecting its regeneration and signal-to-background ratio. If a reliable measurement cannot be performed, ligand-conjugated PEM binding characteristics are also studied using ELISA or isothermal titration calorimetry, which are other established techniques for studying nanoparticle binding.

*In Vivo Tumor Oxygenation Modulation by PEM:*

PEM dispersions formed from PEO-b-PCL, PEO-b-PMCL, PEO-b-PTMC and/or acryl-modified versions of these polymers are tested for their abilities to alter *in vivo* tumor oxygenation upon tail-vein injection into xenotransplanted-tumor bearing mice. The codependent effects of particle size, deformability and concentration on effective Mb delivery, and resultant tumor oxygenation, are also deconstructed. A hyperspectral optical imaging system that can spatially deconstruct real-time kinetic $O_2$ transport is used to assess the efficacy of a given PEM construct to alter mean and minimum tumor oxygen tensions (p$O_2$).[151, 154, 156, 158] While mean p$O_2$s have been previously studied and are readily measured by other techniques, the spatially-distributed minimum tumor p$O_2$s are perhaps the most responsible for driving tumorigenesis and providing the cancer stem cell niche that helps tumors evade effective treatment.[151, 154, 155, 189] A hyperspectral optical imaging system enables the spatial mapping of kinetic p$O_2$s, in real-time, and is used to visualize and quantify the degree of PEM-modulation of low tumor p$O_2$ areas. In addition, mild localized tumor heating increases vessel pore sizes in solid tumors for up to several hours, aiding in nanoparticle extravasation.[190, 191] As such, localized tumor hyperthermia is further capable of increasing $O_2$ delivery by PEMs. Finally, PEM-related myoglobinuria and its effects on creatinine clearance (CCr) is monitored to assess acute post-treatment nephrotoxicity.

*Animal Storage, Handling and PEM Injections:*

Mice are purchased and housed in appropriate animal facilities. Dorsal skin fold window chambers are surgically implanted on each animal approximately 1 week prior to treatment. During the surgical procedure, 10,000 4T1 mammary carcinoma cells are injected onto the mouse dorsum or flank. These cells are engineered to constitutively express RFP, with GFP expression induced in response to HIF-1 activity.[192] The tumors are allowed to grow for approximately 1 week, at which point they are large enough to be hypoxic and have HIF-1 activity.[158] 100 $\mu$L of PEM suspensions are prepared as described above and injected via the tail vein at $t = 0$ and $t = 24$ h. These experimental parameters enable evaluation of the effects of PEMs that have accumulated in the perivascular space, which is expected to peak at approximately 24 h. For hyperthermia studies, a special housing unit is used to vary the window chamber/tumor temperature.[190, 191]

*Visualization and Quantification of Kinetic Tumor Oxygen Modulation by PEM:*

At the time points specified above, hyperspectral imaging is used to evaluate the effects of the various PEM constructs on modifying tumor $pO_2$. Temperatures are adjusted between 34 - 42 °C.[190, 191] Hyperspectral imaging of Mb absorption is used to quantify Mb O2 saturation,[158] while ratiometric evaluation of boron nanoparticle fluorescence and phosphorescence is used to quantify absolute tumor $pO_2$.[153] HIF-1 activity is also evaluated by measuring GFP emission. This enables quantification of both vascular and tissue oxygenation, as well as the presence of the tumor hypoxic phenotype, independently and concurrently.

**Example V**

As discussed above, various embodiment nanoparticles that exhibit high concentrations of biologically active oxygen carriers may accumulate into tumors when injected into the circulation of an animal model of human breast cancer. Within the tumors of these animals, the nanoparticles may result in widespread shutdown of vascular flow, hemorrhage, and necrosis, of the cancer cells but with no obvious toxic side effects to normal tissue.

The following general practices may be used for murine models in the specific example embodiments discussed below.

*Anesthesia During Therapeutic Administration:*

Anesthesia may be performed using isofluorane (1.5-2.5% via nose cone) for imaging. Ketamine/xylazine (100/10 mg/kg, i.p.) may be used for surgical procedures. Animals may be monitored for proper depth of anesthesia by toe pinch response and respiratory rate. They may also be maintained for proper body temperature using a circulating water heating pad. Moisturizing ointment may be placed on their eyes.

*Animal Injection with PEM:*

PEM and empty polymersomes (i.e., a negative control) may be prepared under sterile good laboratory practice (GLP) conditions in 0.9% NaCl USP. The viscosity, pH and osmolality of each injectate may be measured and verified to be within standard physiologic range for blood (e.g. 4-5 kPa, pH 7.4, 290 mOsm). Because of the expected clinical application of the compound to induced tumor specific NME after accumulation, and concerns for potential systemic vasoactivity at higher doses, slow intravenous (IV) injection may be used over the course of 5-10 min. A butterfly needle may be preferentially used, but an intravenous cannula may be taped in place in a superficial vein (short term), or surgically placed some time prior to use (longer term or multiple injections, e.g. for weekly application of three cycles). Initial infusion may be attempted in volumes of 10 ml/kg and at a rate of 1 ml/kg/min; but these may be increased, pending exact PEM concentrations. A maximum volume of 20 mL/kg, infused as at 5 mL/kg/min, may be set in order to avoid significant distress or development of pulmonary lesions.

*Blood Draws:*

Frequent blood draws may be necessary for toxicokinetic studies of animals treated with various doses and schedules of PEM (as per toxicity assessments below). Lateral tarsal (saphenous) vein blood draws may be preferentially used as they enable removal of 7.5% of circulating blood volume without the need for anesthetic. For studies involving mice, this may amount to 0.1 mL blood draws (total blood volume of a 25 g mouse is 1.8 mL). The saphenous vein is on the lateral aspect of the tarsal joint and may be easily located after fur is shaved and the area wiped with alcohol. The animal may be placed in a suitable restrainer, such as a plastic tube, and the operator may extend the hind leg. The vein may be raised by gentle pressure above the joint and the vessel is punctured using the smallest gauge needle that enables sufficiently rapid blood withdrawal without hemolysis (e.g. 25-27 gauge for mice). For small volumes, a simple stab leads to a drop of blood forming immediately at the puncture site and a microhematocrit tube may be used to collect a standard volume. After blood has been collected, pressure over the site may be sufficient to stop further bleeding. Removal of the scab may enable serial sampling. For blood draws that occur no more frequently than a weekly basis, the maximum volume removed per draw may be set at 10% of the circulatory volume (0.2 mL in mouse). For blood draws that occur no more frequently than every 3 weeks, the maximum volume removed per draw may be set at 20% of the circulatory volume (0.4 mL in mouse). For terminal blood draws at sacrifice, cardiac puncture may be employed under general anesthesia.

### Toxicity Assessments:

The kinetic parameters and end points that may be evaluated include clinical signs (hourly for the first 6h, then every 12 hours for 3 days, and weekly thereafter), body weights and changes (2-3 times per week), food consumption (2-3 times per week), abbreviated functional observational battery (2-3 times per week), ophthalmologic assessments (2- 3 times per week), serologic profiles (hematology, coagulation, clinical chemistry, and urinalysis performed (weekly), toxicokinetic parameters (hourly for the first 6h, then every 12 hours for 3 days, and weekly thereafter), gross necropsy findings (upon sacrifice after completion of the third cycle), organ weights (upon sacrifice), and histopathologic examinations (upon sacrifice).

### Window Chamber Surgery:

Animals may be anesthetized with 100/10 mg/kg ketamine/ xylazine. Exidine solution may be used to disinfect the animal's skin and washed off with 70% ethanol. In a sterile field, the animal's dorsal skin may be sutured to a metal c-frame, and a titanium metal frame may then surgically inserted and sutured in place. A 12 mm circular section of the skin may be excised. Approximately 10,000 RCC cells, suspended in 20 microliters of phosphate buffered saline (PBS) may then be injected into the now visible back-side of the underlying skin. A cover glass window may next be inserted and held in place with a spring loaded metal ring. The c-frame may subsequently be removed and the animal may be allowed to recover from anesthesia. Temperature may be maintained throughout the procedure using a heated wax pad. Buprenorphine may be injected subcutaneously immediately post-surgery, and then at 8-12 hours post-surgery, as needed for pain alleviation.

### Imaging NME:

An animal with an active scavenging system may be anaesthetized using isofluorane (1.5-2.5% via nose cone), and positioned on the imaging stage. A Bacitracin/ Neomycin/Polymyxin B (BNP) suspension may be topically applied, and hemoglobin saturation and green fluorescent protein (GFP) (HIF-1) fluorescence may be imaged up to 30 minutes to establish baseline oxygenation levels. The imaging may be performed using a fluorescence microscope (e.g., a Zeiss). A 42-degree Celsius hyperthermia treatment may be imposed for 1 hour, after which the animal may be tail-vein injected with a PEM suspension (e.g., 100 $\mu$L). Imaging may be continued for 1 hour, and follow up imaging sessions may be carried out at 24 and 48 hours post treatment, with a second PEM dose delivered at 24 hours. The total imaging and treatment time for each time point may be less than 2 hours.

### Administration of Sunitinib:

Sunitinib malate salt (a VEGFR TKI) may be purchased (e.g., from LC Laboratories (Japan), 99% USP). Oral formulation may be prepared by four-fold concentrations (e.g. 3.2 mg for a 20 g mouse) in a Cremephor EL/ethanol (50:50) solution. This stock of four-fold concentration may be prepared fresh daily. Final dosing solutions may be generated on the day of use by dilution to normal concentration with endotoxin free distilled water and mixed by vortexing immediately prior to dosing at 40 mg/kg daily (0.8 mg for a 20 g mouse) by oral administration.

### Monitoring of Tumor Growth Via Luciferase Imaging:

786-O cells that have been engineered to constitutively express luciferase may be utilized to generate orthotopically xenografted RCC tumor-bearing animals. Luciferase imaging may be used to monitor tumor size and to track metastases. Tumor luciferase intensity and body weights may be recorded two to three times a week starting with the first day of treatment. Tumor volume may be determined via a preliminary calibration curve to relate total radiance from luciferase to tumor volume, in which animals may be imaged and then sacrificed to measured tumor volume directly.

### Establishment of VEGFR TKI-resistant Disease:

Orthotopically xenografted RCC tumor-bearing animals may be generated and subject to daily treatment with PO Sunitinib (40 mg/kg per day), which may be initiated when tumors reach 100 mm$^3$ and continued until they grow to 1.5 times their initial size. Further therapeutic administration with PEM with and/or without temsirolimus, or with control nanoparticles, may then take place.

**Potential Toxicity, Dosage, and Kinetic Measurements of PEM Formulations**

In various embodiment studies, comprehensive serum chemistry panel and liver histology from mice injected with PEM versus controls (i.e. polymersomes alone or myoglobin alone) may be performed. The minimal effective dose (MED) of PEM necessary to induce NME of RCC may also be identified. For further therapeutic studies, its maximum dose for safe administration may be set as either the STD10 (the severely toxic dose in 10% of animals) or the MTD75 (the maximum tolerated dose that results in less than 15-20% weight loss in 75% of animals), if no dose limiting toxicities (DLTs) are discovered.

*Developing PEM Constructs:*

In various embodiments, PEM constructs may be developed that have a myoglobin encapsulation efficiency of greater than 50%, a weight ratio of encapsulated myoglobin to polymer of greater than 5 wt%, a solution metmyoglobin level that was less than 5% of the total myoglobin, a suspension viscosity between 3-4 cP, a $P_{50}$ similar to free myoglobin, and an order of magnitude smaller NO binding rate constant as that measured for free myoglobin. Such PEM constructs may be developed using biocompatible poly(ethyleneoxide)-block-poly(butadiene) (PEO-b-PBD) copolymers. Further, such PEM constructs may be developed using any of a number of biodegradable copolymers. Encapsulation of the myoglobin may be accomplished using methods including thin film rehydration, thin film direct rehydration, electroporation, and direct rehydration.

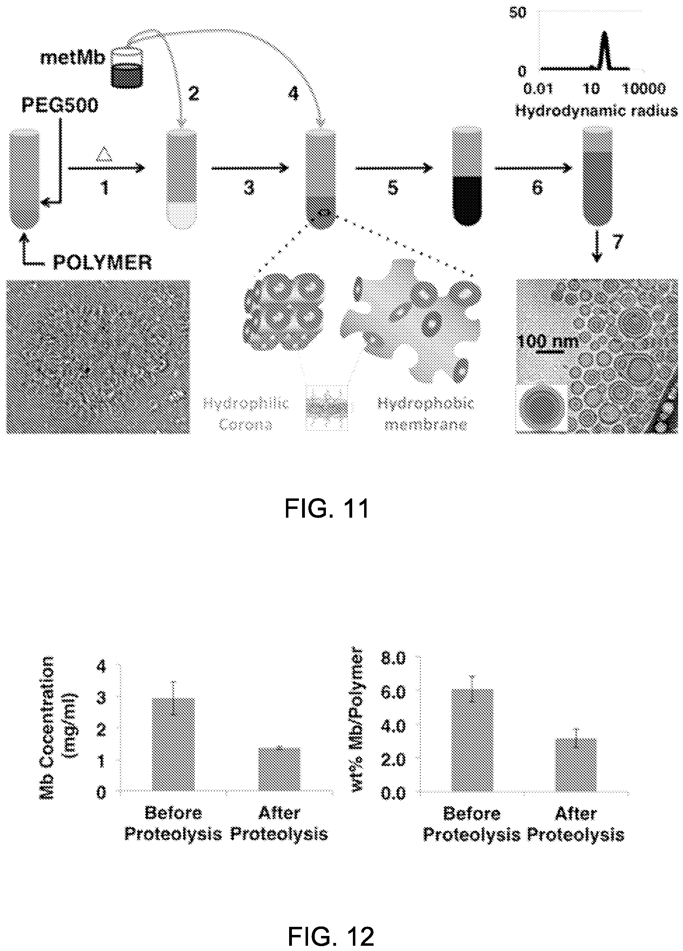

In particular, a modified direct hydration method may be employed to maximize myoglobin loading and myoglobin encapsulation efficiency. Several variables, including temperature, mixing rate, sonication power/time, and myoglobin reduction, may be changed to optimize myoglobin loading in PEM formulations. FIGs. 11 and 12 illustrate steps for generation and measurement of PEM formulations according to some embodiments. As shown in FIG. 11, equivalent amounts of OB18 and PEG polymer (Mw = 500 Da, "PEG500") may be heated for 1 hr at 95°C followed by through mixing and cooling of the sample to room temperature (RT). 10 mg of reduced myoglobin solution (150 mg/mL metmyoglobin (metMb) in 10 mM PBS at pH 7.4) may be added, and reduced by addition of 1 wt% sodium dithionite. The mixture may be agitated and sonicated for 0.5 hr at RT, and the polymer suspension may be further diluted by addition of titrated aliquots (10, 20, 50, and finally 100 nl) of the reduced Mb solution with thorough sonication after each dilution step. The sample may be subjected to further dialysis against sterile PBS buffer at 4°C, and characterization of the resultant PEM formulations by dynamic light scattering (DLS) to determine size. The sample may be subjected to cryogenic transmission electron microscopy (cryo-TEM) to verify morphology.

As shown in the set of graphs in FIG. 12, the sample may further be subjected to UV-vis spectrometry to calculate final concentrations of Mb and weight percentages of Mb to polymer (wt% Mb/polymer) in the final PEM constructs. The sample concentrations of functional Mb may be independently verified by measuring the iron content in the PEM suspensions, using Inductively Coupled Plasma Optical Emission Spectroscopy (ICP-OES), which correlates well to the UV-Vis data (wt% Mb/polymer = 6% by UV-Vis and 8% by ICP-OES). Particle sizes may be tuned between 100 and 200 nm in diameter through selection of polymer composition and molecular weight.

Since the outer and inner surfaces of polymersomes are comprised of hydrophilic polymers (PEG), myoglobin may be encapsulated within the aqueous cavities and bound to the surfaces of polymersomes. To obtain pure PEM, surface-associated myoglobin may be removed by proteolysis using pronase (i.e., a mixture of various proteinases isolated from Streptomyces griseus that digests proteins into individual amino acids). Since pronase cannot cross the polymersome bilayer membrane, it may be used to digest all surface-associated myoglobin while leaving encapsulated Mb unaffected. In brief, pronase solution may be added to PEM samples to obtain a final 4 wt% enzyme concentration. The solution may be further mixed for at least 2 hr at RT, centrifuged, and dialyzed to remove enzyme. Retained Mb in PEM suspensions was subsequently quantified and found to be between 3 wt% (by UV-Vis) and 5 wt% Mb/polymer (by ICP-OES).

Direct hydration may also be utilized to obtain small PEM compositions (i.e., around 100-150 nm in diameter) generated from biodegradable PEO-b-poly(caprolactone) (PEO-b-PCL) and PEO-b-poly(PCL/trimethylene carbonate) (PEO-b-PTMC) copolymers. The wt% myoglobin/polymer as well as myoglobin encapsulation efficiencies may be similar in both biocompatible and biodegradable PEM formulations.

Iron in myoglobin is oxidized to $Fe^{3+}$ when exposed to atmospheric conditions. Thus, various agents may be used for chemical reduction of met-myoglobin in order to enable $O_2$/NO binding, such as sodium dithionite ($Na_2S_2O_4$). Using reduced myoglobin may result in more efficient generation and higher yields of PEM than are achievable by utilizing met-myoglobin. While met-myoglobin may be reduced to oxymyoglobin (with 1 wt% $Na_2S_2O_4$) prior to PEM generation, most PEM may be reoxided to met-myoglobin after long-term storage. Therefore, PEM may be re-reduced back to oxymyoglobin prior to immediate infusion. In various embodiments, such re-reduction may be performed by a process consisting of sonication in dilute quantities of $Na_2S_2O_4$ followed by dialysis, in which effects of sonication power and time may be examined and optimized. To avoid further oxidation, reduced PEM may be used immediately for animal studies.

*Equilibrium binding of gasses:*

Oxygen equilibrium binding curves of PEM and free Mb dispersions may be measured using a Hemox™ Analyzer at physiological temperature (37°C). In brief, samples may be allowed to saturate to a $pO_2$ of 147 mm Hg using compressed air and then deoxygenated using a compressed nitrogen stream. UV-Vis absorbance measurements of oxygenated and deoxygenated samples may be recorded as a function of pO2 via dual wavelength spectroscopy. Oxygen-PEM/myoglobin equilibrium curves may be fit to a four-parameter ($A_0$, A∞, $P_{50}$, n) Hill model:

$$Y = \frac{Abs - Abs_0}{A\infty - A_0} = \frac{pO_2^n}{pO_2^n + P_{50}^n} \qquad \text{(Eq. 1),}$$

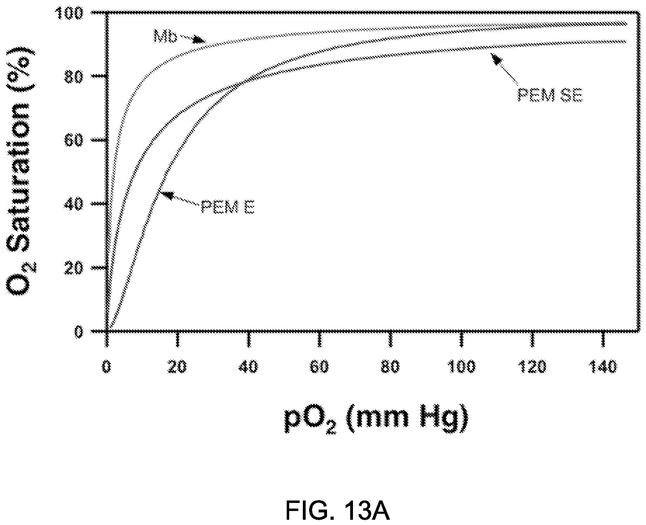

in which A0 and Aoo represent the absorbance at 0 mm Hg and at full O2 saturation, respectively. The $pO_2$ represents the partial pressure of O2, and P50 represents the partial pressure of O2 where PEM/Mb is 50% saturated with oxygen. Lastly, 'n' represents the cooperativity coefficient of PEM/Mb. In example studies, the P50 for PEM before (PEM-SE) and after proteolysis (PEM-E) were found to be 7.9 and 17.1 mm Hg, respectively, which were both significantly higher than the P50 for free Mb (~2 mm Hg). These results are shown in FIG. 13A, which illustrates oxygen saturation of PEM having myoglobin associated both on the surface and in the aqueous cavaties of the polymersomes (i.e. PEM-SE), PEM having myoglobin associated only in the aqueous cavaties of the polymersomes (i.e. PEM-E), and free myoglobin as a function of partial pressure of oxygen. The lower $O_2$ affinity (higher $P_{50}$) of PEM than free myoglobin may be attributed to the shielding effect of the polymersome membrane, which was further found to delay the kinetics of $O_2$ binding to PEM.

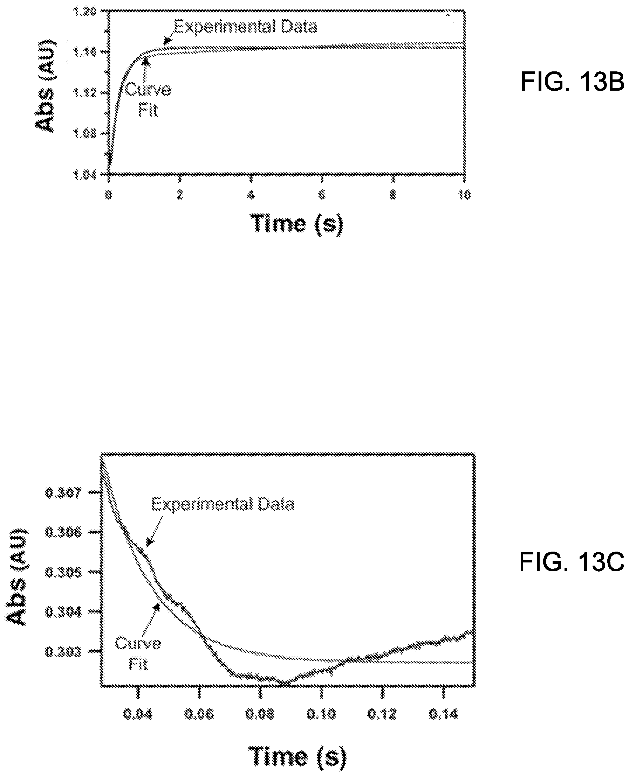

In various embodiments, kinetic measurements of binding/release for various gaseous ligands ($O_2$ and NO) to PEM and free myoglobin may be performed using an Applied Photophysics SX-20 microvolume stopped-flow spectrophotometer. Example measurements of kinetic curves were fit to a first order exponential equation to regress the pseudo rate constants, which were found to be first order in the case of NO and zero-order for $O_2$ dissociation. By measuring kinetic time courses at different NO concentrations, the apparent first order rate constants were plotted against NO concentrations, and the slope of the fitted line yielded the second order rate constant for NO deoxygenation. FIG. 13B shows $O_2$ dissociation time courses of PEM, where all unencapsulated and surface-associated myoglobin had been removed by proteolysis and in which the remaining myoglobin had been reduced to oxymyoglobin in the presence of 1.5 mg/mL of $Na_2S_2O_4$.

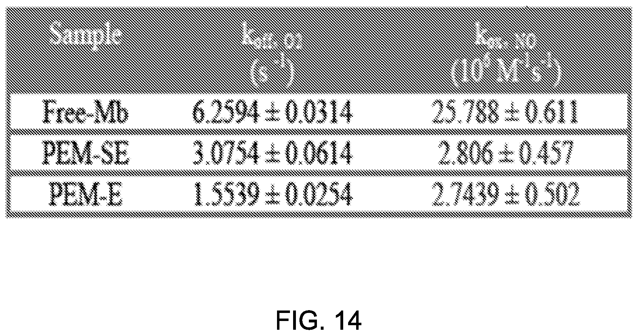

FIG. 14 is a table showing kinetic rate constants for oxygen dissociation ($K_{off, O_2}$) and NO-mediated deoxygenation ($K_{ox, NO}$) for various PEM formulations in comparison to free (unencapsulated) myoglobin (Mb).

FIG. 13C shows the kinetic time course for NO-mediated deoxygenation of PEM in which all unencapsulated and surface-associated myoglobin had been removed by proteolysis and in which the remaining myoglobin had been reduced to oxymyoglobin. The absorbance from the deoxygenation reaction was observed at 437.5 nm in 0.1 M phosphate buffered saline (PBS), pH 7.4 at 20°C. The NO deoxygenation time courses of PEM were acquired based on absorbance changes at 420 nm and 20°C following rapid mixing of oxygenated PEM (7.5 $\mu$M heme) and appropriate dilutions of the NO stock solution.

In various embodiments, the oxygen dissociation constants ($k_{off, O_2}$) as well as NO deoxygenation constants ($k_{ox, NO}$) of PEM-SE and PEM-E may be significantly lower than those of free myoglobin. Such differences may be due to the shielding effect of the thick polymersome membrane, which may effectively serve as a barrier between PEM and gaseous ligands in the environment, thereby delaying $O_2$ dissociation, NO deoxygenation, and the equilibrium binding of these gasses to PEM. The shielding effect is also consistent with observed differences in kinetic and equilibrium binding of gaseous ligands in polymersomes that have both surface associated and encapsulated myoglobin (PEM-SE) as compared to PEM formulations that have been treated with pronase to remove non-specifically bound surface myoglobin (PEM-E). Surface-associated myoglobin may quickly react with exposed gases, and PEM-SE therefore may display higher $k_{off, O_2}$ than PEM-E. Overall, PEM may display an $O_2$ affinity that is higher than RBC-bound hemoglobin and lower than free myoglobin. Examples of kinetic rate constants for oxygen dissociation ($K_{off, O_2}$) and NO-mediated deoxygenation ($K_{ox, NO}$) for various PEM formulations in comparison to free (unencapsulated) myoglobin (Mb). are shown in the table of FIG. 14.

In various embodiments, negative controls may be prepared using near-infrared (NIR) - myoglobin. For example, a near-infrared fluorophore (IRDye800cw NHS) may be covalently attached to the lysines on horse skeletal myoglobin to form the control NIR-myoglobin. In mice models, tail veins may be catheterized using 30G needles and heparinized saline. empty polymersomes (a first control), NIR-myoglobin (a second control), and PEM suspensions may be administered via invtravenous (IV) injection. Following injection, the catheters may be flushed with heparinized saline to ensure accurate dosage.

**Example Study Results**

In example studies, 0.5 x $10^6$ to 1 x $10^6$ 4T1 mouse mammary carcinoma cells were injected into the mammary fat pads of balb/c mice. Once the tumors were greater than 5 mm in diameter, mice were randomized and treated via tail vein catheter with PEMs (at 100 $\mu$L of 2.5 mg/mL Mb, corresponding to 50 mg/mL polymer suspensions), empty polymersomes (at equivalent polymer doses), and NIR-Mb (at equivalent myoglobin doses) (n = 5 per group). NIR-Mb and PEM were reduced to their oxy-myoglobin form prior to treatment, following protocols discussed above. Mice were sacrificed at 24 h (for biodistribution/pharmacokinetic studies) or at 6-9 days (for histology and toxicology assessment following tumor efficacy studies). In both cases, EF-5 was used to stain for hypoxia and Hoechst solution for perfusion. Tumors were collected at sacrifice. Immunofluorescence (IF) was carried out to image EF-5 and Hoechst distribution throughout the tumors. The same tumor sections were also stained with hematoxylin and eosin (H&E) for immunohistologic (IHC) analysis. Blood was collected via cardiac puncture at sacrifice; plasma was isolated and stored at -80 °C for later analyses.

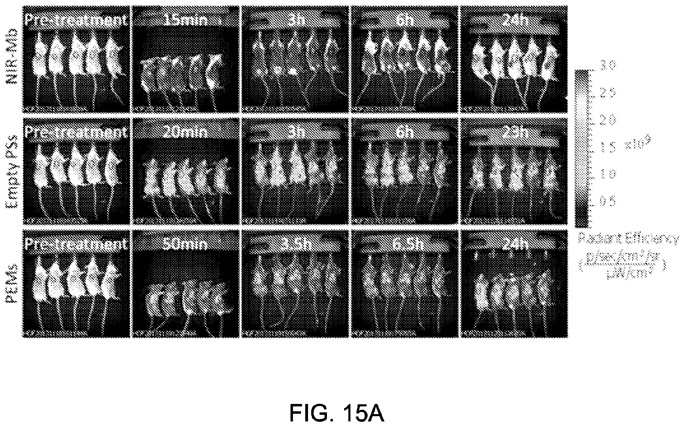

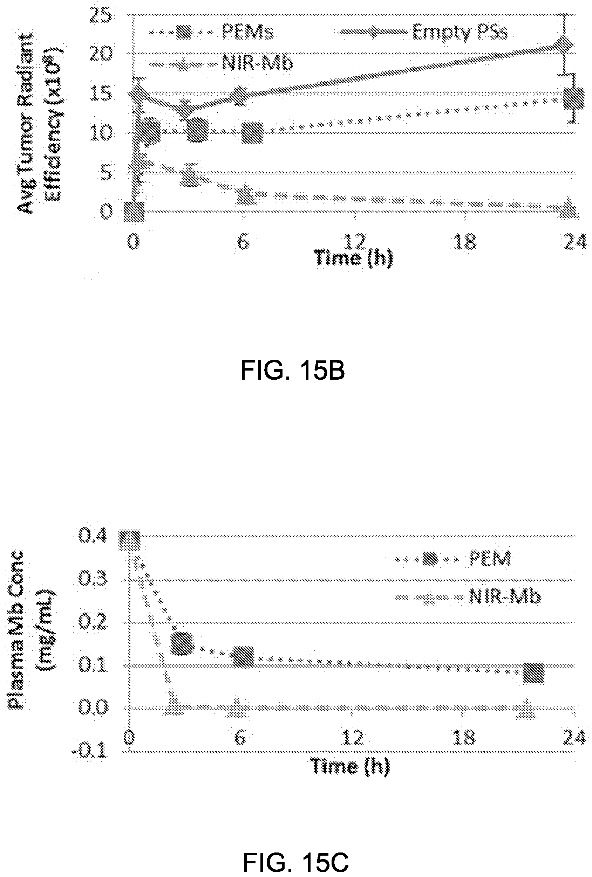

FIG. 15A shows the biodistribution, tumor accumulation, and pharmacokinetics of NIR-Mb, empty polymersomes, and PEM at points following IV injection. Empty polymersomes and PEM constructs had a fluorescent NIR-dye (PZn$_3$) incorporated to enable *in vivo* optical imaging of particle biodistribution, as well as pharmacokinetic determinations of plasma concentrations via quantification of fluorescence signals, which were subsequently compared to initial fluorescence values and correlated to Mb concentrations determined by ICP-OES prior to administration. FIG. 15B shows such plasma concentrations of empty polymersomes, PEM, and NIR-myoglobin as a function of time, while FIG. 15C shows the correlated myogblobin concentrations as a function of time. As illustrated by FIGs. 15A-15C, uptake and pharmacokinetics of PEM appeared to be similar to that of empty polymersomes, which demonstrated a rapid distribution phase, a slower clearance phase, and an overall plasma half-life of approximately 15 h. NIR-Mb, on the other hand, exhibited an extremely rapid plasma clearance (via the kidneys and into the bladder), as expected from the known biodistribution and clearance of free Mb.

For all biodistribution studies, NIR imaging of polymersomes, NIR-Mb and PEM was conducted with an MS® Kinetic Imaging System (Perkin Elmer, AeX= 745 nm, Aem= 810-875 nm) equipped with a Xenogen XGI-8 gas anesthesia system. Fluorescence images were analyzed with Living Image® 4.2 software by measuring radiant efficiency of regions of interest (ROIs). Radiant efficiency ([photons/s]/[$\mu$W/cm$^2$]) corrects for non-uniform excitation and differences in exposure times, providing a consistent measurement between samples.

For the pharmacokinetic studies, 15-100 $\mu$L blood samples were drawn (using a 5.5 mm lancet, Goldenrod) from the submandibular vein of the mouse and collected directly into heparinized capillary tubes (Kimble). The capillary tubes were centrifuged for 3-5 min and imaged immediately using the MS® Kinetic Imaging System. The total radiant efficiency of the plasma in each capillary tube was measured in a region of interest of consistent dimensions. Plasma concentrations of PEM and empty polymersomes were calculated based on a standard curve, which was first determined by adding solutions of known concentrations of PZn$_3$-loaded emissive polymersomes to untreated plasma and measuring fluorescence from the capillary tubes. Pharmacokinetic results were plotted as plasma concentrations of PEM and empty polymersomes versus time. Myoglobin concentration versus time was estimated based on the PZn$_3$ fluorescence of the PEM and the IRDye800 fluorescence of NIR-Mb, respectively, with the assumption that all myoglobin remained associated with the PEM following injection.

***Orthotopic Tumor Response to PEM Administration:***

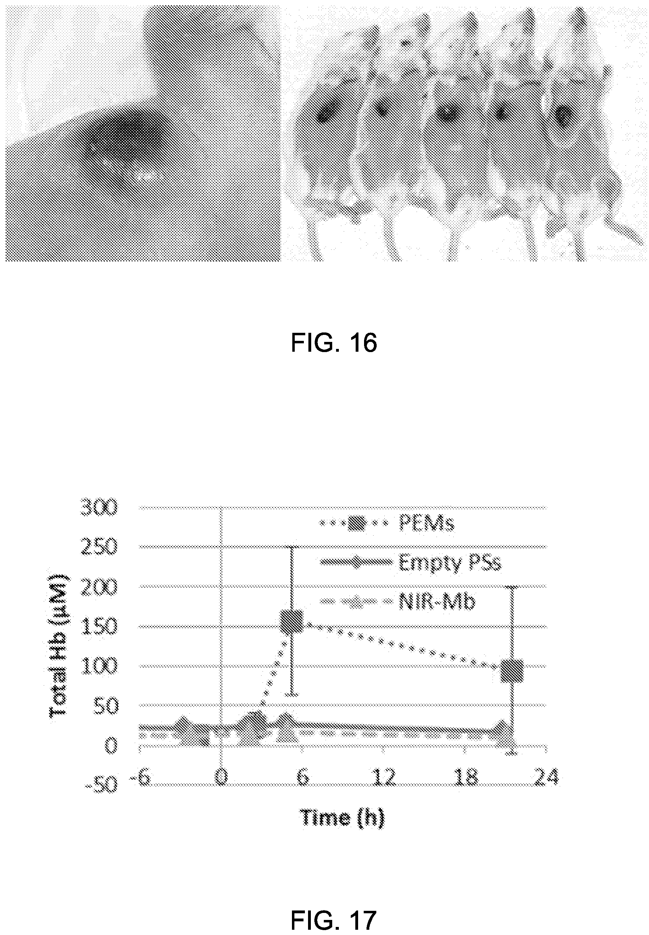

As shown in FIG. 16, within approximately 3-6 hours, 5 of 5 tumors began to change from their initial pink color (as seen in the surrounding normal tissue) to a dark red or black color. These results correspond to an accumulation of RBC-bound hemoglobin (Hb) as assessed by optical spectroscopy (from Zenascope, by Zenalux, Inc.). The spectroscopy results are shown in FIG. 17, which provides total hemoglobin concentration as a function of time for PEM, empty polymersomes, and NIR-myoglobin formulations. Without wishing to be bound to a particle theory, this phenomenon may be due to tumor vascular occlusion and nanoparticle-mediated microvascular embolization (NME) produced by PEM. PEM-induced NME was not seen in the liver, spleen or other normal tissues of PEM biodistribution and thus is shown to be tumor-specific.

High-Resolution Window Chamber Studies of PEM Activity at the Microvascular Level:

Dorsal skin flap window chambers were used to image the tumor microenvironment as described previously.

To carry out window chamber studies, Balb/c mice were shaved and Nair was applied prior to surgery. During surgery, 1-1.5 x 105 4T1 cells were injected into the fascia. 7-9 days following surgery, tumors were visible within the windows. Mice were then treated via tail vein catheter with 150 - 200$\mu$L of 50 mg/mL polymer suspensions, corresponding to empty polymersomes or PEMs. Mice were anesthetized with isofluorane via nose cone and the windows were secured to the microscope stage. Imaging was carried out prior to and during injection of the treatments, and at various time points over the course of the experiment until 48 h.

Empty polymersomes and PEM constructs were imaged within the window chambers using fluorescence imaging of NIR emission from $PZn_3$ fluorophores within their membranes. Brightfield images were also collected over time. Hyperspectral imaging was used to determine hemoglobin concentration and oxygen saturation based on Hb absorbance spectra in blood. With brightfield illumination, a liquid crystal tunable filter was used to collect images over 500-620 nm. Spectral deconvolution for each pixel was carried out using MatLab to create images of oxygenated hemoglobin saturation. The hemodynamic properties of the tumors were also studied. Videos of blood flow in the window chambers were taken with the tunable filter set to 560 nm, where hemoglobin has high absorption. Pixel-by-pixel cross-correlation was applied to the videos and analyzed using MatLab to create maps of flow velocities within tumor blood vessels.

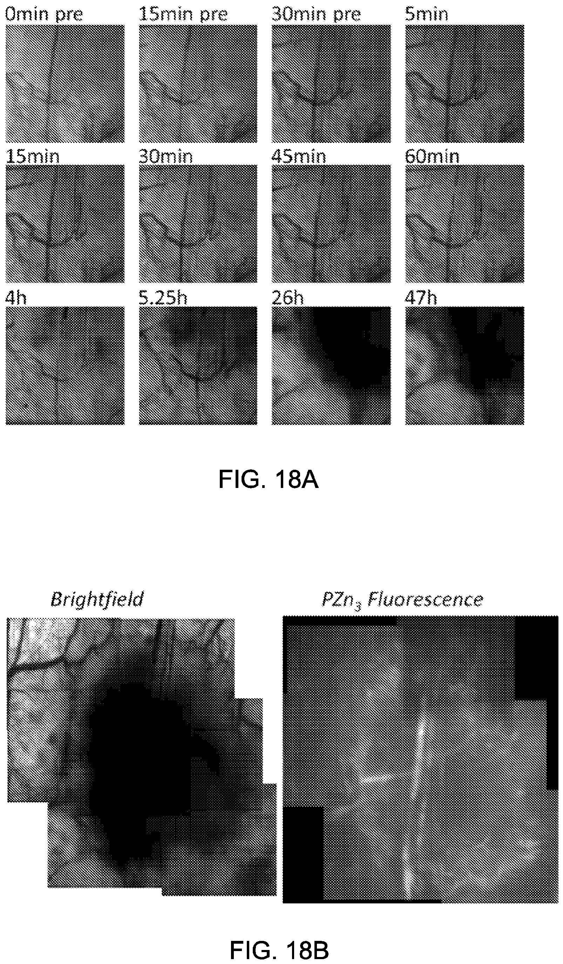

FIG. 18A shows a typical mouse tumor during treatment with PEM and empty polymersomes. Vascular occlusions become apparent within one hour following PEM administration, and more severe over time. By the 4 h time point, darkening of the tumor region becomes pronounced and continues to increase in severity for up to 48 h.

FIG. 18B shows a brightfield image (left) and a near-infrared (NIR)-fluorescence image of using $PZn_3$ (right) of the tumor shown in FIG. 18A at 24 hours after administration of PEM, where the NIR fluorescence corresponds to the localization of PEM within the tumor

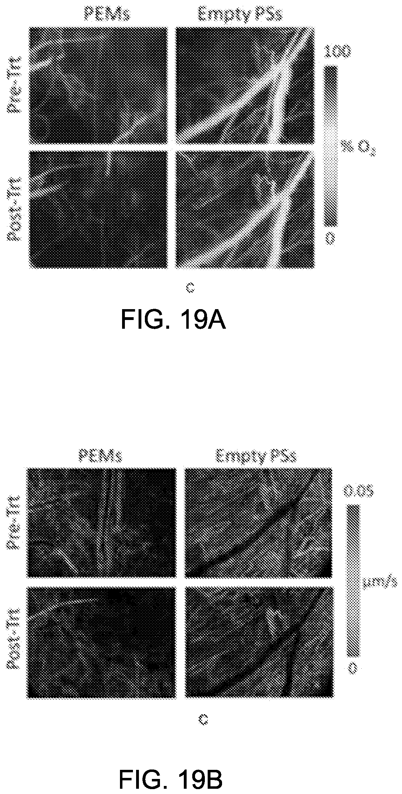

FIGs. 19A and 19B show hemoglobin saturation and blood flow velocity maps for a representative tumor, respectively. Each of these maps show the representative tumor before and after treatment with PEM and empty polymersomes (control). PEM treatment results in a substantial reduction in oxygenated hemoglobin saturation following treatment, as well as a marked decrease in blood flow, which becomes more pronounced in severity over time. These results are not seen with empty polymersome treatments.

### Results of Excised Tumors Demonstrate PEM-induced NME:

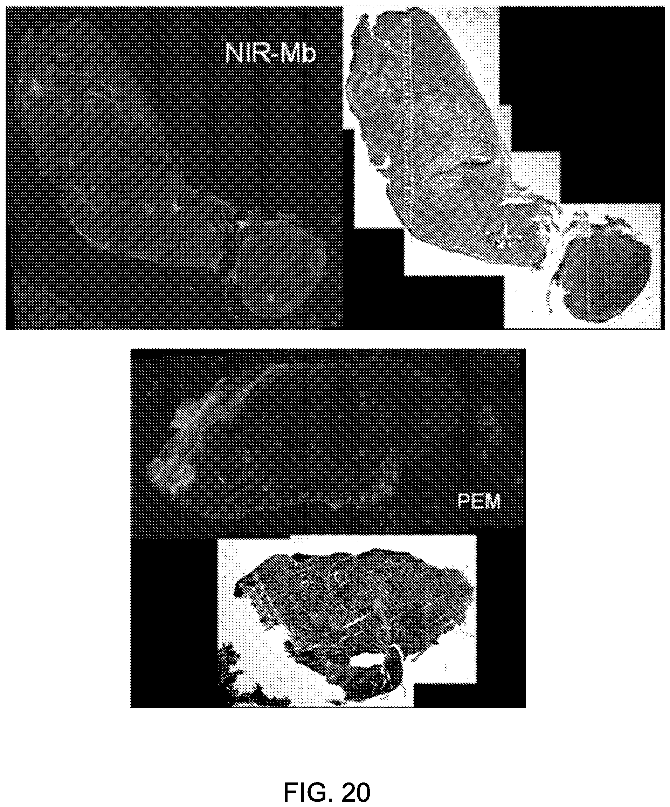

FIG. 20 illustrates representative immunohistochemistry (IHC) and Hematoxylin and Eosin (H&E) images of excised tumors for a NIR-myoglobin control and PEM treated animal. In PEM treated animals, there are vastly enhanced areas of necrosis, which comprise the majority of the tumor sections, as well as near complete obliteration of perfused vessels corresponding to NME of tumors. These effects were not seen with administration of empty polymersomes.

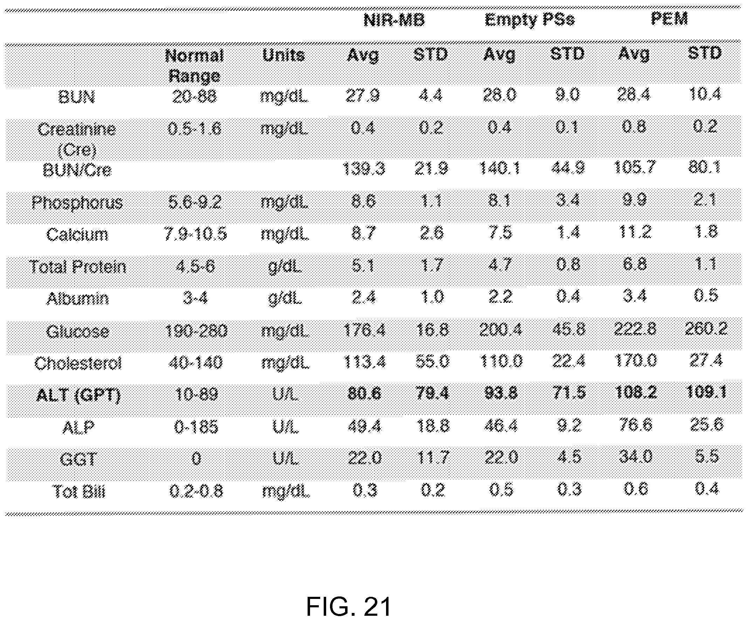

FIG. 21 provides results of a serum chemistry panel done for a set of tumor-bearing mice injected with each of the following treatments (n = 5 mice per treatment): NIR-myoglobin (control), empty polymersomes (control), and PEMs. While there were some elevated individual values distributed throughout the groups, there were no consistently elevated levels for any parameter irrespective of PEM or control treatments.

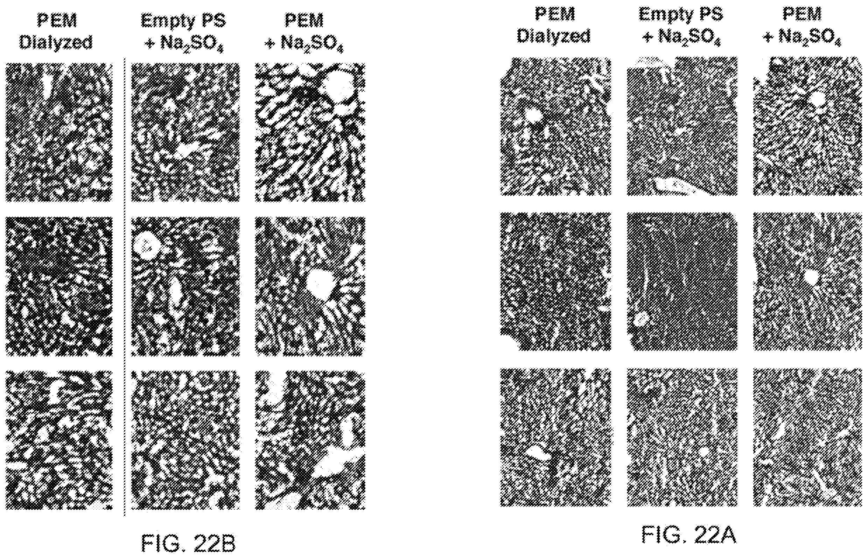

The liver is the major organ of polymersome/PEM accumulation, and thus the histology of livers may be performed to determine any corresponding microvascular damage to normal tissues. In example studies, each treatment suspension may be mixed with $Na_2S_2O_4$ to regenerate oxy-myoglobin prior to infusion. Animals were both directly injected with this solution mixture (of PEM and $Na_2S_2O_4$) as well as with dialyzed PEM suspensions (where $Na_2S_2O_4$ had been removed) to determine any potentiating effects. Livers from orthotoptic 4T1-xenografted mice treated with PEMs and empty polymersomes (control) were also excised upon sacrifice (6-9d post-treatment, 14-17d post-tumor injection) for H&E staining and microscopy. Each treatment suspension was also mixed with $Na_2S_2O_4$ to regenerate oxy-myoglobin prior to infusion. Animals were both directly injected with this solution mixture (PEM and $Na_2S_2O_4$) as well as with dialyzed PEM suspensions (where $Na_2S_2O_4$ had been removed) to determine any potentiating effects. FIGs. 22A and 22B are 20x magnification and 40x magnification H&E images, respectively, of excised livers from tumor-mice treated with PEM and empty polymersomes. As shown in these H&E images, hepatotoxicity was observed from any formulation, again confirming the tumor-specificity of PEM-induced NME.

### Example VI

Based upon the proposed mechanism of action of PEM (i.e. its NME effect on tumors), PEM may be particularly effective as a first-line therapy for the most highly vascularized tumors. An example target may be, clear cell RCC (CC-RCC), which is chemotherapy resistant but responsive to cancer therapeutics that inhibit angiogenesis. In various embodiments, a mouse model of RCC may be used to test the effectiveness of PEM in promoting the selective NME of RCC tumors and inhibiting their growth.

In various studies, ectopic and orthoptopic xenografts of human RCC tumor cells and/or allografts of mouse RCC tumor cells in immunocompromised mice (NCr *nu/nu*) may be used to generate mouse models of RCC. As discussed above, PEM is able to induce tumor-specific NME after a single administration at 10-15 mg/kg Mb (corresponding to 200-300 mg/kg polymer). In order to establish its efficacy for RCC, optimal dose levels that achieve maximal effect with minimal toxicity may be determined.

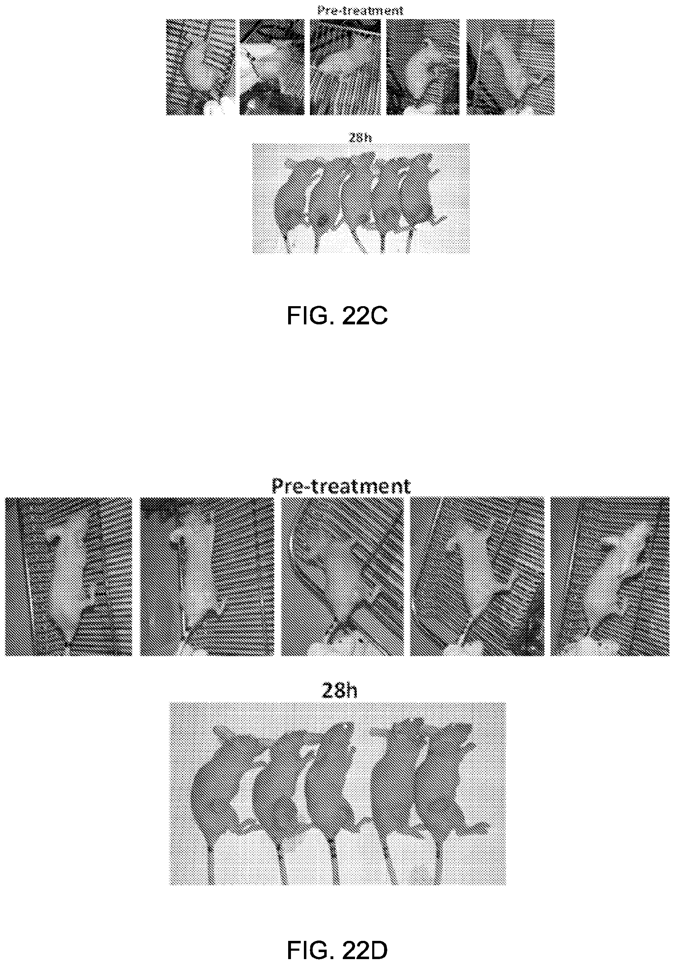

FIG. 22C shows mouse RCC tumors approximately prior to treatment with PEM, and approximately 28 hours after treatment. The change in color from their initial light (as seen in the surrounding normal tissue) to a dark color may correspond to an accumulation of RBC-bound hemoglobin (Hb) as assessed by optical spectroscopy (from Zenascope, by Zenalux, Inc.). In comparison, FIG. 22D shows mouse RCC tumors approximately prior to, and approximately 28 hours following, treatment with empty polymersomes. Any color changes observed in the tumors in FIG. 22D are significantly lower than those in FIG. 22C. Without wishing to be bound to a particular theory, these results may show that PEM-induced NME occurs in mouse tumors other than mammary tumors, such as in RCC tumors.

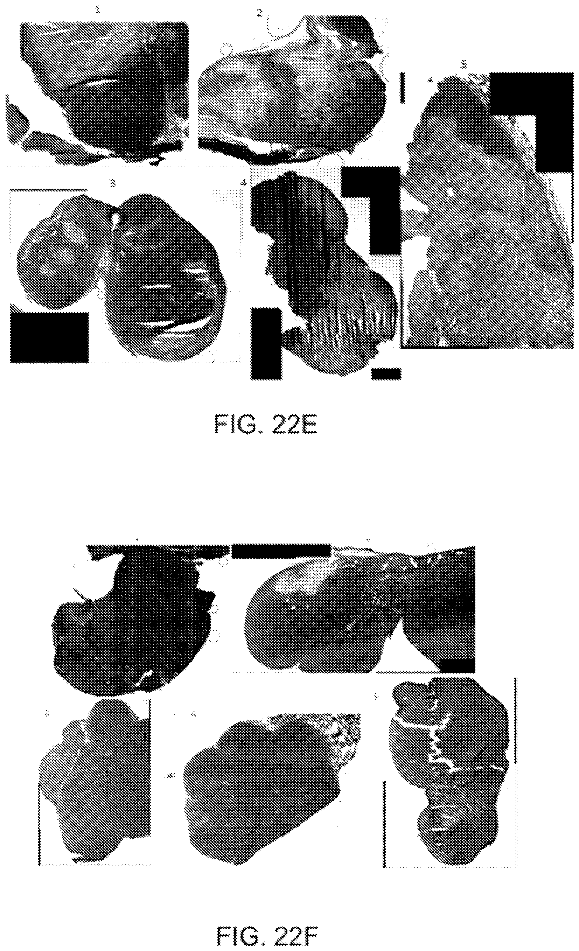

FIGs. 22E and 22F illustrate representative IHC and H&E images of excised tumors from mouse models of RCC treated with PEM and with empty polymersomes, respectively. In the PEM treated animals, there are vastly enhanced areas of necrosis shown in FIG. 22E, which comprise the majority of the tumor sections. Such areas are not similarly shown in FIG. 22F for empty polymersome treatment.

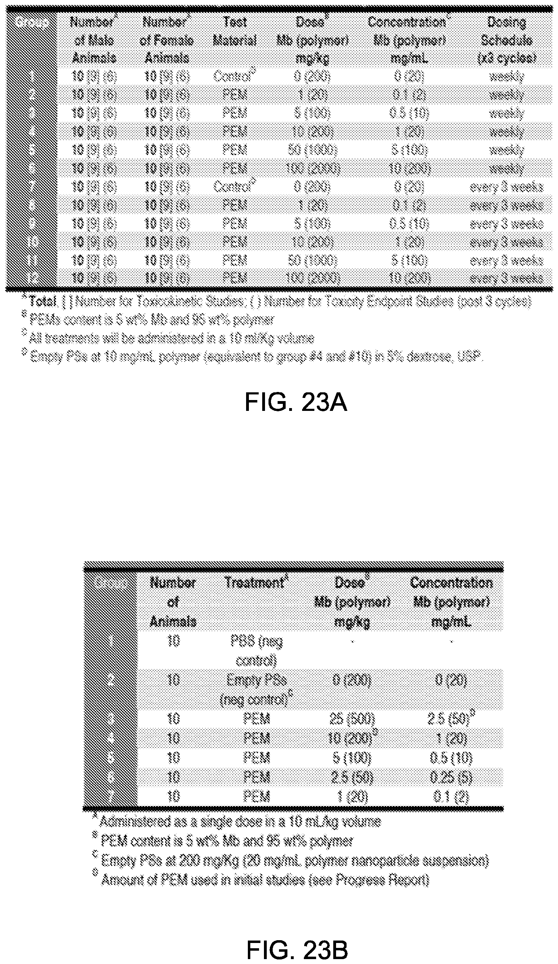

Gram scale preparations of PEM may be generated under GLP conditions, and dose escalation studies may be conducted to determine its therapeutic window as a single agent The table in FIG. 23A provides parameters for studies to determine any DLTs and the table in FIG. 23B provides parameters for studies to determine the MED of PEM to promote NME of RCC. The highest dose of PEM for safe administration may be set as the STD10 (the severely toxic dose in 10% of animals) or the MTD75 (the maximum tolerated dose that results in less than 15-20% weight loss in 75% of animals), if no DLTs are discovered.

Biodegradable PEM Dispersions may be formed using purified human myoglobin and diblock copolymers of PEO(2k)-b-PCL(12k) and PEO(2k)-b-PMCL(9.4k). PEM formation may be performed by a modified direct rehydration protocol as discussed above. For in vivo optical imaging studies, NIR-emissive PEM constructs may be generated via co-incorporation of $PZn_3$ fluorophores as also discussed above. For each PEM batch, particle sizes may be determined by dynamic light scattering (DLS) and viscosity may be measured using a microviscometer, oxygen and NO binding as well as the concentrations of myoglobin, final wt% of myoglobin/polymer, and wt% of met-myoglobin may be determined, following established methodology. *In situ* changes in met-myoglobin level, NO uptake, and myoglobin release from biodegradable PEM formulations may be tested under various solution conditions (e.g. temperature, pH, $pO_2$, and pNO) and at various time points, to verify product stability. The following kinetic parameters and end points may be evaluated: clinical signs (hourly for the first 24 h, then every 12 h for 3 days, and weekly thereafter), body weights and changes (2-3 times per week), food consumption (2-3 times per week), abbreviated functional observational battery (2-3 times/week), ophthalmologic assessments (2-3 times per week), serologic profiles (hematology, coagulation, clinical chemistry, and urinalysis (performed weekly), toxicokinetic parameters (hourly blood draws for the first 24 h, then every 12 h for 3 days, and weekly thereafter, gross necropsy findings (upon sacrifice after completion of third cycle of treatment), organ weights (upon sacrifice), and histopathologic examinations (upon sacrifice).

### Generation of ectopic and orthotopic RCC xenografts in NCr nu/nu mice:

A human RCC cell line, 786-0 (VHL$^{-/-}$) may be obtained from American type culture collection (ATCC), maintained and propagated in RPMI 1640 media (GIBCO), and supplemented with 10% heat-inactivated fetal bovine serum at 37°C and 5% $CO_2$. Female athymic NCr *nu/nu* mice may be implanted subcutaneously with 1 mm$^3$ tumor fragments of 786-0 cells for the ectopic RCC xenograft models. For the orthotopic RCC xenograft models, male athymic NCr *nu/nu* mice may be implanted with similar tumor fragment volumes by making an incision in the renal capsule and placing the donor fragment underneath the capsule sheath.

### Determination of MED to induce NME:

Window chambers may be surgically implanted in ectopically xenografted RCC tumor-bearing animals. PEM suspensions may be prepared as discussed above, and injected via the tail vein when tumors reach 4-6 mm in diameter. Hyperspectral imaging of hemoglobin absorption may be used to quantify hemoglobin oxygen saturation, and HIF-1 activity may be evaluated by measuring GFP emission. To determine PEM location, *in vivo* optical imaging may be performed before and after each tail-vein injection of PEM at t = 0, 2, 4, 6, 24, 48 and 72 h, during which animals may be anesthesized using 1-2.5% isofluorane and maintained on a warming pad.

Since, in contrast to conventional anti-angiogenesis and immune-based therapies, the mechanism of NME is not reliant upon the tumor's expression of specific molecular targets; hence, PEM may prove to be a more uniformly effective first-line therapy of RCC. In various embodiments, PEM may be administered in different dosing levels and schedules, with and without the VEGFR TKI sunitinib, to determine the therapeutic combination that achieves maximal activity against treatment-naive RCC. The preclinical efficacy of PEM may be established if any treatment achieves a duration of tumor growth inhibition that is greater than 1.5 times that of sunitinib alone, and no associated DLTs are observed.

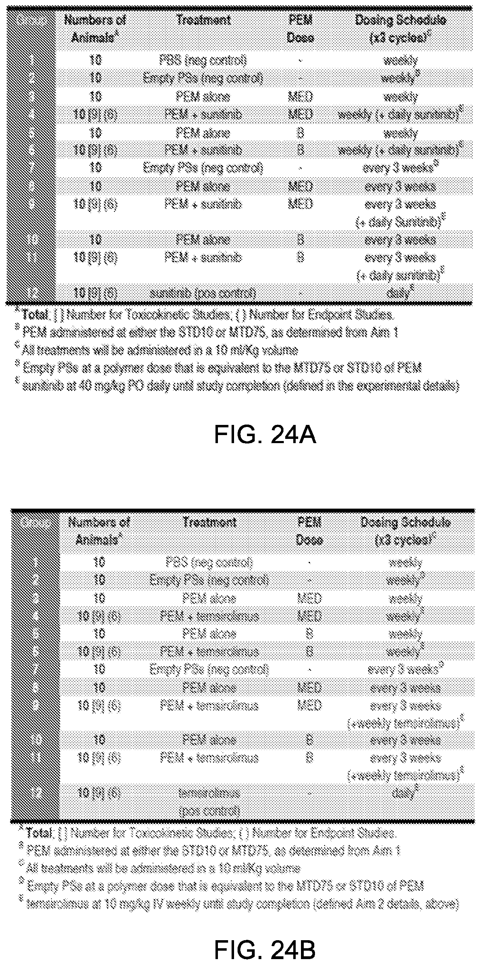

Moreover, as NME works by a different mechanism of action than VEGFR TKIs, PEM may enhance the tumor response seen with these agents. As such, the therapeutic effects may be observed of two different dose levels and two dosing schedules, when PEM is administered as both a single agent and in combination with sunitinib, in murine models of RCC. FIG. 24A provides parameters for a study to develop a single combination of PEM dose level, schedule, and treatment (i.e. either as a single agent or with sunitinib) in a first-line therapy that results in maximal tumor growth inhibition, and no severe toxicities to treated animals.

***Determination of Tumor Growth Inhibition in Response to PEM Dosing Schedules and Combinations:***

786-0 cells that have been engineered to constitutively express luciferase may be utilized to generate orthotopically xenografted RCC tumor-bearing animals. Luciferase imaging may subsequently be used to monitor tumor size and to track metastases. Luciferase intensity and body weights may be recorded two to three times a week, starting with the first day of treatment. A preliminary calibration curve may be generated to relate total tumor radiance to tumor volume, which may be directly measured at the time of sacrifice. Treatments may be initiated when tumors reach 100 mm$^3$ and treatment efficacy may be measured as a percent tumor growth inhibition (TGI) relative to control groups. TGI may be calculated by the Equation 2 below:

$$TGI = (1 - T/C) \times 100 \qquad (\text{Eq. 2}),$$

where T and C represent the mean tumor mass on the last day of therapy in treated (T) and control (C) groups, respectively. A TGI of greater than 50%, in which T is also significantly smaller than C (as assessed by t-test), may be considered efficacious. Error bars may be calculated as the standard error of the means. The general health of animals may be monitored daily until tumors sizes have increased by 5 times, tumors reach a volume of 500 mm$^3$, or signs of significant animal distress are observed (e.g., greater than 15% loss in body mass). Toxicity parameters may be determined for all combinations of PEM and sunitinib. At the end of treatment, all tumors may be removed and surface photomicrographs taken. The tumors may then be fixed in 10% formalin and embedded in paraffin for IHC analyses, as below. Data are assumed to be normal upon transformation, and a t-test may be used to test for statistical significance. Assuming a standard deviation of 50% of the control mean, a minimum of 10 animals per group may be needed to ensure a greater than 80% power of detecting a TGI of more than 50%. Multiple comparisons may be corrected for by false discovery rate.

***IHC Analysis for Inhibition of tumor Angiogenesis, Cell Proliferation, Extent of Apoptosis, and HIF-1 Signaling:***

Inhibition of tumor angiogenesis may be assessed by measuring levels of CD31 and aSMA, cell proliferation by Ki-67 staining, the extent of tumor apoptosis by terminal deoxynucleotidyl transferase-mediated nick end labeling, and tumor hypoxia by Hypoxyprobe-1 Plus Kit, following previously established protocols.

The preclinical efficacy and safety of PEM, as a first line therapy, as a single agent or in combination with the VEGFR TKI sunitinib, may be established if any treatment achieves a duration of TGI that is greater than 1.5 times that of Sunitinib alone, and no DLTs are observed.

In other embodiments, the efficacy of PEM as second-Line therapy (i.e. in VEGFR TKI-resistant RCC) may be determined. PEM may be administered in different dosing levels and schedules, with and without the mTOR-inhibitor temsirolimus, to determine the most effective therapeutic combination against VGFR TKI-resistant RCC.

Currently, mTOR-inhibitors achieve modest response rates against VEGFR TKI-resistant RCC, but are active only for a limited period. Tumor selectivity of NME, coupled with the lack of apparent systemic toxicities, may make PEM particularly suited for this heavily treated patient population. FIG. 24B provides parameters for a study to determine the therapeutic effects of different dose levels and dosing schedules when PEM is administered as a second-line therapy, both as a single agent and in combination with temsirolimus.

In various embodiments, orthotopically xenografted RCC tumor-bearing animals may be generated and subject to treatment with sunitinib (40 mg orally administered per day), which may be initiated when tumors reach 100 mm$^3$ and continued until they grow to 1.5 times their initial size. At this point, they may be randomized for second-line therapy and monitored, as discussed above. The preclinical efficacy and safety of PEM, as a single agent or combination with the mTOR inhibitor temsirolimus, may be established if any treatment achieves a duration of TGI that is greater than 1.5 times that of temsirolimus alone, and no DLTs are observed.

In various embodiments, the benefits in combining PEM with XRT as palliative treatment for RCC may be determined. PEM may be administered after XRT in order to evaluate its ability to prolonging local control of RCC.

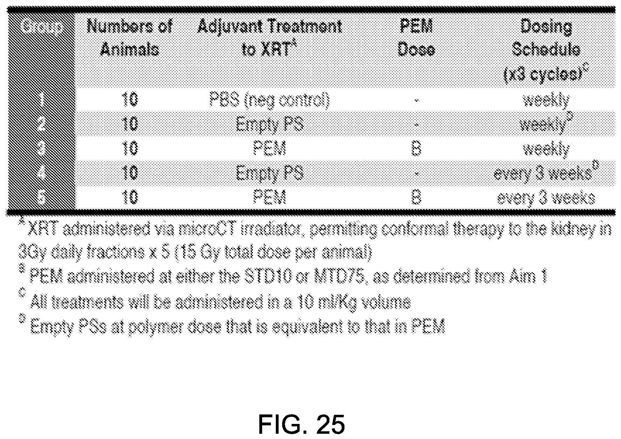

Currently, palliative radiotherapy after nephrectomy is used in high-risk patients to improve local control, but it does not have an OS benefit. These patients have a high propensity for developing distant metastases, which may explain the lack of survival. Thus, PEM may be used to augment XRT as palliative treatment for RCC. FIG. 25 provides parameters for a study to determine a single PEM dose level and schedule in a palliative treatment that results in maximal tumor growth inhibition, and a decreased number of new metastases as measured over weekly intervals for one month. Orthotopically xenografted RCC tumors-bearing animals may be established, as discussed above, and treated daily with a fractionated dose of 3Gy x5 to the renal bed (via PXi X-Rad 225Cx, orthovoltage image-guided irradiator). Following PEM administration, tumor growth may be assessed at weekly intervals for 1 month, and the number of metastases may be determined at sacrifice. The benefit of combining PEM and XRT may be established if administration results in more than 50% TGI; and, there is a 25% reduction in the number of metastases as compared with animals that undergo XRT alone, by one month after treatment.

In various embodiments, PEM scale-up may also be achieved via a tangential flow filtration apparatus, following established techniques. For animal studies, if the MTD75 is not established within the first five initial dose levels, two additional PEM dose levels may be selected (e.g. 200 and 500 mg/kg myoglobin). In addition to 786-0 cells, A498, 769-P, Caki-1, Caki-2, SW839, ACHN, G401 and/or SK-NEP-1 cell lines (all available from ATCC) may be used to generate mouse models of RCC. Further, NOD/SCID mice may be utilized to generate tumor xenografts, if tumors in NCr *nu/nu* mice fail to grow or respond effectively. The transgenic model of cancer of the kidney (i.e., the "TRACK" mouse), which expresses a constitutively active HIF-1a in kidney proximal tubule cells, may alternatively be employed. Different dosages of temsirolimus and/or, alternatively, everolimus may be examined if animals experience excess toxicities with temsirolimus treatment after sunitinib, as assessed for positive control (temsirolimus only) animals. Further, alternative doses (e.g. 30 Gy) or modes of XRT (i.e. SBRT) may be employed if tumor growth is refractory to treatment.

## Claims

1. A composition comprising a nitric oxide (NO)-inhibiting agent and a carrier vehicle, wherein the (NO)-inhibiting agent is for use in treating highly vascularized tumors by causing microvascular embolization in the tumor,

   wherein the carrier vehicle is a nanoparticle, and wherein the nanoparticle is a polymersome,
   wherein the (NO)-inhibiting agent comprises an (NO)-binding molecule;
   wherein NO binding is enabled only at oxygen tensions of less than 10 mm Hg;
   wherein the (NO)-binding molecule is selected from one or more of unmodified human myoglobin, unmodified myoglobin from another biological species, and chemically or genetically modified myoglobin from humans or from another biological species;
   wherein the (NO)-binding molecule is preloaded with oxygen; and
   wherein the NO-inhibiting agent is encapsulated within the carrier vehicle.

2. The composition for use of claim 1, wherein the NO binding is enabled only at oxygen tensions of less than 5 mmHg.

3. The composition for use of claim 1, wherein the composition further comprises at least one of a NO synthase (NOS) inhibitor and an antioxidant co-encapsulated with the NO-binding molecules in the polymersomes.

4. The composition for use of claim 1, wherein the composition further comprises at least one of a chemotherapy agent and an angiogenesis inhibiting agent co-encapsulated with the NO-binding molecules in the polymersomes.

5. The composition for use of claim 1, wherein the composition further comprises at least one of a vascular endothelial growth factor receptor (VEGFR) tyrosine kinase inhibitor (TKI) and a mammalian target of rapamycin (mTOR) inhibitor co-encapsulated with the NO-binding molecules in the polymersomes.

6. The composition for use of claim 1, wherein the polymersome:

   a) comprises a synthetic polymer vesicle, and wherein the NO-inhibiting agent is within an aqueous core of the polymer vesicle;
   b) comprises a synthetic polymer vesicle, and the NO-inhibiting agent is within a membranous portion of the polymer vesicle;
   c) is a uni- or multi-lamellar polymersome;
   d) comprises a plurality of biodegradable polymers, optionally wherein the plurality of biodegradable polymers form a nanoparticle, optionally wherein the nanoparticle is less than 200 or less than 100 nanometers in diameter; or

e) co-encapsulates the NO-inhibiting agent with at least one other radiation-sensitizing or chemotherapeutic agent.

7. The composition for use of claim 6, wherein the composition further comprises:

a plurality of polymersomes configured to accumulate at sites of interest via passive diffusion or via a targeting modality comprised of a conjugation of a targeting molecule separate from the nanoparticles, optionally wherein at least some of the plurality of polymersomes are biodegradable polymer vesicles and at least some of the plurality of polymer vesicles are biocompatible polymer vesicles; and optionally

wherein the biocompatible polymer vesicles are in part comprised of poly(ethylene oxide) or poly(ethylene glycol); wherein the biodegradable polymer vesicles are comprised of at least one block copolymer of poly(ethylene oxide) and poly($\varepsilon$-caprolactone), poly(ethylene oxide) and poly($\gamma$-methyl $\varepsilon$-caprolactone), or poly(ethylene oxide) and poly(trimethylcarbonate); or

wherein the biodegradable polymer vesicles are either pure or blends of multiblock copolymer, wherein the copolymer includes at least one of poly(ethylene oxide) (PEO), poly(lactide) (PLA), poly(glycolide) (PLGA), poly(lactic-co-glycolic acid) (PLGA), poly($\varepsilon$-caprolactone) (PCL), and poly (trimethylene carbonate) (PTMC), poly(lactic acid), poly(methyl $\varepsilon$-caprolactone).

8. The composition for use of claim 7, wherein the polymersome is formed from any one of: PEO-b-PCL, PEO-b-PMCL, and PEO-b-PTMC.

9. A composition for use according to claim 1, wherein the highly vascularized tumor is selected from the group consisting of: renal cell carcinoma (RCC), hepatocellular carcinoma (HCC), glioblastoma multiforme brain tumor (GBM) and multiple myeloma (MM).

10. The composition for use according to claim 1, wherein the composition is delivered to the tumor by introducing the composition into systemic circulation, wherein:
the composition accumulates within the tumor.

11. The composition for use according to claim 1, wherein the NO-inhibiting agent comprises NO-binding molecules that competitively bind oxygen ($O_2$) and NO, and wherein:

the oxygenated NO-binding molecules are introduced into systemic circulation; and
the NO-binding molecules become deoxygenated upon accumulation of the carrier particles in the tumor, thereby enabling the selective scavenging of NO in the tumor microvasculature.

12. The composition for use according to claim 11, wherein the accumulation of the composition in the tumor allows diffusion of NO into the carrier particles, wherein the selective scavenging of NO is performed at least in part by deoxygenation of the encapsulated NO-binding molecules.

13. The composition for use according to claim 1, wherein the selective prevention of normal NO activity in the tumor vasculature causes vasoconstriction and platelet aggregation in the tumor vasculature, optionally wherein the persistent hydrodynamic pressure in the tumor vasculature causes rupture of the platelet aggregation and bleeding into the tumor, and optionally wherein the bleeding into the tumor causes thrombosis of tumor vasculature and necrosis of tumor tissue.

14. The composition for use according to claim 1, wherein the composition is delivered to the tumor in conjunction with at least one of a vascular endothelial growth factor receptor (VEGFR) tyrosine kinase inhibitor (TKI), a mammalian target of rapamycin (mTOR) inhibitor, and radiotherapy.

15. The composition for use according to claim 1, wherein the composition is delivered to the tumor in conjunction with at least one of a chemotherapy agent and an angiogenesis inhibiting agent.

**Patentansprüche**

1. Zusammensetzung, die ein Stickstoffmonoxid (NO) inhibierendes Mittel und ein Trägervehikel umfasst, wobei das (NO) inhibierende Mittel zur Verwendung beim Behandeln von stark vaskularisierten Tumoren durch Bewirken einer mikrovaskulären Embolisation in dem Tumor ist,

wobei das Trägervehikel ein Nanopartikel ist und das Nanopartikel ein Polymersom ist,
wobei das (NO) inhibierende Mittel ein (NO) bindendes Molekül umfasst;
wobei eine NO-Bindung nur bei Sauerstoff-Partialdrücken von weniger als 10 mmHg ermöglicht wird;
wobei das (NO) bindende Molekül aus einem oder mehreren von unmodifiziertem menschlichem Myoglobin, unmodifiziertem Myoglobin von einer anderen biologischen Spezies und chemisch oder genetisch modifiziertem Myoglobin von Menschen oder von einer anderen biologischen Spezies ausgewählt ist;
wobei das (NO) bindende Molekül mit Sauerstoff vorbeladen ist und
wobei das NO inhibierende Mittel innerhalb des Trägervehikels eingekapselt ist.

2. Zusammensetzung zur Verwendung nach Anspruch 1, wobei die NO-Bindung nur bei Sauerstoff-Partialdrücken von weniger als 5 mmHg ermöglicht wird.

3. Zusammensetzung zur Verwendung nach Anspruch 1, wobei die Zusammensetzung weiterhin mindestens einen bzw. eines von einem NO-Synthase-Inhibitor (NOS-Inhibitor) und einem Antioxidans, der bzw. das gemeinsam mit den NO bindenden Molekülen in den Polymersomen eingekapselt ist, umfasst.

4. Zusammensetzung zur Verwendung nach Anspruch 1, wobei die Zusammensetzung weiterhin mindestens eines von einem Chemotherapeutikum und einem Angiogenese inhibierenden Mittel, das gemeinsam mit den NO bindenden Molekülen in den Polymersomen eingekapselt ist, umfasst.

5. Zusammensetzung zur Verwendung nach Anspruch 1, wobei die Zusammensetzung weiterhin mindestens einen von einem Vaskulären-endothelialen-Wachstumsfaktorrezeptor-Tyrosinkinase-Inhibitor (VEGFR-Tyrosinkinase-Inhibitor, VEGFR-TKI) und einem Säugetier-Target-von-Rapamycin-Inhibitor (mTOR-Inhibitor), der gemeinsam mit den NO bindenden Molekülen in den Polymersomen eingekapselt ist, umfasst.

6. Zusammensetzung zur Verwendung nach Anspruch 1, wobei das Polymersom:

a) ein synthetisches Polymervesikel umfasst, und wobei das NO inhibierende Mittel innerhalb eines wässrigen Kerns des Polymervesikels ist;
b) ein synthetisches Polymervesikel umfasst, und wobei das NO inhibierende Mittel innerhalb eines membranösen Abschnitts des Polymervesikels ist;
c) ein uni- oder multilamellares Polymersom ist;
d) mehrere biologisch abbaubare Polymere umfasst, optional wobei die mehreren biologisch abbaubaren Polymere ein Nanopartikel bilden, optional wobei das Nanopartikel einen Durchmesser von weniger als 200 oder weniger als 100 Nanometer aufweist; oder
e) das NO inhibierende Mittel gemeinsam mit mindestens einem anderen strahlungssensibilisierenden Mittel oder Chemotherapeutikum einkapselt.

7. Zusammensetzung zur Verwendung nach Anspruch 6, wobei die Zusammensetzung weiterhin umfasst:

mehrere Polymersome, die dazu konfiguriert sind, sich mittels passiver Diffusion oder mittels einer Targeting-Modalität, die sich aus einer Konjugation eines Targeting-Moleküls, das von den Nanopartikeln getrennt ist, zusammensetzt, an Stellen von Interesse anzusammeln, optional wobei mindestens einige der mehreren Polymersome biologisch abbaubare Polymervesikel sind und mindestens einige der mehreren Polymervesikel biokompatible Polymervesikel sind; und optional
wobei die biokompatiblen Polymervesikel sich zum Teil aus Poly(ethylenoxid) oder Poly(ethylenglykol) zusammensetzen;
wobei die biologisch abbaubaren Polymervesikel sich aus mindestens einem Blockcopolymer von Poly(ethylenoxid) und Poly($\varepsilon$-caprolacton), Poly(ethylenoxid) und Poly($\gamma$-methyl-$\varepsilon$-caprolacton) oder Poly(ethylenoxid) und Poly(trimethylcarbonat) zusammensetzen; oder
wobei die biologisch abbaubaren Polymervesikel reine oder Mischungen von Multiblockcopolymeren sind, wobei das Copolymer mindestens eines von Poly(ethylenoxid) (PEO), Poly(lactid) (PLA), Poly(glykolid) (PLGA), Poly(milchsäure-co-glykolsäure) (PLGA), Poly($\varepsilon$-caprolacton) (PCL) und Poly(trimethylencarbonat) (PTMC), Poly(milchsäure), Poly(methyl-$\varepsilon$-caprolacton) umfasst.

8. Zusammensetzung zur Verwendung nach Anspruch 7, wobei das Polymersom aus einem beliebigen der folgenden gebildet wird: PEO-b-PCL, PEO-b-PMCL und PEO-b-PTMC.

9. Zusammensetzung zur Verwendung nach Anspruch 1, wobei der stark vaskularisierte Tumor aus der Gruppe bestehend aus folgenden ausgewählt ist: Nierenzellkarzinom (RCC), Leberzellkarzinom (HCC), Glioblastoma-multiforme-Hirntumor (GBM) und multiplem Myelom (MM).

10. Zusammensetzung zur Verwendung nach Anspruch 1, wobei die Zusammensetzung durch Einbringen der Zusammensetzung in den Systemkreislauf an den Tumor abgegeben wird, wobei:
die Zusammensetzung sich innerhalb des Tumors ansammelt.

11. Zusammensetzung zur Verwendung nach Anspruch 1, wobei das NO inhibierende Mittel NO bindende Moleküle umfasst, die Sauerstoff ($O_2$) und NO kompetitiv binden, und wobei:

die oxygenierten NO bindenden Moleküle in den Systemkreislauf eingebracht werden und
die NO bindenden Moleküle bei Ansammlung der Trägerpartikel in dem Tumor desoxygeniert werden, wodurch das selektive Einfangen von NO in der Tumormikrovaskulatur ermöglicht wird.

12. Zusammensetzung zur Verwendung nach Anspruch 11, wobei die Ansammlung der Zusammensetzung in dem Tumor eine Diffusion von NO in die Trägerpartikel zulässt, wobei das selektive Einfangen von NO zumindest zum Teil durch Desoxygenierung der eingekapselten NO bindenden Moleküle durchgeführt wird.

13. Zusammensetzung zur Verwendung nach Anspruch 1, wobei die selektive Verhinderung von normaler NO-Aktivität in der Tumorvaskulatur eine Vasokonstriktion und eine Thrombozytenaggregation in der Tumorvaskulatur bewirkt, optional wobei der anhaltende hydrodynamische Druck in der Tumorvaskulatur eine Ruptur der Thrombozytenaggregation und ein Bluten in den Tumor bewirkt und optional wobei das Bluten in den Tumor eine Thrombose der Tumorvaskulatur und eine Nekrose von Tumorgewebe bewirkt.

14. Zusammensetzung zur Verwendung nach Anspruch 1, wobei die Zusammensetzung in Verbindung mit mindestens einem bzw. einer von einem Vaskulären-endothelialen-Wachstumsfaktorrezeptor-Tyrosinkinase-Inhibitor (VEGFR-Tyrosinkinase-Inhibitor, VEGFR-TKI), einem Säugetier-Target-von-Rapamycin-Inhibitor (mTOR-Inhibitor) und einer Radiotherapie an den Tumor abgegeben wird.

15. Zusammensetzung zur Verwendung nach Anspruch 1, wobei die Zusammensetzung in Verbindung mit mindestens einem von einem Chemotherapeutikum und einem Angiogenese inhibierenden Mittel an den Tumor abgegeben wird.

## Revendications

1. Composition comprenant un agent inhibiteur d'oxyde nitrique (NO) et un véhicule transporteur, l'agent inhibiteur de (NO) étant destiné à être utilisé en traitement de tumeurs hautement vascularisées par la provocation d'une embolisation microvasculaire dans la tumeur,
le véhicule transporteur étant une nanoparticule et la nanoparticule étant un polymersome,
l'agent inhibiteur de (NO) comprenant une molécule se liant à (NO) ;
la liaison à NO n'étant permise qu'à des tensions en oxygène inférieures à 10 mm de Hg ;
la molécule se liant à (NO) étant choisie entre la myoglobine humaine non modifiée, la myoglobine non modifiée provenant d'une autre espèce biologique et/ou la myoglobine chimiquement ou génétiquement modifiée provenant d'êtres humains ou provenant d'une autre espèce biologique ;
la molécule se liant à (NO) étant préalablement chargée d'oxygène ; et
l'agent inhibiteur de NO étant encapsulé à l'intérieur du véhicule transporteur.

2. Composition destinée à être utilisée selon la revendication 1, la liaison à NO n'étant permise qu'à des tensions en oxygène inférieures à 5 mm de Hg.

3. Composition destinée à être utilisée selon la revendication 1, la composition comprenant en outre au moins l'un d'un inhibiteur de NO synthase (NOS) et d'un antioxydant co-encapsulé avec les molécules se liant à NO dans les polymersomes.

4. Composition destinée à être utilisée selon la revendication 1, la composition comprenant en outre au moins l'un d'un agent de chimiothérapie et d'un agent inhibiteur d'angiogenèse co-encapsulé avec les molécules se liant à NO dans les polymersomes.

5. Composition destinée à être utilisée selon la revendication 1, la composition comprenant en outre au moins l'un d'un inhibiteur de tyrosine kinase (TKI) du récepteur aux facteurs de croissance de l'endothélium vasculaire (VEGFR) et d'un inhibiteur de la cible de la rapamycine chez les mammifères (mTOR) co-encapsulé avec les molécules se liant à NO dans les polymersomes.

6. Composition destinée à être utilisée selon la revendication 1, le polymersome :

   a) comprenant une vésicule de polymère synthétique et l'agent inhibiteur de NO étant à l'intérieur d'un cœur aqueux de la vésicule de polymère ;
   b) comprenant une vésicule de polymère synthétique et l'agent inhibiteur de NO étant à l'intérieur d'une partie membraneuse de la vésicule de polymère ;
   c) étant un polymersome unilamellaire ou multilamellaire ;
   d) comprenant une pluralité de polymères biodégradables, éventuellement la pluralité de polymères biodégradables formant une nanoparticule, éventuellement la nanoparticule ayant un diamètre inférieur à 200 ou inférieur à 100 nanomètres ; ou
   e) co-encapsulant l'agent inhibiteur de NO avec au moins un autre agent de sensibilisation aux rayonnements ou chimiothérapeutique.

7. Composition destinée à être utilisée selon la revendication 6, la composition comprenant en outre :

   une pluralité de polymersomes conçus pour s'accumuler au niveau de sites présentant de l'intérêt par diffusion passive ou par une modalité de ciblage constituée d'une conjugaison d'une molécule de ciblage séparée des nanoparticules, éventuellement au moins certains de la pluralité de polymersomes étant des vésicules de polymère biodégradable et au moins certaines de la pluralité de vésicules de polymère étant des vésicules de polymère biocompatible ; et éventuellement
   les vésicules de polymère biocompatible étant en partie constituées de poly(oxyde d'éthylène) ou de poly(éthylèneglycol) ;
   les vésicules de polymère biodégradable étant constituées d'au moins un copolymère à blocs de poly(oxyde d'éthylène) et de poly($\varepsilon$-caprolactone), de poly(oxyde d'éthylène) et de poly($\gamma$-méthyl-$\varepsilon$-caprolactone) ou de poly(oxyde d'éthylène) et de poly(carbonate de triméthyle) ; ou
   les vésicules de polymère biodégradable étant soit pures soit des mélanges de copolymère à plusieurs blocs, le copolymère comprenant au moins l'un du poly(oxyde d'éthylène) (POE), du poly(lactide) (PLA), du poly(glycolide) (PLGA), du poly(acide lactique-co-glycolique) (PLGA), de la poly($\varepsilon$-caprolactone) (PCL) et du poly(carbonate de triméthylène) (PTMC), du poly(acide lactique), de la poly(méthyl-$\varepsilon$-caprolactone).

8. Composition destinée à être utilisée selon la revendication 7, le polymersome étant formé à partir de l'un quelconque de : POE-*b*-PCL, POE-*b*-PMCL et POE-*b*-PTMC.

9. Composition destinée à être utilisée selon la revendication 1, la tumeur hautement vascularisée étant choisie dans le groupe constitué par : un carcinome à cellules rénales (CCR), un carcinome hépatocellulaire (CHC), une tumeur cérébrale glioblastome multiforme (GBM) et un myélome multiple (MM).

10. Composition destinée à être utilisée selon la revendication 1, la composition étant administrée à la tumeur par introduction de la composition dans la circulation systémique :
    la composition s'accumulant à l'intérieur de la tumeur.

11. Composition destinée à être utilisée selon la revendication 1, l'agent inhibiteur de NO comprenant des molécules se liant à NO qui se lient de manière compétitive à l'oxygène ($O_2$) et au NO et :

    les molécules se liant à NO oxygénées étant introduites dans la circulation systémique ; et
    les molécules se liant à NO devenant désoxygénées lors de l'accumulation des particules transporteuses dans la tumeur, ce qui permet ainsi le piégeage sélectif de NO dans le système microvasculaire de la tumeur.

12. Composition destinée à être utilisée selon la revendication 11, l'accumulation de la composition dans la tumeur permettant la diffusion de NO dans les particules transporteuses, le piégeage sélectif de NO étant effectué au moins en partie par désoxygénation des molécules se liant à NO encapsulées.

13. Composition destinée à être utilisée selon la revendication 1, le fait d'empêcher sélectivement l'activité normale de NO dans le système vasculaire de la tumeur provoquant une vasoconstriction et une agrégation plaquettaire dans le système vasculaire de la tumeur, éventuellement la pression hydrodynamique persistante dans le système vasculaire de la tumeur provoquant une rupture de l'agrégation plaquettaire et un saignement dans la tumeur, et éventuellement le saignement dans la tumeur provoquant une thrombose de système vasculaire de la tumeur et une nécrose de tissu tumoral.

14. Composition destinée à être utilisée selon la revendication 1, la composition étant administrée à la tumeur conjointement avec au moins l'un d'un inhibiteur de tyrosine kinase (TKI) du récepteur aux facteurs de croissance de l'endothélium vasculaire (VEGFR), d'un inhibiteur de la cible de la rapamycine chez les mammifères (mTOR) et d'une radiothérapie.

15. Composition destinée à être utilisée selon la revendication 1, la composition étant administrée à la tumeur conjointement avec au moins l'un d'un agent de chimiothérapie et d'un agent inhibiteur d'angiogenèse.

FIG. 1

FIG. 2

FIG. 3

FIG. 4A

FIG. 4B

FIG. 4C

FIG. 4D

FIG. 4E

% Oxygen

% Oxygen

FIG. 5

FIG. 6A

FIG. 6B

FIG. 7A

FIG. 7B

FIG. 8A

FIG. 8B

900

```
┌─────────────────────────────────────┐
│ Self-Assembly of a Myoglobin-Based   │
│ Oxygen Carrier (MBOC) in an Aqueous  │──902
│ Solution                             │
└─────────────────────────────────────┘
                    │
                    ▼
┌─────────────────────────────────────┐
│ Stabilization of the MBOC via Chemical│──904
│ Modification                         │
└─────────────────────────────────────┘
                    │
                    ▼
┌─────────────────────────────────────┐
│ Lypholization of the Resultant Construct │──906
└─────────────────────────────────────┘
                    │
                    ▼
┌─────────────────────────────────────┐
│ Dry-Phase Storage                    │──908
└─────────────────────────────────────┘
                    │
                    ▼
┌─────────────────────────────────────┐
│ Point-of-Care Solution Rehydration   │──910
└─────────────────────────────────────┘
                    │
                    ▼
┌─────────────────────────────────────┐
│ In Vivo Delivery of Biodegradable    │
│ MBOCs that Retain their Original     │──912
│ Myoglobin                            │
└─────────────────────────────────────┘
                    │
                    ▼
┌─────────────────────────────────────┐
│ Administer Treatment (e.g., ionizing │──914
│ radiation)                           │
└─────────────────────────────────────┘
```

FIG. 9

1000

Prepare Organic Solution Having
polymers ⟋1002

Expose Organic Solution to a Plastic,
Polytetrafluoroethylene, or Glass Surface ⟋1004

Dehydrate Organic Solution to Create a
Film of Polymers ⟋1006

Rehydrate Film of Polymers in an
Aqueous Solution ⟋1008

Cross-link the Polymers in the Aqueous
Solution via Chemical Modification ⟋1010

FIG. 10

FIG. 11

FIG. 12

FIG. 13A

FIG. 13B

FIG. 13C

| Sample | $k_{off}$ $(s^{-1})$ | $k_{on}$ $(10^6 M^{-1} s^{-1})$ |
|---|---|---|
| Free-Mb | $6.2594 \pm 0.0314$ | $25.788 \pm 0.611$ |
| PEM-SE | $3.0754 \pm 0.0614$ | $2.806 \pm 0.457$ |
| PEM-E | $1.5539 \pm 0.0254$ | $2.7439 \pm 0.502$ |

FIG. 14

FIG. 15A

FIG. 15B

FIG. 15C

FIG. 16

FIG. 17

FIG. 18A

FIG. 18B

FIG. 19A

FIG. 19B

FIG. 20

| | Normal Range | Units | NIR-MB | | Empty PSs | | PEM | |
|---|---|---|---|---|---|---|---|---|
| | | | Avg | STD | Avg | STD | Avg | STD |
| BUN | 20-88 | mg/dL | 27.9 | 4.4 | 28.0 | 9.0 | 28.4 | 10.4 |
| Creatinine (Cre) | 0.5-1.6 | mg/dL | 0.4 | 0.2 | 0.4 | 0.1 | 0.8 | 0.2 |
| BUN/Cre | | | 139.3 | 21.9 | 140.1 | 44.9 | 105.7 | 80.1 |
| Phosphorus | 5.6-9.2 | mg/dL | 8.6 | 1.1 | 8.1 | 3.4 | 9.9 | 2.1 |
| Calcium | 7.9-10.5 | mg/dL | 8.7 | 2.6 | 7.5 | 1.4 | 11.2 | 1.8 |
| Total Protein | 4.5-6 | g/dL | 5.1 | 1.7 | 4.7 | 0.8 | 6.8 | 1.1 |
| Albumin | 3-4 | g/dL | 2.4 | 1.0 | 2.2 | 0.4 | 3.4 | 0.5 |
| Glucose | 190-280 | mg/dL | 176.4 | 16.8 | 200.4 | 45.8 | 222.8 | 260.2 |
| Cholesterol | 40-140 | mg/dL | 113.4 | 55.0 | 110.0 | 22.4 | 170.0 | 27.4 |
| **ALT (GPT)** | 10-89 | U/L | **80.6** | **79.4** | **93.8** | **71.5** | **108.2** | **109.1** |
| ALP | 0-185 | U/L | 49.4 | 18.8 | 46.4 | 9.2 | 76.6 | 25.6 |
| GGT | 0 | U/L | 22.0 | 11.7 | 22.0 | 4.5 | 34.0 | 5.5 |
| Tot Bili | 0.2-0.8 | mg/dL | 0.3 | 0.2 | 0.5 | 0.3 | 0.6 | 0.4 |

FIG. 21

| PEM Dialyzed | Empty PS + Na₂SO₄ | PEM + Na₂SO₄ |
|---|---|---|

FIG. 22B

| PEM Dialyzed | Empty PS + Na₂SO₄ | PEM + Na₂SO₄ |
|---|---|---|

FIG. 22A

FIG. 22C

FIG. 22D

FIG. 22E

FIG. 22F

| Group | Number of Male Animals | Number of Female Animals | Test Material | Dose Mb (polymer) mg/kg | Concentration Mb (polymer) mg/mL | Dosing Schedule (x3 cycles) |
|---|---|---|---|---|---|---|
| | 10 [9] (6) | 10 [9] (6) | Control | 0 (200) | 0 (20) | weekly |
| | 10 [9] (6) | 10 [9] (6) | PEM | 1 (20) | 0.1 (2) | weekly |
| | 10 [9] (6) | 10 [9] (6) | PEM | 5 (100) | 0.5 (10) | weekly |
| | 10 [9] (6) | 10 [9] (6) | PEM | 10 (200) | 1 (20) | weekly |
| | 10 [9] (6) | 10 [9] (6) | PEM | 50 (1000) | 5 (100) | weekly |
| | 10 [9] (6) | 10 [9] (6) | PEM | 100 (2000) | 10 (200) | weekly |
| | 10 [9] (6) | 10 [9] (6) | Control | 0 (200) | 0 (20) | every 3 weeks |
| | 10 [9] (6) | 10 [9] (6) | PEM | 1 (20) | 0.1 (2) | every 3 weeks |
| | 10 [9] (6) | 10 [9] (6) | PEM | 5 (100) | 0.5 (10) | every 3 weeks |
| | 10 [9] (6) | 10 [9] (6) | PEM | 10 (200) | 1 (20) | every 3 weeks |
| | 10 [9] (6) | 10 [9] (6) | PEM | 50 (1000) | 5 (100) | every 3 weeks |
| | 10 [9] (6) | 10 [9] (6) | PEM | 100 (2000) | 10 (200) | every 3 weeks |

Total. [ ] Number for Toxicokinetic Studies; ( ) Number for Toxicity Endpoint Studies (post 3 cycles)

PEMs content is 5 wt% Mb and 95 wt% polymer

All treatments will be administered in a 10 mL/Kg volume

Empty PSs at 10 mg/mL polymer (equivalent to group #4 and #10) in 5% dextrose, USP.

## FIG. 23A

| Group | Number of Animals | Treatment | Dose Mb (polymer) mg/kg | Concentration Mb (polymer) mg/mL |
|---|---|---|---|---|
| | 10 | PBS (neg control) | - | - |
| | 10 | Empty PSs (neg control) | 0 (200) | 0 (20) |
| | 10 | PEM | 25 (500) | 2.5 (50) |
| | 10 | PEM | 10 (200) | 1 (20) |
| | 10 | PEM | 5 (100) | 0.5 (10) |
| | 10 | PEM | 2.5 (50) | 0.25 (5) |
| | 10 | PEM | 1 (20) | 0.1 (2) |

Administered as a single dose in a 10 mL/kg volume

PEM content is 5 wt% Mb and 95 wt% polymer

Empty PSs at 200 mg/Kg (20 mg/mL polymer nanoparticle suspension)

Amount of PEM used in initial studies (see Progress Report)

## FIG. 23B

| Group | Numbers of Animals[A] | Treatment | PEM Dose | Dosing Schedule (x3 cycles)[C] |
|---|---|---|---|---|
| 1 | 10 | PBS (neg control) | - | weekly |
| 2 | 10 | Empty PSs (neg control) | - | weekly[D] |
| 3 | 10 | PEM alone | MED | weekly |
| 4 | 10 [9] (6) | PEM + sunitinib | MED | weekly (+ daily sunitinib)[E] |
| 5 | 10 | PEM alone | B | weekly |
| 6 | 10 [9] (6) | PEM + sunitinib | B | weekly (+ daily sunitinib)[E] |
| 7 | 10 | Empty PSs (neg control) | - | every 3 weeks[D] |
| 8 | 10 | PEM alone | MED | every 3 weeks |
| 9 | 10 [9] (6) | PEM + sunitinib | MED | every 3 weeks (+ daily Sunitinib)[E] |
| 10 | 10 | PEM alone | B | every 3 weeks |
| 11 | 10 [9] (6) | PEM + sunitinib | B | every 3 weeks (+ daily sunitinib)[E] |
| 12 | 10 [9] (6) | sunitinib (pos control) | - | daily[E] |

[A] Total; [ ] Number for Toxicokinetic Studies; ( ) Number for Endpoint Studies.

[B] PEM administered at either the STD10 or MTD75, as determined from Aim 1

[C] All treatments will be administered in a 10 ml/Kg volume

[D] Empty PSs at a polymer dose that is equivalent to the MTD75 or STD10 of PEM

[E] sunitinib at 40 mg/kg PO daily until study completion (defined in the experimental details)

## FIG. 24A

| Group | Numbers of Animals[A] | Treatment | PEM Dose | Dosing Schedule (x3 cycles)[C] |
|---|---|---|---|---|
| 1 | 10 | PBS (neg control) | - | weekly |
| 2 | 10 | Empty PSs (neg control) | - | weekly[D] |
| 3 | 10 | PEM alone | MED | weekly |
| 4 | 10 [9] (6) | PEM + temsirolimus | MED | weekly[E] |
| 5 | 10 | PEM alone | B | weekly |
| 6 | 10 [9] (6) | PEM + temsirolimus | B | weekly[E] |
| 7 | 10 | Empty PSs (neg control) | - | every 3 weeks[D] |
| 8 | 10 | PEM alone | MED | every 3 weeks |
| 9 | 10 [9] (6) | PEM + temsirolimus | MED | every 3 weeks (+weekly temsirolimus)[E] |
| 10 | 10 | PEM alone | B | every 3 weeks |
| 11 | 10 [9] (6) | PEM + temsirolimus | B | every 3 weeks (+weekly temsirolimus)[E] |
| 12 | 10 [9] (6) | temsirolimus (pos control) | - | daily[E] |

[A] Total; [ ] Number for Toxicokinetic Studies; ( ) Number for Endpoint Studies.

[B] PEM administered at either the STD10 or MTD75, as determined from Aim 1

[C] All treatments will be administered in a 10 ml/Kg volume

[D] Empty PSs at a polymer dose that is equivalent to the MTD75 or STD10 of PEM

[E] temsirolimus at 10 mg/kg IV weekly until study completion (defined Aim 2 details, above)

## FIG. 24B

| Group | Numbers of Animals | Adjuvant Treatment to XRT[A] | PEM Dose | Dosing Schedule (x3 cycles)[C] |
|---|---|---|---|---|
| 1 | 10 | PBS (neg control) | - | weekly |
| 2 | 10 | Empty PS | - | weekly[D] |
| 3 | 10 | PEM | B | weekly |
| 4 | 10 | Empty PS | - | every 3 weeks[D] |
| 5 | 10 | PEM | B | every 3 weeks |

[A] XRT administered via microCT irradiator, permitting conformal therapy to the kidney in 3Gy daily fractions x 5 (15 Gy total dose per animal)

[B] PEM administered at either the STD10 or MTD75, as determined from Aim 1

[C] All treatments will be administered in a 10 ml/Kg volume

[D] Empty PSs at polymer dose that is equivalent to that in PEM

## FIG. 25

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2012094679 A
- WO 2011133635 A
- US 5612310 A
- US 09007611

**Non-patent literature cited in the description**

- Cancer Facts and Figures. American Cancer Society, 2010
- Radiation Therapy Management. Care Core National, 2010
- **FELDMANN H.J. ; MOLLS M. ; VAUPEL P.** Blood Flow And Oxygenation Status Of Human Tumors - Clinical Investigations. *STRAHLENTHERAPIE UND ONKOLOGIE,* 1999, vol. 175, 1-9
- **HARRISON L. ; BLACKWELL K.** Hypoxia and Anemia: Factors In Decreased Sensitivity To Radiation Therapy And Chemotherapy. *ONCOLOGIST,* 2004, vol. 9, 31-40
- **HARRISON L.B. ; CHADHA M. ; HILL R.J. ; HU K. ; SHASHA D.** Impact Of Tumor Hypoxia And Anemia On Radiation Therapy Outcomes. *ONCOLOGIST,* 2002, vol. 7, 492-508
- **MUNDT A.J. ; CONNELL P.P. ; CAMPBELL T. ; HWANG J.H. ; ROTMENSCH J. ; WAGGONER S.** Race And Clinical Outcome In Patients With Carcinoma Of The Uterine Cervix Treated With Radiation Therapy. *GYNECOLOGIC ONCOLOGY,* 1998, vol. 71, 151-8
- **TAKESHI K. ; KATSUYUKI K. ; YOSHIAKI T. et al.** Definitive Radiotherapy Combined With High-Dose-Rate Brachytherapy For Stage III Carcinoma Of The Uterine Cervix: Retrospective Analysis Of Prognostic Factors Concerning Patient Characteristics And Treatment Parameters. *INTERNATIONAL JOURNAL OF RADIATION ONCOLOGY BIOLOGY PHYSICS,* 1998, vol. 41, 319-27
- **ROCKWELL S. ; DOBRUCKI I.T. ; KIM E.Y. ; MARRISON S.T. ; VU V.T.** Hypoxia and Radiation Therapy: Past History, Ongoing Research, and Future Promise. *CURRENT MOLECULAR MEDICINE,* 2009, vol. 9, 442-58
- **CARLSON D.J. ; KEALL P.J. ; CHEN Z.J. ; STEWART R.D. ; NATH R. ; BROWN J.M.** Towards Temporal Optimization of Radiation Fractionation: The Kinetic Effects of Tumor Hypoxia, DNA Damage Repair, and Tumor Cell Repopulation. *INTERNATIONAL JOURNAL OF RADIATION ONCOLOGY BIOLOGY PHYSICS,* 2009, vol. 75, S615-S6

- **BROWN J.M.** The Hypoxic Cell: A Target For Selective Cancer Therapy - Eighteenth Bruce F. Cain Memorial Award Lecture. *CANCER RESEARCH,* 1999, vol. 59, 5863-70
- **DIETZ A. ; RUDAT V. ; VANSELOW B. et al.** Rise Of Oxygenation In Cervical Lymph Node Metastasis During The Initial Course Of Radiochemotherapy. *OTOLARYNGOLOGY-HEAD AND NECK SURGERY,* 1999, vol. 121, 789-96
- Evaluation Of The Concept Of "Hypoxic Fraction" As A Descriptor Of Tumor Oxygenation Status, ADV. EXP. MED. BIOL 1997. **EVANS S.M. ; JENKINS W.T. ; SHAPIRO M. ; KOCH C.J.** OXYGEN TRANSPORT TO TISSUE XVIII. 1997, vol. 411, 215-25
- **TEICHER B.A.** Physiological-Mechanisms of Therapeutic Resistance - Blood-Flow and Hypoxia. *HEMATOLOGY-ONCOLOGY CLINICS OF NORTH AMERICA,* 1995, vol. 9, 475-506
- **KAELIN W.G.** ROS: Really Involved In Oxygen Sensing. *Cell Metabolism,* 2005, vol. 1, 357-8
- **ALARCON R. ; KOUMENIS C. ; GEYER R.K. ; MAKI C.G. ; GIACCIA A.J.** Hypoxia Induces Accumulation Through MDM2 Down-Regulation And Inhibition Of E6-Mediated Degradation. *Cancer Research,* 1999, vol. 59, 6046-51
- **GRAEBER T.G. ; OSMANIAN C. ; JACKS T. et al.** Hypoxia-Mediated Selection Of Cells With Diminished Apoptotic Potential In Solid Tumors. *Nature,* 1996, vol. 379, 88-91
- **ROCKWELL S.** Oxygen Delivery: Implications For The Biology And Therapy Of Solid Tumors. *Oncology Research,* 1997, vol. 9, 383-90
- **BRIZEL D.M. ; DODGE R.K. ; CLOUGH R.W. ; DEWHIRST M.W.** Oxygenation Of Head And Neck Cancer: Changes During Radiotherapy And Impact On Treatment Outcome. *Radiotherapy and Oncology,* 1999, vol. 53, 113-7

- **BRIZEL D.M. ; SIBLEY G.S. ; PROSNITZ L.R. ; SCHER R.L. ; DEWHIRST M.W.** Tumor Hypoxia Adversely Affects The Prognosis Of Carcinoma Of The Head And Neck. *International Journal of Radiation Oncology Biology Physics,* 1997, vol. 38, 285-9
- **FYLES A.W. ; MILOSEVIC M. ; WONG R. et al.** Oxygenation Predicts Radiation Response And Survival In Patients With Cervix Cancer. *Radiotherapy And Oncology,* 1998, vol. 48, 149-56
- **HOCKEL M. ; KNOOP C. ; SCHLENGER K. et al.** Intratumoral Po2 Predicts Survival In Advanced Cancer Of The Uterine Cervix. *Radiotherapy and Oncology,* 1993, vol. 26, 45-50
- **HOCKEL M. ; SCHLENGER K. ; ARAL B. ; MITZE M. ; SCHAFFER U. ; VAUPEL P.** Association Between Tumor Hypoxia And Malignant Progression In Advanced Cancer Of The Uterine Cervix. *Cancer Research,* 1996, vol. 56, 4509-15
- **HOCKEL M. ; VORNDRAN B. ; SCHLENGER K. ; BAUSSMANN E. ; KNAPSTEIN P.G.** Tumor Oxygenation - A New Predictive Parameter In Locally Advanced Cancer Of The Uterine Cervix. *Gynecologic Oncology,* 1993, vol. 51, 141-9
- **KNOCKE T.H. ; WEITMANN H.D. ; FELDMANN H.J. ; SELZER E. ; POTTER R.** Intratumoral Po(2)-Measurements As Predictive Assay In The Treatment Of Carcinoma Of The Uterine Cervix. *Radiotherapy and Oncology,* 1999, vol. 53, 99-104
- **ROFSTAD E.K. ; SUNDFOR K. ; LYNG H. ; TROPE C.G.** Hypoxia-Induced Treatment Failure In Advanced Squamous Cell Carcinoma Of The Uterine Cervix Is Primarily Due To Hypoxia Induced Radiation Resistance Rather Than Hypoxia-Induced Metastasis. *British Journal of Cancer,* 2000, vol. 83, 354-9
- **RUDAT V. ; VANSELOW B. ; WOLLENSACK P. et al.** Repeatability And Prognostic Impact Of The Pretreatment Po(2) Histography In Patients With Advanced Head And Neck Cancer. *RADIOTHERAPY AND ONCOLOGY,* 2000, vol. 57, 31-7
- **STADLER P. ; BECKER A. ; FELDMANN H.J. et al.** Influence Of The Hypoxic Subvolume On The Survival Of Patients With Head And Neck Cancer. *INTERNATIONAL JOURNAL OF RADIATION ONCOLOGY BIOLOGY PHYSICS,* 1999, vol. 44, 749-54
- **STADLER P. ; FELDMANN H.J. ; CREIGHTON C. ; KAU R. ; MOLLS M.** Changes In Tumor Oxygenation During Combined Treatment With Split-Course Radiotherapy And Chemotherapy In Patients With Head And Neck Cancer. *RADIOTHERAPY AND ONCOLOGY,* 1998, vol. 48, 157-64

- **KARAR J. ; MAITY A.** Modulating the Tumor Microenvironment to Increase Radiation Responsiveness. *CANCER BIOLOGY & THERAPY,* 2009, vol. 8, 1994-2001
- **BACHE M. ; KAPPLER M. ; SAID H.M. ; STAAB A. ; VORDERMARK D.** Detection And Specific Targeting Of Hypoxic Regions Within Solid Tumors: Current Preclinical And Clinical Strategies. *CURRENT MEDICINAL CHEMISTRY,* 2008, vol. 15, 322-38
- **AHN G.O. ; BROWN M.** Targeting Tumors With Hypoxia-Activated Cytotoxins. *FRONTIERS IN BIOSCIENCE,* 2007, vol. 12, 3483-501
- **NAGASAWA H. ; UTO Y. ; KIRK K.L. ; HORI H.** Design Of Hypoxia-Targeting Drugs As New Cancer Chemotherapeutics. *BIOLOGICAL & PHARMACEUTICAL BULLETIN,* 2006, vol. 29, 2335-42
- **JANSSEN H.L. ; HAUSTERMANS K.M. ; BALM A.J. ; BEGG A.C.** Hypoxia In Head And Neck Cancer: How Much, How Important?. *HEAD AND NECK-JOURNAL FOR THE SCIENCES OF THE HEAD AND NECK,* 2005, vol. 27, 622-38
- **WOUTERS B.G. ; WEPPLER S.A. ; KORITZINSKY M. et al.** Hypoxia As A Target For Combined Modality Treatments. *EUROPEAN JOURNAL OF CANCER,* 2002, vol. 38, 240-57
- **KONDO A. ; SAFAEI R. ; MISHIMA M. ; NIEDNER H. ; LIN X.J. ; HOWELL S.B.** Hypoxia-Induced Enrichment And Mutagenesis Of Cells That Have Lost DNA Mismatch Repair. *CANCER RESEARCH,* 2001, vol. 61, 7603-7
- **GRIGSBY P.W. ; WINTER K. ; WASSERMAN T.H. et al.** Irradiation With Or Without Misonidazole For Patients With Stages IIIB And IVA Carcinoma Of The Cervix: Final Results of RTOG 80-05. *INTERNATIONAL JOURNAL OF RADIATION ONCOLOGY BIOLOGY PHYSICS,* 1999, vol. 44, 513-7
- **LEE D.J. ; COSMATOS D. ; MARCIAL V.A. et al.** Results Of An RTOG Phase-III Trial (RTOG-85-27) Comparing Radiotherapy Plus Etanidazole With Radiotherapy Alone For Locally Advanced Head And Neck Carcinomas. *INTERNATIONAL JOURNAL OF RADIATION ONCOLOGY BIOLOGY PHYSICS,* 1995, vol. 32, 567-76
- **LEE D.J. ; PAJAK T.F. ; STETZ J. ; ORDER S.E. ; WEISSBERG J.B. ; FISCHER J.J.** A Phase-I Phase-II Study Of The Hypoxic Cell Sensitizer Misonidazole As An Adjunct To High Fractional Dose Radiotherapy In Patients With Unresectable Squamous-Cell Carcinoma Of The Head And Neck - A RTOG Randomized Study (79-04). *INTERNATIONAL JOURNAL OF RADIATION ONCOLOGY BIOLOGY PHYSICS,* 1989, vol. 16, 465-70
- **OVERGAARD J.** Sensitization Of Hypoxic Tumor-Cells - Clinical-Experience. *INTERNATIONAL JOURNAL OF RADIATION BIOLOGY,* 1989, vol. 56, 801-11

- **OVERGAARD J. ; BENTZEN S.M. ; KOLSTAD P. et al.** Misonidazole Combined With Radiotherapy In The Treatment Of Carcinoma Of The Uterine Cervix. *INTERNATIONAL JOURNAL OF RADIATION ONCOLOGY BIOLOGY PHYSICS,* 1989, vol. 16, 1069-72
- **OVERGAARD J. ; HANSEN H.S. ; ANDERSEN A.P. et al.** Misonidazole Combined With Split-Course Radiotherapy In The Treatment Of Invasive-Carcinoma Of Larynx And Pharynx - Report From The Dahanca-2 Study. *INTERNATIONAL JOURNAL OF RADIATION ONCOLOGY BIOLOGY PHYSICS,* 1989, vol. 16, 1065-8
- **OVERGAARD J. ; HANSEN H.S. ; OVERGAARD M. et al.** A Randomized Double-Blind Phase III Study Of Nimorazole As A Hypoxic Radiosensitizer Of Primary Radiotherapy In Supraglottic Larynx And Pharynx Carcinoma, Results Of The Danish Head And Neck Cancer Study (DAHANCA) Protocol 5-85. *RADIOTHERAPY AND ONCOLOGY,* 1998, vol. 46, 135-46
- **WASSERMAN T.H. ; LEE D.J. ; COSMATOS D. et al.** Clinical-Trials With Etanidazole (Sr-2508) By The Radiation-Therapy Oncology Group (RTOG). *RADIOTHERAPY AND ONCOLOGY,* 1991, vol. 20, 129-35
- **LIM SH ; LEE JY ; PARK SH et al.** Effect of Combination of Anticancer Agents and Nitroimidazoles on the Survival of Human Hepatocellular Carcinoma Cells under Hypoxic Conditions. *JOURNAL OF THE KOREAN SURGICAL SOCIETY,* 2009, vol. 76, 337-47
- **FENTON B.M. ; LORD E.M. ; PAONI S.E.** Enhancement Of Tumor Perfusion And Oxygenation By Carbogen And Nicotinamide During Single- And Multifraction Irradiation. *RADIATION RESEARCH,* 2000, vol. 153, 75-83
- **ROSENTHAL D.I. ; NURENBERG P. ; BECERRA C.R. et al.** A Phase I Single-Dose Trial Of Gadolinium Texaphyrin (Gd-Tex), A Tumor Selective Radiation Sensitizer Detectable By Magnetic Resonance Imaging. *CLINICAL CANCER RESEARCH,* 1999, vol. 5, 739-45
- **EISBRUCH A. ; ROBERTSON J.M. ; JOHNSTON C.M. et al.** Bromodeoxyuridine Alternating With Radiation For Advanced Uterine Cervix Cancer: A Phase I And Drug Incorporation Study. *JOURNAL OF CLINICAL ONCOLOGY,* 1999, vol. 17, 31-40
- **RISCHIN D. ; PETERS L.J. ; O'SULLIVAN B. et al.** Tirapazamine, Cisplatin, and Radiation Versus Cisplatin and Radiation for Advanced Squamous Cell Carcinoma of the Head and Neck (TROG 02.02, HeadSTART): A Phase III Trial of the Trans-Tasman Radiation Oncology Group. *JOURNAL OF CLINICAL ONCOLOGY,* 2010, vol. 28, 2989-95
- **FOGH S. ; MACHTAY M. ; WERNER-WASIK M. et al.** Phase I Trial Using Patupilone (Epothilone B) And Concurrent Radiotherapy For Central Nervous System Malignancies. *INTERNATIONAL JOURNAL OF RADIATION ONCOLOGY BIOLOGY PHYSICS,* 2010, vol. 77, 1009-16
- **DE LA FOUCHARDIERE C. ; NEGRIER S. ; LABROSSE H. et al.** Phase I Study Of Daily Irinotecan As A Radiation Sensitizer For Locally Advanced Pancreatic Cancer. *INTERNATIONAL JOURNAL OF RADIATION ONCOLOGY BIOLOGY PHYSICS,* 2010, vol. 77, 409-13
- **WILLIAMS K.J. ; ALBERTELLA M.R. ; FITZPATRICK B. et al.** In Vivo Activation Of The Hypoxia-Targeted Cytotoxin AQ4N In Human Tumor Xenografts. *MOLECULAR CANCER THERAPEUTICS,* 2009, vol. 8, 3266-75
- **HAY M.P. ; PRUIJN F.B. ; GAMAGE S.A. et al.** DNA-Targeted 1,2,4-Benzotriazine 1,4-Dioxides: Potent Analogues Of The Hypoxia-Selective Cytotoxin Tirapazamine. *JOURNAL OF MEDICINAL CHEMISTRY,* 2004, vol. 47, 475-88
- **SHIBAMOTO Y. ; ZHOU L. ; HATTA H. ; MORI M. ; NISHIMOTO S.** In Vivo Evaluation Of A Novel Antitumor Prodrug, 1-(2'-Oxopropyl)-5-Fluorouracil (OFU001), Which Releases 5-Fluorouracil Upon Hypoxic Irradiation. *INTERNATIONAL JOURNAL OF RADIATION ONCOLOGY BIOLOGY PHYSICS,* 2001, vol. 49, 407-13
- **KOCH C.J. ; HAHN S.M. ; ROCKWELL K. ; COVEY J.M. ; MCKENNA W.G. ; EVANS S.M.** Pharmacokinetics Of EF5 2-(2-Nitro-1-H-Imidazol-1-Yl)-N-(2,2,3,3,3-Pentafluoropropyl) Acetamide In Human Patients: Implications For Hypoxia Measurements In Vivo By 2-Nitrolmidazoles. *CANCER CHEMOTHER PHARMACOL,* 2001, vol. 48, 177-87
- **VON PAWEL J. ; VON ROEMELING R. ; GATZEMEIER U. et al.** Tirapazamine Plus Cisplatin Versus Cisplatin In Advanced Non-Small-Cell Lung Cancer: A Report Of The International CATAPULT I Study Group. *JOURNAL OF CLINICAL ONCOLOGY,* 2000, vol. 18, 1351-9
- **ROBERTS K.B. ; URDANETA N. ; VERA R. et al.** Interim Results Of A Randomized Trial Of Mitomycin C As An Adjunct To Radical Radiotherapy In The Treatment Of Locally Advanced Squamous-Cell Carcinoma Of The Cervix. *INTERNATIONAL JOURNAL OF CANCER,* 2000, vol. 90, 206-23
- **PAPADOPOULOU M.V. ; JI M. ; RAO M.K. ; BLOOMER W.D.** 4- 3-(2-Nitro-1-Imidazolyl)Propylamino-7-Chloroquinoline Hydrochloride (NLCQ-1), A Novel Bioreductive Compound As A Hypoxia-Selective Cytotoxin. *ONCOLOGY RESEARCH,* 2000, vol. 12, 185-92

- **PAPADOPOULOU M.V. ; JI M. ; RAO M.K. ; BLOOMER W.D.** 4-3-(2-Nitro-1-Imidazolyl)Propylamino -7-Chloroquinoline Hydrochloride (NLCQ-1), A Novel Bioreductive Agent As Radiosensitizer In Vitro And In Vivo: Comparison With Tirapazamine. *ONCOLOGY RESEARCH,* 2000, vol. 12, 325-33
- **CRAIGHEAD P.S. ; PEARCEY R. ; STUART G.** A Phase I/II Evaluation Of Tirapazamine Administered Intravenously Concurrent With Cisplatin And Radiotherapy In Women With Locally Advanced Cervical Cancer. *INTERNATIONAL JOURNAL OF RADIATION ONCOLOGY BIOLOGY PHYSICS,* 2000, vol. 48, 791-5
- **WEITMAN S. ; MANGOLD G. ; MARTY J. et al.** Evidence Of Enhanced In Vivo Activity Using Tirapazamine With Paclitaxel And Paraplatin Regimens Against The MV-522 Human Lung Cancer Xenograft. *CANCER CHEMOTHER PHARMACOL,* 1999, vol. 43, 402-8
- **TREAT J. ; JOHNSON E. ; LANGER C. et al.** Tirapazamine With Cisplatin In Patients With Advanced Non-Small-Cell Lung Cancer: A Phase II Study. *JOURNAL OF CLINICAL ONCOLOGY,* 1998, vol. 16, 3524-7
- **SIEMANN D.W. ; HINCHMAN C.A.** Potentiation Of Cisplatin Activity By The Bioreductive Agent Tirapazamine. *RADIOTHERAPY AND ONCOLOGY,* 1998, vol. 47, 215-20
- **LEE D.J. ; TROTTI A. ; SPENCER S. et al.** Concurrent Tirapazamine And Radiotherapy For Advanced Head And Neck Carcinomas: A Phase II Study. *INTERNATIONAL JOURNAL OF RADIATION ONCOLOGY BIOLOGY PHYSICS,* 1998, vol. 42, 811-5
- **HARRISON L.B. ; RABEN A. ; PFISTER D.G. et al.** A Prospective Phase II Trial Of Concomitant Chemotherapy And Radiotherapy With Delayed Accelerated Fractionation In Unresectable Tumors Of The Head And Neck. *HEAD AND NECK-JOURNAL FOR THE SCIENCES AND SPECIALTIES OF THE HEAD AND NECK,* 1998, vol. 20, 497-503
- **GATZEMEIER U. ; RODRIGUEZ G. ; TREAT J. et al.** Tirapazamine-Cisplatin: The Synergy. *BRITISH JOURNAL OF CANCER,* 1998, vol. 77, 15-7
- **BROWN J.M. ; WANG L.H.** Tirapazamine: Laboratory Data Relevant To Clinical Activity. *ANTI-CANCER DRUG DESIGN,* 1998, vol. 13, 529-39
- **HAFFTY B.G. ; SON Y.H. ; PAPAC R. et al.** Chemotherapy As An Adjunct To Radiation In The Treatment Of Squamous Cell Carcinoma Of The Head And Neck: Results Of The Yale Mitomycin Randomized Trials. *JOURNAL OF CLINICAL ONCOLOGY,* 1997, vol. 15, 268-76
- **ADELSTEIN D.J. ; SAXTON J.P. ; LAVERTU P. et al.** A Phase III Randomized Trial Comparing Concurrent Chemotherapy And Radiotherapy With Radiotherapy Alone In Resectable Stage III And IV Squamous Cell Head And Neck Cancer: Preliminary Results. *HEAD AND NECK-JOURNAL FOR THE SCIENCES AND SPECIALTIES OF THE HEAD AND NECK,* 1997, vol. 19, 567-75
- **SARTORELLI A.C. ; HODNICK W.F. ; BELCOURT M.F. et al.** Mitomycin-C - A Prototype Bioreductive Agent. *ONCOLOGY RESEARCH,* 1994, vol. 6, 501-8
- **DOBROWSKY W.** Mitomycin-C, 5-Fluorouracil And Radiation In Advanced, Locally Recurrent Rectal-Cancer. *BRITISH JOURNAL OF RADIOLOGY,* 1992, vol. 65, 143-7
- **SIVANESARATNAM V. ; JAYALAKSHMI P.** Mitomycin-C Adjuvant Chemotherapy After Wertheim Hysterectomy For Stage-Ib Cervical-Cancer. *CANCER,* 1989, vol. 64, 798-800
- **KEYES S.R. ; ROCKWELL S. ; SARTORELLI A.C.** Enhancement Of Mitomycin-C Cyto-Toxicity To Hypoxic Tumor-Cells By Dicoumarol Invivo And Invitro. *CANCER RESEARCH,* 1985, vol. 45, 213-6
- **KEYES S.R ; ROCKWELL S. ; SARTORELLI A.C.** Porfiromycin As A Bioreductive Alkylating Agent With Selective Toxicity To Hypoxic Emt6 Tumor-Cells Invivo And Invitro. *CANCER RESEARCH,* 1985, vol. 45, 3642-5
- **OVERGAARD J. ; HORSMAN M.R.** Modification Of Hypoxia-Induced Radioresistance In Tumors By The Use Of Oxygen And Sensitizers. *SEMINARS IN RADIATION ONCOLOGY,* 1996, vol. 6, 10-21
- **AQUINO-PARSONS C. ; LIM P. ; GREEN A. ; MINCHINTON A.I.** Carbogen Inhalation In Cervical Cancer: Assessment Of Oxygenation Change. *GYNECOLOGIC ONCOLOGY,* 1999, vol. 74, 259-64
- **BERNIER J. ; DENEKAMP J. ; ROJAS A. et al.** ARCON: Accelerated Radiotherapy With Carbogen And Nicotinamide In Head And Neck Squamous Cell Carcinomas: The Experience Of The Cooperative Group Of Radiotherapy Of The European Organization For Research And Treatment Of Cancer (EORTC). *RADIOTHERAPY AND ONCOLOGY,* 2000, vol. 55, 111-9
- **BUSSINK J. ; KAANDERS J. ; VAN DER KOGEL A.J.** Clinical Outcome And Tumour Microenvironmental Effects Of Accelerated Radiotherapy With Carbogen And Nicotinamide. *ACTA ONCOLOGICA,* 1999, vol. 38, 875-82
- **HOSKIN P.J. ; SAUNDERS M.I. ; DISCHE S.** Hypoxic Radiosensitizers In Radical Radiotherapy For Patients With Bladder Carcinoma - Hyperbaric Oxygen, Misonidazole, And Accelerated Radiotherapy, Carbogen, And Nicotinamide. *CANCER,* 1999, vol. 86, 1322-8

- **MIRALBELL R. ; MORNEX F. ; GREINER R. et al.** Accelerated Radiotherapy, Carbogen, And Nicotinamide In Glioblastoma Multiforme: Report Of European Organization For Research And Treatment Of Cancer Trial 22933. *JOURNAL OF CLINICAL ONCOLOGY,* 1999, vol. 17, 3143-9
- **SAUNDERS M. ; DISCHE S.** Clinical Results Of Hypoxic Cell Radiosensitisation From Hyperbaric Oxygen To Accelerated Radiotherapy, Carbogen And Nicotinamide. *BRITISH JOURNAL OF CANCER,* 1996, vol. 74, S271-S8
- **STUBEN G. ; STUSCHKE M. ; KNUHMANN K. ; HORSMAN M.R. ; SACK H.** The Effect Of Combined Nicotinamide And Carbogen Treatments In Human Tumour Xenografts: Oxygenation And Tumour Control Studies. *RADIOTHERAPY AND ONCOLOGY,* 1998, vol. 48, 143-8
- **ZHAN H.W. ; LIU H.B. ; BAO C.K. ; YE X.J. ; ZHANG H. ; HE G.Q.** Effect Of Carbogen On Tumour Oxygenation And 32P-Colloid Interstitial Irradiation Response. *MEDICAL SCIENCE MONITOR,* 2010, vol. 16, BR11-BR6
- **YU M.H. ; DAI M. ; LIU Q. ; XIU R.J.** Oxygen Carriers And Cancer Chemo- And Radiotherapy Sensitization: Bench To Bedside And Back. *CANCER TREATMENT REVIEWS,* 2007, vol. 33, 757-61
- **HOOGSTEEN I.J. ; MARREST A.M. ; VAN DER KOGEL A.J. ; KAANDERS J.** The Hypoxic Tumour Microenvironment, Patient Selection And Hypoxia-Modifying Treatments. *CLINICAL ONCOLOGY,* 2007, vol. 19, 385-96
- **SHASHA D.** The Negative Impact Of Anemia On Radiotherapy And Chemoradiation Outcomes. *SEMINARS IN HEMATOLOGY,* 2001, vol. 38, 8-15
- **SHASHA D. ; GEORGE M.J. ; HARRISON L.B.** Once-Weekly Dosing Of Epoetin Alfa Increases Hemoglobin And Improves Quality Of Life In Anemic Cancer Patients Receiving Radiation Therapy Either Concomitantly Or Sequentially With Chemotherapy. *BLOOD,* 2000, vol. 96, 1866
- **HENKE M. ; GUTTENBERGER R. ; BARKE A. ; PAJONK F. ; POTTER R. ; FROMMHOLD H.** Erythropoietin For Patients Undergoing Radiotherapy: A Pilot Study. *RADIOTHERAPY AND ONCOLOGY,* 1999, vol. 50, 185-90
- **DUSENBERY K.E. ; MCGUIRE W.A. ; HOLT P.J. et al.** Erythropoietin Increases Hemoglobin During Radiation-Therapy For Cervical-Cancer. *INTERNATIONAL JOURNAL OF RADIATION ONCOLOGY BIOLOGY PHYSICS,* 1994, vol. 29, 1079-84
- **LAVEY R.S. ; DEMPSEY W.H.** Erythropoietin Increases Hemoglobin In Cancer-Patients During Radiation-Therapy. *INTERNATIONAL JOURNAL OF RADIATION ONCOLOGY BIOLOGY PHYSICS,* 1993, vol. 27, 1147-52
- **MCGEE M.C. ; HAMNER J.B. ; WILLIAMS R.F. et al.** Improved Intratumoral Oxygenation Through Vascular Normalization Increases Glioma Sensitivity To Ionizing Radiation. *INTERNATIONAL JOURNAL OF RADIATION ONCOLOGY BIOLOGY PHYSICS,* 2010, vol. 76, 1537-45
- **KATZ D. ; ITO E. ; LIU F.F.** On The Path To Seeking Novel Radiosensitizers. *INTERNATIONAL JOURNAL OF RADIATION ONCOLOGY BIOLOGY PHYSICS,* 2009, vol. 73, 988-96
- **GALI-MUHTASIB H. ; SIDANI M. ; GEARA F. et al.** Quinoxaline 1,4-Dioxides Are Novel Angiogenesis Inhibitors That Potentiate Antitumor Effects Of Ionizing Radiation. *INTERNATIONAL JOURNAL OF ONCOLOGY,* 2004, vol. 24, 1121-31
- **ORDWAY G.A. ; GARRY D.J.** Myoglobin: An Essential Hemoprotein In Striated Muscle. *JOURNAL OF EXPERIMENTAL BIOLOGY,* 2004, vol. 207, 3441-6
- **WITTENBERG J.B. ; WITTENBERG B.A.** Myoglobin Function Reassessed. *JOURNAL OF EXPERIMENTAL BIOLOGY,* 2003, vol. 206, 2011-20
- **KOOYMAN G.L. ; PONGANIS P.J.** The Physiological Basis Of Diving To Depth: Birds And Mammals. *ANNUAL REVIEW OF PHYSIOLOGY,* 1998, vol. 60, 19-32
- **HOCHACHKA P.W.** The Metabolic Implications Of Intracellular Circulation. *PROCEEDINGS OF THE NATIONAL ACADEMY OF SCIENCES OF THE UNITED STATES OF AMERICA,* 1999, vol. 96, 12233-9
- **CONLEY K.E. ; JONES C.** Myoglobin Content And Oxygen Diffusion: Model Analysis Of Horse And Steer Muscle. *AMERICAN JOURNAL OF PHYSIOLOGY-CELL PHYSIOLOGY,* 1996, vol. 271, C2027-C36
- **GALLUZZO M. ; PENNACCHIETTI S ; ROSANO S. ; COMOGLIO P.M. ; MICHIELI P.** Prevention Of Hypoxia By Myoglobin Expression In Human Tumor Cells Promotes Differentiation And Inhibits Metastasis. *JOURNAL OF CLINICAL INVESTIGATION,* 2009, vol. 119, 865-75
- **FLOEGEL U. ; DANG C.V.** Myoglobin Tames Tumor Growth And Spread. *JOURNAL OF CLINICAL INVESTIGATION,* 2009, vol. 119, 766-8
- **BUNN H.F.** The Role Of Hemoglobin Based Substitutes In Transfusion Medicine. *TRANSFUS CLIN. BIOL.,* 1995, vol. 2, 433-9
- **GOTTSCHALK A. ; RAABE A. ; HOMMEL M. ; REMPF C. ; FREITAG M. ; STANDL T.** Influence Of The Hemoglobin Solution HBOC-201 On Tissue Oxygenation In The Rat RIH-Tumor. *ARTIF. CELLS BLOOD SUBSTIT. BIOTECHNOL.,* 2005, vol. 33, 379-89
- **GUNDERSEN S.I. ; PALMER A.F.** Hemoglobin-Based Oxygen Carrier Enhanced Tumor Oxygenation: A Novel Strategy for Cancer Therapy. *BIOTECHNOL. PROG.,* 2008, vol. 24, 1353-64

- **PALAPARTHY R. ; WANG H.S. ; GULATI A.** Current Aspects In Pharmacology Of Modified Hemoglobins. *ADV DRUG DELIV. REV,* 2000, vol. 40, 185-98
- **ROBINSON M.F. ; DUPUIS N.P. ; KUSUMOTO T. ; LIU F. ; MENON K. ; TEICHER B.A.** Increased Tumor Oxygenation And Radiation Sensitivity In 2 Rat-Tumors By A Hemoglobin-Based, Oxygen-Carrying Preparation. *ARTIF. CELLS BLOOD SUBSTIT. IMMOBIL. BIOTECHNOL.,* 1995, vol. 23, 431-8
- **ROWINSKY E.K.** Novel Radiation Sensitizers Targeting Tissue Hypoxia. *ONCOLOGY-NY,* 1999, vol. 13, 61-70
- **TEICHER B.A. ; HERMAN T.S. ; HOPKINS R.E. ; MENON K.** Effect Of A Bovine Hemoglobin Preparation On The Response Of The Fsaiic Fibrosarcoma To Chemotherapeutic Alkylating-Agents. *J. CANCER RES. CLIN. ONCOL.,* 1992, vol. 118, 123-8
- **TEICHER B.A. ; HOLDEN S.A. ; DUPUIS N.P. et al.** Oxygenation Of The Rat-91 Gliosarcoma And The Rat-13672 Mammary-Carcinoma With Various Doses Of A Hemoglobin Solution. *ARTIF. CELLS BLOOD SUBSTIT. IMMOBIL. BIOTECHNOL.,* 1994, vol. 22, 827-33
- **TEICHER B.A. ; HOLDEN S.A. ; MENON K. ; HOPKINS R.E. ; GAWRYL M.S.** Effect Of Hemoglobin Solution On The Response Of Intracranial And Subcutaneous Tumors To Antitumor Alkylating-Agents. *CANCER CHEMOTHER. PHARMACOL.,* 1993, vol. 33, 57-62
- **TEICHER B.A. ; SCHWARTZ G.N. ; SOTOMAYOR E.A. ; ROBINSON M.F. ; DUPUIS N.P. ; MENON K.** Oxygenation Of Tumors By A Hemoglobin Solution. *J. CANCER RES. CLIN. ONCOL.,* 1993, vol. 120, 85-90
- **CHRISTIAN D.A. ; CAI S. ; BOWEN D.M. ; KIM Y. ; PAJEROWSKI J.D. ; DISCHER D.E.** Polymersome Carriers: From Self-Assembly To siRNA And Protein Therapeutics. *EUR. J. PHARM. BIOPHARM.,* 2009, vol. 71, 463-74
- **LEVINE D.H. ; GHOROGHCHIAN P.P. ; FREUDENBERG J. et al.** Polymersomes: A New MultiFunctional Tool For Cancer Diagnosis And Therapy. *METHODS,* 2008, vol. 46, 25-32
- **LETCHFORD K. ; BURT H.** A Review Of The Formation And Classification Of Amphiphilic Block Copolymer Nanoparticulate Structures: Micelles, Nanospheres, Nanocapsules And Polymersomes. *EUR. J. PHARM. BIOPHARM.,* 2007, vol. 65, 259-69
- **DISCHER B.M. ; HAMMER D.A. ; BATES F.S. ; DISCHER D.E.** Polymer Vesicles In Various Media. *CURR. OPIN. COLLOID INTERFACE SCI.,* 2000, vol. 5, 125-31
- **DISCHER D.E. ; ORTIZ V. ; SRINIVAS G. et al.** Emerging Applications Of Polymersomes In Delivery: From Molecular Dynamics To Shrinkage Of Tumors. *PROG. POLYM. SCI.,* 2007, vol. 32, 838-57
- **DISCHER B.M. ; WON Y.Y. ; EGE D.S. et al.** Polymersomes: Tough Vesicles Made From Diblock Copolymers. *SCIENCE,* 1999, vol. 284, 1143-6
- **O'NEIL C.P. ; SUZUKI T. ; DEMURTAS D. ; FINKA A. ; HUBBELL J.A.** A Novel Method For The Encapsulation Of Biomolecules Into Polymersomes Via Direct Hydration. *LANGMUIR,* 2009, vol. 25, 9025-9
- **LEE J.C.M. ; BERMUDEZ H. ; DISCHER B.M. et al.** Preparation, Stability, And In Vitro Performance Of Vesicles Made With Diblock Copolymers. *BIOTECHNOL. BIOENG.,* 2001, vol. 73, 135-45
- **GHOROGHCHIAN P.P. ; LI G.Z. ; LEVINE D.H. et al.** Bioresorbable Vesicles Formed Through Spontaneous Self-Assembly Of Amphiphilic Poly(Ethylene Oxide)-Block-Polycaprolactone. *MACROMOLECULES,* 2006, vol. 39, 1673-5
- **KIM Y.H. ; TEWARI M. ; PAJEROWSKI J.D. et al.** Polymersome Delivery Of siRNA And Antisense Oligonucleotides. *J. CONTROL RELEASE,* 2009, vol. 134, 132-40
- **HEARNDEN V. ; LOMAS H. ; MACNEIL S. et al.** Diffusion Studies of Nanometer Polymersomes Across Tissue Engineered Human Oral Mucosa. *PHARMACEUTICAL RESEARCH,* 2009, vol. 26, 1718-28
- **MENG F.H. ; ENGBERS G.H.M. ; FEIJEN J.** Biodegradable Polymersomes As A Basis For Artificial Cells: Encapsulation, Release And Targeting. *J. CONTROL RELEASE,* 2005, vol. 101, 187-98
- **PHOTOS P.J. ; BACAKOVA L. ; DISCHER B. ; BATES F.S. ; DISCHER D.E.** Polymer Vesicles In Vivo: Correlations With PEG Molecular Weight. *J. CONTROL RELEASE,* 2003, vol. 90, 323-34
- **GHOROGHCHIAN P.P. ; LIN J.J. ; BRANNAN A.K. et al.** Quantitative Membrane Loading Of Polymer Vesicles. *SOFT MATTER,* 2006, vol. 2, 973-80
- **GHOROGHCHIAN P.P. ; FRAIL P.R. ; SUSUMU K. et al.** Near-Infrared-Emissive Polymersomes: Self-Assembled Soft Matter For In Vivo Optical Imaging. *PROC NATL. ACAD. SCI. U.S.A.,* 2005, vol. 102, 2922-7
- **BERMUDEZ H. ; HAMMER D.A. ; DISCHER D.E.** Effect Of Bilayer Thickness On Membrane Bending Rigidity. *LANGMUIR,* 2004, vol. 20, 540-3
- **BERMUDEZ H. ; BRANNAN A.K. ; HAMMER D.A. ; BATES F.S. ; DISCHER D.E.** Molecular Weight Dependence Of Polymersome Membrane Structure, Elasticity, And Stability. *MACROMOLECULES,* 2002, vol. 35, 8203-8

- **MASSIGNANI M. ; LOPRESTI C. ; BLANAZS A. et al.** Controlling Cellular Uptake by Surface Chemistry, Size, and Surface Topology at the Nanoscale. *SMALL,* 2009, vol. 5, 2424-32
- **BLANAZS A. ; MASSIGNANI M. ; BATTAGLIA G. ; ARMES S.P. ; RYAN A.J.** Tailoring Macromolecular Expression at Polymersome Surfaces. *ADVANCED FUNCTIONAL MATERIALS,* 2009, vol. 19, 2906-14
- **VAN DONGEN S.F.M. ; NALLANI M. ; SCHOFFELEN S. ; CORNELISSEN J. ; NOLTE R.J.M. ; VAN HEST J.C.M.** A Block Copolymer For Functionalisation Of Polymersome Surfaces. *MACROMOLECULAR RAPID COMMUNICATIONS,* 2008, vol. 29, 321-5
- **CHRISTIAN N.A. ; MILONE M.C. ; RANKA S.S. et al.** Tat-Functionalized Near-Infrared Emissive Polymersomes For Dendritic Cell Labeling. *BIOCONJUGATE. CHEM.,* 2007, vol. 18, 31-40
- **BINDER W.H. ; SACHSENHOFER R ; FARNIK D ; BLAAS D.** Guiding The Location Of Nanoparticles Into Vesicular Structures: A Morphological Study. *PHYSICAL CHEMISTRY CHEMICAL PHYSICS,* 2007, vol. 9, 6435-41
- **LIN J.J. ; GHOROGHCHIAN P. ; ZHANG Y. ; HAMMER D.A.** Adhesion Of Antibody-Functionalized Polymersomes. *LANGMUIR,* 2006, vol. 22, 3975-9
- **LIN J.J. ; SILAS J.A. ; BERMUDEZ H. ; MILAM V.T. ; BATES F.S. ; HAMMER D.A.** The Effect Of Polymer Chain Length And Surface Density On The Adhesiveness Of Functionalized Polymersomes. *LANGMUIR,* 2004, vol. 20, 5493-500
- **PANGU G.D. ; DAVIS K.P. ; BATES F.S. ; HAMMER D.A.** Ultrasonically Induced Release from Nanosized Polymer Vesicles. *MACROMOL. BIOSCI.,* 2010, vol. 10, 546-54
- **KIM M.S. ; LEE D.S.** Biodegradable And Ph-Sensitive Polymersome With Tuning Permeable Membrane For Drug Delivery Carrier. *CHEMICAL COMMUNICATIONS,* 2010, vol. 46, 4481-3
- **CHEN W. ; MENG F.H. ; CHENG R. ; ZHONG Z.Y.** pH-Sensitive Degradable Polymersomes For Triggered Release Of Anticancer Drugs: A Comparative Study With Micelles. *J CONTROL RELEASE,* 2010, vol. 142, 40-6
- **ROBBINS G.P. ; JIMBO M. ; SWIFT J. ; THERIEN M.J ; HAMMER D.A. ; DMOCHOWSKI I.J.** Photoinitiated Destruction of Composite Porphyrin-Protein Polymersomes. *J AM CHEM SOC,* 2009, vol. 131, 3872
- **KIM K.T. ; CORNELISSEN J. ; NOLTE R.J.M. ; VAN HEST J.C.M.** A Polymersome Nanoreactor with Controllable Permeability Induced by Stimuli-Responsive Block Copolymers. *ADVANCED MATERIALS,* 2009, vol. 21, 2787
- **SANSON C. ; LE MEINS J.F. ; SCHATZ C. ; SOUM A. ; LECOMMANDOUX S.** Temperature Responsive Poly(Trimethylene Carbonate)-Block-Poly(L-Glutamic Acid) Copolymer: Polymersomes Fusion And Fission. *SOFT MATTER,* 2010, vol. 6, 1722-30
- **CASTILLO R.V. ; MULLER A.J. ; RAQUEZ J.M. ; DUBOIS P.** Crystallization Kinetics and Morphology of Biodegradable Double Crystalline PLLA-b-PCL Diblock Copolymers. *MACROMOLECULES,* 2010, vol. 43, 4149-60
- **WANG F. ; WANG Y.C. ; YAN L.F. ; WANG J.** Biodegradable Vesicular Nanocarriers Based On Poly(Epsilon-Caprolactone)-Block-Poly(Ethyl Ethylene Phosphate) For Drug Delivery. *POLYMER,* 2009, vol. 50, 5048-54
- **SCHATZ C. ; LOUGUET S. ; LE MEINS J.F. ; LECOMMANDOUX S.** Polysaccharide-Block-Polypeptide Copolymer Vesicles: Towards Synthetic Viral Capsids. *ANGEW. CHEM. INT. EDIT,* 2009, vol. 48, 2572-5
- **RABOTYAGOVA O.S. ; CEBE P. ; KAPLAN D.L.** Self-Assembly of Genetically Engineered Spider Silk Block Copolymers. *BIOMACROMOLECULES,* 2009, vol. 10, 229-36
- **KATZ J.S. ; LEVINE D.H. ; DAVIS K.P. ; BATES F.S. ; HAMMER D.A. ; BURDICK J.A.** Membrane Stabilization of Biodegradable Polymersomes. *LANGMUIR,* 2009, vol. 25, 4429-34
- **BROMLEY E.H.C ; CHANNON K. ; MOUTEVELIS E. ; WOOLFSON D.N.** Peptide And Protein Building Blocks For Synthetic Biology: From Programming Biomolecules To Self-Organized Biomolecular Systems. *Acs CHEMICAL BIOLOGY,* 2008, vol. 3, 38-50
- **NAJAFI F. ; SARBOLOUKI M.N.** Biodegradable Micelles/Polymersomes From FumaricISebacic Acids And Poly(Ethylene Glycol). *BIOMATERIALS,* 2003, vol. 24, 1175-82
- **RAMEEZ S. ; BAMBA I. ; PALMER A.F.** Large Scale Production of Vesicles by Hollow Fiber Extrusion: A Novel Method for Generating Polymersome Encapsulated Hemoglobin Dispersions. *LANGMUIR,* 2010, vol. 26, 5279-85
- **SHUM H.C. ; KIM J.W. ; WEITZ D.A.** Microfluidic Fabrication Of Monodisperse Biocompatible And Biodegradable Polymersomes With Controlled Permeability. *J AM CHEM SOC,* 2008, vol. 130, 9543-9
- **YILDIZ M.E. ; PRUD'HOMME R.K. ; ROBB I. ; ADAMSON D.H.** Formation And Characterization Of Polymersomes Made By A Solvent Injection Method. *POLYMERS FOR ADVANCED TECHNOLOGIES,* 2007, vol. 18, 427-32
- **DEWHIRST M.W.** Relationships Between Cycling Hypoxia, HIF-1, Angiogenesis And Oxidative Stress. *RADIATION RESEARCH,* 2009, vol. 172, 653-65

- **PALMER G.M. ; BORUTA R.J. ; VIGLIANTI B.L. ; LAN L. ; SPASOJEVIC I. ; DEWHIRST M.W.** Non-Invasive Monitoring Of Intra-Tumor Drug Concentration And Therapeutic Response Using Optical Spectroscopy. *J CONTROL RELEASE,* 2010, vol. 142, 457-64
- **DEWHIRST M.W. ; THRALL D.E. ; PALMER G. et al.** Utility Of Functional Imaging In Prediction Or Assessment Of Treatment Response And Prognosis Following Thermotherapy. *INTERNATIONAL JOURNAL OF HYPERTHERMIA,* 2010, vol. 26, 283-93
- **ZHANG G.Q. ; PALMER G.M. ; DEWHIRST M. ; FRASER C.L.** A Dual-Emissive-Materials Design Concept Enables Tumour Hypoxia Imaging. *NATURE MATERIALS,* 2009, vol. 8, 747-51
- **PALMER G.M. ; VIOLA R.J ; SCHROEDER T. ; YARMOLENKO P.S. ; DEWHIRST M.W. ; RAMANUJAM N.** Quantitative Diffuse Reflectance And Fluorescence Spectroscopy: Tool To Monitor Tumor Physiology In Vivo. *JOURNAL OF BIOMEDICAL OPTICS,* 2009, 14
- **HARDEE M.E. ; DEWHIRST M.W. ; AGARWAL N. ; SORG B.S.** Novel Imaging Provides New Insights into Mechanisms of Oxygen Transport in Tumors. *CURRENT MOLECULAR MEDICINE,* 2009, vol. 9, 435-41
- **SORG B.S. ; HARDEE M.E. ; AGARWAL N. ; MOELLER B.J. ; DEWHIRST M.W.** Spectral Imaging Facilitates Visualization And Measurements Of Unstable And Abnormal Microvascular Oxygen Transport In Tumors. *JOURNAL OF BIOMEDICAL OPTICS,* 2008, 13
- **DEWHIRST M.W. ; CAO Y. ; MOELLER B.** Cycling Hypoxia And Free Radicals Regulate Angiogenesis And Radiotherapy Response. *NATURE REVIEWS CANCER,* 2008, vol. 8, 425-37
- **SORG B.S. ; MOELLER B.J. ; DONOVAN O. ; CAO Y.T. ; DEWHIRST M.W.** Hyperspectral Imaging Of Hemoglobin Saturation In Tumor Microvasculature And Tumor Hypoxia Development. *JOURNAL OF BIOMEDICAL OPTICS,* 2005, 10
- **DEWHIRST M.W. ; CAO Y.T. ; LI C.Y. ; MOELLER B.** Exploring The Role Of HIF-1 In Early Angiogenesis And Response To Radiotherapy. *RADIOTHERAPY AND ONCOLOGY,* 2007, vol. 83, 249-55
- **MOELLER B. ; DEWHIRST M.W.** HIF-I and tumour radiosensitivity. *BRITISH JOURNAL OF CANCER,* 2006, vol. 95, 1-5
- **MOELLER B.J. ; DREHER M.R. ; RABBANI Z.N. et al.** Pleiotropic Effects Of HIF-1 Blockade On Tumor Radiosensitivity. *CANCER CELL,* 2005, vol. 8, 99-110
- **ARIFIN D.R. ; PALMER A.F.** Polymersome Encapsulated Hemoglobin: A Novel Type Of Oxygen Carrier. *BIOMACROMOLECULES,* 2005, vol. 6, 2172-81
- **RAMEEZ S. ; ALOSTA H. ; PALMER A.F.** Biocompatible And Biodegradable Polymersome Encapsulated Hemoglobin: A Potential Oxygen Carrier. *BIOCONJUGATE CHEM,* 2008, vol. 19, 1025-32
- **GHOROGHCHIAN P.P. ; THERIEN M.J. ; HAMMER D.A.** In Vivo Fluorescence Imaging: A Personal Perspective. *WILEY INTERDISCIP. REV-NANOMED NANOBIOTECHNOL.,* 2009, vol. 1, 156-67
- **DUNCAN T.V. ; GHOROGHCHIAN P.P. ; RUBTSOV I.V. ; HAMMER D.A. ; THERIEN M.J.** Ultrafast Excited-State Dynamics Of Nanoscale Near-Infrared Emissive Polymersomes. *J AM. CHEM. SOC,* 2008, vol. 130, 9773-84
- **GHOROGHCHIAN P.P. ; FRAIL P.R. ; LI G.Z. et al.** Controlling Bulk Optical Properties Of Emissive Polymersomes Through Intramembranous Polymer-Fluorophore Interactions. *CHEM. MAT,* 2007, vol. 19, 1309-18
- **GHOROGHCHIAN P.P. ; FRAIL P.R. ; SUSUMU K. et al.** Broad Spectral Domain Fluorescence Wavelength Modulation Of Visible And Near-Infrared Emissive Polymersomes. *J. AM. CHEM. SOC.,* 2005, vol. 127, 15388-90
- **CHRISTIAN N.A. ; BENENCIA F. ; MILONE M.C. et al.** In Vivo Dendritic Cell Tracking Using Fluorescence Lifetime Imaging and Near-Infrared-Emissive Polymersomes. *MOL. IMAGING BIOL.,* 2009, vol. 11, 167-77
- **HEARNDEN V. ; BATTAGLIA G. ; MACNEIL S. ; MURDOCH C. ; THORNHILL M.** Penetration Of Polymersome Drug And Gene Delivery Nanoparticles Into In Vitro Models Of Head And Neck Cancer And Tissue Engineered Oral Mucosa. *ORAL ONCOLOGY,* 2009, 66
- **LI S.L. ; BYRNE B. ; WELSH J. ; PALMER A.F.** Self-AssembledPoly(Butadiene)-B-Poly(Ethylene Oxide) Polymersomes As Paclitaxel Carriers. *BIOTECHNOL. PROG.,* 2007, vol. 23, 278-85
- **AHMED F. ; PAKUNLU R.I. ; BRANNAN A. ; BATES F. ; MINKO T. ; DISCHER D.E.** Biodegradable Polymersomes Loaded With Both Paclitaxel And Doxorubicin Permeate And Shrink Tumors, Inducing Apoptosis In Proportion To Accumulated Drug. *J. CONTROL RELEASE,* 2006, vol. 116, 150-8
- **ZHANG X.L. ; LIU C.S. ; YUAN Y. et al.** Key Parameters Affecting The Initial Leaky Effect Of Hemoglobin-Loaded Nanoparticles As Blood Substitutes. *JOURNAL OF MATERIALS SCIENCE-MATERIALS IN MEDICINE,* 2008, vol. 19, 2463-70
- **DEWHIRST M.W. ; KIMURA H. ; REHMUS S.W.E. et al.** Microvascular Studies On The Origins Of Perfusion-Limited Hypoxia. *BRITISH JOURNAL OF CANCER,* 1996, vol. 74, S247-S51

- **ZUPANCICH J.A. ; BATES F.S. ; HILLMYER M.A.** Aqueous Dispersions Of Poly(Ethylene Oxide)-B-Poly(Gamma-Methyl-Epsilon-Caprolactone) Block Copolymers. *MACROMOLECULES,* 2006, vol. 39, 4286-8
- **HAHN J.S. ; BRAUN R.D. ; DEWHIRST M.W. et al.** Stroma-Free Human Hemoglobin A Decreases R3230Ac Rat Mammary Adenocarcinoma Blood Flow And Oxygen Partial Pressure. *RADIATION RESEARCH,* 1997, vol. 147, 185-94
- **ARIFIN D.R. ; PALMER A.F.** Determination Of Size Distribution And Encapsulation Efficiency Of Liposome-Encapsulated Hemoglobin Blood Substitutes Using Asymmetric Flow Field-Flow Fractionation Coupled With Multi-Angle Static Light Scattering. *BIOTECHNOL PROG,* 2003, vol. 19, 1798-811
- **SAKAI H. ; SATO A. ; SOBOLEWSKI P. et al.** NO And CO Binding Profiles Of Hemoglobin Vesicles As Artificial Oxygen Carriers. *BIOCHIMICA ET BIOPHYSICA ACTA-PROTEINS AND PROTEOMICS,* 2008, vol. 1784, 1441-7
- **SAKAI H. ; HAMADA K. ; TAKEOKA S. ; NISHIDE H. ; TSUCHIDA E.** Functional Evaluation Of Hemoglobin- And Lipidheme-Vesicles As Red Cell Substitutes. *POLYMERS FOR ADVANCED TECHNOLOGIES,* 1996, vol. 7, 639-44
- **FRAUENFELDER H. ; MCMAHON B.H. ; FENIMORE P.W.** Myoglobin: The Hydrogen Atom Of Biology And A Paradigm Of Complexity. *PROCEEDINGS OF THE NATIONAL ACADEMY OF SCIENCES OF THE UNITED STATES OF AMERICA,* 2003, vol. 100, 8615-7
- **HEROLD S. ; EXNER M. ; NAUSER T.** Kinetic And Mechanistic Studies Of The NO Center Dot-Mediated Oxidation Of Oxymyoglobin And Oxyhemoglobin. *BIOCHEMISTRY,* 2001, vol. 40, 3385-95
- **NIENHAUS G.U. ; MOURANT J.R. ; CHU K. ; FRAUENFELDER H.** Ligand-Binding To Heme-Proteins - The Effect Of Light On Ligand-Binding In Myoglobin. *BIOCHEMISTRY,* 1994, vol. 33, 13413-30
- **ANSARI A. ; JONES C.M. ; HENRY E.R. ; HOFRICHTER J. ; EATON W.A.** Conformational Relaxation And Ligand-Binding In Myoglobin. *BIOCHEMISTRY,* 1994, vol. 33, 5128-45
- **MOURANT J.R. ; BRAUNSTEIN D.P. ; CHU K. et al.** Ligand-Binding To Heme-Proteins .2. Transitions In The Heme Pocket Of Myoglobin. *BIOPHYSICAL JOURNAL,* 1993, vol. 65, 1496-507
- **KANNER J. ; HAREL S. ; GRANIT R.** Nitric-Oxide As An Antioxidant. *ARCHIVES OF BIOCHEMISTRY AND BIOPHYSICS,* 1991, vol. 289, 130-6
- **CHANCE B.** Optical Method. *ANNUAL REVIEW OF BIOPHYSICS AND BIOPHYSICAL CHEMISTRY,* 1991, vol. 20, 1-28
- **CHANCE B. ; NIOKA S. ; KENT J. et al.** Time-Resolved Spectroscopy Of Hemoglobin And Myoglobin In Resting And Ischemic Muscle. *ANALYTICAL BIOCHEMISTRY,* 1988, vol. 174, 698-707
- **PRIVALOV P.L. ; GRIKO Y.V. ; VENYAMINOV S.Y. ; KUTYSHENKO V.P.** Cold Denaturation Of Myoglobin. *JOURNAL OF MOLECULAR BIOLOGY,* 1986, vol. 190, 487-98
- **HELCKE G.A. ; INGRAM D.J.E. ; SLADE E.F.** Electron Resonance Studies Of Haemoglobin Derivatives .3. Line-Width And G-Value Measurements Of Acid-Met Myoglobin And Of Met Myoglobin Azide Derivatives. *PROCEEDINGS OF THE ROYAL SOCIETY OF LONDON SERIES B-BIOLOGICAL SCIENCES,* 1968, vol. 169, 275
- **MOELLER B.J. ; RICHARDSON R.A. ; DEWHIRST M.W.** Hypoxia And Radiotherapy: Opportunities For Improved Outcomes In Cancer Treatment. *CANCER AND METASTASIS REVIEWS,* 2007, vol. 26, 241-8
- **KONG G. ; BRAUN R.D. ; DEWHIRST M.W.** Characterization Of The Effect Of Hyperthermia On Nanoparticle Extravasation From Tumor Vasculature. *CANCER RESEARCH,* 2001, vol. 61, 3027-32
- **KONG G. ; BRAUN R.D. ; DEWHIRST M.W.** Hyperthermia Enables Tumor-Specific Nanoparticle Delivery: Effect Of Particle Size. *CANCER RESEARCH,* 2000, vol. 60, 4440-5
- **MOELLER B.J. ; DEWHIRST M.W.** Raising The Bar - How HIF-1 Helps Determine Tumor Radiosensitivity. *CELL CYCLE,* 2004, vol. 3, 1107-10
- **MOZZARELLI A. ; FAGGIANO S. ; BETTATI S ; BRUNO S.** Haemoglobin-based oxygen carriers: research and reality towards an alternative to blood transfusions. *BLOOD TRANSFUS,* 2010, vol. 8 (3), s59-s68
- **ARANDA-ESPINOZA H. ; BERMUDEZ H. ; BATES F.S. ; DISCHER D.E.** Electromechanical Limits of Polymersomes. *PHYSICAL REVIEW LETTERS,* 2001, vol. 87, 208301
- **CHIN A.I. ; LAM J.S. ; FIGLIN R.A. ; BELLDEGRUN A.S.** Surveillance Strategies for Renal Cell Carcinoma Patients Following Nephrectomy. *REVIEWS IN UROLOGY,* 2006, vol. 8, 1-7
- **ATHER M.H. ; MASOOD N, T.S.** Current management of advanced and metastatic renal cell carcinoma. *UROLOGY JOURNAL,* 2010, vol. 7, 1-9
- **BENJAMINI Y. ; HOCHBERG Y.** Controlling the false discovery rate - a practical and powerful approach to multiple testing. *JOURNAL OF THE ROYAL STATISTICAL SOCIETY SERIES B-METHODOLOGICAL,* 1995, vol. 57, 289-300
- **BRAHMER J.R. et al.** Safety and Activity of Anti-PD-LI Antibody in Patients with Advanced Cancer. *NEW ENGLAND JOURNAL OF MEDICINE,* 2012, vol. 366, 2455-65

- **CABRALES P. FJ.** HBOC vasoactivity: interplay between nitric oxide scavenging and capacity to generate bioactive nitric oxide species. *ANTIOXID REDOX SIGNAL,* 2013, vol. 18, 2284-97
- **CAROLINE R. ; JEAN-CHARLES S. ; ALEXANDER M. ME.** Drug of the year: Programmed Death-1 receptor/Programmed Death-1 Ligand-1 receptor monoclonal antibodies. *EUROPEAN JOURNAL OF CANCER,* 2013, vol. 49, 2968-71
- **CHANCE B. et al.** Time-Resolved Spectroscopy of Hemoglobin and Myoglobin in Resting and Isechmic Muscle. *ANALYTICAL BIOCHEMISTRY,* 1988, vol. 174, 698-707
- **CHANCE B.** Optical Method. *ANNUAL REVIEW OF BIOPHYSICS AND BIOPHYSICAL CHEMISTRY,* 1991, vol. 20, 1-28
- **CHANG A. ; FINELLI A. ; BERNS J.S. ; ROSNER M.** Chronic Kidney Disease in Patients With Renal Cell Carcinoma. *ADVANCES IN CHRONIC KIDNEY DISEASE,* 2014, vol. 21, 91-5
- **CHANG T. MS.** Blood replacement with nanobiotechnologically engineered hemoglobin and hemoglobin nanocapsules. *WILEY INTERDISCIPLINARY REVIEWS: NANOMEDICINE AND NANOBIOTECHNOLOGY,* 2010, vol. 2, 418-30
- **CHANG Y. et al.** Sorafenib (BAY 43-9006) inhibits tumor growth and vascularization and induces tumor apoptosis and hypoxia in RCC xenograft models. *CANCER CHEMOTHER PHARMACOL,* 2007, vol. 59, 561-74
- **CHO D. ; SIGNORETTI S. ; REGAN M. ; MIER J.W. ; ATKINS M.B.** The role of mammalian target of rapamycin inhibitors in the treatment of advanced renal cancer. *CLIN CANCER RES,* 2007, vol. 13, 758s-63s
- **CHO D. et al.** The Efficacy of the Novel Dual PI3-Kinase/mTOR Inhibitor NVP-BEZ235 Compared with Rapamycin in Renal Cell Carcinoma. *CLINICAL CANCER RESEARCH,* 2010, vol. 16, 3628-38
- **CHOUEIRI T.K. et al.** Clinical factors associated with outcome in patients with metastatic clear-cell renal cell carcinoma treated with vascular endothelial growth factor-targeted therapy. *CANCER,* 2007, vol. 110, 543-50
- **CHOUEIRI T.K. et al.** Prognostic factors associated with long-term survival in previously untreated metastatic renal cell carcinoma. *ANN ONCOL,* 2007, vol. 18, 249-55
- **CHOUEIRI T.K.** Efficacy of cabozantinib (XL184) in patients (pts) with metastatic, refractory renal cell carcinoma (RCC), 2012. *ASCO Annual Meeting,* 2012
- **CHOUEIRI T.K.** Factors associated with outcome in patients with advanced renal cell carcinoma in the era of antiangiogenic agents. *CLIN GENITOURIN CANCER,* 2008, vol. 6, 15-20
- **CHRISTIAN D.A. ; GARBUZENKO O.B. ; MINKO T. ; DISCHER D.E.** Polymer Vesicles with a Red Cell-like Surface Charge: Microvascular Imaging and in vivo Tracking with Near-Infrared Fluorescence. *MACROMOLECULAR RAPID COMMUNICATIONS,* 2010, vol. 31, 135-41
- **CLIFFORD S.C. ; MAHER E.R.** Von Hippel-Lindau disease: clinical and molecular perspectives. *ADV CANCER RES,* 2001, vol. 82, 85-105
- **CORA N.S. et al.** A randomised, double-blind phase III study of pazopanib in patients with advanced and/or metastatic renal cell carcinoma: Final overall survival results and safety update. *EUROPEAN JOURNAL OF CANCER,* 2013, vol. 49, 1287-96
- **COURTNEY K.D. ; CHOUEIRI T.K.** Optimizing recent advances in metastatic renal cell carcinoma. *CURR ONCOL REP,* 2009, vol. 11, 218-26
- **COURTNEY K.D. ; CHOUEIRI T.K.** Review: Updates on novel therapies for metastatic renal cell carcinoma. *THERAPEUTIC ADVANCES IN MEDICAL ONCOLOGY,* 2010, vol. 2, 209
- **DAVID S.T. et al.** Sickle Erythrocytes Target Cytotoxics to Hypoxic Tumor Microvessels and Potentiate a Tumoricidal Response. *PLOS ONE,* 2013, vol. 8, e52543
- **DOEHN C. et al.** Mode-of-Action, Efficacy, and Safety of a Homologous Multi-Epitope Vaccine in a Murine Model for Adjuvant Treatment of Renal Cell Carcinoma. *EUROPEAN UROLOGY,* 2009, vol. 56, 123-31
- **ESCUDIER B et al.** Randomized, Controlled, Double-Blind, Cross-Over Trial Assessing Treatment Preference for Pazopanib Versus Sunitinib in Patients With Metastatic Renal Cell Carcinoma: PISCES Study. *JOURNAL OF CLINICAL ONCOLOGY,* 2014, vol. 32, 1412-8
- **FELDMAN D.R. et al.** Phase I Trial of Bevacizumab Plus Escalated Doses of Sunitinib in Patients With Metastatic Renal Cell Carcinoma. *JOURNAL OF CLINICAL ONCOLOGY,* 2009, vol. 27, 1432-9
- **FIGLIN R.A. et al.** Overall survival with sunitinib versus interferon (IFN)-alfa as first-line treatment of metastatic renal cell carcinoma (mRCC). *JOURNAL OF CLINICAL ONCOLOGY,* 2008, vol. 26
- **FONTANELLA A.N. et al.** Quantitative Mapping of Hemodynamics in the Lung, Brain, and Dorsal Window Chamber-Grown Tumors Using a Novel, Automated Algorithm. *MICROCIRCULATION,* 2013, vol. 20, 724-35
- **GOLSHAYAN A.R. et al.** Metastatic Sarcomatoid Renal Cell Carcinoma Treated With Vascular Endothelial Growth Factor-Targeted Therapy. *JOURNAL OF CLINICAL ONCOLOGY,* 2009, vol. 27, 235-41

- **GREGORY M.P. et al.** In vivo optical molecular imaging and analysis in mice using dorsal window chamber models applied to hypoxia, vasculature and fluorescent reporters. *NATURE PROTOCOLS,* 2011, vol. 6, 1355-66
- **GREPIN R. et al.** Acceleration of clear cell renal cell carcinoma growth in mice following bevacizumab/Avastin treatment: the role of CXCL cytokines. *ONCOGENE,* 2012, vol. 31, 1683-94
- **GRISANZIO C. et al.** Orthotopic xenografts of RCC retain histological, immunophenotypic and genetic features of tumours in patients. *JOURNAL OF PATHOLOGY,* 2011, vol. 225, 212-21
- **SONPAVDE G. ; CHOUEIRI T.** Precision medicine for metastatic renal cell carcinoma. *UROLOGIC ONCOLOGY,* 2014, vol. 32, 5-15
- **HAI C.** Systems Biology of HBOC-Induced Vasoconstriction. *CURRENT DRUG DISCOVERY TECHNOLOGIES,* 2012, vol. 9, 204-11
- **HILLMAN G.G. et al.** Progression of renal cell carcinoma is inhibited by genistein and radiation in an orthotopic model. *BMC CANCER,* 2007, vol. 7, 4
- **HODI F.S. et al.** MPDL3280A (anti-PDLI): Clinical activity, safety and biomarkers of an engineered PD-L1 antibody in patients with locally advanced or metastatic tumors. *EUROPEAN JOURNAL OF CANCER,* 2013, vol. 49, 184
- **HUDES G. et al.** Temsirolimus, Interferon Alfa, or Both for Advanced Renal-Cell Carcinoma. *NEW ENGLAND JOURNAL OF MEDICINE,* 2007, vol. 356, 2271-81
- **HUTSON TE.** Axitinib versus sorafenib as first-line therapy in patients with metastatic renal cell carcinoma (mRCC). *JOURNAL OF CLINICAL ONCOLOGY,* 2013, vol. 31 (6
- **HYLANDER B.L. et al.** Origin of the vasculature supporting growth of primary patient tumor xenografts. *JOURNAL OF TRANSLATIONAL MEDICINE,* 2013, vol. 11, 1-14
- **JASON S.H. et al.** Stroma-Free Human Hemoglobin A Decreases R3230Ac Rat Mammary Adenocarcinoma Blood Flow and Oxygen Partial Pressure. *RADIATION RESEARCH,* 1997, vol. 147, 185-94
- **CHEN J.Y. ; SCERBO M. ; KRAMER G.** A Review of Blood Substitutes: Examining The History, Clinical Trial Results, and Ethics of Hemoglobin-Based Oxygen Carriers. *CLINICS (SAO PAULO),* 2009, vol. 64, 803-13
- **KAELIN W.G. JR.** The von Hippel-Lindau tumor suppressor protein and clear cell renal carcinoma. *CLIN CANCER RES,* 2007, vol. 13, 680s-684s
- **KAELIN W.G. JR.** The Von Hippel-Lindau Tumor Suppressor Gene and Kidney Cancer. *CLIN CANCER RES,* 2004, vol. 10, 6290s-6295s
- **KERSEY F.R. ; ZHANG G. ; PALMER G.M. ; DEWHIRST M.W. ; FRASER C.L.** Stereocomplexed Poly(lactic acid)-Polyethylene glycol) Nanoparticles with Dual-Emissive Boron Dyes for Tumor Accumulation. *ACS NANO,* 2010, vol. 4, 4989-96
- **KOBAYASHI M. ; MORITA T. ; CHUN N. ; MATSUI A. ; TAKAHASHI M. ; MURAKAMI T.** Effect of host immunity on metastatic potential in renal cell carcinoma: the assessment of optimal in vivo models to study metastatic behavior of renal cancer cells. *TUMOR BIOLOGY,* 2012, vol. 33, 551-9
- **KONDO K. ; KLCO J. ; NAKAMURA E. ; LECHPAMMER M. ; KAELIN W.G. JR.** Inhibition of HIF is necessary for tumor suppression by the von Hippel-Lindau protein. *CANCER CELL,* 2002, vol. 1, 237-46
- **KONDO K. et al.** Comprehensive mutational analysis of the VHL gene in sporadic renal cell carcinoma: relationship to clinicopathological parameters. *GENES CHROMOSOMES CANCER,* 2002, vol. 34, 58-68
- **LARKIN J. et al.** Efficacy of Sequential Treatment with Sunitinib-Everolimus in an Orthotopic Mouse Model of Renal Cell Carcinoma. *ANTICANDER RESEARCH,* 2012, vol. 32, 2399-2406
- **LEE J. ; SIEMANN D.W. ; KOCH C.J. ; LORD E.M.** Direct relationship between radiobiological hypoxia in tumors and monoclonal antibody detection of EF5 cellular adducts. *INTERNATIONAL JOURNAL OF CANCER,* 1996, vol. 67, 372-8
- **LEE Y.S. et al.** Coexpression of erythropoietin and erythropoietin receptor in von Hippel-Lindau disease-associated renal cysts and renal cell carcinoma. *CLIN CANCER RES,* 2005, vol. 11, 1059-1064
- **LEIPING F. ; GANG W. ; SHEVCHUK M.M. ; NANUS D.M ; GUDAS L.J.** Generation of a Mouse Model of Von Hippel-Lindau Kidney Disease Leading to Renal Cancers by Expression of a Constitutively Active Mutant of HIFloc. *CANCER RESEARCH,* 2011, vol. 71, 6848-6856
- **LIN W. et al.** Laboratory Studies: A novel murine model of human renal cell carcinoma spinal metastasis. *JOURNAL OF CLINICAL NEUROSCIENCE,* 2012, vol. 19, 881-3
- **LIU G. ; CHEN C. ; JI J.** Biocompatible and biodegradable polymersomes as delivery vehicles in biomedical applications. *SOFT MATTER,* 2012, vol. 8, 8811-21
- **LOMAS H. et al.** Polymersome-Loaded Capsules for Controlled Release of DNA. *SMALL,* 2011, vol. 7, 2109-19
- **MA W.W. ; ADJEI A.A.** Novel agents on the horizon for cancer therapy. *CA CANCER J CLIN,* 2009, vol. 59, 111-37

- **MALIK A. ; KUNDU J. ; MUKHERJEE S.K. ; CHOWDHURY P.K.** Myoglobin Unfolding in Crowding and Confinement. *JOURNAL OF PHYSICAL CHEMISTRY B,* 2012, vol. 116, 12895-8904
- **MARZILLI L. ; GOLDEN T. ; COTTER R. ; WOODS A.** Peptide sequence information derived by pronase digestion and ammonium sulfate in-source decay matrix-assisted laser desorption/ionization time-of-flight mass spectrometry. *J AM SOC SPECTROM,* 2000, vol. 11, 1000-1008
- **MASSIGNANI M. et al.** Controlling Cellular Uptake by Surface Chemistry, Size, and Surface Topology at the Nanoscale. *SMALL,* 2009, vol. 5, 2424-32
- **MASSIMO C. ; CHUSILP C. ; GABRIEL N.H.** Angiogenesis modulation in cancer research: novel clinical approaches. *NATURE REVIEWS DRUG DISCOVERY,* 2002, vol. 1, 415-426
- **MCGEE M.C. et al.** Improved Intratumoral Oxygenation Through Vascular Normalization Increases Glioma Sensitivity To Ionizing Radiation. *INTERNATIONAL JOURNAL OF RADIATION ONCOLOGY BIOLOGY PHYSICS,* 2010, vol. 76, 1537-1545
- **MELICHAR B. et al.** First-line bevacizumab combined with reduced dose interferon-alpha2a is active in patients with metastatic renal cell carcinoma. *ANN ONCOL,* 2008, vol. 19, 1470-76
- **MENG F. H. ; ENGBERS G.H.M. ; FEIJEN J.** Biodegradable Polymersomes As A Basis For Artificial Cells: Encapsulation, Release And Targeting. *J. CONTROL RELEASE,* 2005, vol. 101, 187-198
- **MERIC-BERNSTAM F. ; GONZALEZ-ANGULO A.M.** Targeting the mTOR signaling network for cancer therapy. *J CLIN ONCOL,* 2009, vol. 27, 2278-287
- **MINORU K. et al.** Investigative Urology: Establishment and Characterization of Transplantable, Luminescence Labeled Rat Renal Cell Carcinoma Cell Lines. *J UROL,* 2009, vol. 183, 2029-35
- **MIRALBELL R. et al.** Accelerated Radiotherapy, Carbogen, And Nicotinamide In Glioblastoma Multiforme: Report Of European Organization For Research And Treatment Of Cancer Trial 22933. *J CLIN ONCOL,* 1999, vol. 17, 3143-49
- **MOELLER B. ; DEWHIRST M.W.** HIF-1 and tumour radiosensitivity. *BRITISH JOURNAL OF CANCER,* 2006, vol. 95, 1-5
- **MOELLER B.J. ; DEWHIRST M.W.** Raising The Bar-How HIF-1 Helps Determine Tumor Radiosensitivity. *CELL CYCLE,* 2004, vol. 3, 1107-10
- **MOELLER B.J. et al.** Pleiotropic Effects Of HIF-1 Blockade On Tumor Radiosensitivity. *CANCER CELL,* 2005, vol. 8, 99-110
- **MOELLER B.J. ; RICHARDSON R.A. ; DEWHIRST M.W.** Hypoxia And Radiotherapy: Opportunities For Improved Outcomes In Cancer Treatment. *CANCER AND METASTASIS REVIEWS,* 2007, vol. 26, 241-48
- **MOLINA A.M. et al.** Phase 1 trial of everolimus plus sunitinib in patients with metastatic renal cell carcinoma. *CANCER,* 2012, vol. 118, 1868-76
- **MOLINO D. ; SEPE J. ; ANASTASIO P. ; DE SANTO N.G.** The history of von Hippel-Lindau disease. *J NEPHROL,* 2006, vol. 19
- **MOORE E.E. et al.** Human Polymerized Hemoglobin for the Treatment of Hemorrhagic Shock when Blood Is Unavailable: The USA Multicenter Trial. *JOURNAL OF THE AMERICAN COLLEGE OF SURGEONS,* 2009, vol. 208, 1-13
- **MOTZER R.J. et al.** A phase 3 comparative study of nivolumab (anti-PD-1; BMS-936558; ONO-4538) versus everolimus in patients with advanced or metastatic renal cell carcinoma (mRCC) previously treated with anti-angiogenic therapy. *BJU INTERNATIONAL,* 2013, vol. 112 (10
- **MOTZER R.J. ; ESCUDIER B. ; OUDARD S. et al.** Efficacy of everolimus in advanced renal cell carcinoma: a double-blind, randomised, placebo-controlled phase III trial. *LANCET,* 2008, vol. 372, 449-56
- **MOTZER R.J. et al.** Phase I Trial of Sunitinib Malate plus Interferon-oc for Patients with Metastatic Renal Cell Carcinoma. *CLINICAL GENITOURINARY CANCER,* 2009, vol. 7, 28-33
- **MOTZER R.J. et al.** Overall survival and updated results for sunitinib compared with interferon alfa in patients with metastatic renal cell carcinoma. *J CLIN ONCOL,* 2009, vol. 27, 3584-90
- **MOTZER R.J. et al.** Dovitinib versus sorafenib for third-line targeted treatment of patients with metastatic renal cell carcinoma: an open-label, randomised phase 3 trial. *LANCET ONCOLOGY,* 2014, vol. 15, 286-96
- **MOTZER R.J. et al.** Tivozanib versus sorafenib as initial targeted therapy for patients with advanced renal cell carcinoma: Results from a phase III randomized, open-label. *J CLIN ONCOL,* 2012, vol. 30 (15), 277s
- **MOURANT J.R. et al.** Ligand-Binding To Heme-Proteins: II. Transitions In The Heme Pocket Of Myoglobin. *BIOPHYSICAL JOURNAL,* 1993, vol. 65, 1496-507
- **MUNDT A..J. ; CONNELL P.P. ; CAMPBELL T. ; HWANG J.H. ; ROTMENSCH J. ; WAGGONER S.** Race And Clinical Outcome In Patients With Carcinoma Of The Uterine Cervix Treated With Radiation Therapy. *GYNECOLOGIC ONCOLOGY,* 1998, vol. 71, 151-8

- **MUSUMECI F. ; RADI M. ; BRULLO C. ; SCHENONE S.** Vascular Endothelial Growth Factor (VEGF) Receptors: Drugs and New Inhibitors. *J. MED. CHEM.,* 2012, vol. 55, 10797-822
- **NAGASAWA H. ; UTO Y. ; KIRK K.L. ; HORI H.** Design Of Hypoxia-Targeting Drugs As New Cancer Chemotherapeutics. *BIO PHARM BULL,* 2006, vol. 29, 2335-42
- **NAJAFI F. ; SARBOLOUKI M.N.** Biodegradable Micelles/Polymersomes From Fumaric/Sebacic Acids And Poly(Ethylene Glycol). *BIOMATERIALS,* 2003, vol. 24, 1175-82
- **NATANSON C. ; KERN S.J. ; LURIE P. ; BANKS S.M. ; WOLFE S.M.** Cell-free hemoglobin-based blood substitutes and risk of myocardial infarction and death: A meta-analysis. *JAMA,* 2008, vol. 299, 2304-12
- **OVERGAARD J. et al.** Misonidazole Combined With Radiotherapy In The Treatment Of Carcinoma Of The Uterine Cervix. *IJROBP,* 1989, vol. 16, 1069-72
- **OVERGAARD J. et al.** Misonidazole Combined With Split-Course Radiotherapy In The Treatment Of Invasive-Carcinoma Of Larynx And Pharynx - Report From The Dahanca-2 Study. *IJROBP,* 1989, vol. 16, 1065-8
- **OVERGAARD J. et al.** A Randomized Double-Blind Phase III Study OfNimorazole As A Hypoxic Radiosensitizer Of Primary Radiotherapy In Supraglottic Larynx And Pharynx Carcinoma, Results Of The Danish Head And Neck Cancer Study (DAHANCA) Protocol 5-85. *RADIOTHERAPY AND ONCOLOGY,* 1998, vol. 46, 135-46
- **CABRALES P.** Examining and mitigating acellular hemoglobin vasoactivity. *ANTIOXID REDOX SIGNAL,* 2013, vol. 18, 2329-41
- **PALMER A.F. ; SUN G. ; HARRIS D.R.** Tangential flow filtration of hemoglobin. *BIOTECHNOLOGY PROGRESS,* 2009, vol. 25, 189-99
- **PALMER G.M. ; VIOLA R.J. ; SCHROEDER T. ; YARMOLENKO P.S. ; DEWHIRST M.W. ; RAMANUJAM N.** Quantitative Diffuse Reflectance And Fluorescence Spectroscopy: Tool To Monitor Tumor Physiology In Vivo. *JOURNAL OF BIOMEDICAL OPTICS,* 2009, vol. 14, 024010
- **PALMER G.M. et al.** In vivo optical molecular imaging and analysis in mice using dorsal window chamber models applied to hypoxia, vasculature and fluorescent reporters. *NAT PROTOC,* 2011, vol. 6, 1355-66
- **PALMER G.M. ; FONTANELLA A.N. ; ZHANG G. ; HANNA G. ; FRASER C.L. ; DEWHIRST M.W.** Optical imaging of tumor hypoxia dynamics. *J BIOMED OPT.,* 2010, vol. 15, 066021
- **PAPADOPOULOU M.V. ; JI M. ; RAO M.K. ; BLOOMER W.D.** 4- 3-(2-Nitro-1-Imidazolyl)Propylamino -7-Chloroquinoline Hydrochloride (NLCQ-1), A Novel Bioreductive Compound As A Hypoxia-Selective Cytotoxin. *ONCOLOGY RESEARCH,* 2000, vol. 12, 185-92
- **PATEL P.H. ; SENICO P.L. ; CURIEL R.E. ; MOTZER R.J.** Phase I Study Combining Treatment with Temsirolimus and Sunitinib Malate in Patients with Advanced Renal Cell Carcinoma. *CLINICAL GENITOURINARY CANCER,* 2009, vol. 7, 24-7
- **PATTON J.N. ; PALMER A.F.** Physical Properties of Hemoglobin-Poly(acrylamide) Hydrogel-Based Oxygen Carriers: Effect of Reaction pH. *LANGMUIR,* 2006, vol. 22, 2212-21
- **P.L. ; GRIKO Y.V. ; VENYAMINOV S.Y. ; KUTYSHENKO V.P.** Cold Denaturation Of Myoglobin. *JOURNAL OF MOLECULAR BIOLOGY,* 1986, vol. 190, 487-98
- **QI W. ; GHOROGHCHIAN P.P. ; LI G. ; HAMMER D.A. ; THERIEN M.J.** Aqueous self-assembly of polyethylene oxide)-block-poly([varepsilon]-caprolactone) (PEO-b-PCL) copolymers: disparate diblock copolymer compositions give rise to nano- and meso-scale bilayered vesicles. *NANOSCALE,* 2013, vol. 5, 10908-15
- **RAMEEZ S. ; BANERJEE U. ; FONTES J. ; ROTH A. ; PALMER A.F.** Reactivity of Polymersome Encapsulated Hemoglobin with Physiologically Important Gaseous Ligands: Oxygen, Carbon Monoxide, and Nitric Oxide. *MACROMOLECULES,* 2012, vol. 45, 2385-9
- **RAVAL R.R. et al.** Contrasting properties of hypoxia-inducible factor 1(H1F-1) and HIF-2 in von Hippel-Lindau-associated renal cell carcinoma. *MOL CELL BIOL,* 2005, vol. 25, 5675-86
- **RINI B. et al.** AMG 386 in combination with sorafenib in patients with metastatic clear cell carcinoma of the kidney. *CANCER,* 2012, vol. 118, 6152-61
- **RINI B. et al.** Bevacizumab plus interferon alfa compared with interferon alfa monotherapy in patients with metastatic renal cell carcinoma: CALGB 90206. *J CLIN ONCOL,* 2008, vol. 26, 5422-8
- **RISCHIN D. et al.** Tirapazamine, Cisplatin, and Radiation Versus Cisplatin and Radiation for Advanced Squamous Cell Carcinoma of the Head and Neck (TROG 02.02, HeadSTART): A Phase III Trial of the Trans-Tasman Radiation Oncology Group. *J CLIN ONCOL,* 2010, vol. 28, 2989-95
- **ROBBINS G.P. ; JIMBO M. ; SWIFT J. ; THERIEN M.J. ; HAMMER D.A. ; DMOCHOWSKI I.J.** Photoinitiated Destruction of Composite Porphyrin-Protein Polymersomes. *J AM CHEM SOC,* 2009, vol. 131, 3872-4

- **ROBERTS K.B. et al.** Interim Results Of A Randomized Trial Of Mitomycin C As An Adjunct To Radical Radiotherapy In The Treatment Of Locally Advanced Squamous-Cell Carcinoma Of The Cervix. *INT. J. CANCER,* 2000, vol. 90, 206-23
- **ROCKWELL S.** Oxygen Delivery: Implications For The Biology And Therapy Of Solid Tumors. *ONCOLOGY RESEARCH,* 1997, vol. 9, 383-90
- **ROFSTAD E.K. ; SUNDFOR K. ; LYNG H. ; TROPE C.G.** Hypoxia-Induced Treatment Failure In Advanced Squamous Cell Carcinoma Of The Uterine Cervix Is Primarily Due To Hypoxia Induced Radiation Resistance Rather Than Hypoxia-Induced Metastasis. *BRITISH JOURNAL OF CANCER,* 2000, vol. 83, 354-9
- **ROWINSKY E.K.** Novel Radiation Sensitizers Targeting Tissue Hypoxia. *ONCOLOGY (WILLISTON PARK),* 1999, vol. 13, 61-70
- **SABATINI D.M.** MTOR and cancer: insights into a complex relationship. *NAT REV CANCER,* 2006, vol. 6, 729-34
- **SAKAI H. et al.** NO And CO Binding Profiles Of Hemoglobin Vesicles As Artificial Oxygen Carriers. *BIOCHIMICA ET BIOPHYSICA ACTA-PROTEINS AND PROTEOMICS,* 2008, vol. 1784, 1441-7
- Pre-clinical models of renal carcinoma and their utility in drug development. **SALUMBIDES B.C. ; LEHET K.M. ; NDIKUYEZE G. ; PILI R.** CURR PROTOC PHARMACOL. December 2009
- **SARTORELLI A.C. et al.** Mitomycin-C - A Prototype Bioreductive Agent. *ONCOLOGY RESEARCH,* 1994, vol. 6, 501-8
- **SAUNDERS M. ; DISCHE S.** Clinical Results Of Hypoxic Cell Radiosensitisation From Hyperbaric Oxygen To Accelerated Radiotherapy, Carbogen And Nicotinamide. *BRITISH JOURNAL OF CANCER,* 1996, vol. 74, 271-8
- **SCHRAML P. et al.** VHL mutations and their correlation with tumour cell proliferation, microvessel density, and patient prognosis in clear cell renal cell carcinoma. *J PATHOL,* 2002, vol. 196, 186-93
- **SHASHA D. ; GEORGE M.J. ; HARRISON L.B.** Once-Weekly Dosing Of Epoetin-Alfa Increases Hemoglobin And Improves Quality Of Life In Anemic Cancer Patients Receiving Radiation Therapy Either Concomitantly Or Sequentially With Chemotherapy. *CANCER,* 2003, vol. 98, 1072-9
- **SHIBAMOTO Y. ; ZHOU L. ; HATTA H. ; MORI M. ; NISHIMOTO S.** In Vivo Evaluation Of A Novel Antitumor Prodrug, 1-(2'-Oxopropyl)-5-Fluorouracil (OFU001), Which Releases 5-Fluorouracil Upon Hypoxic Irradiation. *IJROBP,* 2001, vol. 49, 407-13
- **SHIBUYA M.** Vascular endothelial growth factor-dependent and -independent regulation of angiogenesis. *BMB REP,* 2008, vol. 41, 278-86

- **SIVANAND S. et al.** A Validated Tumorgraft Model Reveals Activity of Dovitinib Against Renal Cell Carcinoma. *SCIENCE TRANSLATIONAL MEDICINE,* 2012, vol. 4, 137ra75
- **SMALDONE M.C. ; FUNG C. ; UZZO R.G. ; HAAS N.B.** Adjuvant and Neoadjuvant Therapies in High-Risk Renal Cell Carcinoma. *HEMATOLOGY/ONCOLOGY CLINICS OF NORTH AMERICA,* 2011, vol. 25, 765-91
- **SONPAVDE G. ; WILLEY C.D. ; SUDARSHAN S.** Fibroblast growth factor receptors as therapeutic targets in clear-cell renal cell carcinoma. *EXPERT OPINION ON INVESTIGATIONAL DRUGS,* 2014, vol. 23, 305-15
- **SONVEAUX P. et al.** Oxygen Regulation of Tumor Perfusion by S-Nitrosohemoglobin Reveals a Pressor Activity of Nitric Oxide. *Circulation Research,* 2005, vol. 96, 1119-26
- **SORG B.S. ; HARDEE M.E. ; AGARWAL N. ; MOELLER B.J. ; DEWHIRST M.W.** Spectral Imaging Facilitates Visualization And Measurements Of Unstable And Abnormal Microvascular Oxygen Transport In Tumors. *J. BIOMED. OPT.,* 2008, vol. 13, 014026
- **SORG B.S. ; MOELLER B.J. ; DONOVAN O. ; CAO Y.T. ; DEWHIRST M.W.** Hyperspectral Imaging Of Hemoglobin Saturation In Tumor Microvasculature And Tumor Hypoxia Development. *JOURNAL OF BIOMEDICAL OPTICS,* 2005, vol. 10, 044004
- **STADLER P. et al.** Influence Of The Hypoxic Subvolume On The Survival Of Patients With Head And Neck Cancer. *IJROBP,* 1999, vol. 44, 749-54
- **STRUBE A. ; STEPINA E. ; MUMBERG D. ; SCHOLZ A. ; HAUFF P. ; KAKONEN S.M.** Characterization of a new renal cell carcinoma bone metastasis mouse model. *CLIN EXP METASTASIS,* 2010, vol. 27, 319-30
- **TAKESHI K. et al.** Definitive Radiotherapy Combined With High-Dose-Rate Brachytherapy For Stage III Carcinoma Of The Uterine Cervix: Retrospective Analysis Of Prognostic Factors Concerning Patient Characteristics And Treatment Parameters. *IJROBP,* 1998, vol. 41, 319-27
- **TANG P.A. ; HENG D.Y.C.** Programmed death 1 pathway inhibition in metastatic renal cell cancer and prostate cancer. *CURRENT ONCOLOGY REPORTS,* 2013, vol. 15, 98-104
- **TEICHER B.A. ; HERMAN T.S. ; HOPKINS R.E. ; MENON K.** Effect Of A Bovine Hemoglobin Preparation On The Response Of The Fsaiic Fibrosarcoma To Chemotherapeutic Alkylating-Agents. *J. CANCER RES CLIN ONCOL,* 1992, vol. 118, 123-8
- **TEICHER B.A. et al.** Oxygenation Of The Rat-9l Gliosarcoma And The Rat-13672 Mammary-Carcinoma With Various Doses Of A Hemoglobin Solution. *ARTIF. CELLS BLOOD SUBSTIT. IMMOBIL. BIOTECHNOL.,* 1994, vol. 22, 827-33

- **TEICHER B.A. ; SCHWARTZ G.N. ; SOTOMAYOR E.A. ; ROBINSON M.F. ; DUPUIS N.P. ; MENON K.** Oxygenation Of Tumors By A Hemoglobin Solution. *J. CANCER RES CLIN ONCOL,* 1993, vol. 120, 85-90
- **TREAT J. et al.** Tirapazamine With Cisplatin In Patients With Advanced Non-Small-Cell Lung Cancer: A Phase II Study. *J CLIN ONCOL,* 1998, vol. 16, 3524-7
- **VON PAWEL J. et al.** Tirapazamine Plus Cisplatin Versus Cisplatin In Advanced Non-Small-Cell Lung Cancer: A Report Of The International CATAPULT I Study Group, Cisplatin and Tirapazamine in Subjects with Advanced Previously Untreated Non-Small-Cell Lung Tumors. *J CLIN ONCOL,* 2000, vol. 18, 1351-9
- **WALSH L. et al.** Efficacy of ablative high-dose-per-fraction radiation for implanted human renal cell cancer in a nude mouse model. *EUROPEAN UROLOGY,* 2006, vol. 50, 795-800
- **WALTER S. et al.** Multipeptide immune response to cancer vaccine IMA901 after single-dose cyclophosphamide associates with longer patient survival. *NATURE MEDICINE,* 2012, vol. 18, 1254-61
- **WANG L. et al.** Encapsulation of Biomacromolecules within Polymersomes by Electroporation. *ANGEW CHEM INT ED ENGL.,* 2012, vol. 51, 11122-5
- **WASSERMAN T.H. et al.** Clinical-Trials With Etanidazole (Sr-2508) By The Radiation-Therapy Oncology Group (RTOG). *RADIOTHERAPY AND ONCOLOGY,* 1991, vol. 20, 129-35
- **WEITMAN S. et al.** Evidence Of Enhanced In Vivo Activity Using Tirapazamine With Paclitaxel And Paraplatin Regimens Against The MV-522 Human Lung Cancer Xenograft. *CANCER CHEMOTHER PHARMACOL,* 1999, vol. 43, 402-8
- **WILLIAMS K.J. et al.** In Vivo Activation Of The Hypoxia-Targeted Cytotoxin AQ4N In Human Tumor Xenografts. *MOLECULAR CANCER THERAPEUTICS,* 2009, vol. 8, 3266-75
- **WILSON D. ; EVANS S. ; JENKINS W. ; VINOGRADOV S. ; ONG E. ; DEWHIRST M.** Oxygen Distributions within R3230AC Tumors Growing in Dorsal Flap Window Chambers in Rats. *ADV EXP MED BIOL.,* 1998, vol. 454, 603-9
- **WOOD C.G. ; FIGLIN R.A.** ADAPT: An Ongoing international phase 3 randomized trial of autologous dendritic cell Immunotherapy (AGS 003) plus standard treatment in advanced renal cell carcinoma (RCC). *BJU INTERNATIONAL,* 2013, vol. 112, 11-2
- **WOUTERS B.G. et al.** Hypoxia As A Target For Combined Modality Treatments. *EUROPEAN JOURNAL OF CANCER,* 2002, vol. 38, 240-57
- **YUEN J.S.P. et al.** Inhibition of angiogenic and non-angiogenic targets by sorafenib in renal cell carcinoma (RCC) in a RCC xenograft model. *BRITISH JOURNAL OF CANCER,* 2011, vol. 104, 941-7
- **ZHAN H.W. ; LIU H.B. ; BAO C.K. ; YE X.J. ; ZHANG H. ; HE G.Q.** Effect Of Carbogen On Tumour Oxygenation And 32P-Colloid Interstitial Irradiation Response. *MED SCI MONIT,* 2010, vol. 16, BR11-6
- **ZHANG G.Q ; PALMER G.M. ; DEWHIRST M. ; FRASER C.L.** A Dual-Emissive-Materials Design Concept Enables Tumour Hypoxia Imaging. *NATURE MATERIALS,* 2009, vol. 8, 747-51
- **ZHANG X.L. et al.** Key Parameters Affecting The Initial Leaky Effect Of Hemoglobin-Loaded Nanoparticles As Blood Substitutes. *J MATER SCI METER MED.,* 2008, vol. 19, 2463-70
- IARC CancerBase No. 10. *International Agency for Research on Cancer,* 2010
- **LAM, JOHN S. ; LEPPERT, JOHN T. ; BELLDEGRUN, ARIE S. ; FIGLIN, ROBERT A.** Novel approaches in the therapy of metastatic renal cell carcinoma. *WORLD JOURNAL OF UROLOGY,* vol. 23, 202-12
- **PATIL S. ; MANOLA J. ; ELSON P. ; NEGRIER S. ; ESCUDIER B. ; EISEN T. ; ATKINS M. ; BUKOWSKI R. ; MOTZER R.J.** Improvement in Overall Survival of Patients with Advanced Renal Cell Carcinoma: Prognostic Factor Trend Analysis from an International Data Set of Clinical Trials. *JUROL.,* 2012, vol. 188, 2095-100
- **SHIH Y.C. ; CHIEN C.R. ; XU Y. ; PAN I.W. ; SMITH G.L. ; BUCHHOLZ T.A.** Economic burden of renal cell carcinoma in the US: Part II--an updated analysis. *PHARMACOECONOMICS,* 2011, vol. 29, 331-41
- **HELFAND C.** Top twenty orphan drugs by 2018. *FREEPHARMA,* 23 July 2013, http://www.fiercepharma.com/special-reports/top-20-orphan-drugs-2018
- Kidney Cancer Drugs: A Global Strategic Business Report. Global Industry Analysts, Inc, 2011
- **VÍTEČEK J. ; LOJEK A. ; VALACCHI G. ; KUBALA L.** Arginine-Based Inhibitors of Nitric Oxide Synthase: Therapeutic Potential and Challenges. *MEDIATORS OF INFLAMMATION,* 2012
- **O'NEIL et al.** *Langmuir,* 2009, vol. 25 (16), 9025-9029